# EUROPEAN PATENT APPLICATION

(11) **EP 4 534 547 A1**
(43) Date of publication of application: **09.04.2025**
(21) Application number: 23816173.1
(22) Date of filing: 02.06.2023
(51) Int. Cl.: C07K 14/00, A61K 39/395, A61K 47/55, A61K 47/64, A61K 47/68, A61K 49/00, A61P 35/00, A61P 43/00, C07K 16/00, C07K 19/00, C12N 1/15, C12N 1/19, C12N 1/21, C12N 5/10, C12N 15/11, C12N 15/62, C12N 15/63

(54) **AFFINITY SUBSTANCE, COMPOUND, AND ANTIBODY AND THEIR SALTS**

(30) Priority: 02.06.2022 JP 2022090443; 06.09.2022 JP 2022141780
(71) Applicant: AJINOMOTO CO., INC., Chuo-ku Tokyo 104-8315 (JP)
(72) Inventor: MATSUDA, Yutaka, Kawasaki-shi, Kanagawa 210-8681 (JP); FUJII, Tomohiro, Kawasaki-shi, Kanagawa 210-8681 (JP); ITO, Kenichiro, Kawasaki-shi, Kanagawa 210-8681 (JP); TAKAHASHI, Kazutoshi, Kawasaki-shi, Kanagawa 210-8681 (JP); MATSUDA, Yoshihiko, Kawasaki-shi, Kanagawa 210-8681 (JP); YAMAGUCHI, Hiroki, Kawasaki-shi, Kanagawa 210-8681 (JP); TSUYOSHI, Naoko, Kawasaki-shi, Kanagawa 210-8681 (JP); NAGANO, Hayato, Kawasaki-shi, Kanagawa 210-8681 (JP); TAKASUGI, Rika, Kawasaki-shi, Kanagawa 210-8681 (JP)
(74) Representative: Mewburn Ellis LLP
(86) International application number: PCT/JP2023/020712
(87) International publication number: WO 2023/234416

(57) **Abstract**

The present invention relates to a technology for easy chemical modification of only one heavy chain in the constituent unit of an antibody (immunoglobulin unit containing two heavy chains and optionally two light chains). More particularly, the present invention relates to a compound or a salt thereof, containing (A) an affinity substance containing first and second affinity moieties each having an affinity to the constant region in a heavy chain of an antibody; and (B) a reactive group for the antibody.

## Description

### TECHNICAL FIELD

The present invention relates to an affinity substance and a salt thereof, as well as a compound, an antibody, and salts thereof, comprising the affinity substance.

### BACKGROUND ART

In recent years, research and development of an antibody-drug conjugate (ADC) have been actively conducted. An ADC, as implied by the name, is a medicine in which a drug (e.g., an anti-cancer agent) is conjugated with an antibody and has a direct cytotoxic activity on cancer cells and the like. A typical ADC is T-DM1 (trade name: Kadcyla^{®}) which is jointly developed by Immunogen and Roche.

ADCs including T-DM1 have had the problem of their nonuniformity from the beginning of their development. For example, a small compound drug is randomly reacted with about 70 to 80 lysine residues in an antibody, and thus a drug antibody ratio (DAR) and a conjugation position are not constant. It is known that such a random conjugation method normally provides a DAR within a range of 0 to 8, producing a plurality of antibody medicines having different number of bonds of a drug. In recent years, it has been reported that when the number of bonds and the bond positions of a drug of an ADC are changed, pharmacokinetics, and a releasing rate and effects of the drug change. Given these circumstances, next-generation ADCs are required to control the number and positions of a drug to be conjugated. It is believed that when the number and positions are constant, the problems of expected efficacy, variations in conjugate medicines, and lot difference, or what is called regulation, will be solved.

Although methods for regioselectively modifying antibodies are being investigated worldwide, most of them are methods of modification using genetic engineering techniques or enzymes. For the genetic engineering methods of modification, problems have been pointed out such as reductions in the expression efficiency of antibodies themselves (reductions in total yield when ADCs are prepared), although regioselectivity and number selectivity can be controlled. In addition, there is a problem in that it takes long years to construct an antibody expression system and the like.

In recent years, a chemical conjugation by affinity peptide (C-CAP) method which can regioselectively modify an antibody by chemical synthetic technique has recently been developed (see Patent Document 1). This method has succeeded in regioselective modification of antibodies by a method that reacts with a peptide reagent in which an NHS-activated ester and a drug are coupled with an affinity peptide with an antibody. However, in the ADC produced by this method, the antibody and the drug are bonded via a linker containing a peptide moiety. The peptide moiety has potential immunogenicity and is susceptible to hydrolysis in a blood. Therefore, the ADC produced by this method has room for improvement in that it contains a peptide moiety in the linker.

As an improved method of the above C-CAP method, techniques which can prepare an antibody which does not contain a peptide moiety as a linker and has a functional substance (e.g., a drug) regioselectively by a chemical synthesis method using a certain compound containing an affinity peptide have been reported (see Patent Documents 2 to 5). In addition, Patent Document 6, which is incorporated herein by reference in its entirety, discloses a technique for producing affinity peptides in large quantities and easily in the preparation of compounds containing the affinity peptides, by utilizing affinity peptides comprising glutamine-glutamic acid-threonine (QET) at the N-terminus. Avoiding the use of linkers containing peptide moieties is desirable in clinical applications. In these techniques, plural positions corresponding to various amino acid residues in CH2 and CH3 domains (e.g., lysine residues, tyrosine residues, serine residues, and threonine residues) have been proposed as the positions of amino acid residues in antibodies that can be regioselectively modified with drugs. However, it is not necessarily easy to regioselectively modify an antibody with a functional substance and control the bonding ratio of the antibody to the functional substance within a desired range. In particular, it is not easy to control the modification to be specific to only one heavy chain in the constituent unit of an antibody (immunoglobulin unit containing two heavy chains).

By the way, Patent Document 6 discloses that (i) a peptide molecule comprising first and second bioactive peptide moieties for proteins such as various receptors (e.g., fibroblast growth factor receptor (FGFR), hepatocyte growth factor receptor (HGFR/c-Met), erythropoietin receptor, thrombopoietin receptor) and a peptide linker can be noncovalently bound to a protein to control the activity of the protein, and that (ii) as the peptide linker, a peptide linker with a certain length, having an amino acid sequence composed of amino acid residues, proline (P), alanine (A), and serine (S), can be used.

### PRIOR ART REFERENCES

### Patent Literature

Patent Document 1: WO 2016/186206
Patent Document 2: WO 2018/199337
Patent Document 3: WO 2019/240287
Patent Document 4: WO 2019/240288
Patent Document 5: WO 2020/090979
Patent Document 6: WO 2021/112249

### DISCLOSURE OF INVENTION

### PROBLEM TO BE SOLVED BY THE INVENTION

An object of the present invention is to develop a technology enabling easy chemical modification of only one heavy chain in the constituent unit of an antibody (in other words, an immunoglobulin unit containing two heavy chains and optionally two light chains).

An additional object of the present invention is to develop an antibody that is easily chemically modified in only one heavy chain of the constituent unit of the antibody while being regioselectively modified.

### MEANS FOR SOLVING PROBLEM

The present inventors have intensively studied to find that use of a compound or a salt thereof comprising (A) an affinity substance comprising first and second affinity moieties each having an affinity to the constant region in a heavy chain of an antibody, and (B) a reactive group for the antibody enables easy chemical modification of only one heavy chain in the constituent unit of the antibody.

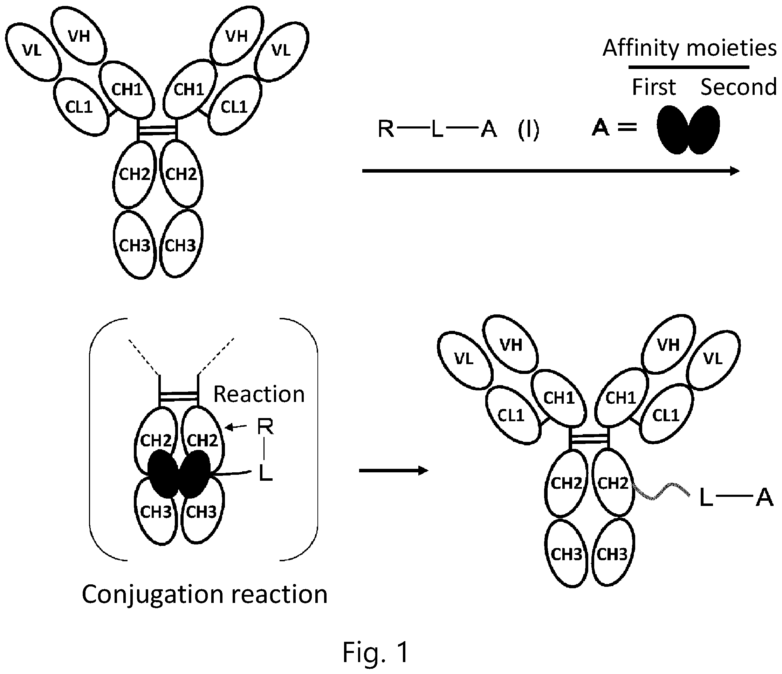

The compound or a salt thereof of the present invention can associate with the two heavy chains of the constituent unit of the antibody via (A) the affinity substance comprising first and second affinity moieties each having an affinity to the constant region in a heavy chain of the antibody, and then can specifically react with the side chain of a specific amino acid residue in one heavy chain of the constituent unit of the antibody via (R) the reactive group for the antibody to produce an affinity substance-modified antibody or a salt thereof in which only one of the two heavy chains of the constituent unit of the antibody is modified (FIG. 1). Without wishing the present invention to be bound by any theory, the mechanism of modifying only one heavy chain of the constituent unit of an antibody by using the compound or a salt thereof of the present invention is as described below. The affinity substance (comprising first and second affinity moieties each having an affinity to the constant region in a heavy chain of an antibody) contained in the compound or a salt thereof of the present invention can stably associate with the constant regions in the two heavy chains of the constituent unit of the antibody, and thus the reactive group contained in the compound or a salt thereof of the present invention can modify only one heavy chain (FIG. 1). At this time, the constant region in the other heavy chain (the constant region in the unmodified heavy chain) is associated with and sterically hindered by the affinity moiety, resulting in disability of other molecules (the compound or a salt thereof of the present invention) to associate with the constant region in the other heavy chain via the affinity substance contained therein. The compound or a salt thereof of the present invention can associate with the constant regions in the two heavy chains via the two affinity moieties contained in the affinity substance, and thus can stably associate with the constituent unit of the antibody to highly prevent the association of other molecules to the constituent unit of the antibody. Therefore, the compound or a salt thereof of the present invention can highly prevent the modification of the constant region in the other heavy chain, allowing for modification of the constant region in only one heavy chain (FIG. 1).

Patent Documents 1 to 5 disclose that use of a compound comprising an affinity substance and a reactive group for an antibody allows for regioselective modification of the antibody with a functional substance, but neither describe nor suggest (1) the challenge of developing a technology that enables easy chemical modification of only one heavy chain of the constituent unit of an antibody, and (2) use of a substance comprising first and second affinity moieties each having an affinity to the constant region in a heavy chain of an antibody as an affinity substance (in particular, a technical idea of using a compound or a salt thereof comprising the affinity substance and a reactive group for an antibody to chemically modify only one heavy chain of the constituent unit of an antibody).

Patent Document 6 discloses that a peptide molecule comprising first and second bioactive peptide moieties for proteins as described above and a certain peptide linker non-covalently binds to the proteins to control the activity of the proteins, but neither describe nor suggest (1) the challenge of developing a technology that enables easy chemical modification of only one heavy chain of the constituent unit of an antibody, and (2) use of a substance comprising first and second affinity moieties each having an affinity to the constant region in a heavy chain of an antibody as an affinity substance (in particular, a technical idea of using a compound or a salt thereof comprising the affinity substance and a reactive group for an antibody to chemically modify only one heavy chain of the constituent unit of an antibody).

The present inventors have also successfully generated an antibody in which only one heavy chain of the constituent unit (the immunoglobulin unit comprising two heavy chains and optionally two light chains) of an antibody is chemically modified by using the compound or a salt thereof of the present invention. Such an antibody is characterized in that it comprises (a) an immunoglobulin unit comprising two heavy chains and optionally two light chains, and (b) a modification unit (e.g., an above-described affinity substance, bioorthogonal functional group, or functional substance), and that (c) the modification unit is introduced to the constant region in only one heavy chain of the immunoglobulin unit.

Specifically, the present invention is as follows.
[1] A compound or a salt thereof, comprising (A) an affinity substance comprising first and second affinity moieties each having an affinity to the constant region in a heavy chain of an antibody; and (B) a reactive group for the antibody.
[2] The compound or a salt thereof according to[1], wherein the constant region is a Fc region.
[3] The compound or a salt thereof according to[1] or [2], wherein the constant region is a CH2 domain.
[4] The compound or a salt thereof according to any one of [1] to [3], wherein the constant region is a human constant region.
[5] The compound or a salt thereof according to any one of [1] to [4], wherein the antibody is IgG.
[6] The compound or a salt thereof according to any one of [1] to [5], wherein the first and second affinity moieties are different affinity moieties.
[7] The compound or a salt thereof according to any one of [1] to [6], wherein the affinity substance is represented by the following formula (A):

   AP1-L_{A}-AP2 (A)

   wherein
   AP1 indicates a first affinity peptide having an affinity to the constant region in a heavy chain of the antibody;
   AP2 indicates a second affinity peptide having an affinity to the constant region in a heavy chain of the antibody; and
   L_{A} indicates a linker.
[8] The compound or a salt thereof according to any one of [1] to [7], wherein the affinity substance (i) (i-1) comprises only a single specific reactive group, and (i-2) is linked via said specific reactive group, to the reactive group for the antibody.
[9] The compound or a salt thereof according to any one of [1] to [8], wherein the affinity substance is an affinity polypeptide comprising first and second affinity peptides each having an affinity to the constant region in a heavy chain of the antibody.
[10] The compound or a salt thereof according to any one of [1] to [6], wherein the affinity polypeptide is represented by the following formula (A'):

   AP1-PL_{A}-AP2 (A')

   wherein
   AP1 indicates a first affinity peptide that has an affinity to the constant region in a heavy chain of the antibody and is present on the N-terminal side of the affinity polypeptide;
   AP2 indicates a second affinity peptide that has an affinity to the constant region in a heavy chain of the antibody and is present on the C-terminal side of the affinity polypeptide; and
   PL_{A} indicates a peptide linker.
[11] The compound or a salt thereof according to any one of [1] to [10], wherein the affinity polypeptide (i) (i-1) comprises only a single amino acid residue having an amino group in its side chain, and (ii-2) is linked to the reactive group for the antibody via said amino group, or (ii) is linked to the reactive group for the antibody via the N-terminal amino group in the first affinity peptide.
[12] The compound or a salt thereof according to [11], wherein the amino acid residue having an amino group in its side chain is a lysine residue.
[13] The compound or a salt thereof according to any one of [1] to [12], wherein the affinity polypeptide further comprises a tripeptide composed of Gln-Glu-Thr (QET) at the N-terminus.
[14] The compound or a salt thereof according to [10], wherein the peptide linker has a length of 20 or more amino acid residues.
[15] The compound or a salt thereof according to any one of [1] to [14], wherein
   one of the first and second affinity peptides is an affinity peptide having an affinity to the constant region in a heavy chain of the antibody and having a single lysine residue; and
   the other of the first and second affinity peptides is an affinity peptide having an affinity to the constant region in a heavy chain of the antibody and having no lysine residue.
[16] The compound or a salt thereof of [15], wherein the affinity peptide having an affinity to the constant region in a heavy chain of the antibody and having a single lysine residue is any one of the following (1) to (4):
   (1) an affinity peptide comprising the amino acid sequence (Fc3K) of RGNCAYHKGQIIWCTYH (SEQ ID NO: 38);
   (2) an affinity peptide comprising an amino acid sequence in which one or two amino acid residues other than lysine or cysteine residue in the amino acid sequence of RGNCAYHKGQIIWCTYH (SEQ ID NO: 38) are replaced by other amino acid residues other than lysine or cysteine residue, and having an affinity to the constant region in a heavy chain of the antibody;
   (3) an affinity peptide comprising the amino acid sequence (Z34CK) of FNKQCQRRFYEALHDPNLNEEQRNARIRSIREEC (SEQ ID NO: 39); and
   (4) an affinity peptide comprising an amino acid sequence in which one or two amino acid residues other than lysine or cysteine residue in the amino acid sequence of FNKQCQRRFYEALHDPNLNEEQRNARIRSIREEC (SEQ ID NO: 39) are replaced by other amino acid residues other than lysine or cysteine residue, and having an affinity to the constant region in a heavy chain of the antibody;
      wherein the two cysteine residues comprised in the amino acid sequence may be cross-linked by a disulfide bond, and/or
      wherein the affinity peptide having an affinity to the constant region in a heavy chain of the antibody and having no lysine residue is any one of the following (5) to (10):
   (5) an affinity peptide comprising the amino acid sequence (Z34CM) of FNMQCQRRFYEALHDPNLNEEQRNARIRSIREEC (SEQ ID NO: 40);
   (6) an affinity peptide comprising an amino acid sequence in which one or two amino acid residues other than cysteine residue in the amino acid sequence of FNMQCQRRFYEALHDPNLNEEQRNARIRSIREEC (SEQ ID NO: 40) are replaced by other amino acid residues other than lysine or cysteine residue, and having an affinity to the constant region in a heavy chain of the antibody;
   (7) an affinity peptide comprising the amino acid sequence (ProAR) of FNREQQNAFYEILHLPNLNEEQRNGFIQSLRDDPSQSANLLAEA (SEQ ID NO: 41);
   (8) an affinity peptide comprising an amino acid sequence in which one or two amino acid residues other than cysteine residue in the amino acid sequence of FNREQQNAFYEILHLPNLNEEQRNGFIQSLRDDPSQSANLLAEA (SEQ ID NO: 41) are replaced by other amino acid residues other than lysine or cysteine residue, and having an affinity to the constant region in a heavy chain of the antibody;
   (9) an affinity peptide comprising the amino acid sequence of RGNCAYHRGQIIWCTYH (SEQ ID NO: 78); and
   (10) an affinity peptide comprising an amino acid sequence in which one or two amino acid residues other than cysteine residue in the amino acid sequence of RGNCAYHRGQIIWCTYH (SEQ ID NO: 78) are replaced by other amino acid residues other than lysine or cysteine residue, and having an affinity to the constant region in a heavy chain of the antibody; and
   wherein the two cysteine residues comprised in said amino acid sequences may be cross-linked by a disulfide bond.
[17] The compound or a salt thereof according to any one of [1] to [16], wherein the compound is represented by the following formula (I):

   R-L-A (I)

   wherein
   R indicates said reactive group;
   L indicates a linker; and
   A indicates said affinity substance.
[18] The compound or a salt thereof according to any one of [1] to [17], wherein the compound or a salt thereof further comprises (iii) a cleavable moiety between (i) said affinity substance and (ii) said reactive group.
[19] The compound or a salt thereof according to [18], wherein the cleavable moiety is a cleavable moiety that can be cleaved to generate a bioorthogonal functional group on said reactive group side.
[20] The compound or a salt thereof according to [19], wherein the compound is represented by the following formula (Ia):

   R-L₁-CLE(B)-L₂-A (Ia)

   wherein
   R indicates said reactive group;
   L₁ indicates a first linker;
   L₂ indicates a second linker;
   CLE(B) represents a cleavable moiety that can be cleaved to generate a bioorthogonal functional group on said reactive group side; and
   A indicates said affinity substance.
[21] The compound or a salt thereof according to [19], wherein the compound is represented by the following formula (Ia-1): 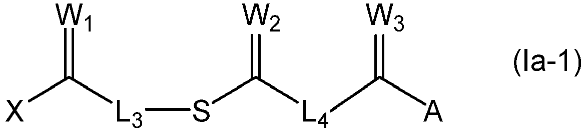 wherein
   X indicates a leaving group;
   W₁, W₂, and W₃ each independently indicate an oxygen atom or a sulfur atom;
   L₃ indicates a third linker;
   L₄ indicates a fourth linker;
   S indicates a sulfur atom; and
   A indicates said affinity substance.
[22] The compound or salt thereof according to [21], wherein the leaving group is selected from the following:
   (a) R_{A}-S, wherein R_{A} indicates a hydrogen atom, a monovalent hydrocarbon group which may have a substituent, or a monovalent heterocyclic group which may have a substituent, and S indicates a sulfur atom;
   (b) R_{A}-O, wherein R_{A} indicates a hydrogen atom, a monovalent hydrocarbon group which may have a substituent, or a monovalent heterocyclic group which may have a substituent, and O indicates an oxygen atom;
   (c) R_{A}-(R_{B}-)N, wherein R_{A} and R_{B} each independently indicate a hydrogen atom, a monovalent hydrocarbon group which may have a substituent, or a monovalent heterocyclic group which may have a substituent, and N indicates a nitrogen atom; or
   (d) a halogen atom.
[23] The compound or a salt thereof according to any one of [1] to [17], wherein the compound or a salt thereof further comprises (iv) a bioorthogonal functional group between (ii) said reactive group and (iii) the cleavable moiety.
[24] The compound or a salt thereof according to [23], wherein the compound is represented by the following formula (Ib): 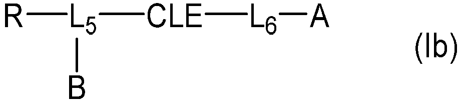 wherein
   R indicates said reactive group;
   L₅ indicates a fifth linker;
   L₆ indicates a sixth linker;
   B indicates a group comprising a bioorthogonal functional group;
   CLE indicates a cleavable moiety; and
   A indicates said affinity substance.
[25] The compound or a salt thereof according to [23], wherein the compound is represented by the following formula (Ib-1): 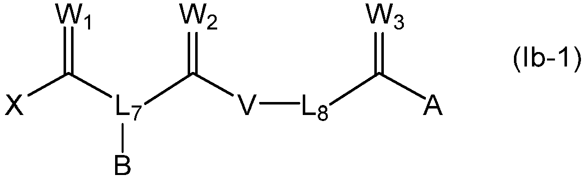 wherein
   X indicates a leaving group;
   W₁, W₂, and W₃ each independently indicate an oxygen atom or a sulfur atom;
   L₇ indicates a seventh linker;
   L₈ indicates an eighth linker;
   B indicates a group comprising a bioorthogonal functional group;
   V indicates an oxygen atom or a sulfur atom; and
   A indicates said affinity substance.
[26] The compound or a salt thereof according to any one of [23] to [25], wherein the bioorthogonal functional group is an azide residue, an alkyne residue, a tetrazine residue, an alkene residue, a thiol residue, a maleimide residue, a thiol residue, a furan residue, or a halocarbonyl residue.
[27] A reagent for derivatizing an antibody, comprising the compound or a salt thereof according to any one of [1] to [26].
[28] An affinity substance-modified antibody or a salt thereof, comprising an affinity substance comprising first and second affinity moieties each having an affinity to the constant region in a heavy chain of the antibody in the constant region in a heavy chain of the antibody.
[29] The affinity substance-modified antibody or a salt thereof according to [28], comprising: (a) an immunoglobulin unit comprising two heavy chains and optionally two light chains; and (b) said affinity substance, (c) wherein said affinity substance is introduced to the constant region in only one heavy chain of said immunoglobulin unit.
[30] The affinity substance-modified antibody or a salt thereof according to [29], wherein said affinity substance is introduced to said constant region in only one heavy chain via modification of the amino group in the side chain of a lysine residue present at one or more positions in said constant region in one heavy chain.
[31] The affinity substance-modified antibody or a salt thereof according to [30], wherein said one or more positions are positions 246/248, 288/290, or 317 in a human IgG heavy chain according to the EU numbering.
[32] The affinity substance-modified antibody or a salt thereof according to any one of [28] to [31], wherein the affinity substance-modified antibody comprises a structural unit represented by the following formula (II): 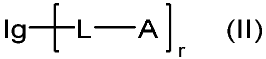 wherein
   Ig indicates an immunoglobulin unit comprising two heavy chains and optionally two light chains;
   L indicates a linker;
   A indicates said affinity substance; and
   the average percent modification r of said immunoglobulin unit with said affinity substance is 65 to 135%.
[33] The affinity substance-modified antibody or a salt thereof according to any one of [28] to [32], wherein said antibody or a salt thereof further comprises (iii') a cleavable moiety between (i') said affinity substance (ii') said antibody.
[34] The affinity substance-modified antibody or a salt thereof according to [33], wherein the cleavable moiety is a cleavable moiety that can be cleaved to generate a bioorthogonal functional group on said immunoglobulin unit.
[35] The affinity substance-modified antibody or a salt thereof according to [34], wherein the affinity substance-modified antibody comprises a structural unit represented by the following formula (IIa): 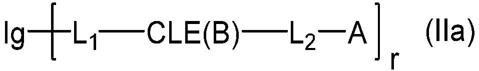 wherein
   Ig indicates said immunoglobulin unit;
   L₁ indicates a first linker;
   L₂ indicates a second linker;
   CLE(B) indicates a cleavable moiety that can be cleaved to generate a bioorthogonal functional group on said immunoglobulin unit side;
   A indicates said affinity substance; and
   the average percent modification r of said immunoglobulin unit with said affinity substance is 65 to 135%.
[36] The affinity substance-modified antibody or a salt thereof according to [34], wherein the affinity substance-modified antibody comprises a structural unit represented by the following formula (IIa-1): 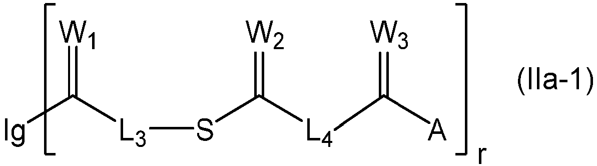 wherein
   Ig indicates said immunoglobulin unit;
   W₁, W₂, and W₃ each independently indicate an oxygen atom or a sulfur atom;
   L₃ indicates a third linker;
   L₄ indicates a fourth linker;
   S indicates a sulfur atom;
   A indicates said affinity substance; and
   the average percent modification r of said immunoglobulin unit with said affinity substance is 65 to 135%.
[37] The affinity substance-modified antibody or a salt thereof according to [33], wherein said antibody or a salt thereof further comprises (iv') a bioorthogonal functional group between (ii') said antibody and (iii') the cleavable moiety.
[38] The affinity substance-modified antibody or a salt thereof according to [37], wherein the affinity substance-modified antibody comprises a structural unit represented by the following formula (IIb): 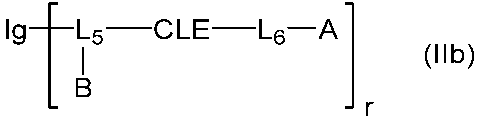 wherein
   Ig indicates said immunoglobulin unit;
   L₅ indicates a fifth linker;
   L₆ indicates a sixth linker;
   B indicates a group comprising a bioorthogonal functional group;
   CLE indicates a cleavable moiety;
   A indicates said affinity substance; and
   the average percent modification r of said immunoglobulin unit with said affinity substance is 65 to 135%.
[39] The affinity substance-modified antibody or a salt thereof according to [37], wherein the affinity substance-modified antibody comprises a structural unit represented by the following formula (IIb-1): 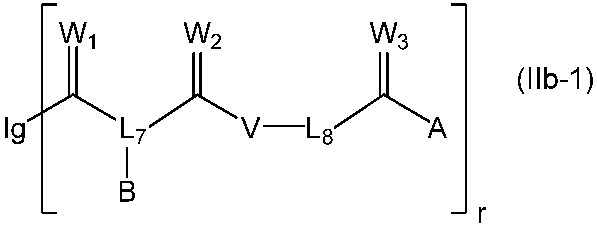 wherein
   Ig indicates said immunoglobulin unit;
   W₁, W₂ and W₃ each independently indicate an oxygen atom or a sulfur atom;
   L₇ indicates a seventh linker;
   L₈ indicates an eighth linker;
   B indicates a group comprising a bioorthogonal functional group;
   V indicates an oxygen atom or a sulfur atom;
   A indicates said affinity substance; and
   the average percent modification r of said immunoglobulin unit with said affinity substance is 65 to 135%.
[40] The affinity substance-modified antibody or a salt thereof according to any one of [28] to [39], wherein the affinity substance-modified antibody further comprises an additional modified moiety.
[41] The affinity substance-modified antibody or a salt thereof according to [40], wherein the additional modified moiety is an additional affinity substance comprising a third affinity moiety having an affinity to the constant region in a heavy chain of the antibody, and said additional affinity substance is comprised in the constant region in a heavy chain of the antibody.
[42] The affinity substance-modified antibody or a salt thereof according to [41], wherein said additional affinity substance is introduced to the constant regions in said two heavy chains via modification of the amino group in the side chain of a lysine residue present at one or more positions in the constant regions in said two heavy chains.
[43] The affinity substance-modified antibody or a salt thereof according to [42], wherein said one or more positions in the constant regions in the two heavy chains are positions 246/248, 288/290, or 317 in a human IgG heavy chain according to the EU numbering.
[44] A method of producing an affinity substance-modified antibody or a salt thereof, the method comprising
   reacting a compound or a salt thereof with an antibody, wherein
   the compound or a salt thereof comprises (A) an affinity substance comprising first and second affinity moieties each having an affinity to the constant region in a heavy chain of the antibody, and (B) a reactive group for the antibody; and
   the antibody comprises an immunoglobulin unit comprising two heavy chains and optionally two light chains,
   to produce an affinity substance-modified antibody or a salt thereof comprising said affinity substance in the constant region in a heavy chain of said immunoglobulin unit.
[45] The method according to [44], wherein the compound or a salt thereof further comprises (iii) a cleavable moiety between (i) said affinity substance and (ii) said reactive group.
[46] The method according to [45], wherein the cleavable moiety is a cleavable moiety that can be cleaved to generate a bioorthogonal functional group on said reactive group side.
[47] The method according to [44], wherein the compound or a salt thereof further comprises (iv) a bioorthogonal functional group between (ii) said reactive group and (iii) the cleavable moiety.
[48] The method according to [44], wherein the affinity substance-modified antibody or a salt thereof is the affinity substance-modified antibody or a salt thereof according to any one of [29] to [43].
[49] An antibody derivative or a salt thereof comprising a bioorthogonal functional group, comprising: (a) an immunoglobulin unit comprising two heavy chains and optionally two light chains; and (b) the bioorthogonal functional group, (c) wherein the bioorthogonal functional group is introduced to the constant region in only one heavy chain of said immunoglobulin unit.
[50] The antibody derivative or a salt thereof according to [49], wherein said bioorthogonal functional group is introduced to said constant region in only one heavy chain via modification of the amino group in the side chain of a lysine residue present at one or more positions in said constant region in one heavy chain.
[51] The antibody derivative or a salt thereof according to [49] or [50], wherein said one or more positions in the constant region in one heavy chain are positions 246/248, 288/290, or 317 in a human IgG heavy chain according to the EU numbering.
[52] The antibody derivative or a salt thereof according to any one of [49] to [51], wherein the antibody derivative or a salt thereof comprising a bioorthogonal functional group comprises a structural unit represented by the following formula (IIIa): 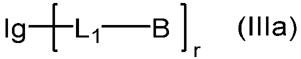 wherein
   Ig indicates said immunoglobulin unit;
   L₁ indicates a first linker;
   B indicates a group comprising a bioorthogonal functional group; and
   the average percent modification r of said immunoglobulin unit with the bioorthogonal functional group is 65 to 135%.
[53] The antibody derivative or a salt thereof according to any one of [49] to [51], wherein the antibody derivative or a salt thereof comprising a bioorthogonal functional group comprises a structural unit represented by the following formula (IIIa-1): 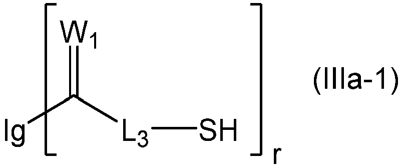 wherein
   Ig indicates said immunoglobulin unit;
   W₁ indicates an oxygen atom or a sulfur atom;
   L₃ indicates a third linker;
   SH indicates a thiol group; and
   the average percent modification r of said immunoglobulin unit with the bioorthogonal functional group is 65 to 135%.
[54] The antibody derivative or a salt thereof according to any one of [49] to [51], wherein the antibody derivative or a salt thereof comprising a bioorthogonal functional group comprises a structural unit represented by the following formula (IIIb): 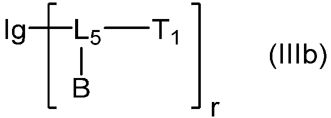 wherein
   Ig indicates said immunoglobulin unit;
   L₅ indicates a fifth linker;
   B indicates a group comprising a bioorthogonal functional group;
   T₁ indicates a monovalent group; and
   the average percent modification r of said immunoglobulin unit with the bioorthogonal functional group is 65 to 135%.
[55] The antibody derivative or a salt thereof according to any one of [49] to [51], wherein the antibody derivative or a salt thereof comprising a bioorthogonal functional group comprises a structural unit represented by the following formula (IIIb-1): 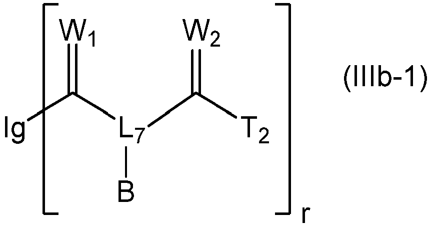 wherein
   Ig indicates said immunoglobulin unit;
   W₁ and W₂ each independently indicate an oxygen atom or a sulfur atom;
   L₇ indicates a seventh linker;
   B indicates a group comprising a bioorthogonal functional group;
   T₂ indicates a monovalent group; and
   the percent modification r of said immunoglobulin unit with the bioorthogonal functional group is 65 to 135%
[56] The antibody derivative or a salt thereof according to any one of [49] to [55], wherein the antibody derivative further comprises an additional modified moiety.
[57] The antibody derivative or a salt thereof according to [56], wherein the additional modified moiety is an additional modified moiety comprising a bioorthogonal functional group, and said additional affinity substance comprising a bioorthogonal functional group is comprised in the constant region in a heavy chain of the antibody.
[58] The antibody derivative or a salt thereof according to [57], wherein said additional modified moiety comprising a bioorthogonal functional group is introduced to the constant regions in said two heavy chains via modification of the amino group in the side chain of a lysine residue present at one or more positions in the constant regions in said two heavy chains.
[59] The antibody derivative or a salt thereof according to [58], wherein said one or more positions in the constant regions in the two heavy chains are positions 246/248, 288/290, or 317 in a human IgG heavy chain according to the EU numbering.
[60] A conjugate of an antibody and a functional substance, or a salt thereof, comprising: (a) an immunoglobulin unit comprising two heavy chains and optionally two light chains; and (b) a functional substance, (c) wherein the functional substance is introduced to the constant region in only one heavy chain of said immunoglobulin unit.
[61] The conjugate or a salt thereof according to [60], wherein the functional substance is introduced to said constant region in only one heavy chain via modification of the amino group in the side chain of a lysine residue present at one or more positions in said constant region in one heavy chain.
[62] The conjugate or a salt thereof according to [60] or [61], wherein said one or more positions in the constant region in one heavy chain are positions 246/248, 288/290, or 317 in a human IgG heavy chain according to the EU numbering.
[63] The conjugate or a salt thereof according to any one of [60] to [62], wherein the conjugate or a salt thereof comprises a structural unit represented by the following formula (IVa): 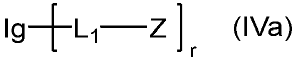 wherein
   Ig indicates said immunoglobulin unit;
   L₁ indicates a first linker;
   Z indicates a functional substance; and
   the average percent modification r of said immunoglobulin unit with the functional substance is 65 to 135%.
[64] The conjugate or a salt thereof according to any one of [60] to [62], wherein the conjugate or a salt thereof comprises a structural unit represented by the following formula (IVa-1): 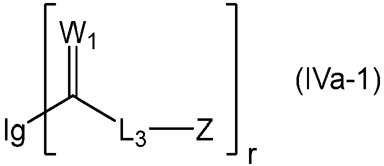 wherein
   Ig indicates said immunoglobulin unit;
   W₁ indicates an oxygen atom or a sulfur atom;
   L₃ indicates a third linker;
   Z indicates a functional substance; and
   the average percent modification r of said immunoglobulin unit with the functional substance is 65 to 135%.
[65] The conjugate or a salt thereof according to any one of [60] to [62], wherein the conjugate or a salt thereof comprises a structural unit represented by the following formula (IVb): 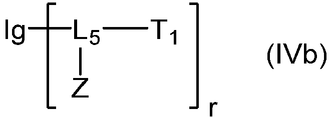 wherein
   Ig indicates said immunoglobulin unit;
   L₅ indicates a fifth linker;
   Z indicates a functional substance;
   T₁ indicates a monovalent group; and
   the average percent modification r of said immunoglobulin unit with the functional substance is 65 to 135%.
[66] The conjugate or a salt thereof according to any one of [60] to [62], wherein the conjugate or a salt thereof comprises a structural unit represented by the following formula (IVb-1): 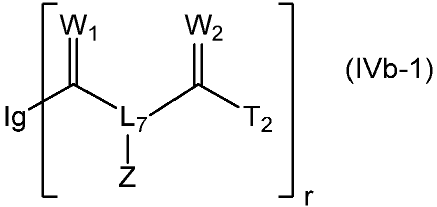 wherein
   Ig indicates said immunoglobulin unit;
   W₁ and W₂ each independently indicate an oxygen atom or a sulfur atom;
   L₇ indicates a seventh linker;
   Z indicates a functional substance;
   T₂ indicates a monovalent group; and
   the percent modification r of said immunoglobulin unit with the functional substance is 65 to 135%.
[67] The conjugate or a salt thereof according to any one of [60] to [66], wherein the functional substance is a drug, a labelling substance, an affinity substance, a transporting substance, or a stabilizer.
[68] The conjugate or a salt thereof according to [67], wherein the affinity substance is a full-length antibody or a fragment thereof.
[69] The conjugate or a salt thereof according to any one of [60] to [68], wherein the conjugate further comprises an additional modified moiety.
[70] The conjugate or a salt thereof according to [69], wherein the additional modified moiety is an additional modified moiety comprising a functional substance, and said additional modified moiety comprising a functional substance is comprised in a constant region in a heavy chain of the antibody.
[71] The conjugate or a salt thereof according to [70], wherein the additional modified moiety comprising a functional substance is introduced into said constant regions in two heavy chains via modification of the amino group in the side chain of a lysine residue present at one or more positions in said constant regions in two heavy chains.
[72] The conjugate or a salt thereof according to [71], wherein said one or more positions in the constant regions in two heavy chains are positions 246/248, 288/290, or 317 in a human IgG heavy chain according to the EU numbering.
[73] A method of producing an antibody or a salt thereof without affinity substance, the method comprising cleaving an affinity substance-modified antibody or a salt thereof comprising (A) an affinity substance comprising first and second affinity moieties each having an affinity to the constant region in a heavy chain of an antibody, and (B) an antibody, and further comprising (C) a cleavable moiety between (A) the affinity substance and (B) the antibody at the cleavable moiety to produce an antibody or a salt thereof without affinity substance.
[74] The method according to [73], wherein
   the cleavable moiety is a cleavable moiety that can be cleaved to generate a bioorthogonal functional group on said antibody side; and
   the antibody or a salt thereof without affinity substance is an antibody derivative or a salt thereof comprising a bioorthogonal functional group.
[75] The method according to [74], wherein the antibody derivative or a salt thereof comprising a bioorthogonal functional group is an antibody derivative or a salt thereof according to any one of [49] to [59].
[76] The method according to [73], wherein the antibody or a salt thereof without affinity substance further comprises a bioorthogonal functional group between said antibody and said cleavable moiety; and
   the antibody or a salt thereof without affinity substance is an antibody derivative or a salt thereof comprising a bioorthogonal functional group.
[77] The method according to [76], wherein the antibody derivative or a salt thereof comprising a bioorthogonal functional group is an antibody derivative or a salt thereof according to any one of [49] to [59].
[78] A method of producing a conjugate or a salt thereof comprising an antibody and a functional substance, the method comprising (1) and (2) below:
   (1) producing an antibody derivative or a salt thereof comprising a bioorthogonal functional group according to the method according to [74]; and
   (2) reacting the antibody derivative or a salt thereof comprising a bioorthogonal functional group with a functional substance to produce a conjugate or a salt thereof comprising the antibody and the functional substance.
[79] The method according to [78], wherein the conjugate or a salt thereof is the conjugate or a salt thereof according to any one of [60] to [72].
[80] A method of producing a conjugate or a salt thereof comprising an antibody and a functional substance, the method comprising (1) and (2) below:
   (1) producing an antibody derivative or a salt thereof comprising a bioorthogonal functional group according to the method according to [76]; and
   (2) reacting the antibody derivative or a salt thereof comprising a bioorthogonal functional group with a functional substance to produce a conjugate or a salt thereof comprising the antibody and the functional substance.
[81] The method according to [78], wherein the conjugate or a salt thereof is the conjugate or a salt thereof according to any one of [60] to [72].
[82] A method of producing a conjugate or a salt thereof comprising an antibody and a functional substance,
   the method comprising reacting an antibody derivative or a salt thereof comprising a bioorthogonal functional group with a functional substance to produce a conjugate or a salt thereof comprising the antibody and the functional substance;
   the antibody derivative or a salt thereof comprising a bioorthogonal functional group is an antibody derivative or a salt thereof comprising a bioorthogonal functional group, comprising: (a) an immunoglobulin unit comprising two heavy chains and optionally two light chains; and (b) the bioorthogonal functional group, (c) wherein the bioorthogonal functional group is introduced to the constant region in only one heavy chain of said immunoglobulin unit; and
   the conjugate or a salt thereof comprising an antibody and a functional substance is a conjugate of an antibody and a functional substance, or a salt thereof, comprising: (a) an immunoglobulin unit comprising two heavy chains and optionally two light chains; and (b) a functional substance, (c) wherein the functional substance is introduced to the constant region in only one heavy chain of said immunoglobulin unit.
[83] The method according to [80], wherein the antibody derivative or a salt thereof comprising a bioorthogonal functional group is an antibody derivative or a salt thereof according to any one of [49] to [59].
[84] The method according to [80], wherein the conjugate or a salt thereof is the conjugate or a salt thereof according to any one of [60] to [72].
[85] An affinity substance or a salt thereof, comprising first and second affinity moieties each having an affinity to the constant region in a heavy chain of an antibody.
[86] The affinity substance or a salt thereof according to [85], wherein the affinity substance is represented by the following formula (A):

   AP1-L_{A}-AP2 (A)

   wherein
   AP1 indicates a first affinity peptide having an affinity to the constant region in a heavy chain of the antibody;
   AP2 indicates a second affinity peptide having an affinity to the constant region in a heavy chain of the antibody; and
   L_{A} indicates a linker.
[87] The affinity substance or a salt thereof according to [85] or [86], wherein the affinity substance comprises only one specific reactive group.
[88] The affinity substance or a salt thereof according to any one of [85] to [87], wherein the affinity substance is an affinity polypeptide comprising first and second affinity peptides each having an affinity to a constant region in a heavy chain of the antibody.
[89] The affinity substance or a salt thereof according to any one of [85] to [88], wherein the affinity polypeptide is represented by the following formula (A'):

   AP1-PL_{A}-AP2 (A')

   wherein
   AP1 indicates a first affinity peptide that has an affinity to the constant region in a heavy chain of the antibody and is present on the N-terminal side of the affinity polypeptide;
   AP2 indicates a second affinity peptide that has an affinity to the constant region in a heavy chain of the antibody and is present on the C-terminal side of the affinity polypeptide; and
   PL_{A} indicates a peptide linker.
[90] The affinity substance or a salt thereof according to any one of [85] to [89], wherein the affinity polypeptide comprises (i) only one amino acid residue having an amino group in its side chain, or (ii) an unprotected N-terminal amino group.
[91] The affinity substance or a salt thereof according to [90], wherein the amino acid residue having an amino group in its side chain is a lysine residue.
[92] The affinity substance or a salt thereof according to any one of [85] to [89], wherein the affinity polypeptide further comprises a tripeptide composed of Gln-Glu-Thr (QET) at the N-terminus.
[93] The affinity substance or a salt thereof according to any one of [89] to [92], wherein the peptide linker has a length of 20 or more amino acid residues.
[94] The affinity substance or a salt thereof according to any one of [85] to [93], wherein
   one of the first and second affinity peptides is an affinity peptide having an affinity to the constant region in a heavy chain of the antibody and having a single lysine residue; and
   the other of the first and second affinity peptides is an affinity peptide having an affinity to a constant region in a heavy chain of the antibody and having no lysine residue.
[95] A polynucleotide encoding an affinity polypeptide comprising first and second affinity peptides each having an affinity to the constant region in a heavy chain of an antibody.
[96] An expression vector comprising the polynucleotide according to [95] and a promoter operably linked thereto.
[97] A host cell comprising an expression unit comprising the polynucleotide according to [95] and a promoter operably linked thereto.
[98] An affinity substance-modified antibody or a salt thereof comprising first and second modified moieties wherein
   the first modified moiety comprises a first affinity substance comprising first and second affinity moieties each having an affinity to the constant region in a heavy chain of an antibody;
   the second modified moiety comprises a second affinity substance comprising third and fourth affinity moieties each having an affinity to the constant region in a heavy chain of the antibody; and
   the first and second modified moieties are contained in the constant region in a heavy chain of the antibody.
[99] The affinity substance-modified antibody or a salt thereof according to [98], comprising (a) an immunoglobulin unit comprising two heavy chains composed of first and second heavy chains and optionally two light chains, and (b) said first and second modified moieties;
   wherein (c) said first modified moiety has been introduced to the constant region in the first heavy chain of said immunoglobulin unit; and
   (d) said second modified moiety has been introduced to the constant region in the second heavy chain of said immunoglobulin unit.
[100] The affinity substance-modified antibody or a salt thereof according to [98] or [99], wherein said first modified moiety has been introduced to the constant region in said first heavy chain via modification of the amino group in the side chain of a lysine residue present at one or more positions in the constant region in said first heavy chain; and said second modified moiety has been introduced to the constant region in said second heavy chain via modification of the amino group in the side chain of a lysine residue present at one or more positions in the constant regions in said second heavy chain.
[101] The affinity substance-modified antibody or a salt thereof according to [100], wherein said one or more positions in the constant region in the first heavy chain and said one or more positions in the constant region in the second heavy chain are positions selected from the group consisting of positions 246/248, 288/290, and 317 in the human IgG heavy chain according to the EU numbering, and combinations thereof.
[102] The affinity substance-modified antibody or a salt thereof according to any one of [98] to [101], wherein the affinity substance-modified antibody comprising the first and second modified moieties comprises a structural unit represented by the following formula (V): 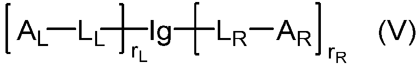 wherein
   Ig indicates an immunoglobulin unit comprising two heavy chains composed of first and second heavy chains and optionally two light chains;
   L_{L} and L_{R} each independently indicate a linker;
   A_{L} indicates said first affinity substance;
   A_{R} indicates said second affinity substance; and
   the average percent modification r_{L} of said immunoglobulin unit with said first modified moiety and the average percent modification r_{R} of said immunoglobulin unit with said second modified moiety are individually 65 to 135%.
[103] The affinity substance-modified antibody or a salt thereof according to any one of [98] to [102], wherein said antibody or a salt thereof further comprises (iii') a first cleavable moiety between (i') said first affinity substance and (ii') said immunoglobulin unit, and/or further comprises (iii") a second cleavable moiety between (i") said second affinity substance and (ii") said immunoglobulin unit.
[104] The affinity substance-modified antibody or a salt thereof according to [103], wherein said first and second cleavable moieties are each a cleavable moiety that can be cleaved to generate a bioorthogonal functional group on said immunoglobulin unit.
[105] The affinity substance-modified antibody or a salt thereof according to any one of [98] to [104], wherein the affinity substance-modified antibody comprising the first and second modified moieties comprises a structural unit represented by the following formula (Va): 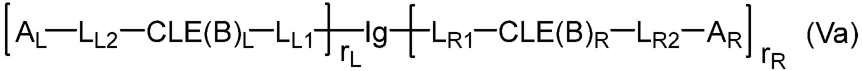 wherein
   Ig indicates said immunoglobulin unit;
   L_{L1} and L_{R1} each independently indicate a first linker;
   L_{L2} and L_{R2} each independently indicate a second linker;
   CLE(B)_{L} and CLE(B)_{R} each independently indicate a cleavable moiety that can be cleaved to generate a bioorthogonal functional group on said immunoglobulin unit side;
   A_{L} indicates said first affinity substance;
   A_{R} indicates said second affinity substance; and
   the average percent modification r_{L} of said immunoglobulin unit with said first modified moiety and the average percent modification r_{R} of said immunoglobulin unit with said second modified moiety are individually 65 to 135%.
[106] The affinity substance-modified antibody or a salt thereof according to any one of [98] to [104], wherein the affinity substance-modified antibody comprising the first and second modified moieties comprises a structural unit represented by the following formula (Va-1): 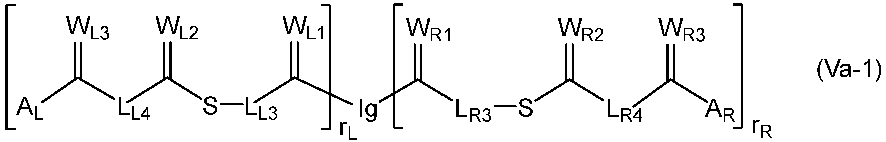 wherein
   Ig indicates said immunoglobulin unit;
   W_{L1}, W_{L2} and W_{L3}, and W_{R1}, W_{R2} and W_{R3} each independently indicate an oxygen atom or a sulfur atom;
   L_{L3} and L_{R3} each independently indicate a third linker;
   L_{L4} and L_{R4} each independently indicate a fourth linker;
   S indicates a sulfur atom;
   A_{L} indicates said first affinity substance;
   A_{R} indicates said second affinity substance; and
   the average percent modification r_{L} of said immunoglobulin unit with said first modified moiety and the average percent modification r_{R} of said immunoglobulin unit with said second modified moiety are individually 65 to 135%.
[107] The affinity substance-modified antibody or a salt thereof according to any one of [98] to [106], wherein said antibody or a salt thereof further comprises (iv') a first bioorthogonal functional group between (ii') said immunoglobulin unit and (iii') said first cleavable moiety, and/or further comprises (iv") a second bioorthogonal functional group between (ii") said immunoglobulin unit and (iii") said second cleavable moiety.
[108] The affinity substance-modified antibody or a salt thereof according to any one of [98] to [107], wherein the affinity substance-modified antibody comprising the first and second modified moieties comprises a structural unit represented by the following formula (Vb): 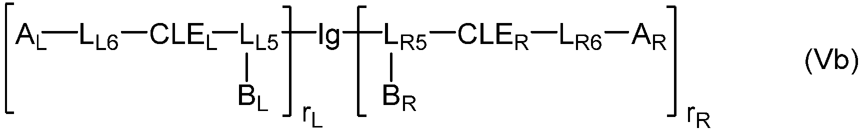 wherein
   Ig indicates said immunoglobulin unit;
   L_{L5} and L_{R5} each independently indicate a fifth linker;
   L_{L6} and L_{R6} each independently indicate a sixth linker;
   B_{L} indicates a first group comprising a first bioorthogonal functional group;
   B_{R} indicates a second group comprising a second bioorthogonal functional group;
   CLE_{L} and CLE_{R} each independently indicate a cleavable moiety;
   A_{L} indicates said first affinity substance;
   A_{R} indicates said second affinity substance; and
   the average percent modification r_{L} of said immunoglobulin unit with said first modified moiety and the average percent modification r_{R} of said immunoglobulin unit with said second modified moiety are individually 65 to 135%.
[109] The affinity substance-modified antibody or a salt thereof according to any one of [98] to [108], wherein the affinity substance-modified antibody comprising the first and second modified moieties comprises a structural unit represented by the following formula (Vb-1): 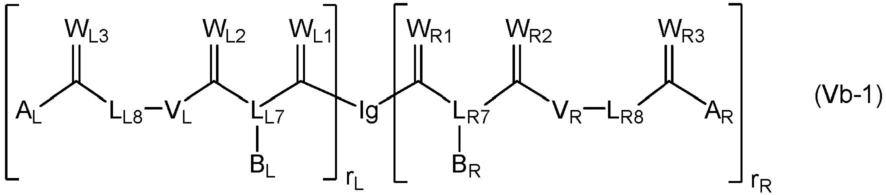 wherein
   Ig indicates said immunoglobulin unit;
   W_{L1}, W_{L2} and W_{L3}, and W_{R1}, W_{R2} and W_{R3} each independently indicate an oxygen atom or a sulfur atom;
   L_{L7} and L_{R7} each independently indicate a seventh linker;
   L_{L8} and L_{R8} each independently indicate an eighth linker;
   B_{L} indicates a first group comprising a first bioorthogonal functional group;
   B_{R} indicates a second group comprising a second bioorthogonal functional group;
   V_{L} and V_{R} each independently indicate an oxygen atom or a sulfur atom;
   A_{L} indicates said first affinity substance;
   A_{R} indicates said second affinity substance; and
   the average percent modification r_{L} of said immunoglobulin unit with said first modified moiety and the average percent modification r_{R} of said immunoglobulin unit with said second modified moiety are individually 65 to 135%.
[110] The affinity substance-modified antibody or a salt thereof according to any one of [98] to [109], wherein said antibody or a salt thereof further comprises (iii') a first cleavable moiety between (i') said first affinity substance and (ii') said immunoglobulin unit, and further comprises (iv") a first bioorthogonal functional group between (ii") said immunoglobulin unit and (iii") the second cleavable moiety; and
   said first cleavable moiety is a cleavable moiety that can be cleaved to generate a second bioorthogonal functional group on said immunoglobulin unit.
[111] The affinity substance-modified antibody or a salt thereof according to any one of [98] to [110], wherein the affinity substance-modified antibody comprising the first and second modified moieties comprises a structural unit represented by the following formula (Vc): 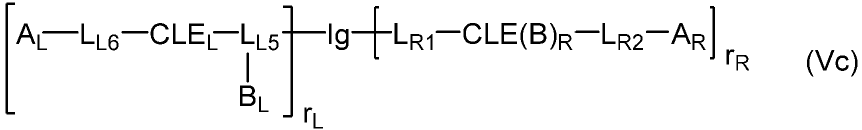 wherein
   Ig indicates said immunoglobulin unit;
   L_{R1} indicates a first linker;
   L_{R2} indicates a second linker;
   L_{L5} indicates a fifth linker;
   L_{L6} indicates a sixth linker;
   B_{L} indicates a group comprising a first bioorthogonal functional group;
   CLE_{L} indicates a first cleavable moiety;
   CLE(B)_{R} indicates a second cleavable moiety that can be cleaved to generate a second bioorthogonal functional group on said immunoglobulin unit side;
   A_{L} indicates said first affinity substance;
   A_{R} indicates said second affinity substance; and
   the average percent modification r_{L} of said immunoglobulin unit with said first modified moiety and the average percent modification r_{R} of said immunoglobulin unit with said second modified moiety are individually 65 to 135%.
[112] The affinity substance-modified antibody or a salt thereof according to any one of [98] to [111], wherein the affinity substance-modified antibody comprising the first and second modified moieties comprises a structural unit represented by the following formula (Vc-1): 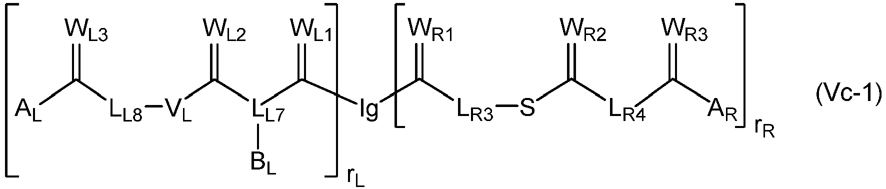 wherein
   Ig indicates said immunoglobulin unit;
   W_{L1}, W_{L2} and W_{L3}, and W_{R1}, W_{R2} and W_{R3} each independently indicate an oxygen atom or a sulfur atom;
   L_{R3} each independently indicates a third linker;
   L_{R4} each independently indicates a fourth linker;
   L_{L7} each independently indicates a seventh linker;
   L_{L8} each independently indicates an eighth linker;
   B_{L} indicates a group comprising a first bioorthogonal functional group;
   V_{L} indicates an oxygen atom or a sulfur atom;
   A_{L} indicates said first affinity substance;
   A_{R} indicates said second affinity substance; and
   the average percent modification r_{L} of said immunoglobulin unit with said first modified moiety and the average percent modification r_{R} of said immunoglobulin unit with said second modified moiety are individually 65 to 135%.
[113] The affinity substance-modified antibody or a salt thereof according to any one of [98] to [112], wherein the affinity substance-modified antibody further comprises an additional modified moiety.
[114] The affinity substance-modified antibody or a salt thereof according to [113], wherein the additional modified moiety is an additional affinity substance comprising a fifth affinity moiety having an affinity to the constant region in a heavy chain of the antibody, and said additional affinity substance is comprised in the constant region in a heavy chain of the antibody.
[115] The affinity substance-modified antibody or a salt thereof according to [114], wherein said additional affinity substance is introduced to the constant regions in said two heavy chains via modification of the amino group in the side chain of a lysine residue present at one or more positions in the constant regions in said two heavy chains.
[116] The affinity substance-modified antibody or a salt thereof according to [115], wherein said one or more positions in the constant regions in the two heavy chains are positions 246/248, 288/290, or 317 in a human IgG heavy chain according to the EU numbering.
[117] A method of producing an affinity substance-modified antibody or a salt thereof comprising first and second modified moieties, the method comprising:
   (1) reacting a compound or a salt thereof comprising (A) a first affinity substance comprising first and second affinity moieties each having an affinity to the constant region in a heavy chain of an antibody, and (B) a first reactive group for the antibody with the antibody comprising an immunoglobulin unit comprising two heavy chains and optionally two light chains to produce an affinity substance-modified antibody or a salt thereof comprising a first modified moiety in which said first affinity substance is contained in the constant region in a heavy chain of said immunoglobulin unit; and
   (2) reacting the affinity substance-modified antibody or a salt thereof comprising said first affinity substance in the constant region in a heavy chain of said immunoglobulin unit with a compound or a salt thereof comprising a second affinity substance comprising third and fourth affinity moieties each having an affinity to the constant region in a heavy chain of the antibody and (B) a second reactive group for the antibody to produce an affinity substance-modified antibody or a salt thereof comprising said first and second modified moieties in the constant region in the heavy chains of said immunoglobulin unit.
[118] The method according to [117], wherein the affinity substance-modified antibody or a salt thereof comprising first and second modified moieties is the affinity substance-modified antibody or a salt thereof according to any one of [98] to [116].
[119] An antibody derivative or a salt thereof comprising first and second modified moieties, comprising
   (a) an immunoglobulin unit comprising two heavy chains composed of first and second heavy chains and optionally two light chains, and
   (b) a first modified moiety comprising a first bioorthogonal functional group and a second modified moiety comprising a second bioorthogonal functional group;
   (c) wherein the first modified moiety has been introduced to the constant region in said first heavy chain;
   (d) wherein the second modified moiety has been introduced to the constant region in said second heavy chain; and
   (e) wherein the first and second modified moieties are different from each other.
[120] The antibody derivative or a salt thereof according to [119], wherein said first modified moiety has been introduced to the constant region in said first heavy chain via modification of the amino group in the side chain of a lysine residue present at one or more positions in the constant region in said first heavy chain; and said second modified moiety has been introduced to the constant region in said second heavy chain via modification of the amino group in the side chain of a lysine residue present at one or more positions in the constant regions in said second heavy chain.
[121] The antibody derivative or a salt thereof according to [119] or [120], wherein said one or more positions in the constant region in the first heavy chain and said one or more positions in the constant region in the second heavy chain are positions selected from the group consisting of positions 246/248, 288/290, and 317 in the human IgG heavy chain according to the EU numbering, and combinations thereof.
[122] The antibody derivative or a salt thereof according to any one of [119] to [121], wherein the antibody derivative or a salt thereof comprising first and second modified moieties comprises a structural unit represented by the following formula (VIa): 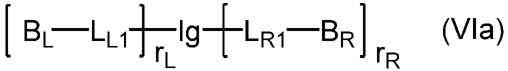 wherein
   Ig indicates said immunoglobulin unit;
   L_{L1} and L_{R1} each independently indicate a first linker;
   B_{L} indicates a first group comprising a first bioorthogonal functional group;
   B_{R} indicates a second group comprising a second bioorthogonal functional group; and
   the average percent modification r_{L} of said immunoglobulin unit with the first modified moiety and the average percent modification r_{R} of said immunoglobulin unit with the second modified moiety are individually 65 to 135%.
[123] The antibody derivative or a salt thereof according to any one of [119] to [122], wherein the antibody derivative or a salt thereof comprising first and second modified moieties comprises a structural unit represented by the following formula (VIa-1): 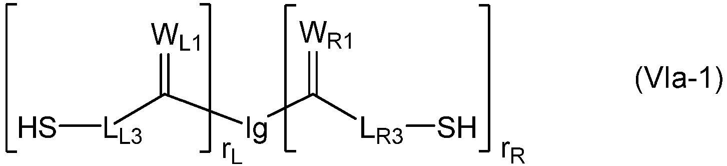 wherein
   Ig indicates said immunoglobulin unit;
   W_{L1} and W_{R1} each independently indicate an oxygen atom or a sulfur atom;
   L_{L3} and L_{R3} each independently indicate a third linker;
   SH indicates a thiol group that is a bioorthogonal functional group; and
   the average percent modification r_{L} of said immunoglobulin unit with the first modified moiety and the average percent modification r_{R} of said immunoglobulin unit with the second modified moiety are individually 65 to 135%.
[124] The antibody derivative or a salt thereof according to any one of [119] to [123], wherein the antibody derivative or a salt thereof comprising first and second modified moieties comprises a structural unit represented by the following formula (VIb): 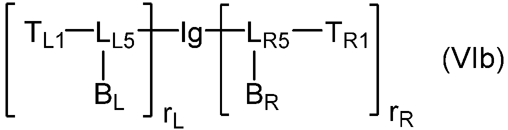 wherein
   Ig indicates said immunoglobulin unit;
   L_{L5} and L_{R5} each independently indicate a fifth linker;
   B_{L} indicates a first group comprising a first bioorthogonal functional group;
   B_{R} indicates a second group comprising a second bioorthogonal functional group;
   T_{L1} and T_{R1} each independently indicate a monovalent group; and
   the average percent modification r_{L} of said immunoglobulin unit with the first modified moiety and the average percent modification r_{R} of said immunoglobulin unit with the second modified moiety are individually 65 to 135%.
[125] The antibody derivative or a salt thereof according to any one of [119] to [124], wherein the antibody derivative or a salt thereof comprising first and second modified moieties comprises a structural unit represented by the following formula (VIb-1): 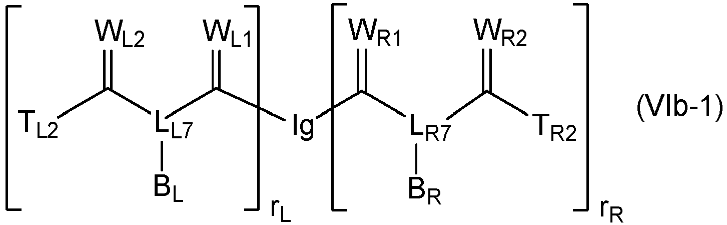 wherein
   Ig indicates said immunoglobulin unit;
   W_{L1} and W_{L2}, and W_{R1} and W_{R2} each independently indicate an oxygen atom or a sulfur atom;
   L_{L7} and L_{R7} each independently indicate a seventh linker;
   B_{L} indicates a first group comprising a first bioorthogonal functional group;
   B_{R} indicates a second group comprising a second bioorthogonal functional group;
   T_{L2} and T_{R2} each independently indicate a monovalent group; and
   the average percent modification r_{L} of said immunoglobulin unit with the first modified moiety and the average percent modification r_{R} of said immunoglobulin unit with the second modified moiety are individually 65 to 135%.
[126] The antibody derivative or a salt thereof according to any one of [119] to [125], wherein the antibody derivative or a salt thereof comprising first and second modified moieties comprises a structural unit represented by the following formula (VIc): 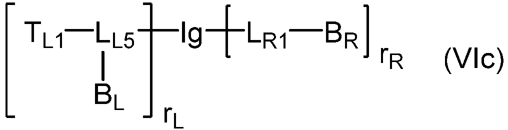 wherein
   Ig indicates said immunoglobulin unit;
   L_{R1} indicates a first linker;
   L_{L5} indicates a fifth linker;
   B_{L} indicates a first group comprising a first bioorthogonal functional group;
   B_{R} indicates a second group comprising a second bioorthogonal functional group;
   T_{L1} indicates a monovalent group; and
   the average percent modification r_{L} of said immunoglobulin unit with the first modified moiety and the average percent modification r_{R} of said immunoglobulin unit with the second modified moiety are individually 65 to 135%.
[127] The antibody derivative or a salt thereof according to any one of [119] to [126], wherein the antibody derivative or a salt thereof comprising first and second modified moieties comprises a structural unit represented by the following formula (VIc-1): 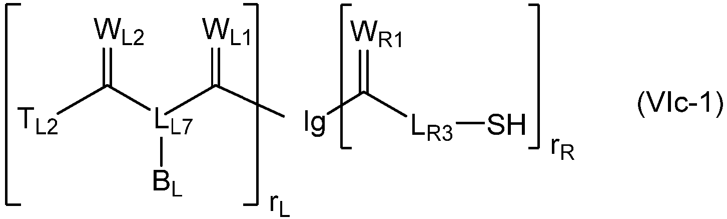 wherein
   Ig indicates said immunoglobulin unit;
   W_{L1} and W_{L2}, and W_{R1} each independently indicate an oxygen atom or a sulfur atom;
   L_{R3} indicates a third linker;
   L_{L7} indicates a seventh linker;
   B_{L} indicates a first group comprising a first bioorthogonal functional group;
   SH indicates a thiol group that is a second bioorthogonal functional group;
   T_{L2} indicates a monovalent group; and
   the average percent modification r_{L} of said immunoglobulin unit with the first modified moiety and the average percent modification r_{R} of said immunoglobulin unit with the second modified moiety are individually 65 to 135%.
[128] The antibody derivative or a salt thereof according to any one of [119] to [127], wherein the antibody derivative further comprises an additional modified moiety.
[129] The antibody derivative or a salt thereof according to [128], wherein the additional modification moiety is an additional modification moiety comprising a bioorthogonal functional group, and said additional modified moiety comprising a bioorthogonal functional group is comprised in the constant region in a heavy chain of the antibody.
[130] The antibody derivative or a salt thereof according to [129], wherein said additional modified moiety comprising a bioorthogonal functional group is introduced to the constant regions in said two heavy chains via modification of the amino group in the side chain of a lysine residue present at one or more positions in the constant regions in said two heavy chains.
[131] The antibody derivative or a salt thereof according to [130], wherein said one or more positions in the constant regions in the two heavy chains are positions 246/248, 288/290, or 317 in a human IgG heavy chain according to the EU numbering.
[132] A conjugate of an antibody and first and second modified moieties, or a salt thereof, comprising (a) an immunoglobulin unit comprising two heavy chains composed of first and second heavy chains and optionally two light chains, and (b) a first modified moiety comprising a first functional substance and a second modified moiety comprising a second functional substance;
   (c) wherein the first modified moiety has been introduced to the constant region in said first heavy chain;
   (d) wherein the second modified moiety has been introduced to the constant region in said second heavy chain; and
   (e) wherein the first and second modified moieties are different from each other.
[133] The conjugate or a salt thereof according to [132], wherein said first modified moiety has been introduced to the constant region in said first heavy chain via modification of the amino group in the side chain of a lysine residue present at one or more positions in the constant region in said first heavy chain; and said second modified moiety has been introduced to the constant region in said second heavy chain via modification of the amino group in the side chain of a lysine residue present at one or more positions in the constant regions in said second heavy chain.
[134] The conjugate or a salt thereof according to [132] or [133], wherein said one or more positions in the constant region in the first heavy chain and said one or more positions in the constant region in the second heavy chain are positions selected from the group consisting of positions 246/248, 288/290, and 317 in the human IgG heavy chain according to the EU numbering, and combinations thereof.
[135] The conjugate or a salt thereof according to any one of [132] to [134], wherein said conjugate or a salt thereof comprises a structural unit represented by the following formula (VIIa): 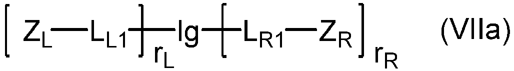 wherein
   Ig indicates said immunoglobulin unit;
   L_{L1} and L_{R1} each independently indicate a first linker;
   Z_{L} indicates a first functional substance;
   Z_{R} indicates a second functional substance; and
   the average percent modification r_{L} of said immunoglobulin unit with the first modified moiety and the average percent modification r_{R} of said immunoglobulin unit with the second modified moiety are individually 65 to 135%.
[136] The conjugate or a salt thereof according to [132] to [135], wherein said conjugate or a salt thereof comprises a structural unit represented by the following formula (VIIa-1): 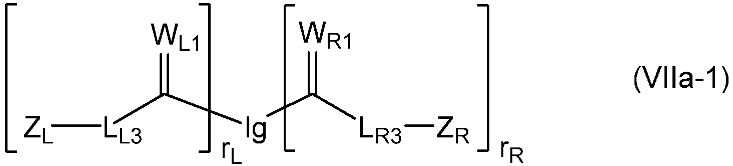 wherein
   Ig indicates said immunoglobulin unit;
   W_{L1} and W_{R1} each independently indicate an oxygen atom or a sulfur atom;
   L_{L3} and L_{R3} each independently indicate a third linker;
   Z_{L} indicates a first functional substance;
   Z_{R} indicates a second functional substance; and
   the average percent modification r_{L} of said immunoglobulin unit with the first modified moiety and the average percent modification r_{R} of said immunoglobulin unit with the second modified moiety are individually 65 to 135%.
[137] The conjugate or a salt thereof according to [132] to [136], wherein said conjugate or a salt thereof comprises a structural unit represented by the following formula (VIIb): 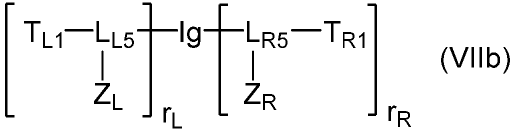 wherein
   Ig indicates said immunoglobulin unit;
   L_{L5} and L_{R5} each independently indicate a fifth linker;
   Z_{L} indicates a first functional substance;
   Z_{R} indicates a second functional substance;
   T_{L1} and T_{R1} each independently indicate a monovalent group; and
   the average percent modification r_{L} of said immunoglobulin unit with the first modified moiety and the average percent modification r_{R} of said immunoglobulin unit with the second modified moiety are individually 65 to 135%.
[138] The conjugate or a salt thereof according to [132] to [137], wherein said conjugate or a salt thereof comprises a structural unit represented by the following formula (VIIb-1): 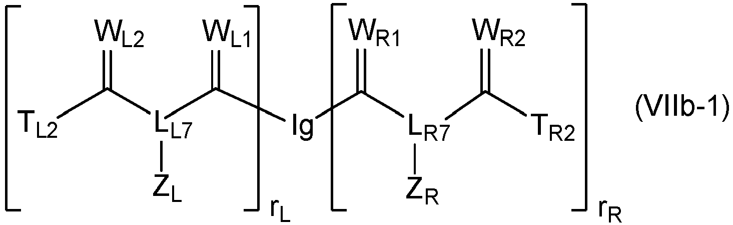 wherein
   Ig indicates said immunoglobulin unit;
   W_{L1} and W_{L2}, and W_{R1} and W_{R2} each independently indicate an oxygen atom or a sulfur atom;
   L_{L7} and L_{R7} each independently indicate a seventh linker;
   Z_{L} indicates a first functional substance;
   Z_{R} indicates a second functional substance;
   T_{L2} and T_{R2} each independently indicate a monovalent group; and
   the average percent modification r_{L} of said immunoglobulin unit with the first modified moiety and the average percent modification r_{R} of said immunoglobulin unit with the second modified moiety are individually 65 to 135%.
[139] The conjugate or a salt thereof according to [132] to [138], wherein said conjugate or a salt thereof comprises a structural unit represented by the following formula (VIIc): 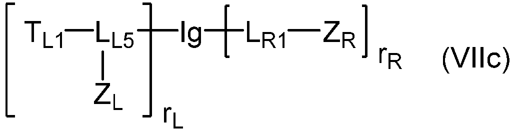 wherein
   Ig indicates said immunoglobulin unit;
   L_{R1} indicates a first linker;
   L_{L5} indicates a fifth linker;
   Z_{L} indicates a first functional substance;
   Z_{R} indicates a second functional substance;
   T_{L1} indicates a monovalent group; and
   the average percent modification r_{L} of said immunoglobulin unit with the first modified moiety and the average percent modification r_{R} of said immunoglobulin unit with the second modified moiety are individually 65 to 135%.
[140] The conjugate or a salt thereof according to [132] to [139], wherein said conjugate or a salt thereof comprises a structural unit represented by the following formula (VIIc-1): 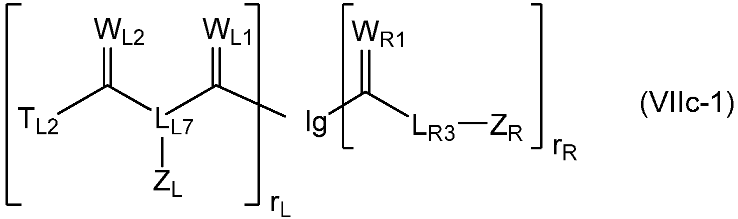 wherein
   Ig indicates said immunoglobulin unit;
   W_{L1} and W_{L2}, and W_{R1} each independently indicate an oxygen atom or a sulfur atom;
   L_{R3} indicates a third linker;
   L_{L7} indicates a seventh linker;
   Z_{L} indicates a first functional substance;
   Z_{R} indicates a second functional substance;
   T_{L2} indicates a monovalent group; and
   the average percent modification r_{L} of said immunoglobulin unit with the first modified moiety and the average percent modification r_{R} of said immunoglobulin unit with the second modified moiety are individually 65 to 135%.
[141] The conjugate or a salt thereof according to [132] to [140], wherein the first and second functional substances are each independently a drug, a labelling substance, an affinity substance, a transporting substance, or a stabilizer.
[142] The conjugate or a salt thereof according to [132] to [141], wherein one of the first and second functional substances is a full-length antibody or a fragment thereof, or both of the first and second functional substances are each independently a full-length antibody or a fragment thereof.
[143] The conjugate or a salt thereof according to [132] to [142], wherein the conjugate further comprises an additional modified moiety.
[144] The conjugate or a salt thereof according to [143], wherein the additional modified moiety is an additional modified moiety comprising a functional substance, and said additional modified moiety comprising a functional substance is contained in a constant region in a heavy chain of the antibody.
[145] The conjugate or a salt thereof according to [144], wherein the additional modified moiety comprising a functional substance is introduced into said constant regions in two heavy chains via modification of the amino group in the side chain of a lysine residue present at one or more positions in said constant regions in two heavy chains.
[146] The conjugate or a salt thereof according to [145], wherein said one or more positions in the constant regions in two heavy chains are positions 246/248, 288/290, or 317 in a human IgG heavy chain according to the EU numbering.

### ADVANTAGEOUS EFFECTS OF INVENTION

According to the present invention, only one heavy chain of the constituent unit (immunoglobulin unit comprising two heavy chains) of an antibody can be easily modified.

In addition, according to the present invention, an antibody that is easily modified in only one heavy chain of the constituent unit of the antibody, while being regioselectively modified.

### BRIEF DESCRIPTION OF THE DRAWINGS

FIG. 1 shows a schematic diagram showing the modification of a constituent unit of an antibody with the compound of the present invention or a salt thereof represented by the formula (I).
FIG. 2 shows a diagram showing an outline of an embodiment of the present invention.
FIG. 3 shows a diagram showing an outline of another embodiment of the present invention.
FIG. 4 shows a diagram showing an outline of a still another embodiment of the present invention.
FIG. 5 shows a diagram showing an outline of a still another embodiment of the present invention.
FIG. 6 shows a diagram showing an outline of a still another embodiment of the present invention.

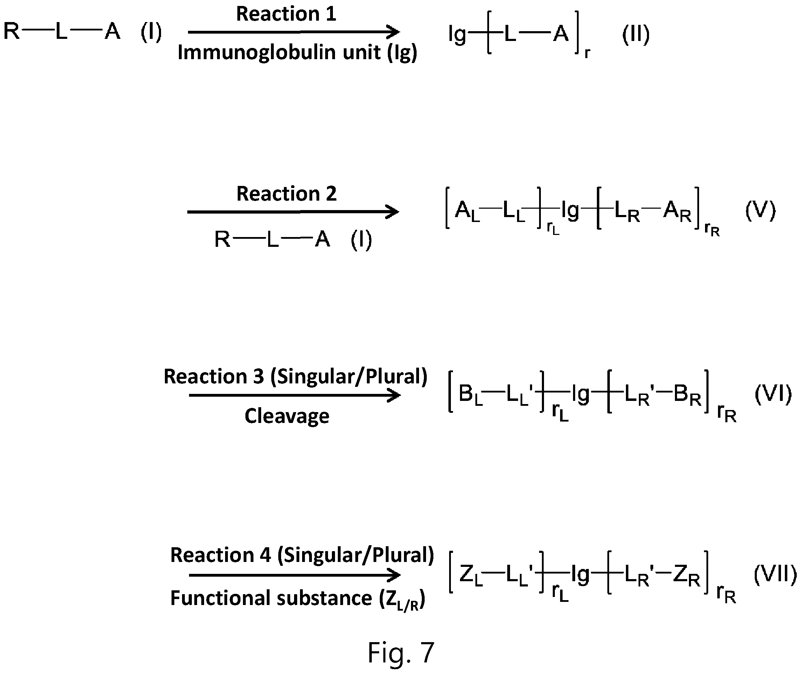

FIG. 7 shows a diagram showing an outline of an embodiment of the present invention.

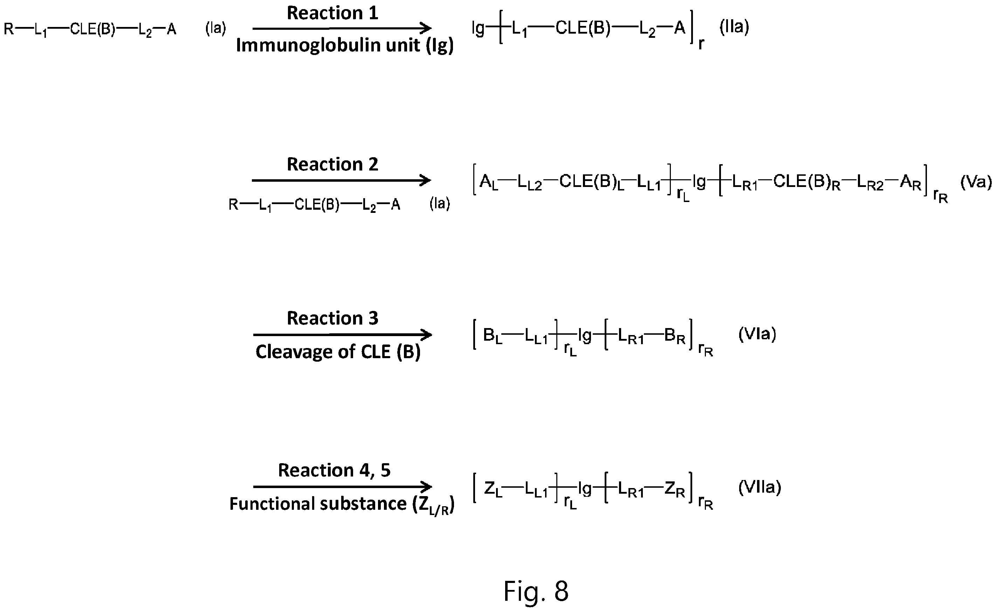

FIG. 8 shows a diagram showing an outline of another embodiment of the present invention.

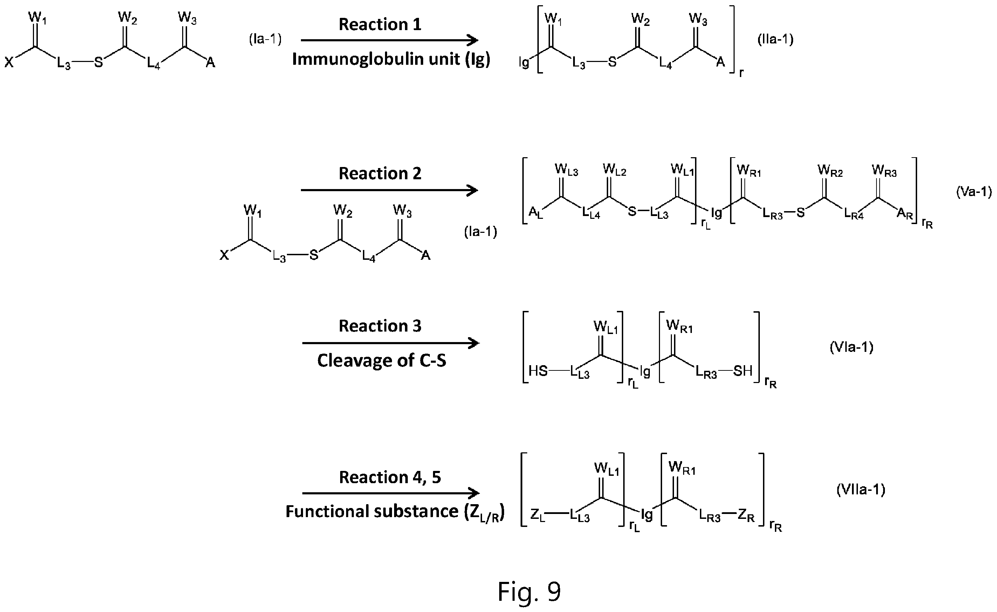

FIG. 9 shows a diagram showing an outline of a still another embodiment of the present invention.

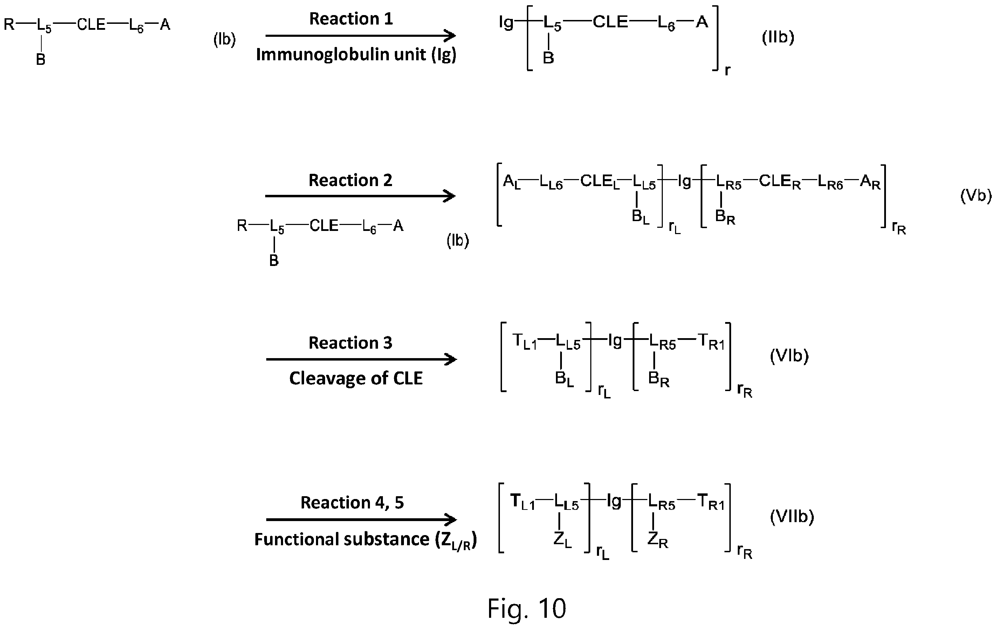

FIG. 10 shows a diagram showing an outline of a still another embodiment of the present invention.

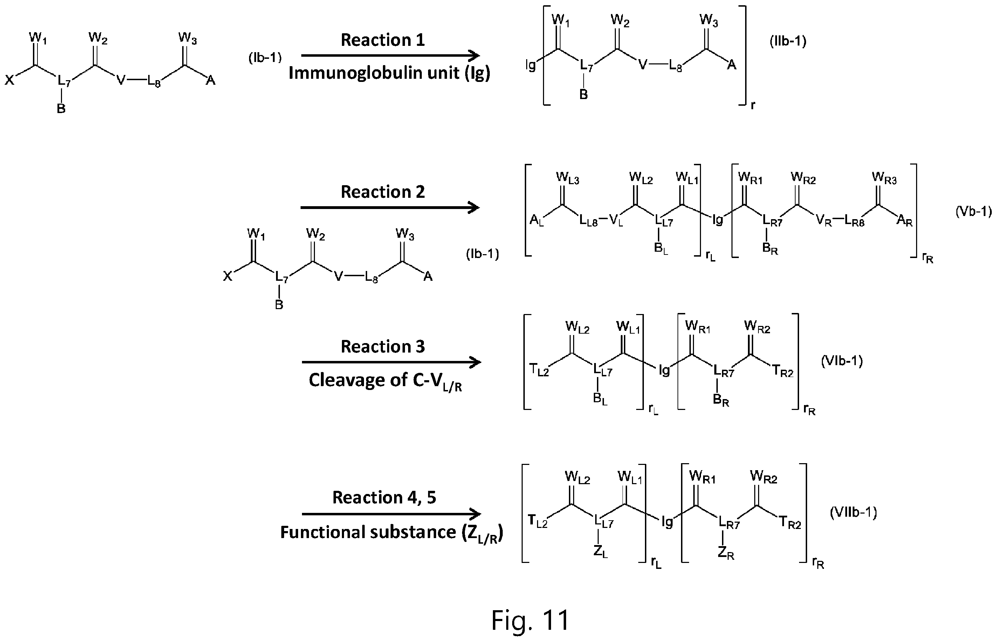

FIG. 11 shows a diagram showing an outline of a still another embodiment of the present invention.

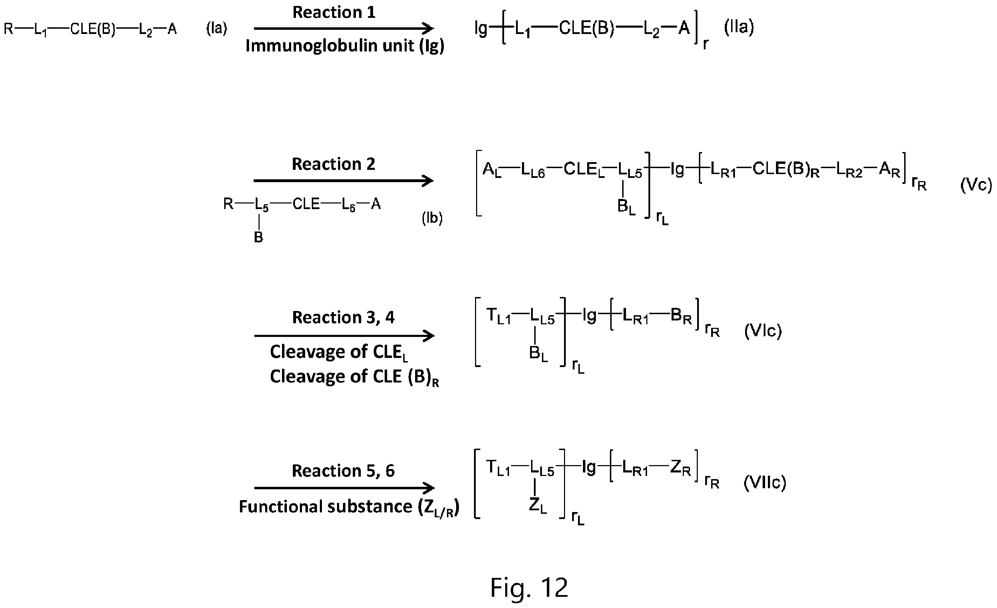

FIG. 12 shows a diagram showing an outline of a still another embodiment of the present invention.

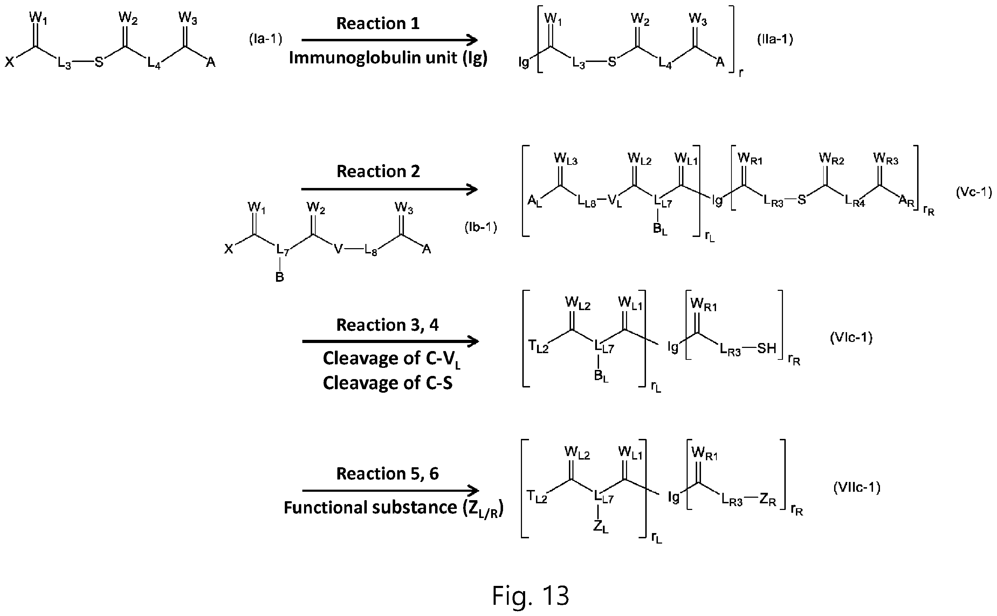

FIG. 13 shows a diagram showing an outline of a still another embodiment of the present invention.

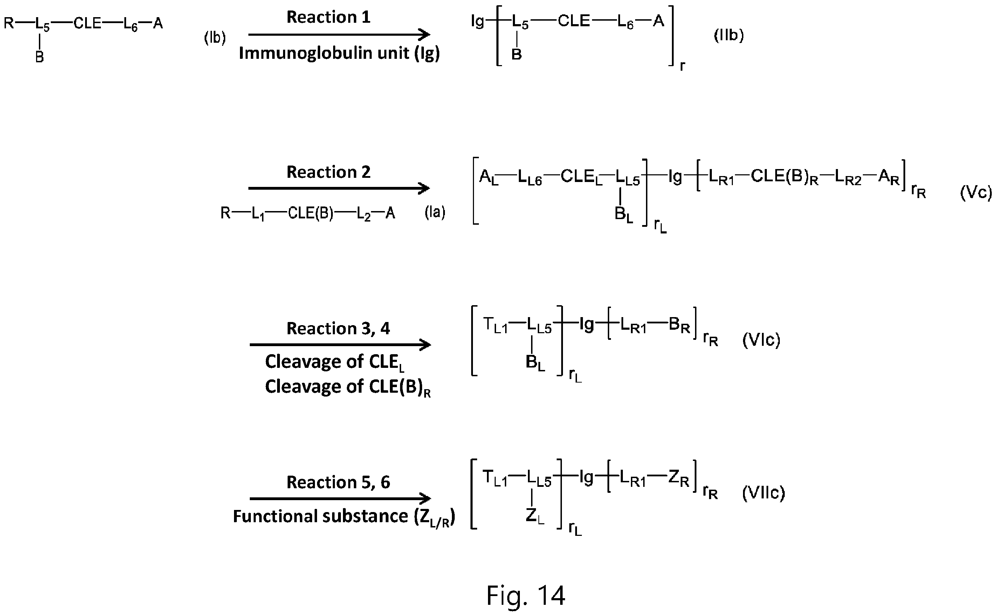

FIG. 14 shows a diagram showing an outline of a still another embodiment of the present invention.

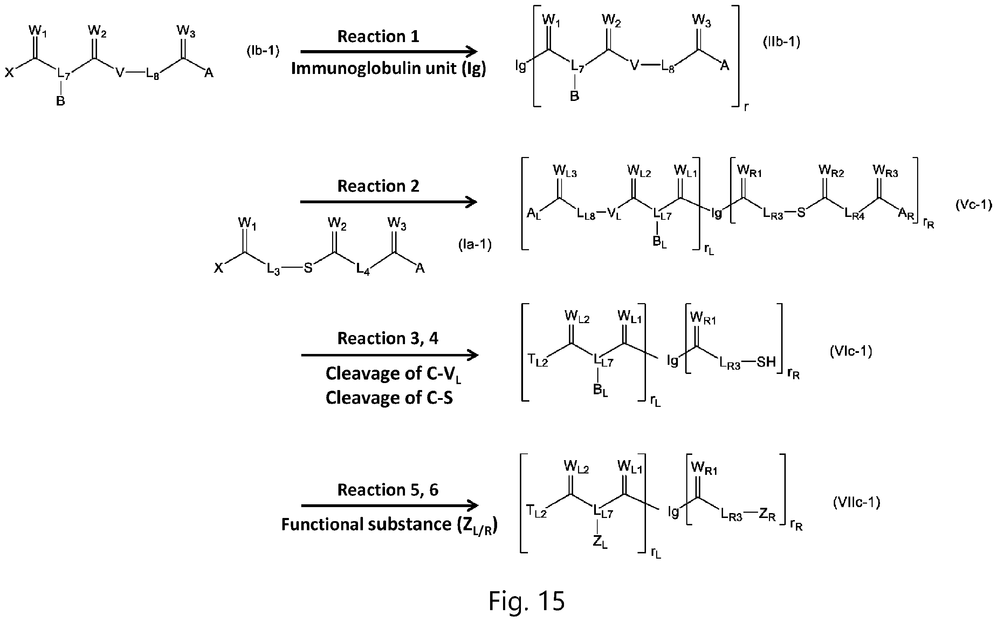

FIG. 15 shows a diagram showing an outline of a still another embodiment of the present invention.
FIG. 16 shows a diagram showing the expression of the polypeptides in the transformants.
FIG. 17-1 illustrates a sensorgram showing the affinity of the polypeptide QET-Z34CM-PA32-Fc3K (SEQ ID NO: 1) for the Fc region of an IgG1 antibody.
FIG. 17-2 illustrates a sensorgram showing the affinity of the polypeptide QET-Z34CM-PA48-Fc3K (SEQ ID NO: 2) for the Fc region of an IgG1 antibody.
FIG. 17-3 illustrates a sensorgram showing the affinity of the polypeptide QET-Fc3-PA32-Z34CM (SEQ ID NO: 3) for the Fc region of an IgG1 antibody.
FIG. 17-4 illustrates a sensorgram showing the affinity of the polypeptide QET-Fc3K-PA48-Z34CM (SEQ ID NO: 4) for the Fc region of an IgG1 antibody.
FIG. 17-5 illustrates a sensorgram showing the affinity of the polypeptide QET-Fc3K-PA32-ProAR (SEQ ID NO: 5) for the Fc region of an IgG1 antibody.
FIG. 17-6 illustrates a sensorgram showing the affinity of the polypeptide QET-Fc3K-PA48-ProAR (SEQ ID NO: 6) for the Fc region of an IgG1 antibody.
FIG. 17-7 illustrates a sensorgram showing the affinity of the polypeptide QET-ProAR-PA32-Z34CK (SEQ ID NO: 7) for the Fc region of an IgG1 antibody.
FIG. 17-8 illustrates a sensorgram showing the affinity of the polypeptide QET-ProAR-PA48-Z34CK (SEQ ID NO: 8) for the Fc region of an IgG1 antibody.
FIG. 18 shows a sensorgram showing the results from the determination of the peptide/antibody bonding ratio.
FIG. 19-1 shows the modification site of a lysine residue within the constant region in a heavy chain of an antibody, as determined by LC-MS/MS.
FIG. 19-2 shows the modification of the lysine residue at the position 246 or 248 in a heavy chain according to the EU numbering, as determined by CID spectrum.
FIG. 19-3 shows the selectivity of the modification to the lysine residue at the position 248, as determined by BioPharma Finder.
FIG. 20-1 shows a sensorgram showing the affinity between EGFR and cetuximab.
FIG. 20-2 shows a sensorgram showing the affinity between neonatal Fc receptor (FcRn) and cetuximab.
FIG. 20-3 shows a sensorgram showing the pH-dependent affinity between FcRn and cetuximab.
FIG. 21-1 shows a sensorgram showing the affinity between EGFR and a bi-specific antibody (Cetuximab-Trastuzumab Fab) (M-3).
FIG. 21-2 shows a sensorgram showing the affinity between HER2 and a bi-specific antibody (Cetuximab-Trastuzumab Fab) (M-3).
FIG. 21-3 shows a sensorgram showing the affinity between FcRn and a bi-specific antibody (Cetuximab-Trastuzumab Fab) (M-3).
FIG. 21-4 shows a sensorgram showing the pH-dependent affinity between FcRn and a bi-specific antibody (Cetuximab-Trastuzumab Fab) (M-3).
FIG. 22-1 shows a sensorgram showing the affinity between EGFR and a bi-specific antibody (Trastuzumab-Cetuximab Fab) (M-4).
FIG. 22-2 shows a sensorgram showing the affinity between HER2 and a bi-specific antibody (Trastuzumab-Cetuximab Fab) (M-4).
FIG. 22-3 shows a sensorgram showing the affinity between FcRn and a bi-specific antibody (Trastuzumab-Cetuximab Fab) (M-4).
FIG. 22-4 shows a sensorgram showing the pH-dependent affinity between FcRn and a bi-specific antibody (Trastuzumab-Cetuximab Fab) (M-4).
FIG. 23-1 shows a sensorgram showing the affinity between EGFR and a Tri-specific antibody (M-2).
FIG. 23-2 shows a sensorgram showing the affinity between HER2 and a Tri-specific antibody (M-2).
FIG. 23-3 shows a sensorgram showing the affinity between PD-1 and a Tri-specific antibody (M-2).
FIG. 23-4 shows a sensorgram showing the affinity between FcRn and a Tri-specific antibody (M-2).
FIG. 23-5 shows a sensorgram showing the pH-dependent affinity between FcRn and a Tri-specific antibody (M-2).
FIG. 24-1 shows the positive rates of SKBR-3 cells (HER2-positive) and A-431 cells (EGFR-positive) bound to cetuximab (Cmab), trastuzumab (Tmab), the bi-specific antibody (Cetuximab-Trastuzumab Fab) (M-3) (Trastuzumab-Cetuximab Fab) (M-4), and the tri-specific antibody (Pembrolizumab Fab-Cetuximab-Trastuzumab Fab) (M-2), as determined by flow cytometry.
FIG. 24-2 shows the evaluation of cetuximab (Cmab) binding to SKBR-3 cells (HER2-positive), A-431 cells (EGFR-positive), and T cells (PD-1-positive), as determined by flow cytometry.
FIG. 24-3 shows the evaluation of trastuzumab (Tmab) binding to SKBR-3 cells (HER2-positive) and A-431 cells (EGFR-positive), as determined by flow cytometry.
FIG. 24-4 shows the evaluation of the bi-specific antibody (Cetuximab-Trastuzumab Fab) (M-3) binding to SKBR-3 cells (HER2-positive) and A-431 cells (EGFR-positive), as determined by flow cytometry.
FIG. 24-5 shows the evaluation of the bi-specific antibody (Trastuzumab-Cetuximab Fab) (M-4) binding to SKBR-3 cells (HER2-positive) and A-431 cells (EGFR-positive), as determined by flow cytometry.
FIG. 24-6 shows the evaluation of pembrolizumab (Pbl) binding to T cells (PD-1-positive), as determined by flow cytometry.
FIG. 24-7 shows the evaluation of the tri-specific antibody (Pembrolizumab Fab-Cetuximab-Trastuzumab Fab) (M-2) binding to SKBR-3 cells (HER2-positive), A-431 cells (EGFR-positive), and T cells (PD-1-positive), as determined by flow cytometry.
FIG. 24-8 shows the positive rates of T cells (PD-1-positive) bound to cetuximab (Cmab), pembrolizumab (Pbl), and the tri-specific antibody (Pembrolizumab Fab-Cetuximab-Trastuzumab Fab) (M-2), as determined by flow cytometry.
FIG. 25 shows SDS-PAGE for the expression of CD3-VHH-PA24H6-AzF (V-1).
FIG. 26 shows the evaluation results for the affinity of CD3-VHH(V-1).
FIG. 27 shows the evaluation results for the affinity of CD3-VHH(V-1).
FIG. 28 shows SDS-PAGE for six affinity peptides.

### EMBODIMENTS FOR CARRYING OUT THE INVENTION

### 1. Definitions of General Terms

Terms and phrases used herein to describe a particular invention or item may also be used to describe another invention or item. Therefore, the definitions, examples, and preferred examples of the terms and phrases used to describe a particular invention or item may be the same as those for another invention or item described using such terms and phrases.

In the present invention, the term "antibody" is as follows. The term "immunoglobulin unit" corresponds to a divalent monomer unit that is a constituent unit of such an antibody, and is an immunoglobulin unit comprising two heavy chains and optionally two light chains. Therefore, definitions, examples, and preferred examples of the origin, type (polyclonal or monoclonal, isotype, and full-length antibody or antibody fragment), antigen, position of an amino acid residue (e.g., lysine residue), and regioselectivity of the immunoglobulin unit are similar to those of the antibody described below and used interchangeably with the expression "antibody."

The origin of the antibody is not particularly limited, and for example, the antibody may be derived from an animal such as a mammal or a bird (e.g., a domestic fowl). The immunoglobulin unit is preferably derived from a mammal. Examples of such a mammal include primates (e.g., humans, monkeys, and chimpanzees), rodents (e.g., mice, rats, guinea pigs, hamsters, and rabbits), pets (e.g., dogs and cats), domestic animals (e.g., cows, pigs, and goats), and work animals (e.g., horses and sheep). Primates and rodents are preferred, and humans are more preferred.

The type of antibody may be a polyclonal antibody or a monoclonal antibody. The antibody may be a divalent antibody (e.g., IgG, IgD, or IgE) or a tetravalent or higher antibody (e.g., IgA antibody or IgM antibody). The antibody is preferably a monoclonal antibody. Examples of the monoclonal antibody include chimeric antibodies, humanized antibodies, human antibodies, antibodies with a certain sugar chain added (e.g., an antibody modified so as to have a sugar chain-binding consensus sequence such as an N-type sugar chain-binding consensus sequence), bi-specific antibodies, Fc region proteins, Fc-fusion proteins, and disulfide bond-reduced antibodies. Examples of the isotype of the monoclonal antibody include IgG (e.g., IgG1, IgG2, IgG3, and IgG4), IgM, IgA, IgD, IgE, and IgY. In the present invention, as the monoclonal antibody, a full-length antibody or a variable region, and an antibody fragment comprising a CH1 domain and a CH2 domain can be used, but a full-length antibody is preferred. The antibody is preferably a human IgG monoclonal antibody, and more preferably a human IgG full-length monoclonal antibody.

As an antigen of the antibody, any antigen can be used. Examples of such an antigen include proteins [including oligopeptides and polypeptides, or they may be proteins modified with a biomolecule such as a sugar (e.g., glycoproteins)], sugar chains, nucleic acids, and small compounds. The antibody may be preferably an antibody that recognizes a protein as an antigen. Examples of the protein include cell membrane receptors, cell membrane proteins other than cell membrane receptors (e.g., extracellular matrix proteins), ligands, and soluble receptors.

More specifically, the protein as an antigen of the antibody may be a disease target protein. Examples of the disease target protein include the following.

### (1) Cancerous Region

PD-L1, GD2, PDGFRα (platelet-derived growth factor receptor), CD22, HER2, phosphatidylserine (PS), EpCAM, fibronectin, PD-1, VEGFR-2, CD33, HGF, gpNMB, CD27, DEC-205, folic acid receptors, CD37, CD19, Trop2, CEACAM5, S1P, HER3, IGF-1R, DLL4, TNT-1/B, CPAAs, PSMA, CD20, CD105 (Endoglin), ICAM-1, CD30, CD16A, CD38, MUC1, EGFR, KIR2DL1, KIR2DL2, NKG2A, tenascin-C, IGF (insulin-like growth factor), CTLA-4, mesothelin, CD138, c-Met, Ang2, VEGF-A, CD79b, ENPD3, folic acid receptor α, TEM-1, GM2, Glypican 3, macrophage inhibitory factor, CD74, Notch1, Notch2, Notch3, CD37, TLR-2, CD3, CSF-1R, FGFR2b, HLA-DR, GM-CSF, EphA3, B7-H3, CD123, gpA33, Frizzled7 receptor, DLL4, VEGF, RSPO, LIV-1, SLITRK6, Nectin-4, CD70, CD40, CD19, SEMA4D (CD100), CD25, MET, Tissue Factor, IL-8, EGFR, cMet, KIR3DL2, Bst1(CD157), P-Cadherin, CEA, GITR, TAM (tumor associated macrophage), CEA, DLL4, Ang2, CD73, FGFR2, CXCR4, LAG-3, GITR, Fucosyl GM1, IGF-1, Angiopoietin 2, CSF-1R, FGFR3, OX40, BCMA, ErbB3, CD137(4-1BB), PTK7, EFNA4, FAP, DR5, CEA, Ly6E, CA6, CEACAM5, LAMP1, tissue factor, EPHA2, DR5, B7-H3, FGFR4, FGFR2, α2-PI, A33, GDF15, CAIX, CD166, ROR1, GITR, BCMA, TBA, LAG-3, EphA2, TIM-3, CD-200, EGFRvIII, CD16A, CD32B, PIGF, Axl, MICA/B, Thomsen-Friedenreich, CD39, CD37, CD73, CLEC12A, Lgr3, transferrin receptors, TGFβ, IL-17, 5T4, RTK, Immune Suppressor Protein, NaPi2b, Lewis blood group B antigen, A34, Lysil-Oxidase, DLK-1, TROP-2, α9 Integrin, TAG-72(CA72-4), and CD70.

### (2) Autoimmune Diseases and Inflammatory Diseases

IL-17, IL-6R, IL-17R, INF-α, IL-5R, IL-13, IL-23, IL-6, ActRIIB, β7-Integrin, IL-4αR, HAS, Eotaxin-1, CD3, CD19, TNF-α, IL-15, CD3ε, Fibronectin, IL-1β, IL-1α, IL-17, TSLP (Thymic Stromal Lymphopoietin), LAMP (Alpha4 Beta 7 Integrin), IL-23, GM-CSFR, TSLP, CD28, CD40, TLR-3, BAFF-R, MAdCAM, IL-31R, IL-33, CD74, CD32B, CD79B, IgE (immunoglobulin E), IL-17A, IL-17F, C5, FcRn, CD28, TLR4, MCAM, B7RP1, CXCR1/2 Ligands, IL-21, Cadherin-11, CX3CL1, CCL20, IL-36R, IL-10R, CD86, TNF-α, IL-7R, Kv1.3, α9 Integrin, and LIFHT.

### (3) Brain or Nerve Diseases

CGRP, CD20, β amyloid, β amyloid protofibril, Calcitonin Gene-Related Peptide Receptor, LINGO (Ig Domain Containing 1), a Synuclein, extracellular tau, CD52, insulin receptors, tau protein, TDP-43, SOD1, TauC3, and JC virus.

### (4) Infectious Diseases

Clostridium Difficile toxin B, cytomegalovirus, RS viruses, LPS, S. Aureus Alpha-toxin, M2e protein, Psl, PcrV, S. Aureus toxin, influenza A, Alginate, Staphylococcus aureus, PD-L1, influenza B, Acinetobacter, F-protein, Env, CD3, enteropathogenic Escherichia coli, Klebsiella, and Streptococcus pneumoniae.

### (5) Hereditary Rare Diseases

amyloid AL, SEMA4D (CD100), insulin receptors, ANGPTL3, IL4, IL13, FGF23, adrenocorticotropic hormone, transthyretin, and huntingtin.

### (6) Eye Diseases

Factor D, IGF-1R, PGDFR, Ang2, VEGF-A, CD-105 (Endoglin), IGF-1R, and β amyloid.

### (7) Bone and Orthopedic Region

Sclerostin, Myostatin, Dickkopf-1, GDF8, RNAKL, HAS, and Siglec-15.

### (8) Blood Diseases

vWF, Factor IXa, Factor X, IFNγ, C5, BMP-6, Ferroportin, and TFPI.

### (9) Other Diseases

BAFF (B cell activating factor), IL-1β, PCSK9, NGF, CD45, TLR-2, GLP-1, TNFR1, C5, CD40, LPA, prolactin receptors, VEGFR-1, CB1, Endoglin, PTH1R, CXCL1, CXCL8, IL-1β, AT2-R, and IAPP.

Specific examples of the monoclonal antibody include specific chimeric antibodies (e.g., rituximab, basiliximab, infliximab, cetuximab, siltuximab, dinutuximab, and altertoxaximab), specific humanized antibodies (e.g., daclizumab, palivizumab, trastuzumab, alemtuzumab, omalizumab, efalizumab, bevacizumab, natalizumab (IgG4), tocilizumab, eculizumab (IgG2), mogamulizumab, pertuzumab, obinutuzumab, vedolizumab, pembrolizumab (IgG4), mepolizumab, elotuzumab, daratumumab, ixekizumab (IgG4), reslizumab (IgG4), and atezolizumab), and specific human antibodies (e.g., adalimumab (IgG1), panitumumab, golimumab, ustekinumab, canakinumab, ofatumumab, denosumab (IgG2), ipilimumab, belimumab, raxibacumab, ramucirumab, nivolumab, dupilumab (IgG4), secukinumab, evolocumab (IgG2), alirocumab, necitumumab, brodalumab (IgG2), and olaratumab) (cases not referring to the IgG subtype indicate that they are IgG1).

According to the present invention, a specific amino acid residue in the constant region in a heavy chain of an antibody can be regioselectively modified. Examples of such a specific amino acid residue include a lysine residue, a tyrosine residue, a serine residue, and a threonine residue. For example, in human IgG such as human IgG1, the following amino acid residues present in the heavy chain constant region can be exposed to the antibody surface. Thus, these amino acid residues can be used to introduce specific cleavable moieties (the positions of the amino acid residues are in accordance with the EU numbering; see *http:*//*www.imgt.org*/*IMGTScientificChart*/*Numbering*/*Hu_IGHGnber.html).*
(1) Exposed lysine residue
   CH2 domain (e.g., positions 246, 248, 274, 288, 290, 317, 320, and 322)
   CH3 domain (e.g., positions 360, 414, and 439)
(2) Exposed tyrosine residue
   CH2 domain (e.g., positions 278, 296, and 300)
   CH3 domain (e.g., position 436)
(3) Exposed serine residue
   CH2 domain (e.g., positions 254, 267, and 298)
   CH3 domain (e.g., positions 400, 415, and 440)
(4) Exposed threonine residue
   CH2 domain (e.g., positions 256 and 289)
   CH3 domain (e.g., positions 335 and 359)

The positions of an amino acid residue in the antibody and the position of the constant region in a heavy chain (e.g., CH2 domain) are in accordance with the EU numbering (see, *http:*//*www.imgt.org*/*IMGTScientificChart*/*Numbering*/*Hu_IGHGnber. html*). For example, when human IgG is a target, the lysine residue at the position 246 corresponds to the 16th amino acid residue in the human IgG CH2 region; the lysine residue at the position 248 corresponds to the 18th amino acid residue in the human IgG CH2 region; the lysine residue at the position 288 corresponds to the 58th amino acid residue in the human IgG CH2 region; the lysine residue at the position 290 corresponds to the 60th amino acid residue in the human IgG CH2 region; and the lysine residue at the position 317 corresponds to the 87th amino acid residue in the human IgG CH2 region. The notation at the position 246/248 indicates that the lysine residue at the position 246 or 248 is a target. The notation at the position 288/290 indicates that the lysine residue at the position 288 or 290 is a target.

Preferably, for a specific amino acid residue in the constant region in a heavy chain to be regioselectively modified, a lysine residue (e.g., the lysine residue at the position 246/248 or 288/290) can be regioselectively modified. **In** the present specification, "regioselective" or "regioselectivity" refers to a state in which even though a specific amino acid residue is not present locally in a specific region in the antibody, a certain structural unit capable of binding to the specific amino acid residue in the antibody is present locally in a specific region in the antibody. Consequently, expressions related to regioselectivity such as "regioselectively having," "regioselective binding," and "binding with regioselectivity" mean that the possession rate or the binding rate of a certain structural unit in the target region comprising one or more specific amino acid residues is higher at a significant level than the possession rate or the binding rate of the structural unit in the non-target region comprising a plurality of amino acid residues of the same type as the specific amino acid residues in the target region. Such regioselectivity may be 50% or more, preferably 60% or more, more preferably 70% or more, even more preferably 80% or more, and particularly preferably 90% or more, 95% or more, 96% or more, 97% or more, 98% or more, 99% or more, 99.5% or more, or 100%. According to the present invention, a specific lysine residue in a heavy chain of an antibody can be regioselectively modified without utilizing a linker containing a peptide. The peptide moiety has potential immunogenicity and is susceptible to hydrolysis in a blood. Therefore, avoiding the use of a linker containing a peptide moiety is desirable in clinical applications.

In the present invention, as long as a specific amino acid residue in the constant region in a heavy chain (e.g., a lysine residue at a specific position) is regioselectively modified, a specific amino acid residue at another position may be further regioselectively modified. For example, a method for regioselectively modifying a specific amino acid residue at a certain position in an antibody is described in WO 2018/199337 A, WO 2019/240288 A, WO 2019/240287 A, and WO 2020/090979 A. As such a specific amino acid residue, an amino acid residue (e.g., a lysine residue, an aspartic acid residue, a glutamic acid residue, an asparagine residue, a glutamine residue, a threonine residue, a serine residue, a tyrosine residue, or a cysteine residue) having a side chain that is easily modified (e.g., an amino group, a carboxy group, an amide group, a hydroxy group, or a thiol group) can be used. However, a lysine residue having a side chain comprising an amino group, a tyrosine residue having a side chain comprising a hydroxy group, a serine residue, a threonine residue, or a cysteine residue having a side chain comprising a thiol group may be preferred, and a lysine residue may be more preferred (that is, two lysine residues of the lysine residues at the position 246/248, 288/290, and 317 may be regioselectively double-modified, or three lysine residues of them may be regioselectively triple-modified).

### Halogen atom

Examples of the halogen atom include a fluorine atom, a chlorine atom, a bromine atom, and an iodine atom.

### Monovalent group

Examples of the monovalent group include a monovalent hydrocarbon group and a monovalent heterocyclic group.

The monovalent group may be substituted by one or more (e.g., 1 to 10, preferably 1 to 8, more preferably 1 to 6, even more preferably 1 to 5, particularly preferably 1 to 3) substituents as described below.

### Monovalent hydrocarbon group and related terms

Examples of the monovalent hydrocarbon group include a monovalent chain hydrocarbon group, a monovalent alicyclic hydrocarbon group, and a monovalent aromatic hydrocarbon group.

The monovalent chain hydrocarbon group means a hydrocarbon group comprising only a chain structure and does not comprise any cyclic structure in the main chain thereof. Note that the chain structure may be linear or branched. Examples of the monovalent chain hydrocarbon group include alkyl, alkenyl, and alkynyl. The alkyl, alkenyl, and alkynyl may be linear or branched.

The alkyl is preferably C1-12 alkyl, more preferably C1-6 alkyl, and even more preferably C1-4 alkyl. When the alkyl has a substituent, the number of carbon atoms does not include the number of carbon atoms of the substituent. Examples of the C1-12 alkyl include methyl, ethyl, n-propyl, i-propyl, n-butyl, s-butyl, isobutyl, t-butyl, pentyl, hexyl, heptyl, octyl, nonyl, decyl, and dodecyl.

The alkenyl is preferably C2-12 alkenyl, more preferably C2-6 alkenyl, and even more preferably C2-4 alkenyl. When the alkenyl has a substituent, the number of carbon atoms does not include the number of carbon atoms of the substituent. Examples of the C2-12 alkenyl include vinyl, propenyl, and n-butenyl.

The alkynyl is preferably C2-12 alkynyl, more preferably C2-6 alkynyl, and even more preferably C2-4 alkynyl. When the alkyl has a substituent, the number of carbon atoms does not include the number of carbon atoms of the substituent. Examples of the C2-12 alkynyl include ethynyl, propynyl, and n-butynyl.

The monovalent chain hydrocarbon group is preferably alkyl.

The monovalent alicyclic hydrocarbon group means a hydrocarbon group comprising only an alicyclic hydrocarbon as a cyclic structure and not comprising any aromatic ring, in which the alicyclic hydrocarbon may be monocyclic or polycyclic. Note that the monovalent alicyclic hydrocarbon group is not necessarily required to comprise only an alicyclic hydrocarbon but may comprise a chain structure in part thereof. Examples of the monovalent alicyclic hydrocarbon group include cycloalkyl, cycloalkenyl, and cycloalkynyl, which may be monocyclic or polycyclic.

The cycloalkyl is preferably C3-12 cycloalkyl, more preferably C3-6 cycloalkyl, and even more preferably C5_6 cycloalkyl. When the cycloalkyl has a substituent, the number of carbon atoms does not include the number of carbon atoms of the substituent. Examples of the C3-12 cycloalkyl include cyclopropyl, cyclobutyl, cyclopentyl, and cyclohexyl.

The cycloalkenyl is preferably C3-12 cycloalkenyl, more preferably C3-6 cycloalkenyl, and even more preferably C5-6 cycloalkenyl. When the cycloalkenyl has a substituent, the number of carbon atoms does not include the number of carbon atoms of the substituent. Examples of the C3-12 cycloalkenyl include cyclopropenyl, cyclobutenyl, cyclopentenyl, and cyclohexenyl.

The cycloalkynyl is preferably C3-12 cycloalkynyl, more preferably C3-6 cycloalkynyl, and even more preferably C5-6 cycloalkynyl. When the cycloalkynyl has a substituent, the number of carbon atoms does not include the number of carbon atoms of the substituent. Examples of the C3-12 cycloalkynyl include cyclopropynyl, cyclobutynyl, cyclopentynyl, and cyclohexynyl.

The monovalent alicyclic hydrocarbon group is preferably cycloalkyl.

The monovalent aromatic hydrocarbon group means a hydrocarbon group comprising an aromatic cyclic structure. Note that the monovalent aromatic hydrocarbon group is not necessarily required to comprise only an aromatic ring and may comprise a chain structure or alicyclic hydrocarbon in part thereof, in which the aromatic ring may be monocyclic or polycyclic. The monovalent aromatic hydrocarbon group is preferably C6-12 aryl, more preferably C6-10 aryl, and even more preferably C6 aryl. When the monovalent aromatic hydrocarbon group has a substituent, the number of carbon atoms does not include the number of carbon atoms of the substituent. Examples of the C6-12 aryl include phenyl and naphthyl.

The monovalent aromatic hydrocarbon group is preferably phenyl.

Among these groups, the monovalent hydrocarbon group is preferably alkyl, cycloalkyl, or aryl.

### Monovalent heterocyclic group and related terms

The monovalent heterocyclic group refers to a group obtained by removing one hydrogen atom from a heterocycle of a heterocyclic compound. The monovalent heterocyclic group is a monovalent aromatic heterocyclic group or a monovalent nonaromatic heterocyclic group. The monovalent heterocyclic group preferably comprises one or more selected from the group consisting of an oxygen atom, a sulfur atom, a nitrogen atom, a phosphorus atom, a boron atom, and a silicon atom, and more preferably comprises one or more selected from the group consisting of an oxygen atom, a sulfur atom, and a nitrogen atom as a hetero atom comprised in the heterocyclic group.

The monovalent aromatic heterocyclic group is preferably a C1-15 aromatic heterocyclic group, more preferably a C1-9 aromatic heterocyclic group, and even more preferably a C1-6 aromatic heterocyclic group. When the monovalent aromatic heterocyclic group has a substituent, the number of carbon atoms does not include the number of carbon atoms of the substituent. Examples of the monovalent aromatic heterocyclic group include pyrrolyl, furanyl, thiophenyl, pyridinyl, pyridazinyl, pyrimidinyl, pyrazinyl, triazinyl, pyrazolyl, imidazolyl, thiazolyl, isothiazolyl, oxazolyl, isoxazolyl, triazolyl, tetrazolyl, indolyl, purinyl, anthraquinolyl, carbazonyl, fluorenyl, quinolinyl, isoquinolinyl, quinazolinyl, and phthalazinyl.

The monovalent nonaromatic heterocyclic group is preferably a C2-15 nonaromatic heterocyclic group, more preferably a C2-9 nonaromatic heterocyclic group, and even more preferably a C2-6 nonaromatic heterocyclic group. When the monovalent nonaromatic heterocyclic group has a substituent, the number of carbon atoms does not include the number of carbon atoms of the substituent. Examples of the monovalent nonaromatic heterocyclic group include oxiranyl, aziridinyl, azetidinyl, oxetanyl, thietanyl, pyrrolidinyl, dihydrofuranyl, tetrahydrofuranyl, dioxolanyl, tetrahydrothiophenyl, pyrolinyl, imidazolidinyl, oxazolidinyl, piperidinyl, dihydropyranyl, tetrahydropyranyl, tetrahydrothiopyranyl, morpholinyl, thiomorpholinyl, piperazinyl, dihydrooxazinyl, tetrahydrooxazinyl, dihydropyrimidinyl, and tetrahydropyrimidinyl.

Among these groups, the monovalent heterocyclic group is preferably a five-membered or six-membered heterocyclic group.

### Divalent group

The divalent group is a group having a main chain structure containing one group or 2 or more (e.g., 2 to 10, preferably 2 to 8, more preferably 2 to 6, even more preferably 2 to 5, and particularly preferably 2 or 3) groups selected from the group consisting of a divalent linear hydrocarbon group, a divalent cyclic hydrocarbon group, a divalent heterocyclic group, -C(=O)-, -C(=S)-, -NR₁-, -C(=O)-NR₁-, -NR₁-C(=O)-, - C(=S)-NR₁-, -NR₁-C(=S)-, -O-, -S-, -(O-R₂)ₙ-, and -(S-R₂)ₘ-. R₁ indicates a hydrogen atom or a substituent as described below. R₂ indicates a divalent linear hydrocarbon group, a divalent cyclic hydrocarbon group, or a divalent heterocyclic group. n and m are each an integer of 1 to 10, preferably an integer of 1 to 8, more preferably an integer of 1 to 6, even more preferably an integer of 1 to 5, and particularly preferably an integer of 1 to 3.

The divalent linear hydrocarbon group is a linear alkylene, a linear alkenylene, or a linear alkynylene.

The linear alkylene is a C1-6 linear alkylene, and is preferably a C1-4 linear alkylene. Examples of the linear alkylene include methylene, ethylene, n-propylene, n-butylene, n-pentylene, and n-hexylene.

The linear alkenylene is a C2-6 linear alkenylene, and is preferably a C2-4 linear alkenylene. Examples of the linear alkenylene include ethylenylene, n-propynylene, n-butenylene, n-pentenylene, and n-hexenylene.

The linear alkynylene is a C2-6 linear alkynylene, and is preferably a C2-4 linear alkynylene. Examples of the linear alkynylene include ethynylene, n-propynylene, n-butynylene, n-pentynylene, and n-hexynylene.

The divalent linear hydrocarbon group is preferably a linear alkylene.

The divalent cyclic hydrocarbon group is an arylene or a divalent nonaromatic cyclic hydrocarbon group.

The arylene is preferably a C6-14 arylene, more preferably a C6-10 arylene, and particularly preferably a C6 arylene. Examples of the arylene include phenylene, naphthylene, and anthracenylene.

The divalent nonaromatic cyclic hydrocarbon group is preferably a C3-12 monocyclic or polycyclic divalent nonaromatic cyclic hydrocarbon group, more preferably a C4-10 monocyclic or polycyclic divalent nonaromatic cyclic hydrocarbon group, and particularly preferably a C5-8 monocyclic divalent nonaromatic cyclic hydrocarbon group. Examples of the divalent nonaromatic cyclic hydrocarbon group include cyclopropylene, cyclobutylene, cyclopentylene, cyclohexylene, cycloheptylene, and cyclooctylene.

The divalent cyclic hydrocarbon group is preferably an arylene.

The divalent heterocyclic group is a divalent aromatic heterocyclic group or a divalent nonaromatic heterocyclic group. The divalent heterocyclic group preferably comprises, as a hetero atom forming a heterocycle, one or more selected from the group consisting of an oxygen atom, a sulfur atom, a nitrogen atom, a phosphorous atom, a boron atom, and a silicon atom, and more preferably comprises one or more selected from the group consisting of an oxygen atom, a sulfur atom, and a nitrogen atom.

The divalent aromatic heterocyclic group is preferably a C3-15 divalent aromatic heterocyclic group, more preferably a C3-9 divalent aromatic heterocyclic group, and particularly preferably a C3-6 divalent aromatic heterocyclic group. Examples of the divalent aromatic heterocyclic group include pyrrolediyl, furandiyl, thiophenediyl, pyridinediyl, pyridazinediyl, pyrimidinediyl, pyrazinediyl, triazinediyl, pyrazolediyl, imidazolediyl, thiazolediyl, isothiazolediyl, oxazolediyl, isoxazolediyl, triazolediyl, tetrazolediyl, indolediyl, purinediyl, anthraquinonediyl, carbazolediyl, fluorenediyl, quinolinediyl, isoquinolinediyl, quinazolinediyl, and phthalazinediyl.

The divalent nonaromatic heterocyclic group is preferably a C3-15 nonaromatic heterocyclic group, more preferably a C3-9 nonaromatic heterocyclic group, and particularly preferably a C3-6 nonaromatic heterocyclic group. Examples of the divalent nonaromatic heterocyclic group include pyrroldionediyl, pyrrolinedionediyl, oxiranediyl, aziridinediyl, azetidinediyl, oxetanediyl, thietanediyl, pyrrolidinediyl, dihydrofurandiyl, tetrahydrofurandiyl, dioxolanediyl, tetrahydrothiophenediyl, pyrrolinediyl, imidazolidinediyl, oxazolidinediyl, piperidinediyl, dihydropyrandiyl, tetrahydropyrandiyl, tetrahydrothiopyrandiyl, morpholinediyl, thiomorpholinediyl, piperazinediyl, dihydrooxazinediyl, tetrahydrooxazinediyl, dihydropyrimidinediyl, and tetrahydropyrimidinediyl.

The divalent heterocyclic group is preferably a divalent aromatic heterocyclic group.

Preferably, the divalent group is a divalent group having a main chain structure containing one group selected from the group consisting of alkylene, arylene, -C(=O)-, - NR₁-, -C(=O)-NR₁-, -NR₁-C(=O)-, -O-, and -(O-R₂)ₙ-; or
a divalent group having a main chain structure containing two or more selected from the group consisting of alkylene, arylene, -C(=O)-, -NR₁-, -C(=O)-NR₁-, -NR₁-C(=O)-, -O-, and -(O-R₂)ₙ-;
R₁ is a hydrogen atom or alkyl;
R₂ is alkylene or arylene; and
n may be an integer of 1 to 5 (or 1, 2, 3, 4, or 5).

The alkylene, arylene, and alkyl are similar to those described above.

The main chain structure in the divalent group may be substituted by one or more (e.g., 1 to 10, preferably 1 to 8, more preferably 1 to 6, even more preferably 1 to 5, particularly preferably 1 to 3) substituents as described below.

### (Substituent)

Examples of the substituents include:
(i) a halogen atom;
(ii) a monovalent hydrocarbon group;
(iii) a monovalent heterocyclic group;
(iv) an aralkyl;
(v) Rₐ-O-, Rₐ-C(=O)-, Rₐ-O-C(=O)-, or Rₐ-C(=O)-O- (Rₐ indicates a hydrogen atom, or a monovalent hydrocarbon group); or
(vi) NR_{b}R_{c}-, NR_{b}R_{c}-C(=O)-, NR_{b}R_{c}-C(=O)-O-, or R_{b}-C(=O)-NR_{c}- (R_{b} and R_{c} are the same or different from each other, and each represent a hydrogen atom or a monovalent hydrocarbon group);
(vii) a nitro group, a sulfate group, a sulfonate group, a cyano group, and a carboxyl group.

The definitions, examples, and preferred examples of the halogen atom, the monovalent hydrocarbon group, and the monovalent heterocyclic group in the substituent are similar to those described above.

The aralkyl refers to arylalkyl. The definitions, examples, and preferred examples of the aryl and the alkyl in the arylalkyl are as described above. The aralkyl is preferably C3-15 aralkyl. Examples of such an aralkyl include benzoyl, phenethyl, naphthylmethyl, and naphthylethyl.

Preferably, the substituent may be:
(i) a halogen atom;
(ii) a C1-12 alkyl, a C1-12 phenyl, or a C1-12 naphthyl;
(iii) a C3-15 aralkyl;
(iv) a 5- or 6-membered heterocycle;
(v) Rₐ-O-, Rₐ-C(=O)-, Rₐ-O-C(=O)-, or Rₐ-C(=O)-O- (where Rₐ represents a hydrogen atom, or a C1-12 alkyl);
(vi) NR_{b}R_{c}-, NR_{b}R_{c}-C(=O)-, NR_{b}R_{c}-C(=O)-O-, or R_{b}-C(=O)-NR_{c}- (where R_{b} and R_{c} are the same or different from each other, and each represents a hydrogen atom or a C1-12 alkyl); or
(vii) the same groups as listed in the above (vii).

More preferably, the substituent may be:
(i) a halogen atom;
(ii) a C1-12 alkyl;
(iii) Rₐ-O-, Rₐ-C(=O)-, Rₐ-O-C(=O)-, or Rₐ-C(=O)-O, (where Rₐ represents a hydrogen atom or a C1-12 alkyl);
(iv) NR_{b}R_{c}-, NR_{b}R_{c}-C(=O)-, NR_{b}R_{c}-C(=O)-O-, or R_{b}-C(=O)-NR_{c}- (where R_{b} and R_{c} are the same as or different from each other, and each represent a hydrogen atom or a C1-12 alkyl); or
(v) the same groups as listed in the above (vii).

Even more preferably, the substituent may be:
(i) a halogen atom;
(ii) a C1-6 alkyl;
(iii) Rₐ-O-, Rₐ-C(=O)-, Rₐ-O-C(=O)-, or Rₐ-C(=O)-O- (where Rₐ represents a hydrogen atom or a C1-6 alkyl);
(iv) NR_{b}R_{c}-, NR_{b}R_{c}-C(=O)-, NR_{b}R_{c}-C(=O)-O-, or R_{b}-C(=O)-NR_{c}- (where R_{b} and R_{c} are the same as or different from each other, and each represent a hydrogen atom or a C1-6 alkyl); or
(v) the same groups as listed in the above (vii).

Particularly preferably, the substituent may be:
(i) a halogen atom;
(ii) a C1-4 alkyl;
(iii) Rₐ-O-, Rₐ-C(=O)-, Rₐ-O-C(=O)-, or Rₐ-C(=O)-O- (where Rₐ represents a hydrogen atom or a C1-4 alkyl);
(iv) NR_{b}R_{c}-, NR_{b}R_{c}-C(=O)-, NR_{b}R_{c}-C(=O)-O-, or R_{b}-C(=O)-NR_{c}- (where R_{b} and R_{c} are the same as or different from each other, and each represent a hydrogen atom or a C1-4 alkyl); or
(v) the same groups as listed in the above (vii).

### (Bioorthogonal functional group)

Bioorthogonal functional groups refer to groups that do not react with biological components (e.g., amino acids, proteins, nucleic acids, lipids, carbohydrates, and phosphates), or react slowly with biological components but selectively with non-biological components. Bioorthogonal functional groups are well known in the art (see, e.g., Sharpless K. B. et al., Angew. Chem. Int. Ed. 40,2004 (2015*); B*ertozzi C. R. et al., Science 291,2357(2001*);* Bertozzi C. R. et al., Nature Chemical Biology 1,13 (2005*)).*

In the present invention, bioorthogonal functional groups for proteins are used as bioorthogonal functional groups. This is because antibodies to be derivatized with the reagent of the present invention are proteins. The bioorthogonal functional groups for proteins are groups that do not react with the side chains of the 20 naturally-occurring amino acid residues constituting proteins, or react slowly with the side chains but react with a target functional group. The 20 naturally-occurring amino acids constituting proteins are alanine (A), asparagine (N), cysteine (C), glutamine (Q), glycine (G), isoleucine (I), leucine (L), methionine (M), phenylalanine (F), proline (P), serine (S), threonine (T), tryptophan (W), tyrosine (Y), valine (V), aspartic acid (D), glutamic acid (E), arginine (R), histidine (H), and lysine (L). Of these 20 naturally-occurring amino acids, glycine that has no side chain (i.e., the side chain is a hydrogen atom), and alanine, isoleucine, leucine, phenylalanine, and valine with a side chain that is a hydrocarbon group (i.e., the side chain does not contain any hetero atom selected from the group consisting of a sulfur atom, a nitrogen atom, and an oxygen atom) are inactive to common reactions. Thus, the bioorthogonal functional groups for proteins are groups that do not react not only with the side chains of these amino acids wherein the side chains are inactive to common reactions, but also with the side chains of asparagine, glutamine, methionine, proline, serine, threonine, tryptophan, tyrosine, aspartic acid, glutamic acid, arginine, histidine, and lysine, or react slowly with them but react with a target functional group.

Examples of such a bioorthogonal functional group include azide residues, aldehyde residues, thiol residues, alkene residues (in other words, they should have a vinylene (ethenylene) moiety that is the smallest unit having a carbon-carbon double bond. The same shall apply hereinafter), alkyne residues (in other words, they should have an ethynylene moiety that is the smallest unit having a carbon-carbon triple bond. The same shall apply hereinafter), halogen residues, tetrazine residues, nitrone residues, hydroxylamine residues, nitrile residues, hydrazine residues, ketone residues, boronic acid residues, cyanobenzothiazole residues, allyl residues, phosphine residues, maleimide residue, disulfide residues, thioester residues, α-halocarbonyl residues (e.g., carbonyl residues having a fluorine atom, a chlorine atom, a bromine atom, or an iodine atom at the alpha position. The same shall apply hereinafter), isonitrile residues, sydnone residues, and selenium residues.

The bioorthogonal functional group may be protected or unprotected. The bioorthogonal functional group refers to an unprotected bioorthogonal functional group or a protected bioorthogonal functional group. The unprotected bioorthogonal functional group corresponds to the bioorthogonal functional group as described above. The protected bioorthogonal functional group is a group that generate a bioorthogonal functional group by cleavage of the protecting group. The cleavage of the protecting group can be performed by a specific treatment under conditions that cannot cause protein denaturation or degradation (e.g., cleavage of an amide bond) (mild conditions). Examples of such a specific treatment include (a) treatment with one or more substance selected from the group consisting of an acidic substance, a basic substance, a reducing agent, an oxidizing agent, and an enzyme, (b) treatment with physicochemical stimulation selected from the group consisting of light, or (c) incubation using a cleavable linker comprising a self-degradable cleavable moiety. Such protecting groups and the conditions for cleaving them are common technical knowledge in the art (e.g., G. Leriche, L. Chisholm, A. Wagner, Bioorganic & Medicinal Chemistry. 20,571 (2012*);* Feng P. et al., Jounal of American Chemical Society. 132,1500 (2010*).;* Bessodes M. et al., Journal of Controlled Release, 99,423 (2004*).;* DeSimone, J. M., Journal of American Chemical Society. 132,17928 (2010*);* Thompson, D. H., Journal of Controlled Release, 91,187 (2003*);* Schoenmarks, R. G., Journal of Controlled Release, 95,291 (2004*)).* For the mild conditions, the reaction conditions (e.g., the reaction temperature, the reaction time, and the reaction solution) are as described later.

Examples of the protected bioorthogonal functional group include disulfide residues, ester residues, acetal residues, ketal residues, imine residues, and vicinaldiol residues.

Preferably, the bioorthogonal functional group is an unprotected bioorthogonal functional group.

More preferably, the bioorthogonal functional group may be a specific bioorthogonal functional group that has excellent responsiveness with other bioorthogonal functional groups (e.g., reaction level and/or reaction specificity). Examples of such a bioorthogonal functional group include azide residues, alkyne residues (preferably, ring groups that may be substituted by a substituent as described above and have a carbon-carbon triple bond), tetrazine residues, alkene residues, thiol residues, maleimide residues, thiol residues, furan residues, and halocarbonyl residues. Examples of a combination of two bioorthogonal functional group that can be reacted with each other include a combination of an azide residue and an alkyne residue, a combination of a tetrazine residue and an alkene residue, a combination of a tetrazine residue and an alkyne residue, a combination of a thiol residue and a maleimide residue, a combination of a furan residue and a maleimide residue, a combination of a thiol residue and a halocarbonyl residue (a replacement reaction replaces the halogen with thiol), and a combination of a thiol residue and another thiol residue (generation of a disulfide bond).

### Functional substance

The functional substance is not limited to a particular substance as long as it is a substance imparting any function to the antibody; examples thereof include drugs, labelling substances, affinity substances, transporting substances, and stabilizers; preferred may be drugs, labelling substances, affinity substances, and transporting substances. The functional substance may be a single functional substance or a substance in which two or more functional substances are linked with each other.

The drug may be a drug for any disease. Examples of such a disease include cancer (e.g., lung cancer, stomach cancer, colon cancer, pancreatic cancer, renal cancer, liver cancer, thyroid cancer, prostatic cancer, bladder cancer, ovarian cancer, uterine cancer, bone cancer, skin cancer, a brain tumor, and melanoma), autoimmune diseases and inflammatory diseases (e.g., allergic diseases, articular rheumatism, and systemic lupus erythematosus), brain or nerve diseases (e.g., cerebral infarction, Alzheimer's disease, Parkinson disease, and amyotrophic lateral sclerosis), infectious diseases (e.g., microbial infectious diseases and viral infectious diseases), hereditary rare diseases (e.g., hereditary spherocytosis and nondystrophic myotonia), eye diseases (e.g., age-related macular degeneration, diabetic retinopathy, and retinitis pigmentosa), diseases in the bone and orthopedic field (e.g., osteoarthritis), blood diseases (e.g., leukosis and purpura), and other diseases (e.g., diabetes, metabolic diseases such as hyperlipidemia, liver diseases, renal diseases, lung diseases, circulatory system diseases, and digestive system diseases). The drug may be a prophylactic or therapeutic agent for a disease, or a relief agent for side effects.

More specifically, the drug may be an anti-cancer agent. Examples of the anti-cancer agent include chemotherapeutic agents, toxins, and radioisotopes, and substances comprising them. Examples of chemotherapeutic agents include DNA injuring agents, antimetabolites, enzyme inhibitors, DNA intercalating agents, DNA cleaving agents, topoisomerase inhibitors, DNA binding inhibitors, tubulin binding inhibitors, cytotoxic nucleosides, and platinum compounds. Examples of toxins include bacteriotoxins (e.g., diphtheria toxin) and phytotoxins (e.g., ricin). Examples of radioisotopes include radioisotopes of a hydrogen atom (e.g., ³H), radioisotopes of a carbon atom (e.g., ¹⁴C), radioisotopes of a phosphorous atom (e.g., ³²P), radioisotopes of a sulfur atom (e.g., ³⁵S), radioisotopes of yttrium (e.g., ⁹°Y), radioisotopes of technetium (e.g., ^{99m}Tc), radioisotopes of indium (e.g., ¹¹¹In), radioisotopes of an iodide atom (e.g., ¹²³I, ¹²⁵I, ¹²⁹I, and ¹³¹I), radioisotopes of samarium (e.g., ¹⁵³Sm), radioisotopes of rhenium (e.g., ¹⁸⁶Re), radioisotopes of astatine (e.g., ²¹¹At), and radioisotopes of bismuth (e.g., ²¹²Bi). More specific examples of the drug include auristatin (MMAE, MMAF), maytansine (DM1, DM4), PBD (pyrrolobenzodiazepine), IGN, camptothecin analogs, calicheamicin, duocarmycin, eribulin, anthracycline, dmDNA31, and tubricin.

The labelling substance is a substance that makes detection of a target (e.g., a tissue, a cell, or a substance) possible. Examples of the labelling substance include enzymes (e.g., peroxidase, alkaline phosphatase, luciferase, and β-galactosidase), affinity substances (e.g., streptavidin, biotin, digoxigenin, and aptamers), fluorescent substances (e.g., fluorescein, fluorescein isothiocyanate, rhodamine, green-fluorescent proteins, and red-fluorescent proteins), luminescent substances (e.g., luciferin, aequorin, acridinium esters, tris(2,2'-bipyridyl) Duthenium, and luminol), and radioisotopes (e.g., those described above) or substances comprising them.

The affinity substance is a substance having an affinity to a target. Examples of the affinity substance include affinity proteins or peptides, such as antibodies, aptamers, lectins, and complementary strands for target nucleic acids. The affinity substance is preferably an affinity protein or an affinity peptide, and more preferably may be an antibody. The types of animals from which the antibodies used as functional substances are derived are the same as those described above.

The type of antibody used as a functional substance may be a polyclonal antibody or a monoclonal antibody. The antibody may be a divalent antibody (e.g., IgG, IgD, or IgE) or a tetravalent or higher antibody (e.g., IgA antibody or IgM antibody). The antibody is preferably a monoclonal antibody. Examples of the monoclonal antibody include chimeric antibodies, humanized antibodies, human antibodies, antibodies with a certain sugar chain added (e.g., an antibody modified so as to have a sugar chain-binding consensus sequence such as an N-type sugar chain-binding consensus sequence), bi-specific antibodies, Fc region proteins, Fc-fusion proteins, and disulfide bond-reduced antibodies. Examples of the isotype of the monoclonal antibody include IgG (e.g., IgG1, IgG2, IgG3, and IgG4), IgM, IgA, IgD, IgE, and IgY. Examples of the antibody used as a functional substance included a full-length antibody and a fragment (fragment antibody) thereof. The fragment antibody preferably maintains the binding properties for a desired antigen, and examples thereof include Fab, Fab', F(ab')₂, scFv, a VHH antibodies.

The antigenicity of the antibody used as the functional substance may be the same as or different form the antigenicity of the immunoglobulin unit in the antibody, the antibody derivative, and the conjugate of the present invention, and is preferably different. The origin of the antibody used as the functional substance may be the same as or different from the origin of the immunoglobulin unit, and is preferably different. Therefore, the antibody used as the functional substance may be a specific chimeric antibody, a specific humanized antibody, or a specific human antibody, or an antibody derived from it, as referred to in the specific examples of monoclonal antibodies described above. The antibody used as the functional substance may also be IgG1, IgG2, IgG3, or IgG4, or an antibody derived from it, as referred to in the specific examples of monoclonal antibodies described above.

The transporting substances are substances capable of transporting compounds. Preferred transporting substances include substances that can encapsulate compounds within a protein shell (e.g., multimer) (e.g., ferritin, viral particles, virus-like particles).

The stabilizer is a substance that makes stabilization of an antibody possible. Examples of the stabilizer include diols, glycerin, nonionic surfactants, anionic surfactants, natural surfactants, saccharides, and polyols.

The functional substance may also be a peptide, a protein, a nucleic acid, an organic compound, an inorganic compound, a sugar chain, a lipid, a high molecular polymer, a metal (e.g., gold), or a chelator. Examples of the peptide include a cell membrane permeable peptide, a blood-brain barrier permeable peptide, and a peptide medicament. Examples of the protein include enzymes, cytokines, fragment antibodies, lectins, interferons, serum albumin, antibodies, and ferritin. Examples of the nucleic acid include DNA, RNA, and artificial nucleic acids. Examples of the nucleic acid also include RNA interference inducible nucleic acids (e.g., siRNA), aptamers, and antisense nucleic acids. Examples of the organic compound include low molecular weight organic compounds such as proteolysis targeting chimeras, dyes, and photodegradable compounds. Examples of the inorganic compound include silica, talc, and alumina.

### Salt

In the present invention, examples of the term "salt" include salts with inorganic acids, salts with organic acids, salts with inorganic bases, salts with organic bases, and salts with amino acids. Examples of salts with inorganic acids include salts with hydrogen chloride, hydrogen bromide, phosphoric acid, sulfuric acid, and nitric acid. Examples of salts with organic acids include salts with formic acid, acetic acid, trifluoroacetic acid, lactic acid, tartaric acid, fumaric acid, oxalic acid, maleic acid, citric acid, succinic acid, malic acid, benzenesulfonic acid, and p-toluenesulfonic acid. Examples of salts with inorganic bases include salts with alkali metals (e.g., sodium and potassium), alkaline-earth metals (e.g., calcium and magnesium), other metals such as zinc and aluminum, and ammonium. Examples of salts with organic bases include salts with trimethylamine, triethylamine, propylenediamine, ethylenediamine, pyridine, ethanolamine, monoalkyl ethanolamine, dialkyl ethanolamine, diethanolamine, and triethanolamine. Examples of salts with amino acids include salts with basic amino acids (e.g., arginine, histidine, lysine, and ornithine) and acidic amino acids (e.g., aspartic acid and glutamic acid). The salt is preferably a salt with an inorganic acid (e.g., hydrogen chloride) or a salt with an organic acid (e.g., trifluoroacetic acid).

### 2. Affinity substances or salts thereof, and their related invention

### 2-1. Affinity substance or a salt thereof

The present invention provides an affinity substance or a salt thereof comprising first and second affinity moieties each having an affinity to the constant region in a heavy chain of an antibody.

The affinity substance used in the present invention comprises first and second affinity moieties each having an affinity to the constant region in a heavy chain of the constituent unit of an antibody. The constituent unit of an antibody is an immunoglobulin unit comprising two heavy chains and optionally two light chains. Therefore, the constituent unit of an antibody is an immunoglobulin unit comprising two heavy chains and two light chains, or an immunoglobulin unit comprising two heavy chains but not two light chains. Examples of the antibody comprising an immunoglobulin unit comprising two heavy chains and two light chains include divalent antibodies (e.g., IgG, IgD, and IgE), and tetravalent or higher antibodies (e.g., IgA antibodies and IgM antibodies), chimeric antibodies, humanized antibodies, human antibodies, bi-specific antibodies, and disulfide bond-reduced antibodies. Examples of the antibody comprising an immunoglobulin unit comprising two heavy chains but not two light chains include Fc region proteins, Fc-fusion proteins, and disulfide bond-reduced antibodies. This is because a Fc region comprises CH2 domains and CH3 domains as heavy chains, but not any light chains.

The first and second affinity moieties may be the same or different from each other. When the first and second affinity moieties are different from each other, the first and second affinity moieties may be affinity substances having affinities to different regions in the constant regions in the heavy chains of an antibody (e.g., an affinity substance wherein one of the first and second affinity moieties has an affinity to the CH2 domain, while the other has an affinity to the CH3 domain), or may be different affinity moieties having affinities to the same regions in the constant regions in the heavy chains of an antibody (e.g., an affinity substance wherein both the first and second affinity moieties have affinities to the CH2 domain or the CH3 domain), and is preferably different affinity moieties having affinities to the same regions in the constant regions in the heavy chains of an antibody. With respect to the first and second affinity moieties, in order to, for example, prevent association of first and second affinity moieties to the constant region in a single heavy chain, such a relationship is preferred that the first association site of the first affinity moiety for the constant region in a heavy chain and the second association site of the second affinity moiety for the constant region in a heavy chain sterically interfere with each other, and as a result, one association can reduce the other association. Such a relationship, in the constituting unit of an antibody (the immunoglobulin unit comprising two heavy chains and optionally two light chains), can prevent two compound molecules each comprising an affinity substance and a reactive group for an antibody (a first molecule and a second molecule) from competing with each other for association with a single antibody constituting unit (the immunoglobulin unit), so that specific modification of the constant region in only one heavy chain can be facilitated. Examples of the first and second affinity moieties include polymeric substances comprising a certain structural unit [e.g., peptides (including oligopeptides, polypeptides, proteins), nucleic acids (including oligonucleic acids and polysaccharides), and sugars (including oligosaccharides and polysaccharides)], and nonpolymeric substances (e.g., small compounds). As substances that can be used as the first and second affinity moieties each having an affinity to the constant region in a heavy chain of an antibody, many substances such as peptides, nucleic acids, sugars, and small compounds have been reported (see, for example, publications such as WO2007/004748, WO2008/054030, WO2013/027796, WO2016/186206, WO2018/199337, WO2019/240287, WO2019/240288, and WO2020/090979; Nomura Y et al., Nucleic Acids Res., 2010 Nov; 38(21): 7822-9*;* Miyakawa S et al., RNA., 2008 Jun; 14(6): 1154-63*,* and scientific articles described later). The first and second affinity moieties are preferably the same.

The substances that can be used as the first and second affinity moieties each having an affinity to the constant region in a heavy chain of an antibody can be obtained by any method. For example, such substances can be obtained by screening (e.g., high-throughput screening, phage display, SELEX, mRNA display, ribosome display, cDNA display, and yeast display) a library of any substances (e.g., small compound library, peptide library, aptamer library, sugar library, phage library, mRNA library, and cDNA library) for substances having an affinity to the constant region in a heavy chain of an antibody. In the case of screening for substances having an affinity to a specific region (e.g., the CH2 domain or the CH3 domain) in the Fc region or the constant region of an antibody, partial peptides present in specific regions (e.g., CH1, CH2, and CH3) in the Fc region of various antibodies (e.g., IgG, IgA, IgM, IgD, and IgE) can be used to efficiently obtain substances that can selectively bind to such regions.

The first and second affinity moieties can each have an affinity to the constant region in a heavy chain of an antibody. For example, the first and second affinity moieties may each have an affinity to the Fc region in a heavy chain of an antibody. Alternatively, the first and second affinity moieties can each have an affinity to the CH1 domain, the CH2 domain, or the CH3 domain, or a region spanning them (e.g., a flanking region between the CH1 domain and the CH2 domain, or a flanking region between the CH2 domain and the CH3 domain) as the constant region of a heavy chain. The first and second affinity moieties may have affinities to the same or different CHX domain(s) (X is 1, 2, or 3), and preferably have affinities to the same domain. As used herein, there is no particular limitation in the phrase "have an affinity to the CHX domain" as long as the first and second affinity moieties each have an affinity to at least a partial region in the CHX domain, and the first and second affinity moieties may each have an affinity to a partial region in the CHX domain or an affinity to a region (e.g., flanking region) spanning the CHX domain and another CHX domain. Therefore, the first and second affinity moieties each having an affinity to the CH2 domain may each have an affinity to only a partial region in the CH2 domain, or an affinity to a region spanning the CH2 domain and the CH1 or CH3 domain (e.g., the flanking region between the CH1 domain and the CH2 domain, or the flanking region between the CH2 domain and the CH3 domain). The first and second affinity moieties each having an affinity to the CH2 domain may preferably have an affinity to only a partial region in the CH2 domain, or an affinity to a region spanning the CH2 domain and the CH3 domain (e.g., the flanking region between the CH2 domain and the CH3 domain), and more preferably may each have an affinity to only a partial region in the CH2 domain.

In the affinity substance or a salt thereof of the present invention, the first and second affinity moieties may be directly linked, or a linker may be inserted between the first affinity substance and the second affinity moiety. The first and second affinity moieties may be directly linked in the cases where the distance between the first and second affinity moieties does not need to be adjusted due to such reasons that the binding sites of the first and second affinity moieties for the constant region in a heavy chain of an antibody are in proximity to each other, or the sizes of the first affinity substance and the second affinity moiety are sufficiently large. Conversely, a linker can be inserted between the first affinity substance and the second affinity moiety in the cases where the distance between the first and second affinity moieties need to be adjusted due to such reasons that the binding sites of the first and second affinity moieties for the constant region in a heavy chain of an antibody are not in proximity to each other, or the sizes of the first affinity substance and the second affinity moiety are not sufficiently large. A divalent group can be used as the linker. The divalent group may be substituted or unsubstituted. Examples of the divalent group include those described above. Examples of the substituent when the divalent group is substituted include those described above. As the linker, for example, substances such as peptides, nucleic acids, sugars, other polymeric substances (e.g., polyethyleneglycol), and divalent hydrocarbon groups (e.g., alkyl chains) may be used. A person skilled in the art can appropriately determine the presence of a linker and the type of the linker according to the types of the first and second affinity moieties to be used. Preferably, a linker may be inserted in order to optimize the bindings of the first and second affinity moieties to the constant region in a heavy chain of an antibody.

The constant region in a heavy chain of an antibody to which the first and second affinity moieties each have an affinity may be derived from an animal (e.g., a mammal or a bird) as described above. The constant region in a heavy chain of an antibody may preferably be a mammal constant region, more preferably a primate constant region or a rodent constant region, and even more preferably a human constant region.

The constant region in a heavy chain of an antibody to which the first and second affinity moieties each have an affinity may be the constant region of a divalent antibody (e.g., IgG, IgD, or IgE), or a tetravalent or higher antibody (e.g., IgA antibody or IgM antibody). Such a constant region is preferably the constant region of a divalent antibody (e.g., IgG, IgD, or IgE), and more preferably the constant region of IgG.

Preferably, the first and second affinity moieties may be affinity peptides each having an affinity to the constant region in a heavy chain of an antibody. Many peptides have been reported as such affinity peptides. For example, examples of such affinity peptides include:
(1) various IgG-binding peptides having an affinity to a specific region (the CH2 domain) of human IgG in general (i.e., human IgG1, IgG2, IgG3, and IgG4. The same shall apply hereinafter) (see, for example, WO2008/054030, WO2013/027796, and WO2016/186206);
(2) Protein A Mimetic (PAM) peptides having an affinity to a specific region (the CH2 domain) of human IgG in general (see, for example, Fassina G et al., JOURNAL OF MOLECULAR RECOGNITION, 1996, VOL. 6, 564-569*);*
(3) EPIHRSTLTALL (SEQ ID NO: 27) having an affinity to a specific region (the CH2 domain) of human IgG in general (see, for example, Ehrlich G. K et al., J. Biochem. Biophys. Methods, 2001, VOL.49, 443-454*);*
(4) (NH₂-Cys1-X1-X2-X3-X4)₂-Lys-Gly-OH having an affinity to a specific region (the Fc region) of human IgG in general (see, for example, Ruvo M et al., ChemBioChem, 2005, VOL.6, 1242-1253*);*
(5) FARLVSSIRY (SEQ ID NO: 28), FGRLVSSIRY (SEQ ID NO: 29), and TWKTSRISIF (SEQ ID NO: 30) having an affinity to a specific region (the Fc region) of human IgG in general (see, for example, Krook M et al., Journal of Immunological Methods, 1998, VOL.221, 151-157*);*
(6) QSYP (SEQ ID NO: 31) having an affinity to a specific region of human IgG in general (see, for example, Jacobs J. M. et al., Bio. Techniques, 2003, VOL.34, 132-141*);*
(7) HWRGWV (SEQ ID NO: 32), HYFKFD (SEQ ID NO: 33), and HFRRHL (SEQ ID NO: 34) having an affinity to a specific region (the Fc region) of human IgG in general (see, for example, Carbonell R. G. et al., Journal of Chromatography A, 2009, VOL.1216, 910-918*);*
(8) DAAG (SEQ ID NO: 35) having an affinity to a specific region (the Fc region) of human IgG in general (see, for example, Lund L. N. et al., Journal of Chromatography A, 2012, VOL.1225, 158-167*);*
(9) Fc-I, Fc-II, and Fc-III having an affinity to a specific region (the Fc region) of human IgG in general (see, for example, Warren L. Delano et al., Science, 2000, VOL.287, 1279-1283*;* WO2001/045746); and
(10) NARKFYKG (SEQ ID NO: 36) and NKFRGKYK (SEQ ID NO: 37) having an affinity to a specific region (the Fc region) of human IgG in general (see, for example, Biochemical Engineering Journal, 2013, VOL. 79, 33-40*);*
(11) protein A, protein G, protein L, and protein Z, or fragments thereof, having an affinity to a specific region (the Fc region) of human IgG in general (for example, Moks T et al., Eur J Biochem. 1986 May 2; 156(3): 637-43*;* Sjobring UJ et al., Biol Chem. 1991 Jan 5; 266(1): 399-405*;* Graille M el al., Structure. 2001 Aug; 9(8): 679-87*;* Nilsson B et al., Protein Eng. 1987 Feb-Mar; 1(2): 107-13*);*
(12) various IgG-binding peptides having an affinity to a specific region (the Fc region or the CH2 domain) of human IgG in general (see, for example, WO2018/199337, WO2019/240287, WO2019/240288, and WO2020/090979).

The affinity peptide can be obtained by a screening method as described above (e.g., the above-described method using a library, or the above-described display method).

As the amino acid residues constituting the affinity peptide, the common 20 natural amino acids constituting proteins, or unnatural amino acid residues can be used. Examples of the common 20 natural amino acids constituting proteins include L-alanine (A), L-asparagine (N), L-cysteine (C), L-glutamine (Q), L-isoleucine (I), L-leucine (L), L-methionine (M), L-phenylalanine (F), L-proline (P), L-serine (S), L-threonine (T), L-tryptophan (W), L-tyrosine (Y), L-valine (V), L-aspartic acid (D), L-glutamic acid (E), L-arginine (R), L-histidine (H), and L-lysine (K), and glycine (G) (hereinafter, L is omitted).

In a specific embodiment, one of the first and second affinity peptides is an affinity peptide having one lysine residue, and the other is an affinity peptide having no lysine residue.

Many peptides have been reported as the affinity peptide having an affinity to the constant region in a heavy chain of an antibody and having a single lysine residue (see, for example, WO2016/186206, WO2018/199337, WO2019/240287, WO2019/240288, and WO2020/090979). Therefore, in the present invention, such peptides can be used as one of the first and second affinity peptides.

More specifically, the following may be used as the affinity peptide having an affinity to the constant region in a heavy chain of an antibody and having a single lysine residue:
(1) the affinity peptides comprising the amino acid sequences of SEQ ID NOs: 39 to 72 in WO2018/199337,
(2) the affinity peptides comprising the amino acid sequences of SEQ ID NOs: 5, 6, 37 to 100 in WO2019/240288;
(3) the affinity peptides comprising the amino acid sequences of SEQ ID NOs: 5, 8 to 57, 68 to 92 in WO2019/240287;
(4) the affinity peptides having QET at the N-terminus (SEQ ID NOs: 7 to 10, 22 to 25, 52, and 53 in WO2020/090979); and
(5) affinity peptides having one lysine residue, of the affinity peptides (1) to (12) listed as examples of the affinity peptide.

In a specific embodiment, examples of the affinity peptide having an affinity to the constant region in a heavy chain of an antibody and having a single lysine residue include the following (1) to (4):
(1) an affinity peptide comprising the amino acid sequence (Fc3K) of RGNCAYHKGQIIWCTYH (SEQ ID NO: 38);
(2) an affinity peptide comprising an amino acid sequence in which one or two amino acid residues other than lysine or cysteine residue in the amino acid sequence of RGNCAYHKGQIIWCTYH (SEQ ID NO: 38) are replaced by other amino acid residues other than lysine or cysteine residue, and having an affinity to the constant region in a heavy chain of the antibody;
(3) an affinity peptide comprising the amino acid sequence (Z34CK) of FNKQCQRRFYEALHDPNLNEEQRNARIRSIREEC (SEQ ID NO: 39); and
(4) an affinity peptide comprising an amino acid sequence in which one or two amino acid residues other than lysine or cysteine residue in the amino acid sequence of FNKQCQRRFYEALHDPNLNEEQRNARIRSIREEC (SEQ ID NO: 39) are replaced by other amino acid residues other than lysine or cysteine residue, and having an affinity to the constant region in a heavy chain of the antibody.

Here, the two cysteine residues contained in the amino acid sequence may be cross-linked by a disulfide bond.

Many peptides have been reported as the affinity peptide having an affinity to the constant region in a heavy chain of an antibody and having no lysine residue. The lysine residue in the affinity peptide having an affinity to the constant region in a heavy chain of an antibody and having one lysine residue is introduced, in many cases, not to maintain the affinity to the constant region in a heavy chain of an antibody, but to allow it to covalently bind to another moiety (e.g., moiety compound comprising a reactive group) to derivatize the affinity substance (see, for example, WO2016/186206, WO2018/199337, WO2019/240287, WO2019/240288, and WO2020/090979). Thus, even if such a lysine residue is replaced with another amino acid residue, the affinity to the constant region in a heavy chain of an antibody can be maintained. Therefore, as the affinity peptide having an affinity to the constant region in a heavy chain of an antibody and having no lysine residue, an affinity peptide having an affinity to the constant region in a heavy chain of an antibody and having a single lysine residue, wherein the lysine residue is replaced with another amino acid residue (preferably a common natural amino acid residue constituting proteins, other than lysine residue or cysteine residue) and wherein the affinity peptide has an affinity to the constant region in a heavy chain of an antibody can be used.

More specifically, as the affinity peptide having an affinity to the constant region in a heavy chain of an antibody and having no lysine residue, the following may be used:
(1) SEQ ID NOs: 20 to 38, and 73 to 75 (when Xaa1 is other than lysine residue), and SEQ ID NO: 92 in WO2018/199337;
(2) SEQ ID NOs: 7, 11 to 14, and 108 in WO2019/240288;
(3) affinity peptides in which the lysine residue has been replaced with another amino acid residue (preferably another amino acid residue other than cysteine residue), of the above-described (1) to (4) listed as examples of the affinity peptide having an affinity to the constant region in a heavy chain of an antibody and having a single lysine residue; and
(4) affinity peptides having no lysine residue, of the affinity peptides (1) to (12) listed as examples of the affinity peptide.

In a specific embodiment, examples of the affinity peptide having no lysine residue include the following (5) to (10):
(5) an affinity peptide comprising the amino acid sequence (Z34CM) of FNMQCQRRFYEALHDPNLNEEQRNARIRSIREEC (SEQ ID NO: 40);
(6) an affinity peptide comprising an amino acid sequence in which one or two amino acid residues other than cysteine residue in the amino acid sequence of FNMQCQRRFYEALHDPNLNEEQRNARIRSIREEC (SEQ ID NO: 40) are replaced by other amino acid residues other than lysine or cysteine residue, and having an affinity to the constant region in a heavy chain of the antibody;
(7) an affinity peptide comprising the amino acid sequence (ProAR) of FNREQQNAFYEILHLPNLNEEQRNGFIQSLRDDPSQSANLLAEA (SEQ ID NO: 41);
(8) an affinity peptide comprising an amino acid sequence in which one or two amino acid residues other than cysteine residue in the amino acid sequence of FNREQQNAFYEILHLPNLNEEQRNGFIQSLRDDPSQSANLLAEA (SEQ ID NO: 41) are replaced by other amino acid residues other than lysine or cysteine residue, and having an affinity to the constant region in a heavy chain of the antibody;
(9) an affinity peptide comprising the amino acid sequence of RGNCAYHRGQIIWCTYH (SEQ ID NO: 78); and
(10) an affinity peptide comprising an amino acid sequence in which one or two amino acid residues other than cysteine residue in the amino acid sequence of RGNCAYHRGQIIWCTYH (SEQ ID NO: 78) are replaced by other amino acid residues other than lysine or cysteine residue, and having an affinity to the constant region in a heavy chain of the antibody.

Here, the two cysteine residues contained in the amino acid sequence may be cross-linked by a disulfide bond.

The amino residue replacement may be conservative replacement. The term "conservative replacement" refers to replacement of a given amino acid residue with an amino acid residue having a similar side chain. Families of the amino acid residues having a similar side chain is well known in the art. Examples of such families include amino acids having a basic side chain (e.g., lysine, arginine, and histidine), amino acids having an acidic side chain (e.g., aspartic acid, and glutamic acid), amino acids having an uncharged polar side chain (e.g., asparagine, glutamine, serine, threonine, tyrosine, and cysteine), amino acids having a nonpolar side chain (e.g., glycine, alanine, valine, leucine, isoleucine, proline, phenylalanine, methionine, and tryptophan), amino acids having a β-branched side chain (e.g., threonine, valine, and isoleucine), amino acids having an aromatic side chain (e.g., tyrosine, phenylalanine, tryptophan, and histidine), amino acids having a hydroxyl group-containing (e.g., alcoholic or phenolic) side chain (e.g., serine, threonine, tyrosine), and amino acids containing a sulfur-containing side chain (e.g., cysteine and methionine). The amino acids having an uncharged polar side chain and the amino acids having a nonpolar side chain may be collectively referred to as neutral amino acids. Preferably, the conservative replacement of amino acids may be replacement between aspartic acid and glutamic acid, replacement among arginine and lysine and histidine, replacement between tryptophan and phenylalanine, replacement between phenylalanine and valine, replacement among leucine and isoleucine and alanine, and replacement between glycine and alanine.

When the first and second affinity moieties are affinity peptides in the affinity substance or a salt thereof of the present invention, the first and second affinity peptides may be directly linked, or a linker may be inserted between the first affinity peptide and the second affinity peptide. From the viewpoint of optimizing the bindings of the first and second affinity peptides to the constant region in a heavy chain of an antibody, a linker is preferably inserted. When the first and second affinity moieties are affinity peptides, a preferred linker is a peptide linker.

The number of the amino acid residues constituting the peptide linker can be appropriately set according to conditions such as the types of the amino acid residues (e.g., α-amino acids, β-amino acids, and γ-amino acids, and preferably α-amino acid). For example, the peptide linker may be composed of 20 or more amino acid residues. The peptide linker may be composed of 22 or more, 24 or more, 26 or more, 28 or more, 30 or more, 32 or more, 34 or more, 36 or more, 38 or more, or 40 or more amino acid residues. The peptide linker may also be composed of less than 50, less than 49, less than 48, less than 47, less than 46, or less than 45 amino acid residues.

As the amino acid residues constituting the peptide linker, the above-described natural amino acid or unnatural amino acid residues can be used. Preferably, the amino acid residue constituting the peptide linker may only include the above-described natural amino acid residues. Examples of suitable amino acid residues for the peptide linker include, but not limited to, alanine, proline, serine, and glycine. As the peptide linker, those disclosed in WO2021/112249 and WO2011/144756 can also be used.

Preferably, the affinity substance or a salt thereof of the present invention may be an affinity substance or a salt thereof in which the first and second affinity moieties are affinity peptides, and a linker is contained in the first affinity peptide and the second affinity peptide. Such an affinity substance can be represented by the following formula (A):

AP1-L_{A}-AP2 (A)

wherein
AP1 indicates a first affinity peptide having an affinity to the constant region in a heavy chain of the antibody;
AP2 indicates a second affinity peptide having an affinity to the constant region in a heavy chain of the antibody; and
L_{A} indicates a linker.

In formula (A) and other formulae presented in relation to the present invention, -(hyphen) indicates that two units present on both sides thereof are covalently bonded to each other. Therefore, in the formula (A), AP1 is covalently bound to L, L is covalently bound to both AP1 and AP2, and AP2 is covalently bound to L.

The definitions, examples, and preferred examples of the first and second affinity peptides represented by AP1 and AP2, respectively, are as described above.

The linker represented by L_{A} is a divalent group. The divalent group may be substituted or unsubstituted. Examples of the divalent group include those described above. Examples of the substituent when the divalent group is substituted include those described above. Preferably, as the linker, for example, substances such as peptides, nucleic acids, sugars, other polymeric substances (e.g., polyethyleneglycol), and divalent hydrocarbon groups (e.g., alkyl chains) may be used.

More preferably, the affinity substance or a salt thereof of the present invention may be an affinity polypeptide or a salt thereof in which the first and second affinity moieties are affinity peptides, and a peptide linker is contained in the first affinity peptide and the second affinity peptide. Such an affinity polypeptide can be represented by the following formula (A'):

AP1-PL_{A}-AP2 (A')

wherein
AP1 indicates a first affinity peptide that has an affinity to the constant region in a heavy chain of the antibody and is present on the N-terminal side of the affinity polypeptide;
AP2 indicates a second affinity peptide that has an affinity to the constant region in a heavy chain of the antibody and is present on the C-terminal side of the affinity polypeptide; and
PL_{A} indicates a peptide linker.

The affinity substance or a salt thereof such as an affinity polypeptide can comprise natural amino acid residues or unnatural amino acid residues as described above as amino acid residues constituting the affinity substance or a salt thereof. When the affinity substance only comprises natural amino acid residues as described above, the affinity substance can be produced, for example, via a polypeptide expression system using host cells, a cell-free synthesis system, or an organic synthesis system (e.g., solid phase synthesis). When the affinity substance comprises unnatural amino acid residues as described above, the affinity substance can be produced, for example, via an organic synthesis system (e.g., solid phase synthesis). Preferably, the affinity substance may only comprise natural amino acid residues in order to allow for mass production of the affinity substance via a polypeptide expression system using host cells, or a cell-free synthesis system.

When the affinity substance of the present invention is an affinity substance or a salt thereof such as an affinity polypeptide, the amino group and carboxy group present at the ends of the affinity substance can be appropriately protected. Examples of a protecting group for the N-terminal amino group include an alkylcarbonyl group (an acyl group) (e.g., an acetyl group, a propoxy group, and a butoxycarbonyl group such as a tert-butoxycarbonyl group), an alkyloxycarbonyl group (e.g., a fluorenylmethoxycarbonyl group), an aryloxycarbonyl group, and an arylalkyl(aralkyl)oxycarbonyl group (e.g., a benzyloxycarbonyl group). Preferably, the N-terminal amino group may be alkylated, formylated, or acetylated. Examples of a protecting group for the C-terminal carboxy group include a group capable of forming an ester or an amide. Examples of the group capable of forming an ester or an amide include an alkyloxy group (e.g., methyloxy, ethyloxy, propyloxy, butyloxy, pentyloxy, and hexyloxy), an aryloxy group (e.g., phenyloxy and naphthyloxy), an aralkyloxy group (e.g., benzyloxy), and an amino group.

When the N-terminal amino acid of the affinity substance is glutamic acid (E) or glutamine (Q), its side chain can be used to protect the N-terminus. When the N-terminal amino acid is glutamic acid, the protected N-terminal glutamic acid can have a cyclic structure of pyroglutamic acid. When the N-terminal amino acid is glutamine, the N-terminal amino group (NH₂) can react with the amide group present at its side chain (pyroglutamylation), and as a result, the protected N-terminal glutamine can have a pyroglutamic acid-type cyclic structure. Therefore, the N-terminal amino acid may preferably be glutamic acid or glutamine.

When the affinity substance is an affinity polypeptide, the affinity polypeptide may further comprise a tripeptide consisting of Gln-Glu-Thr (QET) at the N-terminus. In this case, a polypeptide expression system using host cells enables protection of the N-terminal amino group via pyroglutamylation of Q, and consequently mass and simple secretory production of the affinity polypeptide (see Examples, WO2013/062029, WO2020/090979). In this case, a signal peptide such as a signal peptide (CspBss) composed of the amino acid sequence of MFNNRIRTAALAGAIAISTAASGVAIPAFA (SEQ ID NO: 42) can be attached at the N-terminus side of QET (see Examples, WO2013/062029, and WO2020/090979).

The affinity substance or a salt thereof of the present invention can be used, for example, as a synthetic intermediate for the compound or a salt thereof of the present invention comprising an affinity substance and a reactive group for an antibody. Thus, in order to easily achieve uniform synthesis of the compound or a salt thereof of the present invention comprising a reactive group for an antibody in addition to an affinity substance, the affinity substance or a salt thereof of the present invention may be derivatized to contain only one specific reactive group that allows for a specific reaction with a moiety compound containing the reactive group for an antibody. When the affinity substance comprises only one specific reactive group, both the affinity substance and the reactive group for an antibody can be specifically reacted via the specific reactive group in the affinity substance, so that the compound or a salt thereof of the present invention comprising the affinity substance and the reactive group for an antibody can be easily produced as a uniform compound.

Examples of the specific reactive group as described above include:
(1) amino groups (NH₂, NHR₃, NR₃R₄, wherein R₃ and R₄ each independently are a monovalent group as described above, preferably a monovalent hydrocarbon group, more preferably an alkyl group, and even more preferably a C1-6 alkyl group);
(2) residues capable of reacting with an amino group, such as activated ester residues (e.g., N-hydroxysuccinimide residues), vinyl sulfone residues, sulfonyl chloride residues, isocyanate residues, isothiocyanate residues, aldehyde residues, 1,4,7,10-tetraazacyclododecane-1,4,7,10-tetraacetate residues, 2-imino-2-methoxyethyl residues, and diazonium terephthalic acid residues;
(3) a carboxyl group (COOH);
(4) residues capable of reacting with a carboxyl group, such as the amino groups as described above;
(5) hydroxyl groups (OH) (including alcoholic and phenolic hydroxyl groups); and
(6) residues capable of reacting with a hydroxyl group, such as diazonium residues, diazodicarboxylate residues, and 2,3-dihydro-1H-pyrazin-6-on residues.

The specific reactive group may preferably be (1) to (4). More preferably, the specific reactive group may be (1) or (2), or may be (3) or (4). Alternatively, more preferably, the specific reactive group may be (1) or (3). The specific reactive group may even more preferably be (1), and particularly preferably an amino group (NH₂).

The affinity substance or a salt thereof may comprise only one amino acid residue containing a specific reactive group. **In** such a case, the affinity polypeptide may comprise (a) only one amino acid residue having a specific reactive group (e.g., an amino group, a carboxyl group, or a hydroxyl group) in its side chain (e.g., a lysine residue, an aspartic acid residue, a glutamic acid residue, a tyrosine residue, a threonine residue, or a serine residue). When the affinity polypeptide comprises only one lysine residue having an amino group in its side chain, the N-terminus of the affinity polypeptide is preferably protected (the C-terminus may also be protected). When the affinity polypeptide comprises only one amino acid residue having a carboxyl group in its side chain (e.g., an aspartic acid residue or a glutamic acid residue), the C-terminus of the affinity polypeptide is preferably protected (the N-terminus may also be protected).

Alternatively, the affinity polypeptide may be a polypeptide comprising only one amino group as a specific reactive group by not containing any amino acid residue having an amino group in its side chain (e.g., a lysine residue) and having an amino group at the N-terminus. The affinity polypeptide may also be a polypeptide comprising only one carboxyl group as a specific reactive group by not containing any amino acid residue having a carboxyl group in its side chain (e.g., an aspartic acid residue or a glutamic acid residue) and having a carboxyl group at the C-terminus.

Preferably, the affinity polypeptide comprising only one specific reactive group may be a polypeptide comprising an amino acid residue having an amino group in its side chain. When the affinity polypeptide is produced via an organic synthesis system (e.g., a solid phase synthesis), not also a lysine residue that is a natural amino acid constituting proteins but also other amino acid residues having an amino group in the side chain (e.g., ornithine) can be used. More preferably, the affinity polypeptide comprising only one specific reactive group may be a polypeptide comprising only one lysine residue having an amino group (NH₂) in its side chain in order to allow for easy production not only via an organic synthesis system but also via a polypeptide expression system using host cells and a cell-free synthesis system. In this case, the lysine residue may be contained in either the first affinity peptide, the second affinity peptide, or the peptide linker. The lysine residue, when contained in the peptide linker, may be contained at a position close to the first affinity peptide or the second affinity peptide (e.g., a position separated by 10 or less, 5 or less, or 1 to 3 amino acid residues from the first affinity peptide or the second affinity peptide). Preferably, the lysine residue is contained in either the first affinity peptide or the second affinity peptide.

Preferably, the first and second affinity peptides represented by AP1 and AP2, respectively, in the affinity substance or a salt thereof represented by the formula (A), and the linker represented by L_{A} may be those further having the following characteristic (1) or (2), preferably having the characteristic (1).

(1) Affinity substance or a salt thereof comprising only one amino group as the specific reactive group:
   (1-1) an affinity substance or a salt thereof, wherein (i) the first affinity peptide is an affinity peptide comprising only one amino acid residue having an amino group in its side chain (preferably, a lysine residue), and having a protected N-terminus; (ii) the second affinity peptide is an affinity peptide comprising no amino acid residue having an amino group in its side chain; and the linker is a linker comprising no amino group;
   (1-2) an affinity substance or a salt thereof, wherein (i) the first affinity peptide is an affinity peptide comprising no amino acid residue having an amino group in its side chain, and having an unprotected N-terminus; (ii) the second affinity peptide is an affinity peptide comprising no amino acid residue having an amino group in its side chain; and the linker is a linker comprising no amino group, affinity substance or a salt thereof;
   (1-3) an affinity substance or a salt thereof, wherein (i) the first affinity peptide is an affinity peptide comprising no amino acid residue having an amino group in its side chain, and having a protected N-terminus; (ii) the second affinity peptide is an affinity peptide comprising only one amino acid residue having an amino group in its side chain (preferably, a lysine residue); and the linker is a linker comprising no amino group; (1-4) an affinity substance or a salt thereof, wherein (i) the first affinity peptide is an affinity peptide comprising no amino acid residue having an amino group in its side chain, and having a protected N-terminus; (ii) the second affinity peptide is an affinity peptide comprising no amino acid residue having an amino group in its side chain; and the linker is a linker comprising only one amino group.
   The affinity substance or a salt thereof comprising only one amino group as the specific reactive group may be an affinity substance or a salt thereof comprising no group that can react with an amino group (e.g., carboxy group) in order to reduce undesired reactions (e.g., intramolecular reactions or intermolecular reactions). Therefore, in (1-1) to (1-4) described above, the first affinity peptide may be one comprising no amino acid residue having a carboxy group in its side chain; the second affinity peptide may be one comprising no amino acid residue having a carboxy group in its side chain and/or having a protected C-terminus; and the linker may be a linker comprising no carboxy group.
(2) Affinity substance or a salt thereof comprising only one carboxy group as the specific reactive group:
   (2-1) an affinity substance or a salt thereof, wherein (i)the first affinity peptide is an affinity peptide comprising only one amino acid residue having a carboxy group in its side chain (preferably, an acidic amino acid residue such as aspartic acid or glutamic acid); (ii) the second affinity peptide is an affinity peptide comprising no amino acid residue having a carboxy group in its side chain; and the linker is a linker comprising no carboxy group;
   (2-2) an affinity substance or a salt thereof, wherein (i) the first affinity peptide is an affinity peptide comprising no amino acid residue having a carboxy group in its side chain; (ii) the second affinity peptide is an affinity peptide comprising only one amino acid residue having a carboxy group in its side chain (preferably, an acidic amino acid residue such as aspartic acid or glutamic acid), and having a protected C-terminus; and the linker is a linker comprising no carboxy group;
   (2-3) (i) the first affinity peptide is an affinity peptide comprising no amino acid residue having a carboxy group in its side chain; (ii) the second affinity peptide is an affinity peptide comprising no amino acid residue having a carboxy group in its side chain, and having an unprotected C-terminus; and the linker is a linker comprising no carboxy group, affinity substance or a salt thereof;
   (2-4) an affinity substance or a salt thereof, wherein (i) is an affinity peptide comprising no amino acid residue having a carboxy group in its side chain; (ii)the second affinity peptide is an affinity peptide comprising no amino acid residue having a carboxy group in its side chain, and having a protected C-terminus; and the linker is a linker comprising only one carboxy group.

The affinity substance or a salt thereof comprising only one carboxy group as the specific reactive group may be an affinity substance or a salt thereof comprising no group that can react with a carboxy group (e.g., amino group) in order to reduce undesired reactions (e.g., intramolecular reactions or intermolecular reactions). Therefore, in (2-1) to (2-4) described above, the first affinity peptide may be one comprising no amino acid residue having an amino group in its side chain, and having a protected N-terminus; the second affinity peptide may be one comprising no amino acid residue having an amino group in its side chain; and the linker may be a linker comprising no amino group.

Preferably, the first and second affinity peptides represented by AP1 and AP2, respectively, in the affinity substance or a salt thereof represented by the formula (A'), and the peptide linker represented by PL_{A} may be those further having the following characteristic (1') or (2'), preferably having the characteristic (1').

(1') Affinity substance or a salt thereof comprising only one amino group as the specific reactive group:
   (1-1') an affinity substance or a salt thereof, wherein (i) the first affinity peptide is an affinity peptide comprising only one amino acid residue having an amino group in its side chain (preferably, a lysine residue), and having a protected N-terminus; (ii) the second affinity peptide is an affinity peptide comprising no amino acid residue having an amino group in its side chain; and the peptide linker is a peptide linker comprising no amino acid residue comprising an amino group in its side chain;
   (1-2') an affinity substance or a salt thereof, wherein (i) the first affinity peptide is an affinity peptide comprising no amino acid residue having an amino group in its side chain, and having an unprotected N-terminus; (ii) the second affinity peptide is an affinity peptide comprising no amino acid residue having an amino group in its side chain; and the peptide linker is a peptide linker comprising no amino acid residue comprising an amino group in its side chain;
   (1-3') an affinity substance or a salt thereof, wherein (i) the first affinity peptide is an affinity peptide comprising no amino acid residue having an amino group in its side chain, and having a protected N-terminus; (ii) the second affinity peptide is an affinity peptide comprising only one amino acid residue having an amino group in its side chain (preferably, a lysine residue); and the peptide linker is a peptide linker comprising no amino acid residue comprising an amino group in its side chain;
   (1-4') an affinity substance or a salt thereof, wherein (i) the first affinity peptide is an affinity peptide comprising no amino acid residue having an amino group in its side chain, and having a protected N-terminus; (ii) the second affinity peptide is an affinity peptide comprising no amino acid residue having an amino group in its side chain; and the peptide linker is a peptide linker comprising only one amino acid residue comprising an amino group in its side chain.
   The affinity substance or a salt thereof comprising only one amino group as the specific reactive group may be an affinity substance or a salt thereof comprising no group that can react with an amino group (e.g., carboxy group) in order to reduce undesired reactions (e.g., intramolecular reactions or intermolecular reactions). Therefore, in (1-1) to (1-4) described above, the first affinity peptide may be one comprising no amino acid residue having a carboxy group in its side chain; the second affinity peptide may be one comprising no amino acid residue having a carboxy group in its side chain and/or having a protected C-terminus; and the peptide linker may be a peptide linker comprising no amino acid residue comprising a carboxy group in its side chain.
(2') Affinity substance or a salt thereof comprising only one carboxy group as the specific reactive group:
   (2-1') an affinity substance or a salt thereof, wherein (i) the first affinity peptide is an affinity peptide comprising only one amino acid residue having a carboxy group in its side chain (preferably, an acidic amino acid residue such as aspartic acid or glutamic acid); (ii) the second affinity peptide is an affinity peptide comprising no amino acid residue having a carboxy group in its side chain; and the peptide linker is a peptide linker comprising no amino acid residue comprising a carboxy group in its side chain; (2-2') an affinity substance or a salt thereof, wherein (i) the first affinity peptide is an affinity peptide comprising no amino acid residue having a carboxy group in its side chain; (ii) the second affinity peptide is an affinity peptide comprising only one amino acid residue having a carboxy group in its side chain (preferably, an acidic amino acid residue such as aspartic acid or glutamic acid), and having a protected C-terminus; and the peptide linker is a peptide linker comprising no amino acid residue comprising a carboxy group in its side chain;
   (2-3') an affinity substance or a salt thereof, wherein (i) the first affinity peptide is an affinity peptide comprising no amino acid residue having a carboxy group in its side chain; (ii) the second affinity peptide is an affinity peptide comprising no amino acid residue having a carboxy group in its side chain, and having an unprotected C-terminus; and the peptide linker is a peptide linker comprising no amino acid residue comprising a carboxy group in its side chain;
   (2-4') an affinity substance or a salt thereof, wherein (i) the first affinity peptide is an affinity peptide comprising no amino acid residue having a carboxy group in its side chain; (ii)the second affinity peptide is an affinity peptide comprising no amino acid residue having a carboxy group in its side chain, and having a protected C-terminus; and the peptide linker is a linker comprising only one amino acid residue having a carboxy group in its side chain.

The affinity substance or a salt thereof comprising only one carboxy group as the specific reactive group may be an affinity substance or a salt thereof comprising no group that can react with a carboxy group (e.g., amino group) in order to reduce undesired reactions (e.g., intramolecular reactions or intermolecular reactions). Therefore, in (2-1') to (2-4') described above, the first affinity peptide may be one comprising no amino acid residue having an amino group in its side chain, and having a protected N-terminus; the second affinity peptide may be one comprising no amino acid residue having an amino group in its side chain; and the peptide linker may be a linker comprising no amino acid residue comprising an amino group in its side chain.

### 2-2. Related Invention of Affinity Substance or Salt Thereof

When the affinity substance or a salt thereof of the present invention is an affinity polypeptide comprising first and second affinity peptides each having an affinity to the constant region in a heavy chain of an antibody, such an affinity polypeptide can be prepared by using a host cell comprising an expression unit comprising polynucleotide encoding the affinity polypeptide and a promoter operably linked thereto, or using a cell-free system. The present invention also provides such a polynucleotide and such a host cell, as well as an expression vector that can be used to prepare such a host cell.

The polynucleotide of the present invention is a polynucleotide encoding the affinity polypeptide of the present invention. The polynucleotide of the present invention may be DNA or RNA, and preferably DNA.

The host cell of the present invention can be prepared, for example, by a method using an expression vector comprising the polynucleotide of the present invention (e.g., a competent cell method or an electroporation method), or a genome modifying technique. When the expression vector is an integrative vector that undergoes homologous recombination with the genomic DNA of the host cell, the expression unit can be integrated into the genomic DNA of the host cell by transformation. On the other hand, when the expression vector is a non-integrative vector that does not undergo homologous recombination with the genomic DNA of the host cell, the expression unit is not integrated into the genomic DNA of the host cell by transformation, and can exist in the host cell, independently of the genomic DNA, keeping the form of an expression vector. Alternatively, genome editing technology (e.g., CRISPR/Cas system, and Transcription Activator-Like Effector Nucleases (TALEN)) can be used to integrate an expression unit into the genomic DNA of a host cell, and modify an expression unit inherent to a host cell.

The present invention also provides an expression vector comprising the polynucleotide of the present invention and a promoter operably linked thereto. The expression vector of the present invention may further comprise elements such as a terminator, a ribosomal binding site, and a drug resistance gene that function in the host cell. Examples of the drug resistance gene include resistance genes against drugs such as tetracycline, ampicillin, kanamycin, hygromycin, and phosphinothricin.

For homologous recombination with the genomic DNA of the host cell, the expression vector may further comprise a region that enables homologous recombination with the genome of the host cell. For example, the expression vector may be designed such that the expression unit contained therein is positioned between a pair of homologous regions (e.g., homology arms that are homologous to a specific sequence in the genome of the host cell, or loxPs, or FRTs). The genomic region of the host cell, where the expression unit is to be introduced (the target of the homologous region) is not particularly limited, and may be a locus of a gene that is highly expressed in the host cell.

The expression vector may be a plasmid, a virus vector, a phage, or an artificial chromosome. The expression vector may be an integrative vector or a non-integrative vector. The integrated vector may be such a type of vector that its entirety is incorporated into the genome of the host cell. Alternatively, the integrative vector may be such a type of vector that only a part thereof (e.g., expression unit) is incorporated into the genome of the host cell. Additionally, the expression vector may be a DNA vector or an RNA vector (e.g., retrovirus). The expression vector may also be an expression vector that is generally used. Examples of such an expression vector include pUC (e.g., pUC19 and pUC18), pSTV, pBR (e.g., pBR322), pHSG (e.g., pHSG299, pHSG298, pHSG399, and pHSG398), RSF (e.g., RSF1010), pACYC (e.g., pACYC177 and pACYC184), pMW (e.g., pMW119, pMW118, pMW219, and pMW218), pQE (e.g., pQE30), and their derivatives.

Examples of the host cell that can be used to express the affinity polypeptide of the present invention include various prokaryotic cells including *Escherichia spp.,* such as *Escherichia coli, Corynebacterium spp.* (e.g., *Corynebacterium glutamicum),* and *Bacillus spp.* (e.g., *Bacillus subtilis);* and various eucaryotic cells including *Saccharomyces spp.* (e.g., *Saccharomyces cerevisiae*), *Pichia spp.* (e.g., *Pichia stipitis*)*,* and *Aspergillus spp.* (e.g., *Aspergillus oryzae*). Alternatively, insect cells, plant cells, or animal cells (e.g., mammalian cells such as Chinese hamster ovary (CHO) cells) can be used as the host. A strain that is deficient in a certain gene may be used as the host. Examples of the host cells include host cells carrying the expression vector in the cytoplasm and host cells where the target gene is introduced to the genome.

When the affinity polypeptide of the present invention comprises a tripeptide consisting of Gln-Glu-Thr (QET) at the N-terminus, it is preferable to use an affinity polypeptide that can be prepared by the method for secretory production of polypeptides using coryneform bacteria as the host (WO2013/062029). This method enables attaching the N-terminal three residues Gln-Glu-Thr (QET) in the Csp mature protein to a target polypeptide at the N-terminus, and furthermore enables preparing polypeptides comprising a glutamine residue (Q) at the N-terminus easily and in large quantities, and therefore is suitable for preparation of affinity polypeptides. In this case, various signal peptides such as a signal peptide (CspBss) composed of the amino acid sequence of MFNNRIRTAALAGAIAISTAASGVAIPAFA (SEQ ID NO: 42) can be attached at the N-terminus side of QET (see Examples, WO2013/062029, and WO2020/090979). Examples of the coryneform bacterium that can be used in the method include *Corynebacterium spp.* (e.g., *Corynebacterim glutamicum* and *Corynebacterium stationis*) and *Brevibacterium spp.*

The host cells of the present invention can be cultured, for example, in a culture medium having the composition described below using a certain culture apparatus (e.g., test tube, flask, or jar fermenter). The culture conditions can be set as appropriate. Specifically, the culture temperature may be 10°C to 37°C, pH may be 6.5 to 7.5, and the culture time may be 1 h to 100 h. Additionally, the dissolved oxygen concentration may be controlled during the culture. In this case, the dissolved oxygen concentration (DO level) in the culture medium can be used as an indicator of control. By controlling the aeration and stirring conditions, the relative dissolved oxygen concentration (DO level) when the atmospheric oxygen concentration is considered as 21% can be maintained, for example, at no less than 1-10%, preferably no less than 3-8%. Additionally, the culture may be batch culture or fed-batch culture. In the case of fed-batch culture, the culture can be continued by sequentially adding solutions that serve as carbon sources and a solution containing phosphate to the culture medium, either continuously or discontinuously.

As the promoter for expression of the polynucleotide of the present invention, promoters that are usually used in production of heterologous proteins in *E. coli* can be used, and examples thereof include strong promoters such as PhoA, PhoC, T7 promoters, lac promoters, trp promoters, trc promoters, tac promoters, PR promoters and PL promoters of lambda phage, and T5 promoters, and preferred are PhoA, PhoC, and lac. Examples of the vector that may be used include pUC (e.g., pUC19 and pUC18), pSTV, pBR (e.g., pBR322), pHSG (e.g., pHSG299, pHSG298, pHSG399, and pHSG398), RSF (e.g., RSF1010), pACYC (e.g., pACYC177 and pACYC184), pMW (e.g., pMW119, pMW118, pMW219, and pMW218), pQE (e.g., pQE30), and their derivatives.

Additionally, a terminator that is a transcription termination sequence may be linked downstream of the polynucleotide of the present invention. Examamples of such a terminator include T7 terminators, fd phage terminators, T4 terminators, terminators of the tetracycline resistance gene, and terminators of the *E. coli* trpA gene.

As the culture medium, a culture medium that is commonly used to culture E. *coli,* such as M9 medium with casamino acids or LB medium, may be used. The culture medium may contain certain carbon sources, nitrogen sources, and coenzymes (e.g., pyridoxine hydrochloride). Specifically, for example, peptone, yeast extract, NaCl, glucose, MgSO₄, ammonium sulfate, potassium dihydrogen phosphate, ferric sulfate, and manganese sulfate may be used. The culture conditions and production induction conditions are appropriately selected according to the types of the marker and the promoter of the vector used, and the host bacteria.

To collect the affinity polypeptide of the present invention, for example, the following method is used. The affinity polypeptide of the present invention can be obtained as debris or lysates by collecting the transformed cells of the present invention and then disrupting (e.g., sonication or homogenization) or lysing (e.g., lysozyme treatment) them. In the case where the affinity polypeptide is secreted or leaked out of the cells, the culture can be centrifuged or filtered through membranes to obtain a sterile solution containing the affinity polypeptide. Such a debris, lysate, or sterile solution is subjected to extraction, precipitation, filtration, column chromatography, or other techniques to obtain the affinity polypeptide of the present invention.

### 3. Compound or a salt thereof

The compound or a salt thereof of the present invention comprises (A) an affinity substance comprising first and second affinity moieties each having an affinity to the constant region in a heavy chain of an antibody, and (B) a reactive group for the antibody. The definitions, examples, and preferred examples of the affinity substance and the components constituting them (e.g., the affinity moiety, such as an affinity peptide, and the linker between the affinity moieties, such as a peptide linker) are as described above.

Among the amino acid residues constituting the antibody (protein), reactive groups for amino acid residues with reactive side chains can be used as reactive groups for antibodies. Of the 20 naturally-occurring amino acids constituting proteins as described above, glycine that has no side chain, and alanine, isoleucine, leucine, phenylalanine, and valine with a side chain that is a hydrocarbon group are inactive to common reactions. Therefore, the reactive group for an antibody is a group that can react with the side chain of any one or two or more (e.g., 2, 3, or 4) of 14 amino acids consisting of asparagine, glutamine, methionine, proline, serine, threonine, tryptophan, tyrosine, aspartic acid, glutamic acid, arginine, histidine, and lysine. One or two or more (e.g., 2, 3, or 4) reactive group may be contained in the compound or a salt thereof of the present invention depending on the conditions such as the amino acid composition of the antibody, and preferably one reactive group may be contained in the compound or a salt thereof of the present invention.

Preferably, the reactive group for an antibody is a group that can react with the side chain of any one amino acid of 14 amino acids constituting proteins as described above. The reactive group for an antibody is more preferably a reactive group specific to the side chain of any one amino acid of lysine, tyrosine, tryptophan, and cysteine, even more preferably a reactive group specific to the side chain of any one amino acid of lysine, tyrosine, and tryptophan, and particularly preferably a reactive group specific to the side chain of lysine or tyrosine, in particular, of the side chain of lysine. For the details of such reactive groups, see, for example, WO2016/186206, WO2018/199337, WO2019/240287, WO2019/240288, and WO2020/090979.

The reactive group specific to the side chain of a lysine residue is a group that can specifically react with the amino group (NH₂) present in the side chain of a lysine residue, including, for example, activated ester residues (e.g., N-hydroxysuccinimide residues), vinyl sulfone residues, sulfonyl chloride residues, isocyanate residues, isothiocyanate residues, aldehyde residues, 1,4,7, 10-tetraazacyclododecane-1,4,7, 10-tetraacetate residues, 2-imino-2-methoxyethyl residues, diazonium terephthalic acid residues, α-halogenated acetamido, α-halogenated methylketone. The reaction between the reactive group specific to the side chain of a lysine residue and the amino group (NH₂) present in the side chain of a lysine residue can produce a linking moiety, for example, an amide residue, a urea residue, a pyridine residue, a carbamate residue, or a sulfoneamide residue.

In a specific embodiment, the compound or a salt thereof of the present invention may be represented by the following formula (I):

R-L-A (I)

wherein
R indicates a reactive group for an antibody;
L indicates a linker; and
A indicates an affinity substance comprising first and second affinity moieties each having an affinity to the constant region in a heavy chain of an antibody. The definitions, examples, and preferred examples of the reactive group for an antibody represented by R and the affinity substance represented by A are as described above.

The linker is a divalent group. The divalent group may be substituted or unsubstituted. Examples of the divalent group include those described above. Examples of the substituent when the divalent group is substituted include those described above.

**In** a specific embodiment, the compound or a salt thereof of the present invention may further comprise a cleavable moiety between the affinity substance and the reactive group for an antibody. **In** this case, the compound or a salt thereof represented by the formula (I) may comprise a linker containing a cleavable moiety.

The cleavable moiety is a moiety that can be cleaved by a specific treatment under conditions that cannot cause protein denaturation or degradation (e.g., cleavage of an amide bond) (mild conditions). Therefore, the cleavable moiety can be a moiety (a bond other than amide bond) that can be cleaved by a specific treatment under mild conditions. Such a specific treatment may be, for example, (a) treatment with one or more substance selected from the group consisting of an acidic substance, a basic substance, a reducing agent, an oxidizing agent, and an enzyme as described above, (b) treatment with physicochemical stimulation such as light, or (c) incubation using a cleavable linker comprising a self-degradable cleavable moiety. Such cleavable linkers and the conditions for cleaving them are common technical knowledge in the art (e.g., G. Leriche, L. Chisholm, A. Wagner, Bioorganic & Medicinal Chemistry. 20,571 (2012*);* Feng P. et al., Jounal of American Chemical Society. 132,1500 (2010*).;* Bessodes M. et al., Journal of Controlled Release, 99,423 (2004*).;* DeSimone, J. M., Journal of American Chemical Society. 132,17928 (2010*);* Thompson, D. H., Journal of Controlled Release, 91,187 (2003*);* Schoenmarks, R. G., Journal of Controlled Release, 95,291 (2004*)).* For the mild conditions, the reaction conditions (e.g., the reaction temperature, the reaction time, and the reaction solution) are as described later. Examples of the cleavable moiety as described above include disulfide residues, acetal residues, ketal residues, ester residues, carbamoyl residues, alkoxyalkyl residues, imine residues, tertiary alkyloxycarbamate residues (e.g., tert-butyloxycarbamate residues), silane residues, hydrazone-containing residues (e.g., hydrazone residues, acylhydrazone residues, and bisarylhydrazone residues), phosphoramidite residues, aconytyl residues, trityl residues, azo residues, vicinaldiol residues, selenium residues, residue containing an aromatic ring having an electron-withdrawing group, coumarin-containing residues, sulfone-containing residues, unsaturated bond-containing chain residues, and glycosyl residues.

The aromatic ring group having an electron-withdrawing group is preferably one having an aromatic ring group selected from the group consisting of aryl, aralkyl, an aromatic heterocyclic group, and alkyl having an aromatic heterocyclic group, and more preferably aralkyl, or alkyl having an aromatic heterocyclic group. The electron-withdrawing group is preferably attached at the 2-position of the ring. Even more preferably, the aromatic ring-containing residue having an electron-withdrawing group is, for example, aralkyl (e.g., benzyl) having an electron-withdrawing group at the 2-position. Examples of the electron-withdrawing group include halogen atoms, alkyl substituted with a halogen atom (e.g., trifluoromethyl), a boronic acid residue, mesyl, tosyl, triflate, nitro, cyano, phenyl groups, and keto groups (e.g., acyl).

The definitions, examples, and preferred examples of the groups such as alkyl, acyl (or alkylcarbonyl), alkoxy (or alkyloxy), aryl, and aralkyl found as prefixes, suffixes, and other terms in connection with the names of residues as cleavable moieties are similar to those described above.

Examples of the ester residues include usual ester residues composed of carbon atoms and oxygen atoms [e.g., alkyl esters (e.g., tertiary alkyloxycarbonyl such as tert-butyl oxycarbonyl), aryl esters (e.g., phenacyl esters and 2-(diphenylphosphino)benzoate), glycosyl ester residues, ortho ester residues], ester residues containing sulfur atoms and oxygen atoms (e.g., thioester residues such as α-thiophenyl ester residues and alkyl thioester residues), ester residues containing phosphorus and oxygen atoms (e.g., phosphodiester residues, and phosphotriester residues), and activated ester residues (e.g., N-hydroxysuccinimide residues).

Examples of the sulfone-containing residue include sulfone residues and quinolinyl benzene sulfonate residues.

The silane residue preferably is a silane residue having a group selected from the group consisting of alkyl, aryl, aralkyl, and alkoxy. Examples of such a silane residue include dialkyldialkoxysilane residues (e.g., dimethyldialkoxysilane and diethyldialkoxysilane), and diaryldialkoxysilane residues (e.g., diphenyldialkoxysilane).

The alkoxyalkyl (or alkyloxyalkyl) residue is a group in which alkyloxy and alkyl as descried above are combined, and examples thereof include, but not limited to, methoxymethyl residues, ethoxymethyl residues, methoxyethyl residues, and ethoxyethyl residue.

The unsaturated bond-containing chain residue is a residue containing an unsaturated bond moiety composed solely of carbon atoms [e.g., vinyl (ester) that is the smallest unit having a carbon-carbon double bond, or acetylenyl (ethinyl) that is the smallest unit having a carbon-carbon triple bond], or a residue containing an unsaturated bond moiety (e.g., aldehyde or cyano) composed of carbon atoms and hetero atoms (e.g., a nitrogen atom, a sulfur atom, and an oxygen atom). Examples of the unsaturated bond-containing chain residue include vinyl ether residues, cyanoethyl residues, ethylene residues, and malondialdehyde residues.

Examples of the acidic substance (also referred to as electrophilic reagent) include inorganic acidic substances such as hydrochloric acid, sulfuric acid, and nitric acid, and organic acidic substances such as formic acid, acetic acid, 4-(2-hydroxyethyl)-1-piperazinepropanesulfonic acid, 3-morphorinopropanesulfonic acid, sodium dihydrogenphosphate, citric acid, dodecyl sulfate, N-dodecanoylsarcosinate, and trifluoroacetic acid. Examples of the moiety that can be cleaved by the acidic substance include alkyloxyarylalkyl residues, tertiary alkyloxycarbamate residues, acetal residues, silane residues, imine residues, vinyl ether residues, β-thiopropionate residues, trityl residues, hydrazone residues, aconytyl residues, ortho ester residues, carbamoyl residues, and 2-(diphenylphosphino)benzoate residues.

Examples of the basic substance (also referred to as nucleophilic reagent) include inorganic basic substances such as sodium hydroxide, potassium hydroxide, sodium acetate, potassium acetate, and ammonium acetate, and organic basic substances such as hydroxylamine, triethylamine, and N,N'-diisopropylamine. Examples of the moiety that can be cleaved by the basic substance include silane residues, cyanoethyl residues, sulfone residues, ethylene residues, glycosyl disuccinate residues, α-thiophenyl ester residues, unsaturated vinylsulfide residues, malondialdehyde residues, acylhydrazone residues, and alkylthioester residues.

Examples of the reducing agent include cysteine, dithiothreitol, reduced glutathione, and β-mercaptoethanol. Examples of the moiety that can be cleaved by the reducing agent include disulfide residues, alkoxyalkyl residues, and azo residue.

Examples of the oxidizing agent include sodium periodate and oxidized glutathione. Examples of the moiety that can be cleaved by the oxidizing agent include vicinaldiol residues and selenium residues.

Examples of the enzyme include trypsin, papain, TEV, thrombin, cathepsin B, cathepsin D, cathepsin K, caspases, proteases, matrix metalloproteinases, lipases, endoglycosidases, and PNGase F. Examples of the moiety that can be cleaved by the enzyme include ester residues, phosphodiester residues, and glycosyl residues.

Examples of moiety that can be cleaved by light include 2-nitrobenzyl residues, phenacyl ester residues, 8-quinolinebenzenesulfonate residues, coumarin residues, phosphotriester residues, bisarylhydrazone residues, and bimanedithiopropionic acid residues.

Example of the self-degradable cleavable moiety include activated ester residues (e.g., N-hydroxysuccinimide residues).

When the compound or a salt thereof of the present invention comprises a cleavable moiety, the cleavable moiety may be one that can be cleaved to generate a bioorthogonal functional group on the reactive group side. Examples of such a cleavable moiety include disulfide residues, ester residues (including typical ester residues, and other ester residues described above, such as thioester residues), acetal residues (including typical ester residues, and other acetal residues such as thioacetal residues), ketal residues, imine residues, and vicinaldiol residues.

The compound or a salt thereof of the present invention, when comprising a cleavable moiety that can be cleaved to generate a bioorthogonal functional group on the reactive group side, may be represented by the following formula (Ia):

R-L₁-CLE(B)-L₂-A (Ia)

wherein
R indicates a reactive group for an antibody;
L₁ indicates a first linker;
L₂ indicates a second linker;
CLE(B) represents a cleavable moiety that can be cleaved to generate a bioorthogonal functional group on the reactive group side; and
A indicates an affinity substance comprising first and second affinity moieties each having an affinity to the constant region in a heavy chain of an antibody. The definitions, examples, and preferred examples of the reactive group for an antibody represented by R, the affinity substance represented by A, and the cleavable moiety that can be cleaved to generate a bioorthogonal functional group on the reactive group side, represented by CLE(B), are as described above.

The first linker represented by L1 and the second linker represented by L2 may be the same or different divalent groups. The divalent group may be substituted or unsubstituted. Examples of the divalent group include those described above. Examples of the substituent when the divalent group is substituted include those described above.

In a specific embodiment, the total number of atoms constituting the main chains in the first and second linkers may be 2 to 10. The total number of such atoms may be 3 or more, or 4 or more. The total number of such atoms may be 9 or less, 8 or less, or 7 or less. More specifically, the total number of such atoms may be 3 to 9, 4 to 8, or 4 to 7.

The number of atoms constituting the main chains in the first and second linkers may be each 1 to 9. The number of such atoms may be 2 or more, or 3 or more. The number of such atoms may be 8 or less, 7 or less, or 6 or less. More specifically, the number of such atoms may be 2 to 8, 3 to 7, or 3 to 6.

The main chains in the first linker and the second linker are composed of a chain structure, a cyclic structure, or a structure containing a combination thereof. When the main chain has a chain structure comprising no cyclic structure, the number of atoms of the main chain can be determined by counting the number of atoms in the chain structure. On the other hand, when the main chain has a structure comprising a cyclic structure, the number of atoms in the main chain can be determined by counting the specific number of the atoms constituting the cyclic structure as the number of atoms in the main chain. Specifically, the number of atoms of the main chain in the cyclic structure can be determined by counting the number of atoms of the shortest route linking two bonds in the cyclic structure (see, e.g., the following thick routes (a) to (d)). When the main chain has a structure comprising a combination of a chain structure and a cyclic structure, the number of atoms of the main chain can be determined by adding the number of atoms in the chain structure not comprising the cyclic structure to the number of atoms of the shortest route linking two bonds in the cyclic structure. The counting of the number of atoms in the main chain is the same for other linkers. 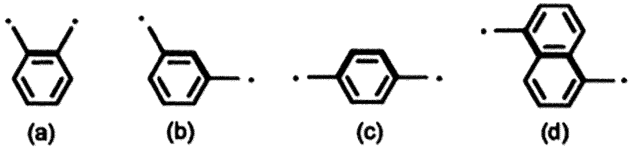 • is a bond.

In the case of (a), the shortest route is the thick route, and thus the number of atoms in the divalent cyclic structure counted as the number of atoms of the main chain is two.

In the case of (b), the shortest route is the thick route, and thus the number of atoms in the divalent cyclic structure counted as the number of atoms of the main chain is three.

In the case of (c), both routes are the shortest routes (equidistant), and thus the number of atoms in the divalent cyclic structure counted as the number of atoms in the main chain is four.

In the case of (d), a route of a condensation site is the shortest route, and thus the number of atoms in the divalent cyclic structure counted as the number of atoms in the main chain is four.

**In** a specific embodiment, the compound or a salt thereof of the present invention may be a compound or a salt thereof represented by the following formula (Ia-1): 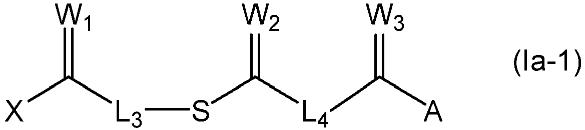 wherein
X indicates a leaving group;
W₁, W₂ and W₃ each independently indicate an oxygen atom or a sulfur atom;
L₃ indicates a third linker;
L₄ indicates a fourth linker;
S indicates a sulfur atom; and
A indicates an affinity substance comprising first and second affinity moieties each having an affinity to the constant region in a heavy chain of an antibody. The definitions, examples, and preferred examples of the affinity substance represented by A are as described above.

The leaving group represented by X is a group that can be eliminated by a reaction between a carbon atom of C=W₁ adjacent to X and an amino group. A person skilled in the art can appropriately design such a leaving group. Examples of such leaving groups include:
(a) R_{A}-S, wherein R_{A} indicates a hydrogen atom, a monovalent hydrocarbon group which may have a substituent, or a monovalent heterocyclic group which may have a substituent, and S indicates a sulfur atom;
(b) R_{A}-O, wherein R_{A} indicates a hydrogen atom, a monovalent hydrocarbon group which may have a substituent, or a monovalent heterocyclic group which may have a substituent, and O indicates an oxygen atom;
(c) R_{A}-(R_{B}-)N, wherein R_{A} and R_{B} each independently indicate a hydrogen atom, a monovalent hydrocarbon group which may have a substituent, or a monovalent heterocyclic group which may have a substituent, and N indicates a nitrogen atom; or
(d) a halogen atom.

Preferably, the leaving group represented by X may be:
(a) R_{A}-S, wherein R_{A} indicates a hydrogen atom, a monovalent hydrocarbon group which may have a substituent, or a monovalent heterocyclic group which may have a substituent, and S indicates a sulfur atom;
(b) R_{A}-O, wherein R_{A} indicates a hydrogen atom, a monovalent hydrocarbon group which may have a substituent, or a monovalent heterocyclic group which may have a substituent, and O indicates an oxygen atom; or
(c) R_{A}-(R_{B}-)N, wherein R_{A} and R_{B} each independently indicate a hydrogen atom, a monovalent hydrocarbon group which may have a substituent, or a monovalent heterocyclic group which may have a substituent, and N indicates a nitrogen atom.

More preferably, the leaving group represented by X may be:
(a) R_{A}-S, wherein R_{A} indicates a hydrogen atom, a monovalent hydrocarbon group which may have a substituent, or a monovalent heterocyclic group which may have a substituent, and S indicates a sulfur atom; or
(b) R_{A}-O, wherein R_{A} indicates a hydrogen atom, a monovalent hydrocarbon group which may have a substituent, or a monovalent heterocyclic group which may have a substituent, and O indicates an oxygen atom.

Even more preferably, the leaving group represented by X may be:
(a) R_{A}-S, wherein R_{A} indicates a hydrogen atom, a monovalent hydrocarbon group which may have a substituent, or a monovalent heterocyclic group which may have a substituent, and S indicates a sulfur atom.

Particularly preferably, the leaving group represented by X may be:
(a') R_{A}-S wherein R_{A} indicates a monovalent aromatic hydrocarbon group (e.g., phenyl) which may have a substituent, and S indicates a sulfur atom.

W₁, W₂ and W₃ each independently indicate an oxygen atom or a sulfur atom. Preferably, W₁, W₂ and W₃ may be an oxygen atom.

The third linker represented by L₃ and the fourth linker represented by L₄ may be the same or different divalent groups. The divalent group may be substituted or unsubstituted. Examples of the divalent group include those described above. Examples of the substituent when the divalent group is substituted include those described above.

In a specific embodiment, the total number of atoms constituting the main chains in the third and fourth linkers may be 2 to 10. The total number of such atoms may be 3 or more, or 4 or more. The total number of such atoms may be 9 or less, 8 or less, or 7 or less. More specifically, the total number of such atoms may be 3 to 9, 4 to 8, or 4 to 7.

The number of atoms constituting the main chains in the third and fourth linkers may be 1 to 9. The number of such atoms may be 2 or more, or 3 or more. The number of such atoms may be 8 or less, 7 or less, or 6 or less. More specifically, the number of such atoms may be 2 to 8, 3 to 7, or 3 to 6.

The compound or a salt thereof of the present invention, when it comprises a cleavable moiety, may further comprise a bioorthogonal functional group between the reactive group for an antibody and the cleavable moiety. The bioorthogonal functional group is as described above. Preferred examples of the bioorthogonal functional group include azide residues, alkyne residues (preferably, ring groups that may be substituted by a substituent as described above and have a carbon-carbon triple bond), tetrazine residues, alkene residues, thiol residues, maleimide residues, thiol residues, furan residues, and halocarbonyl residues.

The compound or a salt thereof of the present invention, when it further comprises a bioorthogonal functional group between the reactive group for an antibody and the cleavable moiety, may be represented by the following formula (Ib): 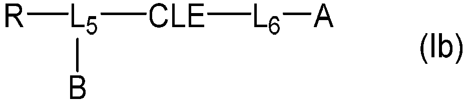 wherein
R indicates a reactive group for an antibody;
L₅ indicates a fifth linker;
L₆ indicates a sixth linker;
B indicates a group containing a bioorthogonal functional group;
CLE indicates a cleavable moiety; and
A indicates an affinity substance comprising first and second affinity moieties each having an affinity to the constant region in a heavy chain of an antibody. The definitions, examples, and preferred examples of the reactive group for an antibody represented by R, the affinity substance represented by A, and the cleavable moiety represented by CLE are as described above.

The fifth linker represented by L₅ and the sixth linker represented by L₆ may be the same or different divalent groups. The divalent group may be substituted or unsubstituted. Examples of the divalent group include those described above. Examples of the substituent when the divalent group is substituted include those described above.

In a specific embodiment, the total number of atoms constituting the main chains in the fifth and sixth linkers may be 2 to 10. The total number of such atoms may be 3 or more, or 4 or more. The total number of such atoms may be 9 or less, 8 or less, or 7 or less. More specifically, the total number of such atoms may be 3 to 9, 4 to 8, or 4 to 7.

The number of atoms constituting the main chains in the fifth and sixth linkers may be each 1 to 9. The number of such atoms may be 2 or more, or 3 or more. The number of such atoms may be 8 or less, 7 or less, or 6 or less. More specifically, the number of such atoms may be 2 to 8, 3 to 7, or 3 to 6.

The group comprising a bioorthogonal functional group represented by B may be a group consisting of a bioorthogonal functional group, or may be a group comprising a bioorthogonal functional group and other moieties. Examples of the other moieties include a linking moiety between the bioorthogonal functional group and the linker. The linking moiety is, for example, a divalent group. The divalent group may be substituted or unsubstituted. The definitions, examples, and preferred examples of the bioorthogonal functional group, the divalent group, and the substituent when the divalent group is substituted are as described above.

In a specific embodiment, the compound or a salt thereof of the present invention may be a compound or a salt thereof represented by the following formula (Ib-1): 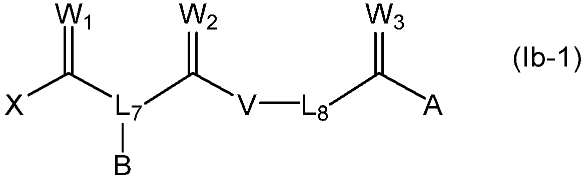 wherein
X indicates a leaving group;
W₁, W₂ and W₃ each independently indicate an oxygen atom or a sulfur atom;
L₇ indicates a seventh linker;
L₈ indicates an eighth linker;
B indicates a group containing a bioorthogonal functional group;
V indicates an oxygen atom or a sulfur atom; and
A indicates an affinity substance comprising first and second affinity moieties each having an affinity to the constant region in a heavy chain of an antibody. The definitions, examples, and preferred examples of the leaving group represented by X, the group containing a bioorthogonal functional group represented by B, and the affinity substance represented by A are as described above.

W₁, W₂ and W₃ each independently indicate an oxygen atom or a sulfur atom. Preferably, W₁, W₂ and W₃ may be an oxygen atom.

The seventh linker represented by L₇ and the eighth linker represented by L₈ may be the same or different divalent groups. The divalent group may be substituted or unsubstituted. Examples of the divalent group include those described above. Examples of the substituent when the divalent group is substituted include those described above.

In a specific embodiment, the total number of atoms constituting the main chains in the seventh and eighth linkers may be 2 to 10. The total number of such atoms may be 3 or more, or 4 or more. The total number of such atoms may be 9 or less, 8 or less, or 7 or less. More specifically, the total number of such atoms may be 3 to 9, 4 to 8, or 4 to 7.

The number of atoms constituting the main chains in the seventh and eighth linkers may be each 1 to 9. The number of such atoms may be 2 or more, or 3 or more. The number of such atoms may be 8 or less, 7 or less, or 6 or less. More specifically, the number of such atoms may be 2 to 8, 3 to 7, or 3 to 6.

V indicates an oxygen atom or a sulfur atom. Preferably, V may be a sulfur atom.

The compound or a salt thereof of the present invention can easily modify only one heavy chain of the constituent unit of an antibody. The compound or a salt thereof of the present invention can also provide an antibody that is easily modified in only one heavy chain of the constituent unit of the antibody, while being regioselectively modified.

The production of a series of compounds or salts thereof as described above can be performed by reacting the affinity substance of the present invention with a moiety compound containing a reactive group for an antibody. For example, such a reaction may be carried out at a suitable temperature (e.g., about -10 to 30°C) in a suitable organic solvent system (e.g., an organic solvent containing an alkyl halide (e.g., methyl halide) such as CH₂Cl₂ and an amine such as triethylamine). The reaction time is, for example, 1 minute to 20 hours, preferably 10 minutes to 15 hours, more preferably 20 minutes to 10 hours, and even more preferably 30 minutes to 8 hours.

The determination of the formation of a series of compounds or salts thereof as described above, which depends on their specific raw materials and the molecular weights of the products, can be performed, for example, by electrophoresis, chromatography (e.g., gel permutation chromatography, ion-exchange chromatography, reversed phase column chromatography, and HPLC), NMR, or mass spectrometry. Such compounds or salts thereof can be purified as appropriate by any method such as chromatography (e.g., the chromatography described above and affinity chromatography).

### 4. Affinity substance-modified antibody or a salt thereof

### 4-1. Affinity substance-modified antibody or a salt thereof, comprising at least one affinity substance (comprising first and second affinity moieties)

The present invention provides an affinity substance-modified antibody or a salt thereof, comprising an affinity substance comprising first and second affinity moieties each having an affinity to the constant region in a heavy chain of the antibody in the constant region in a heavy chain of the antibody. The definitions, examples, and preferred examples of the affinity substance, and the antibody, and the components constituting them (e.g., the affinity moiety, such as an affinity peptide, the linker between the affinity moieties, such as a peptide linker, and the constant region) are as described above.

Preferably, the affinity substance-modified antibody or a salt thereof may comprise (a) a constituent unit of an antibody (an immunoglobulin unit comprising two heavy chains and optionally two light chains), and (b) an affinity substance, wherein (c) the affinity substance is introduced to the constant region in only one heavy chain of the immunoglobulin unit (i.e., an affinity substance-modified antibody is introduced to the constant region in one heavy chain of the immunoglobulin unit, and no affinity substance-modified antibody is introduced to the constant region in the other heavy chain). The definitions, examples, and preferred examples of the antibody, the immunoglobulin unit, and the affinity substance, and the components constituting them (e.g., the constant region) are as described above.

The affinity substance-modified antibody or a salt thereof can comprise an affinity substance via modification of the functional group in the side chain of any one or two or more (e.g., 2, 3, or 4) of 14 amino acid residues consisting of asparagine, glutamine, methionine, proline, serine, threonine, tryptophan, tyrosine, aspartic acid, glutamic acid, arginine, histidine, and lysine present in the constant region (preferably the Fc region or the CH2 domain). The affinity substance-modified antibody or a salt thereof can preferably comprise an affinity substance via modification of the functional group in the side chain of any one of lysine, tyrosine, tryptophan, or cysteine present in the constant region (preferably the Fc region or the CH2 domain), more preferably via modification of the functional group in the side chain of any one of lysine, tyrosine, or tryptophan, even more preferably via modification of the functional group in the side chain of lysine or tyrosine, and particularly preferably via modification of the amino group in the side chain of lysine. The positions of these amino acid residues in the constant region are as described above. The position of modification in the antibody or a salt thereof by the affinity substance can be determined by peptide mapping. The modification may be regioselective as described above. Thus, the immunoglobulin unit in the formulae (II), (IIa), (IIa-1), (IIb), and (IIb-1) described below may regioselectively have a corresponding modification unit via a functional group in the side chain of the amino acid residue described above.

Preferably, the affinity substance-modified antibody can comprise the affinity substance via modification of the amino group in the side chain of one or more (preferably one or two, more preferably one) lysine residue in the constant region (preferably the Fc region or the CH2 domain) in one heavy chain of the constituent unit (the immunoglobulin unit comprising two heavy chains and optionally two light chains) of an antibody (in other words, comprises the affinity substance via the amino group in the side chain of a lysine residue in the constant region in one heavy chain of the immunoglobulin unit, but does not comprise the affinity substance via the amino group in the side chain of a lysine residue in the constant region in the other heavy chain). More specifically, the position of the one or more (preferably one or two, more preferably one) lysine residue may be the position 246/248, 288/290, or 317 in the human IgG heavy chain according to the EU numbering (see, for example, WO2016/186206, WO2018/199337, WO2019/240287, WO2019/240288, WO2020/009165, and WO2020/090979). The modification may be regioselective as described above. Thus, the immunoglobulin unit in the formulae (II), (IIa), (IIa-1), (IIb), and (IIb-1) described below may regioselectively have a corresponding modification unit via the amino group in the side chain of the lysine residue described above.

The affinity substance-modified antibody or a salt thereof can be produced by reacting the compound or a salt thereof of the present invention with an antibody or a salt thereof comprising an immunoglobulin unit comprising two heavy chains and optionally two light chains. The equivalence of the compound or salt thereof of the present invention to the antibody (the compound of the present invention or salt thereof/antibody) to the antibody in the reaction is not particularly limited, since it varies depending on factors such as the type of the compound of the present invention or salt thereof and antibody. It is, for example, 1 to 100, preferably 2 to 80, more preferably 4 to 60, even more preferably 5 to 40, and particularly preferably 6 to 20.

Such a reaction can be appropriately carried out under a condition that cannot cause protein denaturation/degradation (e.g., cleavage of amide bond) (mild conditions). For example, such a reaction under the mild conditions can be carried out at room temperature (e.g., about 15 to 30°C) in a suitable reaction system, such as a buffer. The pH of the buffer is, for example, 5 to 9, preferably 5.5 to 8.5, and more preferably 6.0 to 8.0. The buffer may contain a suitable catalyst. The reaction time is, for example, 1 minute to 20 hours, preferably 10 minutes to 15 hours, more preferably 20 minutes to 10 hours, and even more preferably 30 minutes to 8 hours. For details of such a reaction, see, e.g., G. J. L. Bernardes et al., Chem. Rev., 115,2174 (2015*);* G. J. L. Bernardes et al., Chem. Asian. J., 4,630 (2009*);* B. G. Davies et al., Nat. Commun., 5,4740 (2014*);* A. Wagner et al., Bioconjugate. Chem., 25,825 (2014*).*

In a specific embodiment, the affinity substance-modified antibody or a salt thereof may be an antibody or a salt thereof, comprising a structural unit represented by the following formula (II): 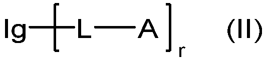 wherein
Ig indicates an immunoglobulin unit comprising two heavy chains and optionally two light chains;
L indicates a linker;
A indicates an affinity substance comprising first and second affinity moieties each having an affinity to the constant region in a heavy chain of an antibody; and
the average percent modification r of the immunoglobulin unit with the affinity substance is 65 to 135%. The definitions, examples, and preferred examples of the immunoglobulin unit represented by Ig, the linker represented by L, and the affinity substance represented by A, and the antibody are as described above (e.g., for the linker represented by L, see the linker represented by L in the compound of the formula (I)).

The average percent modification r of the immunoglobulin unit with the affinity substance is 65 to 135%. The average percent modification r may be 66% or more, 67% or more, 68% or more, 69% or more, 70% or more, 72% or more, 74% or more, 76% or more, 78% or more, 80% or more, 82% or more, 84% or more, 86% or more, 88% or more, 90% or more, 92% or more, 94% or more, or 96% or more. The average percent modification r may also be 130% or less, 125% or less, 120% or less, 115% or less, 110% or less, 105% or less, 100% or less, 98% or less, 96% or less, 94% or less, 92% or less, 90% or less, 88% or less, 86% or less, 84% or less, 82% or less, or 80% or less. The average percent modification r can be determined by mass spectrometry (DAR calculator (Agilent software) can be used in combination. see Examples).

The average percent modification r may preferably be 65 to 100%, more preferably 70 to 100%, even more preferably 75 to 100%, and particularly preferably 80 to 100%, 85 to 100%, 90 to 100%, or 95 to 100%. In these average percent modifications, the upper limit may be a value that is not more than the average percent modification described above, such as 98% or less or 96% or less. Alternatively, the average percent modification r may be 96 to 100%, 97 to 100%, 98 to 100%, 99 to 100%, or 100%.

The extent of the aforementioned average percent modification r can also be similarly applied to other average percent modifications r. In other words, the extent of the average percent modification r described above can be similarly applied not only to the average percent modification r described later with affinity substances but also to the average percent modification r described later with any modifications (e.g., bioorthogonal functional groups, functional substances).

An antibody or a salt thereof comprising a structural unit represented by the formula (II) can be produced by reacting the compound or a salt thereof represented by the formula (I) with an antibody or a salt thereof comprising an immunoglobulin unit comprising two heavy chains and optionally two light chains.

In a specific embodiment, the affinity substance-modified antibody or a salt thereof may further comprise a cleavable moiety between the affinity substance and the antibody (immunoglobulin unit). In this case, the antibody or a salt thereof comprising a structural unit represented by the formula (II) may comprise a linker containing a cleavable moiety. The definition, examples, and preferred examples of the cleavable moiety are as described above.

When the affinity substance-modified antibody or a salt thereof comprises a cleavable moiety, the cleavable moiety may be one that can be cleaved to generate a bioorthogonal functional group on the antibody (immunoglobulin unit) side. Examples of such a cleavable moiety include disulfide residues, ester residues (including typical ester residues, and other ester residues described above, such as thioester residues), acetal residues (including typical ester residues, and other acetal residues such as thioacetal residues), ketal residues, imine residues, and vicinaldiol residues.

The affinity substance-modified antibody or a salt thereof, when comprising a cleavable moiety that can be cleaved to generate a bioorthogonal functional group on the antibody (immunoglobulin unit) side, may be an antibody or a salt thereof comprising a structural unit represented by the following formula (IIa): 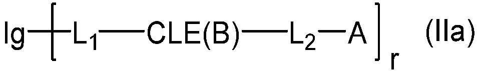 wherein
Ig indicates an immunoglobulin unit comprising two heavy chains and optionally two light chains;
L₁ indicates a first linker;
L₂ indicates a second linker;
CLE(B) indicates a cleavable moiety that can be cleaved to generate a bioorthogonal functional group on the immunoglobulin unit side;
A indicates an affinity substance comprising first and second affinity moieties each having an affinity to the constant region in a heavy chain of an antibody; and
the average percent modification r of the immunoglobulin unit with the affinity substance is 65 to 135%. The definitions, examples, and preferred examples of the immunoglobulin unit represented by Ig, the first linker represented by L₁, the second linker represented by L₂, the cleavable moiety represented by CLE(B), the affinity substance represented by A, and the average percent modification represented by r, and the antibody are as described above.

An antibody or a salt thereof comprising a structural unit represented by the formula (IIa) can be produced by reacting the compound or a salt thereof represented by the formula (Ia) with an antibody or a salt thereof comprising an immunoglobulin unit comprising two heavy chains and optionally two light chains.

In a specific embodiment, the affinity substance-modified antibody or a salt thereof may be an antibody or a salt thereof, comprising a structural unit represented by the following formula (IIa-1): 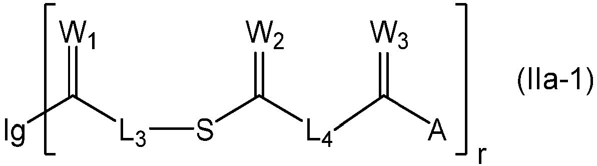 wherein
Ig indicates an immunoglobulin unit comprising two heavy chains and optionally two light chains;
W₁, W₂ and W₃ each independently indicate an oxygen atom or a sulfur atom;
L₃ indicates a third linker;
L₄ indicates a fourth linker;
S indicates a sulfur atom;
A indicates an affinity substance comprising first and second affinity moieties each having an affinity to the constant region in a heavy chain of an antibody; and
the average percent modification r of the immunoglobulin unit with the affinity substance is 65 to 135%. The definitions, examples, and preferred examples of the immunoglobulin unit represented by Ig, the atoms represented by W₁, W₂, and W₃, the third linker represented by L₃, the fourth linker represented by L₄, the affinity substance represented by A, and the average percent modification represented by r, and the antibody are as described above.

An antibody or a salt thereof comprising a structural unit represented by the formula (IIa-1) can be produced by reacting the compound or a salt thereof represented by the formula (Ia-1) with an antibody or a salt thereof comprising an immunoglobulin unit comprising two heavy chains and optionally two light chains.

The affinity substance-modified antibody or a salt thereof, when it comprises a cleavable moiety, may further comprise a bioorthogonal functional group between the antibody (immunoglobulin unit) and the cleavable moiety. The bioorthogonal functional group is as described above. Preferred examples of the bioorthogonal functional group include azide residues, alkyne residues (preferably, ring groups that may be substituted by a substituent as described above and have a carbon-carbon triple bond), tetrazine residues, alkene residues, thiol residues, maleimide residues, thiol residues, furan residues, and halocarbonyl residues.

The affinity substance-modified antibody or a salt thereof, when it further comprises a bioorthogonal functional group between the antibody (immunoglobulin unit) and the cleavable moiety, may be an antibody or a salt thereof comprising a structural unit represented by the following formula (IIb): 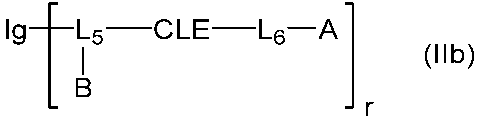 wherein
Ig indicates an immunoglobulin unit comprising two heavy chains and optionally two light chains;
L₅ indicates a fifth linker;
L₆ indicates a sixth linker;
B indicates a group containing a bioorthogonal functional group;
CLE indicates a cleavable moiety;
A indicates an affinity substance comprising first and second affinity moieties each having an affinity to the constant region in a heavy chain of an antibody; and
the average percent modification r of the immunoglobulin unit with the affinity substance is 65 to 135%. The definitions, examples, and preferred examples of the immunoglobulin unit represented by Ig, the fifth linker represented by L₅, the sixth linker represented by L₆, the group containing a bioorthogonal functional group represented by B, the cleavable moiety represented by CLE, the affinity substance represented by A, and the average percent modification represented by r, and the antibody are as described above.

An antibody or a salt thereof comprising a structural unit represented by the formula (IIb) can be produced by reacting the compound or a salt thereof represented by the formula (Ib) with an antibody or a salt thereof comprising an immunoglobulin unit comprising two heavy chains and optionally two light chains.

In a specific embodiment, the affinity substance-modified antibody or a salt thereof may be an antibody or a salt thereof, comprising a structural unit represented by the following formula (IIb-1): 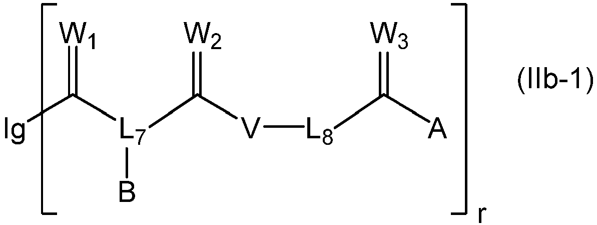 wherein
Ig indicates an immunoglobulin unit comprising two heavy chains and optionally two light chains;
W₁, W₂ and W₃ each independently indicate an oxygen atom or a sulfur atom;
L₇ indicates a seventh linker;
L₈ indicates an eighth linker;
B indicates a group containing a bioorthogonal functional group;
V indicates an oxygen atom or a sulfur atom;
A indicates an affinity substance comprising first and second affinity moieties each having an affinity to the constant region in a heavy chain of an antibody; and
the percent modification r of the immunoglobulin unit with the affinity substance is 65 to 135%. The definitions, examples, and preferred examples of the immunoglobulin unit represented by Ig, the atoms represented by W₁, W₂ and W₃, the seventh linker represented by L₇, the eighth linker represented by L₈, the group containing a bioorthogonal functional group represented by B, the atom represented by V, the affinity substance represented by A, and the average percent modification represented by r, and the antibody are as described above.

An antibody or a salt thereof comprising a structural unit represented by the formula (IIb-1) can be produced by reacting the compound or a salt thereof represented by the formula (Ib-1) with an antibody or a salt thereof comprising an immunoglobulin unit comprising two heavy chains and optionally two light chains.

The affinity substance-modified antibody or a salt thereof may further comprise an additional modified moiety. Various methods are known as methods for modifying antibodies. Thus, in the present invention, the affinity substance-modified antibody or a salt thereof can be modified to further comprise an additional modified moiety. The additional modified moiety may be introduced to a heavy chain or a light chain of an antibody, and preferably in a heavy chain of an antibody (particularly in the constant region in a heavy chain).

In a specific embodiment, the additional modified moiety may be an additional affinity substance comprising a third affinity moiety having an affinity to the constant region in a heavy chain of the antibody. The term "affinity moiety" in the term "third affinity moiety," and the term "affinity substance" in the term "additional affinity substance" are the same as those described above. The third affinity moiety may be the same as or different from the first and/or second affinity moieties, and is preferably different.

In a specific embodiment, the additional modified moiety comprising a third affinity moiety having an affinity to the constant region in a heavy chain of an antibody may be introduced to the constant regions in said two heavy chains via modification of the amino group in the side chain of a lysine residue present at one or more positions in the constant regions in said two heavy chains. The affinity substance-modified antibody or a salt thereof may comprise an additional modified moiety via modification of the amino group in the side chain of one or more (preferably one or two, and more preferably one) lysine residue in the constant regions (preferably the Fc regions or the CH2 domains) in the two heavy chains of the constituent unit (the immunoglobulin unit comprising two heavy chains and optionally two light chains) of an antibody. More specifically, the position of the one or more (preferably one or two, more preferably one) lysine residue may be the position 246/248, 288/290, or 317 in the human IgG heavy chain according to the EU numbering (see, for example, WO2016/186206, WO2018/199337, WO2019/240287, WO2019/240288, WO2020/009165, and WO2020/090979). The position at which the additional modified moiety comprising a third affinity moiety having an affinity to the constant region in a heavy chain of an antibody is preferably different from the position at which the affinity substance comprising first and second affinity moieties each having an affinity to the constant region in a heavy chain of the antibody. For example, when the position at which the affinity substance is introduced is the lysine residue at the position 246/248, the position at which the additional modified moiety is introduced is preferably the lysine residue at the position 288/290, or 317, and more preferably the lysine residue at the position 288/290. When the position at which the affinity substance is introduced is the lysine residue at the position 288/290, the position at which the additional modified moiety is introduced is preferably the lysine residue at the position 246/248, or 317, and more preferably the lysine residue at the position 246/248. When the position at which the affinity substance is introduced is the lysine residue at the position 317, the position at which the additional modified moiety is introduced is preferably the lysine residue at the position 246/248 or 288/290.

### 4-2. Affinity substance-modified antibody or a salt thereof, comprising at least two affinity substances (comprising first, second, third, and fourth affinity moieties)

The present invention also provides an affinity substance-modified antibody or a salt thereof comprising first and second modified moieties, wherein the first modified moiety contains a first affinity substance comprising first and second affinity moieties each having an affinity to the constant region in a heavy chain of an antibody; the second modified moiety contains a second affinity substance comprising third and fourth affinity moieties each having an affinity to the constant region in a heavy chain of the antibody; and the first and second modified moieties are contained in the constant region in a heavy chain of the antibody. The definitions, examples, and preferred examples of the affinity substance, the antibody, and the components constituting them (e.g., the affinity moiety, such as an affinity peptide, the linker between the affinity moieties, such as a peptide linker, and the constant region) are as described above. The first and second modified moieties may be the same or different from each other and are preferably different from each other.

Preferably, such an affinity substance-modified antibody or a salt thereof may comprise (a)a constituent unit (an immunoglobulin unit comprising two heavy chains and optionally two light chains) of an antibody, and (b) the first and second modified moieties, wherein (c) the first modified moiety is introduced to the constant region in the first heavy chain of the immunoglobulin unit, and (d) the second modified moiety is introduced to the constant region in the second heavy chain of the immunoglobulin unit. The definitions, examples, and preferred examples of the antibody, the immunoglobulin unit, and the affinity substance, and the components constituting them (e.g., the constant region) are as described above.

The affinity substance-modified antibody or a salt thereof can comprise the first modified moiety comprising a first affinity substance and the second modified moiety comprising a second affinity substance via modification of the functional group in the side chain of any one or two or more (e.g., 2, 3, or 4) of 14 amino acid residues consisting of asparagine, glutamine, methionine, proline, serine, threonine, tryptophan, tyrosine, aspartic acid, glutamic acid, arginine, histidine, and lysine present in the constant region (preferably the Fc region or the CH2 domain). The affinity substance-modified antibody or a salt thereof can preferably comprise the first modified moiety comprising a first affinity substance and the second modified moiety comprising the second affinity substance via modification of the functional group in the side chain of any one of lysine, tyrosine, tryptophan, or cysteine present in the constant region (preferably the Fc region or the CH2 domain), more preferably via modification of the functional group in the side chain of any one of lysine, tyrosine, or tryptophan, even more preferably via modification of the functional group in the side chain of lysine or tyrosine, and particularly preferably via modification of the amino group in the side chain of lysine. The positions of these amino acid residues in the constant region are as described above. The position of modification in the antibody or a salt thereof by the affinity substance can be determined by peptide mapping. The modification may be regioselective as described above. Thus, the immunoglobulin unit in the formula described below may regioselectively have a corresponding modification unit via a functional group in the side chain of the amino acid residue described above.

Preferably, the affinity substance-modified antibody can comprise the first modified moiety comprising the first affinity substance via modification of the amino group in the side chain of one or more (preferably one or two, and more preferably one) lysine residue in the constant region (preferably the Fc region or the CH2 domain) in the first heavy chain of the constituent unit (the immunoglobulin unit comprising two heavy chains and optionally two light chains) of an antibody, and can comprise the second modified moiety comprising the second affinity substance via modification of the amino group in the side chain of one or more (preferably one or two, and more preferably one) lysine residue in the constant region (preferably the Fc region or the CH2 domain) in the second heavy chain. More specifically, the position of the one or more (preferably one or two, more preferably one) lysine residue may be the position 246/248, 288/290, or 317 in the human IgG heavy chain according to the EU numbering. The modification may be regioselective as described above. Thus, the immunoglobulin unit in the formula described below may regioselectively have a corresponding modification unit via the amino group in the side chain of the lysine residue described above.

The affinity substance-modified antibody or a salt thereof can be produced by:
(1) reacting the compound or a salt thereof of the present invention with an antibody comprising an immunoglobulin unit comprising two heavy chains and optionally two light chains to produce an affinity substance-modified antibody or a salt thereof containing a first modified moiety in which the first affinity substance is contained in the constant region in a heavy chain of the immunoglobulin unit; and
(2) reacting the affinity substance-modified antibody or a salt thereof comprising the first affinity substance in the constant region in a heavy chain of the immunoglobulin unit with the compound or a salt thereof of the present invention to produce an affinity substance-modified antibody or a salt thereof containing the first and second modified moieties in the constant region of a heavy chain of the immunoglobulin unit.

The compound or a salt thereof of the present invention used in the step (1) and the compound or a salt thereof of the present invention used in the step (2) may be the same or different from each other, and is preferably different from each other. The equivalence of the compound or salt thereof of the present invention to the antibody (the compound of the present invention or salt thereof/antibody) to the antibody in the reaction is not particularly limited, since it varies depending on factors such as the type of the compound of the present invention or salt thereof and antibody. It is, for example, 1 to 100, preferably 2 to 80, more preferably 4 to 60, even more preferably 5 to 40, and particularly preferably 6 to 20.

Such a reaction can be appropriately carried out under a condition that cannot cause protein denaturation/degradation (e.g., cleavage of amide bond) (mild conditions). For example, such a reaction under the mild conditions is the same as those described above.

In a specific embodiment, the affinity substance-modified antibody or a salt thereof may be an antibody or a salt thereof, comprising a structural unit represented by the following formula (V): 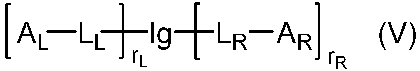 wherein
Ig indicates an immunoglobulin unit comprising two heavy chains composed of first and second heavy chains and optionally two light chains;
L_{L} and L_{R} each independently indicate a linker;
A_{L} indicates said first affinity substance;
A_{R} indicates said second affinity substance; and
the average percent modification r_{L} of said immunoglobulin unit with said first modified moiety and the average percent modification r_{R} of said immunoglobulin unit with said second modified moiety are individually 65 to 135%.

**In** a specific embodiment, the affinity substance-modified antibody or a salt thereof may further comprise (iii') a first cleavable moiety between (i') the first affinity substance and (ii') the immunoglobulin unit, and/or further comprise (iii") a second cleavable moiety between (i") the second affinity substance and (ii") the immunoglobulin unit. The first and second cleavable moieties are the same as the cleavable moiety. The first and second cleavable moieties may be the same or different from each other.

When the affinity substance-modified antibody or a salt thereof comprises a cleavable moiety, the cleavable moiety may be one that can be cleaved to generate a bioorthogonal functional group on the immunoglobulin unit side. Examples of such a cleavable moiety include disulfide residues, ester residues (including typical ester residues, and other ester residues described above, such as thioester residues), acetal residues (including typical ester residues, and other acetal residues such as thioacetal residues), ketal residues, imine residues, and vicinaldiol residues.

The affinity substance-modified antibody or a salt thereof, when comprising a cleavable moiety that can be cleaved to generate a bioorthogonal functional group on the immunoglobulin unit side, may be an antibody or a salt thereof comprising a structural unit represented by the following formula (Va): 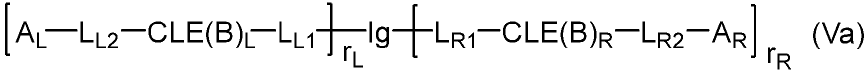 wherein
Ig indicates said immunoglobulin unit;
L_{L1} and L_{R1} each independently indicate a first linker;
L_{L2} and L_{R2} each independently indicate a second linker;
CLE(B)_{L} and CLE(B)_{R} each independently indicate a cleavable moiety that can be cleaved to generate a bioorthogonal functional group on said immunoglobulin unit side;
A_{L} indicates said first affinity substance;
A_{R} indicates said second affinity substance; and
the average percent modification r_{L} of said immunoglobulin unit with said first modified moiety and the average percent modification r_{R} of said immunoglobulin unit with said second modified moiety are individually 65 to 135%.

An antibody or a salt thereof comprising a structural unit represented by the formula (Va) can be produced by reacting the compound or a salt thereof represented by the formula (Ia) with an antibody or a salt thereof comprising an immunoglobulin unit comprising two heavy chains and optionally two light chains.

**In** a specific embodiment, the affinity substance-modified antibody or a salt thereof may be an antibody or a salt thereof, comprising a structural unit represented by the following formula (Va-1): 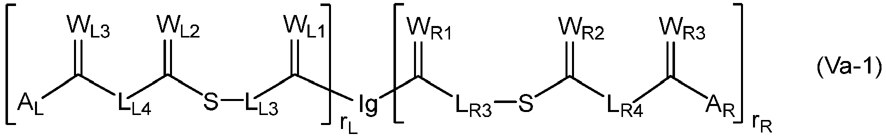 wherein
Ig indicates said immunoglobulin unit;
W_{L1}, W_{L2} and W_{L3}, and W_{R1}, W_{R2} and W_{R3} each independently indicate an oxygen atom or a sulfur atom;
L_{L3} and L_{R3} each independently indicate a third linker;
L_{L4} and L_{R4} each independently indicate a fourth linker;
S indicates a sulfur atom;
A_{L} indicates said first affinity substance;
A_{R} indicates said second affinity substance; and
the average percent modification r_{L} of said immunoglobulin unit with said first modified moiety and the average percent modification r_{R} of said immunoglobulin unit with said second modified moiety are individually 65 to 135%.

An antibody or a salt thereof comprising a structural unit represented by the formula (Va-1) can be produced by reacting the compound or a salt thereof represented by the formula (Ia-1) with an antibody or a salt thereof comprising an immunoglobulin unit comprising two heavy chains and optionally two light chains.

The affinity substance-modified antibody or a salt thereof may further comprise (iv') a first bioorthogonal functional group between (ii') the immunoglobulin unit and (iii') the first cleavable moiety, and/or further comprise (iv") a second bioorthogonal functional group between (ii") the immunoglobulin unit and (iii") the second cleavable moiety. The bioorthogonal functional group is as described above. Preferred examples of the bioorthogonal functional group include azide residues, alkyne residues (preferably, ring groups that may be substituted by a substituent as described above and have a carbon-carbon triple bond), tetrazine residues, alkene residues, thiol residues, maleimide residues, thiol residues, furan residues, and halocarbonyl residues.

The affinity substance-modified antibody or a salt thereof, when it further comprises a bioorthogonal functional group between the immunoglobulin unit and the cleavable moiety, may be an antibody or a salt thereof comprising a structural unit represented by the following formula (Vb): 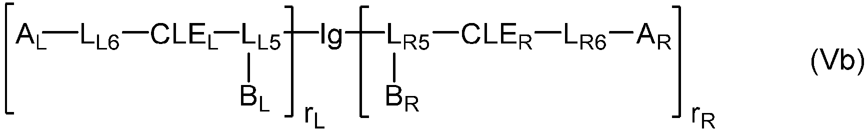 wherein
Ig indicates said immunoglobulin unit;
L_{L5} and L_{R5} each independently indicate a fifth linker;
L_{L6} and L_{R6} each independently indicate a sixth linker;
B_{L} indicates a first group comprising a first bioorthogonal functional group;
B_{R} indicates a second group comprising a second bioorthogonal functional group;
CLE_{L} and CLE_{R} each independently indicate a cleavable moiety;
A_{L} indicates said first affinity substance;
A_{R} indicates said second affinity substance; and
the average percent modification r_{L} of said immunoglobulin unit with said first modified moiety and the average percent modification r_{R} of said immunoglobulin unit with said second modified moiety are individually 65 to 135%.

An antibody or a salt thereof comprising a structural unit represented by the formula (Vb) can be produced by reacting the compound or a salt thereof represented by the formula (Ib) with an antibody or a salt thereof comprising an immunoglobulin unit comprising two heavy chains and optionally two light chains.

In a specific embodiment, the affinity substance-modified antibody or a salt thereof may be an antibody or a salt thereof, comprising a structural unit represented by the following formula (Vb-1): 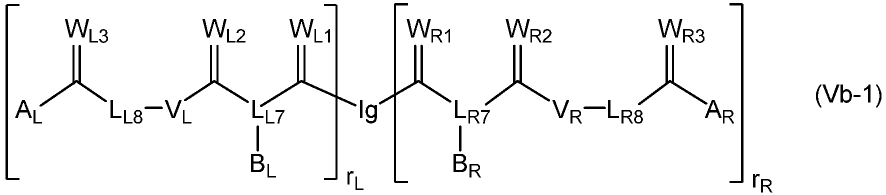 wherein
Ig indicates said immunoglobulin unit;
W_{L1}, W_{L2} and W_{L3}, and W_{R1}, W_{R2} and W_{R3} each independently indicate an oxygen atom or a sulfur atom;
L_{L7} and L_{R7} each independently indicate a seventh linker;
L_{L8} and L_{R8} each independently indicate an eighth linker;
B_{L} indicates a first group comprising a first bioorthogonal functional group;
B_{R} indicates a second group comprising a second bioorthogonal functional group;
V_{L} and V_{R} each independently indicate an oxygen atom or a sulfur atom;
A_{L} indicates said first affinity substance;
A_{R} indicates said second affinity substance; and
the average percent modification r_{L} of said immunoglobulin unit with said first modified moiety and the average percent modification r_{R} of said immunoglobulin unit with said second modified moiety are individually 65 to 135%.

An antibody or a salt thereof comprising a structural unit represented by the formula (Vb-1) can be produced by reacting the compound or a salt thereof represented by the formula (Ib-1) with an antibody or a salt thereof comprising an immunoglobulin unit comprising two heavy chains and optionally two light chains.

The affinity substance-modified antibody or a salt thereof may further comprise (iii') a first cleavable moiety between (i') the first affinity substance and (ii') the immunoglobulin unit, and further comprise (iv") a first bioorthogonal functional group between (ii") the immunoglobulin unit and (iii") the second cleavable moiety. In this case, the first cleavable moiety may be a cleavable moiety that can be cleaved to generate a second bioorthogonal functional group on the immunoglobulin unit side.

In a specific embodiment, the affinity substance-modified antibody or a salt thereof may be an antibody or a salt thereof, comprising a structural unit represented by the following formula (Vc): 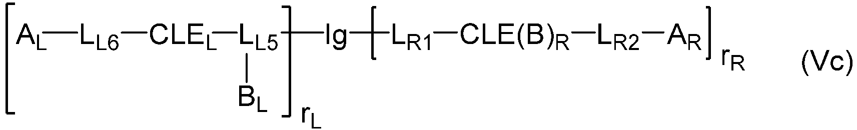 wherein
Ig indicates said immunoglobulin unit;
L_{R1} indicates a first linker;
L_{R2} indicates a second linker;
L_{L5} indicates a fifth linker;
L_{L6} indicates a sixth linker;
B_{L} indicates a group comprising a first bioorthogonal functional group;
CLE_{L} indicates a first cleavable moiety;
CLE(B)_{R} indicates a second cleavable moiety that can be cleaved to generate a second bioorthogonal functional group on said immunoglobulin unit side;
A_{L} indicates said first affinity substance;
A_{R} indicates said second affinity substance; and
the average percent modification r_{L} of said immunoglobulin unit with said first modified moiety and the average percent modification r_{R} of said immunoglobulin unit with said second modified moiety are individually 65 to 135%.

An antibody or a salt thereof comprising a structural unit represented by the formula (Vc) can be produced by reacting the compound or a salt thereof represented by the formula (Ia) and the compound or a salt thereof represented by the formula (Ib) with an antibody or a salt thereof comprising an immunoglobulin unit comprising two heavy chains and optionally two light chains.

In a specific embodiment, the affinity substance-modified antibody or a salt thereof may be an antibody or a salt thereof, comprising a structural unit represented by the following formula (Vc-1): 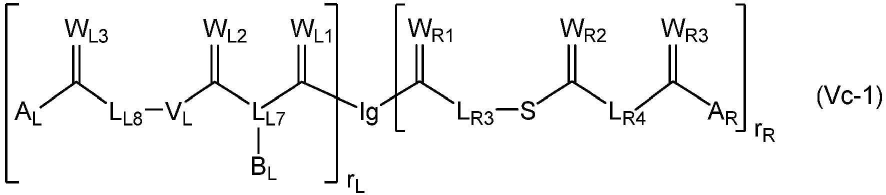 wherein
Ig indicates said immunoglobulin unit;
W_{L1}, W_{L2} and W_{L3}, and W_{R1}, W_{R2} and W_{R3} each independently indicate an oxygen atom or a sulfur atom;
L_{R3} each independently indicates a third linker;
L_{R4} each independently indicates a fourth linker;
L_{L7} each independently indicates a seventh linker;
L_{L8} each independently indicates an eighth linker;
B_{L} indicates a group comprising a first bioorthogonal functional group;
V_{L} indicates an oxygen atom or a sulfur atom;
A_{L} indicates said first affinity substance;
A_{R} indicates said second affinity substance; and
the average percent modification r_{L} of said immunoglobulin unit with said first modified moiety and the average percent modification r_{R} of said immunoglobulin unit with said second modified moiety are individually 65 to 135%.

An antibody or a salt thereof comprising a structural unit represented by the formula (Vc-1) can be produced by reacting the compound or a salt thereof represented by the formula (Ia-1) and a compound or a salt thereof represented by the formula (Ib-1) with an antibody or a salt thereof comprising an immunoglobulin unit comprising two heavy chains and optionally two light chains.

In the formulae (V), (Va), (Va-1), (Vb), (Vb-1), (Vc), and (Vc-1), the definitions, examples, and preferred examples of the immunoglobulin unit represented by Ig, and the antibody are as described above.

As used herein, the subscript "L" on any symbol is used for convenience for a symbol positioned on the Left side of the immunoglobulin unit (Ig). On the other hand, the subscript "R" on any symbol is used for convenience for a symbol positioned on the Right side of the immunoglobulin unit (Ig). The meanings of the symbols with the subscripts "L" and "R" are the same as the meanings of the symbols excluding the subscripts "L" and "R."

Therefore, in the formulae (V), (Va), (Va-1), (Vb), (Vb-1), (Vc), and (Vc-1), the symbols with the subscripts "L" and "R" are as described below.

W_{L1} and W_{R1} are the same as W₁, and are each preferably an oxygen atom.

W_{L2} and W_{R2} are the same as W₂, and are each preferably an oxygen atom.

W_{L3} and W_{R3} are the same as W₃, and are each preferably an oxygen atom.

The linkers represented by L_{L} and L_{R} are the same as the linker represented by L. The linkers represented by L_{L} and L_{R} may be the same or different from each other, and are preferably different from each other.

The first linkers represented by L_{L1} and L_{R1} are the same as the first linker represented by L₁. The first linkers represented by L_{L1} and L_{R1} may be the same or different from each other, and are preferably different from each other.

The second linkers represented by L_{L2} and L_{R2} are the same as the second linker represented by L₂. The second linkers represented by L_{L2} and L_{R2} may be the same or different from each other, and are preferably different from each other.

The third linkers represented by L_{L3} and L_{R3} are the same as the third linker represented by L₃. The third linkers represented by L_{L3} and L_{R3} may be the same or different from each other, and are preferably different from each other.

The fourth linkers represented by L_{L4} and L_{R4} are the same as the fourth linker represented by L₄. The fourth linkers represented by L_{L4} and L_{R4} may be the same or different from each other, and are preferably different from each other.

The fifth linkers represented by L_{L5} and L_{R5} are the same as the fifth linker represented by L₅. The first linkers represented by L_{L5} and L_{R5} may be the same or different from each other, and are preferably different from each other.

The sixth linkers represented by L_{L6} and L_{R6} are the same as the sixth linker represented by L₆. The sixth linkers represented by L_{L6} and L_{R6} may be the same or different from each other, and are preferably different from each other.

The seventh linkers represented by L_{L7} and L_{R7} are the same as the seventh linker represented by L₇. The seventh linkers represented by L_{L7} and L_{R7} may be the same or different from each other, and are preferably different from each other.

The eighth linkers represented by L_{L8} and L_{R8} are the same as the eighth linker represented by L₈. The eighth linkers represented by L_{L8} and L_{R8} may be the same or different from each other, and are preferably different from each other.

The cleavable moieties represented by CLE(B)_{L} and CLE(B)_{R} are the same as the cleavable moiety represented by CLE(B). The cleavable moieties represented by CLE(B)_{L} and CLE(B)_{R} may be the same or different from each other, and are preferably different from each other.

The first group comprising a first bioorthogonal functional group represented by B_{L} and the second group comprising a second bioorthogonal functional group represented by B_{R} are the same as the group containing a bioorthogonal functional group represented by B. The first group comprising a first bioorthogonal functional group represented by B_{L} and the second group comprising a second bioorthogonal functional group represented by B_{R} may be the same or different from each other, and are preferably different from each other.

The cleavable moieties represented by CLE_{L} and CLE_{R} are the same as the cleavable moiety represented by CLE. The cleavable moieties represented by CLE_{L} and CLE_{R} may be the same or different from each other, and are preferably different from each other.

The affinity substances represented by A_{L} and A_{R} are the same as the affinity substance represented by A. The affinity substances represented by A_{L} and A_{R} may be the same or different from each other.

The extents of the average percent modifications represented by r_{L} and r_{R}, and the method for determining them are the same as the average percent modification represented by r. The average percent modification represented by r_{L} and r_{R} may be the same or different from each other, and are preferably different from each other.

The determination of the formation of the affinity substance-modified antibody or a salt thereof of interest, which depends on its specific raw materials and the molecular weight of a product, can be performed by electrophoresis, chromatography (e.g., gel permutation chromatography, ion-exchange chromatography, reversed phase column chromatography, and HPLC), or mass spectrometry, for example. Determination of regioselectivity can be performed by peptide mapping. Peptide mapping can be performed by protease treatment and mass spectrometry, for example. For the protease, an endoprotease is preferred. Examples of such an endoprotease include trypsin, chymotrypsin, Glu-C, Lys-N, Lys-C, and Asp-N. The determination of the number of the introduced affinity substances can be performed by using mass spectrometry (DAR calculator (Agilent software)) in combination. The affinity substance-modified antibody or a salt thereof can be purified as appropriate by any method such as chromatography (e.g., chromatography described above and affinity chromatography).

The affinity substance-modified antibody or a salt thereof may further comprise an additional modified moiety. Various methods are known as methods for modifying antibodies. Thus, in the present invention, the affinity substance-modified antibody or a salt thereof can be modified to further comprise an additional modified moiety. The additional modified moiety may be introduced to a heavy chain or a light chain of an antibody, and preferably in a heavy chain of an antibody (particularly in the constant region in a heavy chain).

**In** a specific embodiment, the additional modified moiety may be an additional affinity substance comprising a fifth affinity moiety having an affinity to the constant region in a heavy chain of the antibody. The term "affinity moiety" in the term "fifth affinity moiety," and the term "affinity substance" in the term "additional affinity substance" are the same as those described above. The fifth affinity moiety may be the same as or different from the first, second, third and/or fourth affinity moieties, and is preferably different from them.

In a specific embodiment, the additional modified moiety comprising a fifth affinity moiety having an affinity to the constant region in a heavy chain of an antibody may be introduced to the constant regions in said two heavy chains via modification of the amino group in the side chain of a lysine residue present at one or more positions in the constant regions in said two heavy chains. The affinity substance-modified antibody or a salt thereof may comprise an additional modified moiety via modification of the amino group in the side chain of one or more (preferably one or two, and more preferably one) lysine residue in the constant regions (preferably the Fc regions or the CH2 domains) in the two heavy chains of the constituent unit (the immunoglobulin unit comprising two heavy chains and optionally two light chains) of an antibody. More specifically, the position of the one or more (preferably one or two, more preferably one) lysine residue may be the position 246/248, 288/290, or 317 in the human IgG heavy chain according to the EU numbering (see, for example, WO2016/186206, WO2018/199337, WO2019/240287, WO2019/240288, WO2020/009165, and WO2020/090979). The position at which the additional modified moiety comprising a fifth affinity moiety having an affinity to the constant region in a heavy chain of an antibody is preferably different from the positions at which the first modified moiety comprising the first affinity substance comprising the first and second affinity moieties each having an affinity to the constant region in a heavy chain of the antibody and the second modified moiety comprising the second affinity substance comprising the third and fourth affinity moieties each having an affinity to the constant region in a heavy chain of the antibody are introduced. For example, when the position at which both the first modified moiety and the second modified moiety are introduced is the lysine residue at the position 246/248, the position at which the additional modified moiety is introduced is preferably the lysine residue at the position 288/290, or 317, and more preferably the lysine residue at the position 288/290. When the position at which both the first modified moiety and the second modified moiety are introduced is the lysine residue at the position 288/290, the position at which the additional modified moiety is introduced is preferably the lysine residue at the position 246/248, or 317, and more preferably the lysine residue at the position 246/248. When the position at which both the first modified moiety and the second modified moiety are introduced is the lysine residue at the position 317, the position at which the additional modified moiety is introduced is preferably the lysine residue at the position 246/248 or 288/290.

### 5. Antibody or a salt thereof without affinity substance

### 5-1. Outline

The antibody or a salt thereof without affinity substance can be produced by using an affinity substance-modified antibody or a salt thereof, comprising (A) an affinity substance comprising first and second affinity moieties having an affinity to the constant region in a heavy chain of an antibody (immunoglobulin unit) and (B) an antibody (immunoglobulin unit), and further comprising (C) a cleavable moiety between (A) the affinity substance and (B) the antibody (immunoglobulin unit).

More specifically, the method of producing an antibody or a salt thereof without affinity substance may be the method 1-1 or 1-2 described below.

(Method 1-1) A method of producing an antibody or a salt thereof without affinity substance, the method comprising cleaving an affinity substance-modified antibody or a salt thereof comprising (A) an affinity substance comprising first and second affinity moieties each having an affinity to the constant region in a heavy chain of an antibody(immunoglobulin unit), and (B) an antibody (immunoglobulin unit), and further comprising (C) a cleavable moiety between (A) the affinity substance and (B) the antibody (immunoglobulin unit) at the cleavable moiety to produce an antibody or a salt thereof without affinity substance.
(Method 1-2) A method of producing an antibody or a salt thereof without affinity substance, the method comprising the following (1) and (2):
   (1) reacting a compound or a salt thereof comprising (A) an affinity substance comprising first and second affinity moieties each having an affinity to the constant region in a heavy chain of an antibody (immunoglobulin unit) and (B) a reactive group for the antibody (immunoglobulin unit), and further comprising (C) a cleavable moiety between (A) the affinity substance and (B) the reactive group with an antibody or a salt thereof comprising an immunoglobulin unit comprising two heavy chains and optionally two light chains to produce an affinity substance-modified antibody or a salt thereof comprising a cleavable moiety between the affinity substance and the antibody; and
   (2) cleaving the affinity substance-modified antibody or a salt thereof comprising a cleavable moiety between the affinity substance and the antibody at the cleavable moiety to produce an antibody or a salt thereof without affinity substance.

The cleavage treatment may be (a) treatment with one or more substance selected from the group consisting of an acidic substance, a basic substance, a reducing agent, an oxidizing agent, and an enzyme as described above, (b) treatment with physicochemical stimulation such as light, or (c) incubation using a cleavable linker comprising a self-degradable cleavable moiety. For the cleavage treatment, reference can also be made to WO2019/240287, WO2019/240288, WO2020/009165, and WO2020/090979.

Such a cleavage reaction can be appropriately carried out under a condition that cannot cause protein denaturation/degradation (e.g., cleavage of amide bond) (mild conditions). For example, such mild conditions are as described above. When the cleavable moiety is an ester (e.g., a typical ester, or another ester such as thioester), the cleavage reaction can be performed through incubation in a hydroxylamine hydrochloride solution (e.g., pH4.0 to 8.0, 10 mM to 10 M) for an appropriate time (e.g., 1 hour) (e.g., Vance, N. et al., Bioconjugate Chem. 2019, 30, 148-160).

The determination of the formation of the antibody or a salt thereof without affinity substance obtained in the cleavage reaction, which depends on its specific raw materials and the molecular weight of a product, can be performed by electrophoresis, chromatography (e.g., gel permutation chromatography, ion-exchange chromatography, reversed phase column chromatography, and HPLC), or mass spectrometry, for example. Determination of regioselectivity can be performed by peptide mapping as described above. The determination of the number of the introduced affinity substances can be performed by using mass spectrometry (DAR calculator (Agilent software)) in combination. The affinity substance-modified antibody or a salt thereof can be purified as appropriate by any method such as chromatography (e.g., chromatography described above and affinity chromatography).

By the way, when the affinity substance-modified antibody or a salt thereof comprising a cleavable moiety between (A) an affinity substance comprising first and second affinity moieties each having an affinity to the constant region in a heavy chain of an antibody (immunoglobulin unit) and (B) an antibody (immunoglobulin unit) comprises, as (a) the cleavable moiety, a cleavable moiety that can be cleaved to generate a bioorthogonal functional group on the antibody (immunoglobulin unit) side, or comprises (b) a bioorthogonal functional group between the antibody (immunoglobulin unit) and the cleavable moiety, an antibody derivative or a salt thereof comprising a bioorthogonal functional group can be produced as the antibody or a salt thereof without affinity substance.

An antibody derivative or a salt thereof comprising a bioorthogonal functional group can be reacted with a functional substance to produce a conjugate of an antibody and a functional substance, or a salt thereof as an antibody or a salt thereof without affinity substance.

Hereinafter, as antibodies or salts thereof without affinity substance, (1) antibody derivatives comprising a bioorthogonal functional group or salts thereof, and (2) conjugates of an antibody and a functional substance, or salts thereof will be described in detail.

### 5-2. Antibody derivative comprising at least one bioorthogonal functional group or a salt thereof

The present invention provides an antibody derivative or a salt thereof comprising a bioorthogonal functional group, comprising (a) a constituent unit (immunoglobulin unit comprising two heavy chains and optionally two light chains) of an antibody, and (b) a bioorthogonal functional group, wherein (c) the bioorthogonal functional group is introduced to the constant region in only one heavy chain of the immunoglobulin unit (that is, the bioorthogonal functional group is introduced to the constant region in one heavy chain of the immunoglobulin unit, while no bioorthogonal functional group is introduced to the constant region in the other heavy chain). The definitions, examples, and preferred examples of the antibody, the immunoglobulin unit, and the bioorthogonal functional group, and the components constituting them (e.g., the constant region) are as described above.

The antibody derivative or a salt thereof can comprise a bioorthogonal functional group via modification of the functional group in the side chain of any one or two or more (e.g., 2, 3, or 4) of 14 amino acid residues consisting of asparagine, glutamine, methionine, proline, serine, threonine, tryptophan, tyrosine, aspartic acid, glutamic acid, arginine, histidine, and lysine present in the constant region (preferably the Fc region or the CH2 domain). The antibody derivative or a salt thereof can preferably comprise a bioorthogonal functional group via modification of the functional group in the side chain of any one of lysine, tyrosine, tryptophan, or cysteine present in the constant region (preferably the Fc region or the CH2 domain), more preferably via modification of the functional group in the side chain of any one of lysine, tyrosine, or tryptophan, even more preferably via modification of the functional group in the side chain of lysine or tyrosine, and particularly preferably via modification of the amino group in the side chain of lysine. The positions of these amino acid residues in the constant region are as described above. The position of modification in the antibody or a salt thereof by the bioorthogonal functional group can be determined by peptide mapping. The modification may be regioselective as described above. Thus, the immunoglobulin unit in the formulae (IIIa), (IIIa-1), (IIIb), and (IIIb-1) described below may regioselectively have a corresponding modification unit via a functional group in the side chain of the amino acid residue described above.

Preferably, the antibody derivative can comprise the bioorthogonal functional group via modification of the amino group in the side chain of one or more (preferably one or two, more preferably one) lysine residue in the constant region (preferably the Fc region or the CH2 domain) in one heavy chain of the constituent unit (the immunoglobulin unit comprising two heavy chains and optionally two light chains) of an antibody (in other words, comprises the bioorthogonal functional group via the amino group in the side chain of a lysine residue in the constant region in one heavy chain of the immunoglobulin unit, but does not comprise the bioorthogonal functional group via the amino group in the side chain of a lysine residue in the constant region in the other heavy chain). More specifically, the position of one or more (preferably one or two, more preferably one) lysine residue may be the position 246/248, 288/290, or 317 in the human IgG heavy chain according to the EU numbering (see, for example, WO2016/186206, WO2018/199337, WO2019/240287, WO2019/240288, and WO2020/090979). The modification may be regioselective as described above. Thus, the immunoglobulin unit in the formulae (IIIa), (IIIa-1), (IIIb), and (IIIb-1) described below may regioselectively have a corresponding modification unit via the amino group in the side chain of the lysine residue described above.

In a specific embodiment, the antibody derivative or a salt thereof may be an antibody or a salt thereof comprising a structural unit represented by the following formula (IIIa): 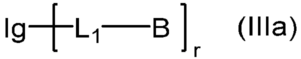 wherein
Ig indicates an immunoglobulin unit comprising two heavy chains and optionally two light chains;
L₁ indicates a first linker;
B indicates a group containing a bioorthogonal functional group; and
the average percent modification r of the immunoglobulin unit with the bioorthogonal functional group is 65 to 135%.
The definitions, examples, and preferred examples of the immunoglobulin unit represented by Ig, the first linker represented by L₁, the group containing a bioorthogonal functional group represented by B, and the average percent modification represented by r, and the antibody are as described above. A particularly preferred bioorthogonal functional group is a thiol group.

In the formula (IIIa), the molecular weight of the partial structure represented by L₁-B may be 700 or less. When the molecular weight of the partial structure represented by L₁-B is 700 or less, the antibody derivative or a salt thereof having a bioorthogonal functional group has a very small ratio of the molecular weight of the partial structure to the molecular weight of the entire antibody and thus is relatively difficult to be purified based on the difference in the molecular weight. However, according to the present invention, which allows for a high degree of control of DAR, antibody derivatives exhibiting the desired DAR can be obtained with high purity without necessarily requiring purification based on the difference in the molecular weight. The molecular weight of the partial structure represented by L₁-B may be preferably 600 or less, more preferably 500 or less, even more preferably 400 or less, and particularly preferably 300 or less, 250 or less, 200 or less, or 100 or less.

In another specific embodiment, the antibody derivative or a salt thereof may be an antibody or a salt thereof comprising a structural unit represented by the following formula (IIIa-1): 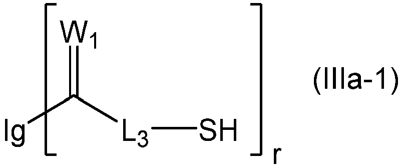 wherein
Ig indicates an immunoglobulin unit comprising two heavy chains and optionally two light chains;
W₁ indicates an oxygen atom or a sulfur atom;
L₃ indicates a third linker;
SH indicates a thiol group; and
the average percent modification r of the immunoglobulin unit with the bioorthogonal functional group is 65 to 135%. The definitions, examples, and preferred examples of the immunoglobulin unit represented by Ig, the atom represented by W₁, the third linker represented by L₃, and the average percent modification represented by r, and the antibody are as described above.

In the formula (IIIa-1), the molecular weight of the partial structure represented by C(=W₁)-L₃-SH may be 700 or less. The molecular weight of the partial structure represented by C(=W₁)-L₃-SH may be preferably 600 or less, more preferably 500 or less, even more preferably 400 or less, and particularly preferably 300 or less, 250 or less, 200 or less, 150 or less, or 100 or less.

In the formula (IIIa-1), the third linker represented by L₃ may be (CH₂)ₙ₁. n1 is an integer of 1 to 10. Preferably, n1 may be an integer of 2 or more. n1 may also be an integer of 9 or less, 8 or less, 7 or less, 6 or less, 5 or less, 4 or less, 3 or less, or 2 or less. Particularly preferably, n1 is 2.

In still another specific embodiment, the antibody derivative or a salt thereof may be an antibody or a salt thereof comprising a structural unit represented by the following formula (IIIb): 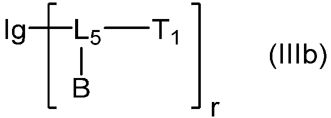 wherein
Ig indicates an immunoglobulin unit comprising two heavy chains and optionally two light chains;
L₅ indicates a fifth linker;
B indicates a group containing a bioorthogonal functional group;
T₁ indicates a monovalent group; and
the average percent modification r of the immunoglobulin unit with the bioorthogonal functional group is 65 to 135%. The definitions, examples, and preferred examples of the immunoglobulin unit represented by Ig, the fifth linker represented by L₅, the group containing a bioorthogonal functional group represented by B, and the average percent modification represented by r, and the antibody are as described above. A particularly preferred bioorthogonal functional group is an azide group.

T₁ is a monovalent group, which can be generated by cleavage of the cleavable moiety. The monovalent group may be substituted or unsubstituted. Examples of the monovalent group include those described above. Examples of the substituent when the monovalent group is substituted include those described above.

In a specific embodiment, the monovalent group represented by T₁ may be a hydroxyamino group that may be substituted. The hydroxyamino group that may be substituted can be represented by the following formula (α):

NRᵢ-ORᵢᵢ (α)

wherein
Rᵢ and Rᵢᵢ each independently indicate a hydrogen atom or a monovalent hydrocarbon group.

Here, the monovalent hydrocarbon group may be substituted or unsubstituted. The definitions, examples, and preferred examples of the monovalent hydrocarbon group, and the substituent when the monovalent hydrocarbon group is substituted are as described above. Preferably, the hydroxyamino group that may be substituted may be NH-ORᵢᵢ (where Rᵢᵢ indicates an alkyl group). More preferably, the hydroxyamino group that may be substituted may be NH-ORᵢᵢ (where Rᵢᵢ indicates a C1-6 alkyl group).

In the formula (IIIb), the molecular weight of the partial structure represented by L₅(-B)-T₁ may be 700 or less. The molecular weight of the partial structure represented by L₅(-B)-T₁ may be preferably 600 or less, more preferably 500 or less, even more preferably 400 or less, and particularly preferably 300 or less, 250 or less, 200 or less, or 100 or less.

In still another specific embodiment, the antibody derivative or a salt thereof may be an antibody or a salt thereof comprising a structural unit represented by the following formula (IIIb-1): 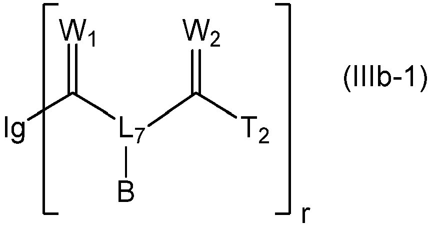 wherein
Ig indicates an immunoglobulin unit comprising two heavy chains and optionally two light chains;
W₁ and W₂ each independently indicate an oxygen atom or a sulfur atom;
L₇ indicates a seventh linker;
B indicates a group containing a bioorthogonal functional group;
T₂ indicates a monovalent group; and
the percent modification r of the immunoglobulin unit with the bioorthogonal functional group is 65 to 135%. The definitions, examples, and preferred examples of the immunoglobulin unit represented by Ig, the atom represented by W₁ and W₂, the seventh linker represented by L₇, the group containing a bioorthogonal functional group represented by B, and the average percent modification represented by r, and the antibody are as described above. A particularly preferred bioorthogonal functional group is an azide group.

T₂ is a monovalent group, which can be generated by cleavage of the cleavable moiety. The monovalent group may be substituted or unsubstituted. Examples of the monovalent group include those described above. Examples of the substituent when the monovalent group is substituted include those described above. The monovalent group represented by T₂ may be a hydroxyamino group that may be substituted. The details of the hydroxyamino group that may be substituted are the same as described for T₁.

In the formula (IIIb-1), the molecular weight of the partial structure represented by C(=W₁)-L₇(-B)-C(=W₂)-T₂ may be 700 or less. The molecular weight of the partial structure represented by C(=W₁)-L₇(-B)-C(=W₂)-T₂ may be preferably 600 or less, more preferably 500 or less, even more preferably 400 or less, and particularly preferably 300 or less, 250 or less, 200 or less, or 100 or less.

In the formula (IIIb-1), the seventh linker represented by L₇ may be (CH₂)ₙ₂. n2 is an integer of 1 to 10. Preferably, n2 may be an integer of 2 or more or 3 or more. n2 may also be an integer of 9 or less, 8 or less, 7 or less, 6 or less, 5 or less, 4 or less, or 3 or less. Particularly preferably, n2 may be 3.

In the formula (IIIb-1), a group comprising a bioorthogonal functional group may be NH-C(=O)-(CH₂)ₙ₃-N₃. n3 is an integer of 1 to 10. Preferably, n3 may be an integer of 2 or more, or 3 or more, or 4 or more. n3 may also be an integer of 9 or less, 8 or less, 7 or less, 6 or less, 5 or less, or 4 or less. Particularly preferably, n3 may be 4.

The antibody derivative or a salt thereof comprising a bioorthogonal functional group can be produced by using an affinity substance-modified antibody or a salt thereof, comprising (A) an affinity substance comprising first and second affinity moieties having an affinity to the constant region in a heavy chain of an antibody (immunoglobulin unit) and (B) an antibody (immunoglobulin unit), and further comprising (C) a cleavable moiety between (A) the affinity substance and (B) the antibody (immunoglobulin unit).

In a specific embodiment, inclusion of (a) a cleavable moiety that can be cleaved to generate a bioorthogonal functional group on the antibody (immunoglobulin unit) side in the affinity substance-modified antibody or a salt thereof enables production of an antibody derivative or a salt thereof comprising a bioorthogonal functional group.

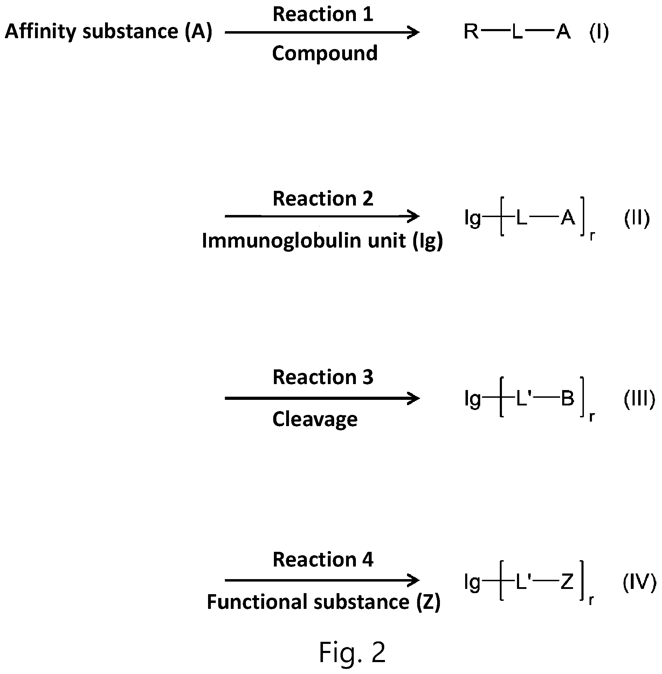

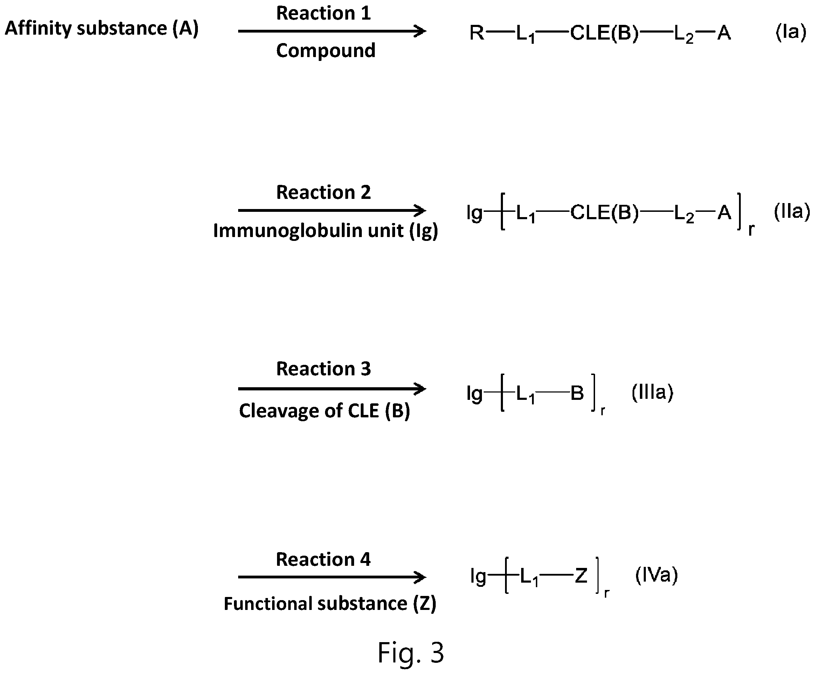

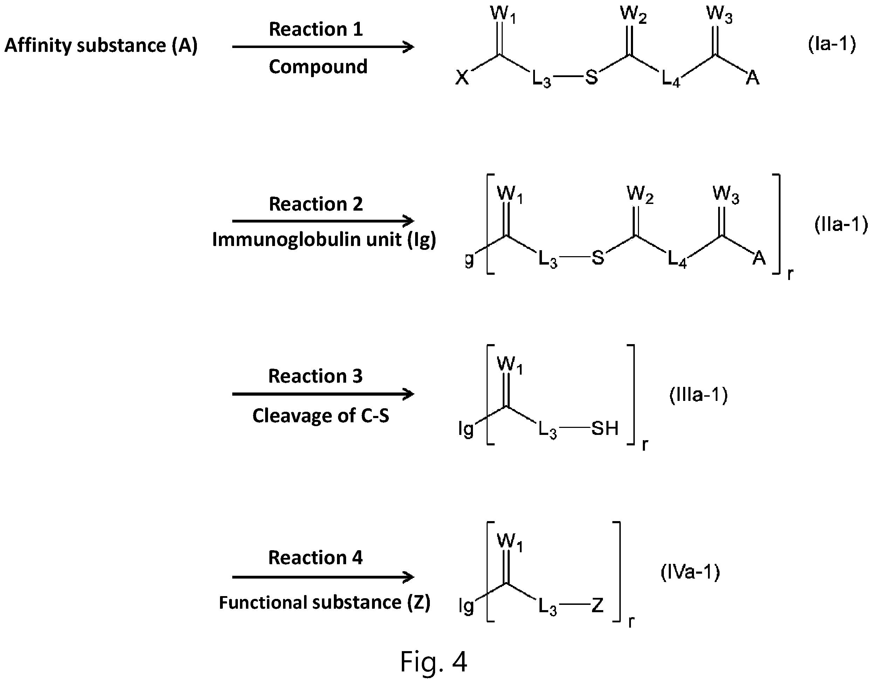

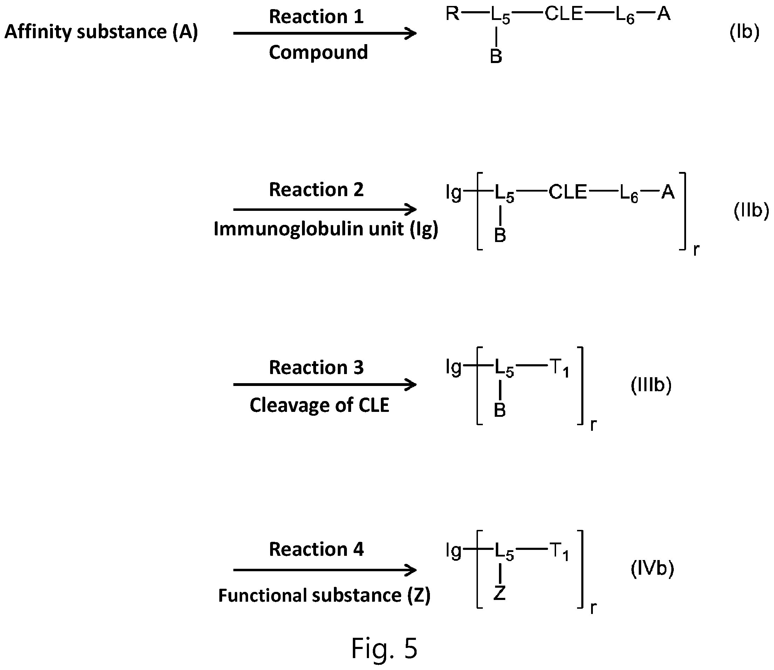

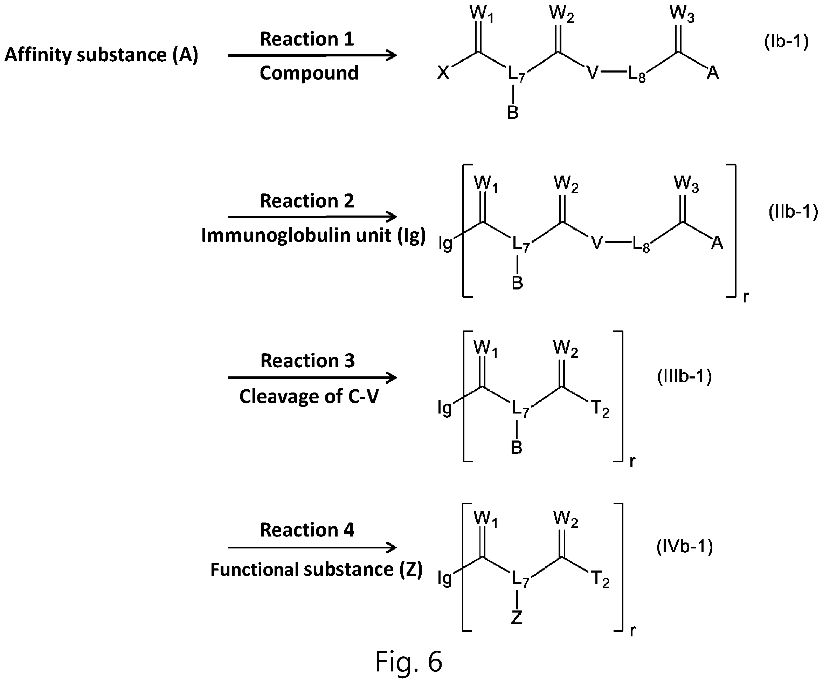

More specifically, examples of such a production method include (2-1) to (2-6) described below (FIGS. 2 to 6).

(Method 2-1) A method of producing an antibody derivative or a salt thereof comprising a bioorthogonal functional group, the method comprising cleaving an affinity substance-modified antibody or a salt thereof comprising (A) an affinity substance comprising first and second affinity moieties each having an affinity to the constant region in a heavy chain of an antibody (immunoglobulin unit), and (B) an antibody (immunoglobulin unit), and further comprising (C) a cleavable moiety between (A) the affinity substance and (B) the antibody (immunoglobulin unit) (wherein the cleavable moiety is a cleavable moiety that can be cleaved to generate a bioorthogonal functional group on the antibody (immunoglobulin unit) side) at the cleavable moiety to produce an antibody derivative or a salt thereof comprising a bioorthogonal functional group.
(Method 2-2) A method of producing an antibody derivative or a salt thereof comprising a bioorthogonal functional group, the method comprising the following (1) and (2):
   (1) reacting a compound or a salt thereof comprising (A) an affinity substance comprising first and second affinity moieties each having an affinity to the constant region in a heavy chain of an antibody (immunoglobulin unit), and (B) a reactive group for an antibody (immunoglobulin unit), and further comprising (C) a cleavable moiety between (A) the affinity substance and (B) the reactive group (wherein the cleavable moiety is a cleavable moiety that can be cleaved to generate a bioorthogonal functional group on the reactive group for an antibody (immunoglobulin unit)) with an antibody or a salt thereof comprising an immunoglobulin unit comprising two heavy chains and optionally two light chains to produce an affinity substance-modified antibody or a salt thereof comprising a cleavable moiety between the affinity substance and the antibody (wherein the cleavable moiety is a cleavable moiety that can be cleaved to generate a bioorthogonal functional group on the antibody side); and
   (2) cleaving the affinity substance-modified antibody or a salt thereof comprising a cleavable moiety between the affinity substance and the antibody at the cleavable moiety to produce an antibody derivative or a salt thereof comprising a bioorthogonal functional group.
(Method 2-3) A method of producing an antibody derivative or a salt thereof comprising a bioorthogonal functional group, the method comprising cleaving an antibody or a salt thereof comprising a structural unit represented by the formula (IIa) at the cleavable moiety to produce an antibody derivative or a salt thereof comprising a structural unit represented by the formula (IIIa).
(Method 2-4) A method of producing an antibody derivative or a salt thereof comprising a bioorthogonal functional group, the method comprising the following (1) and (2):
   (1) reacting the compound or a salt thereof represented by the formula (Ia) with an antibody or a salt thereof comprising an immunoglobulin unit comprising two heavy chains and optionally two light chains to produce an antibody or a salt thereof comprising a structural unit represented by the formula (IIa); and
   (2) cleaving the antibody or a salt thereof comprising a structural unit represented by the formula (IIa) at the cleavable moiety to produce an antibody derivative or a salt thereof comprising a structural unit represented by the formula (IIIa).
(Method 2-5) A method of producing an antibody derivative or a salt thereof comprising a bioorthogonal functional group, the method comprising cleaving an antibody or a salt thereof comprising a structural unit represented by the formula (IIa-1) at the cleavable moiety to produce an antibody derivative or a salt thereof comprising a structural unit represented by the formula (IIIa-1).
(Method 2-6) A method of producing an antibody derivative or a salt thereof comprising a bioorthogonal functional group, the method comprising the following (1) and (2):
   (1) reacting the compound or a salt thereof represented by the formula (Ia-1) with an antibody or a salt thereof comprising an immunoglobulin unit comprising two heavy chains and optionally two light chains to produce an antibody or a salt thereof comprising a structural unit represented by the formula (IIa-1); and
   (2) cleaving the antibody or a salt thereof comprising a structural unit represented by the formula (IIa-1) at the cleavable moiety to produce an antibody derivative or a salt thereof comprising a structural unit represented by the formula (IIIa-1).

The method 2-1 may be performed with the method 2-3 or 2-5. The method 2-2 may be performed with the method 2-4 or 2-6. The methods 2-2, 2-4, and 2-6 may further comprise reacting an affinity substance of the present invention with a moiety compound containing a reactive group for an antibody to produce a compound or a salt thereof of the present invention (FIGS 2 to 6).

In another specific embodiment, inclusion of (b) a bioorthogonal functional group between the antibody (immunoglobulin unit) and the cleavable moiety in the affinity substance-modified antibody or a salt thereof enables production of an antibody derivative or a salt thereof comprising a bioorthogonal functional group.

More specifically, examples of such a production method include (2-7) to (2-12) described below (FIGS. 2 to 6).

(Method 2-7) A method of producing an antibody derivative or a salt thereof comprising a bioorthogonal functional group, the method comprising cleaving an affinity substance-modified antibody or a salt thereof comprising (A) an affinity substance comprising first and second affinity moieties each having an affinity to the constant region in a heavy chain of an antibody (immunoglobulin unit), and (B) an antibody (immunoglobulin unit), and further comprising (C) a cleavable moiety between (A) the affinity substance and (B) the antibody (immunoglobulin unit), and (D) a bioorthogonal functional group between the antibody (immunoglobulin unit) and the cleavable moiety at the cleavable moiety to produce an antibody derivative or a salt thereof comprising a bioorthogonal functional group.
(Method 2-8) A method of producing an antibody derivative or a salt thereof comprising a bioorthogonal functional group, the method comprising the following (1) and (2):
   (1) reacting a compound or a salt thereof comprising (A) an affinity substance comprising first and second affinity moieties each having an affinity to the constant region in a heavy chain of an antibody (immunoglobulin unit) and (B) a reactive group for the antibody (immunoglobulin unit), and further comprising (C) a cleavable moiety between (A) the affinity substance and (B) the reactive group, and (D) a bioorthogonal functional group between the reactive group and the cleavable moiety with an antibody or a salt thereof comprising an immunoglobulin unit comprising two heavy chains and optionally two light chains to produce an affinity substance-modified antibody or a salt thereof comprising a cleavable moiety between the affinity substance and the antibody and (D) a bioorthogonal functional group between the antibody (immunoglobulin unit) and the cleavable moiety; and
   (2) cleaving the affinity substance-modified antibody or a salt thereof comprising a cleavable moiety between the affinity substance and the antibody at the cleavable moiety to produce an antibody derivative or a salt thereof comprising a bioorthogonal functional group.
(Method 2-9) A method of producing an antibody derivative or a salt thereof comprising a bioorthogonal functional group, the method comprising cleaving an antibody or a salt thereof comprising a structural unit represented by the formula (IIb) at the cleavable moiety to produce an antibody derivative or a salt thereof comprising a structural unit represented by the formula (IIIb).
(Method 2-10) A method of producing an antibody derivative or a salt thereof comprising a bioorthogonal functional group, the method comprising the following (1) and (2):
   (1) reacting the compound or a salt thereof represented by the formula (Ib) with an antibody or a salt thereof comprising an immunoglobulin unit comprising two heavy chains and optionally two light chains to produce an antibody or a salt thereof comprising a structural unit represented by the formula (IIb); and
   (2) cleaving the antibody or a salt thereof comprising a structural unit represented by the formula (IIb) at the cleavable moiety to produce an antibody derivative or a salt thereof comprising a structural unit represented by the formula (IIIb).
(Method 2-11) A method of producing an antibody derivative or a salt thereof comprising a bioorthogonal functional group, the method comprising cleaving an antibody or a salt thereof comprising a structural unit represented by the formula (IIb-1) at the cleavable moiety to produce an antibody derivative or a salt thereof comprising a structural unit represented by the formula (IIIb-1).
(Method 2-12) A method of producing an antibody derivative or a salt thereof comprising a bioorthogonal functional group, the method comprising the following (1) and (2):
   (1) reacting the compound or a salt thereof represented by the formula (Ib-1) with an antibody or a salt thereof comprising an immunoglobulin unit comprising two heavy chains and optionally two light chains to produce an antibody or a salt thereof comprising a structural unit represented by the formula (IIb-1); and
   (2) cleaving the antibody or a salt thereof comprising a structural unit represented by the formula (IIb-1) at the cleavable moiety to produce an antibody derivative or a salt thereof comprising a structural unit represented by the formula (IIIb-1).

The method 2-7 may be performed with the method 2-9 or 2-11. The method 2-8 may be performed with the method 2-10 or 2-11. The methods 2-8, 2-10, and 2-12 may further comprise reacting an affinity substance of the present invention with a moiety containing a reactive group for an antibody to produce a compound or a salt thereof of the present invention (FIGS 2 to 6).

The antibody derivative or a salt thereof may further comprise an additional modified moiety. Various methods are known as methods for modifying antibodies. Thus, in the present invention, the antibody derivative or a salt thereof can be modified to further comprise an additional modified moiety. The additional modified moiety may be introduced to a heavy chain or a light chain of an antibody, and preferably in a heavy chain of an antibody (particularly in the constant region in a heavy chain).

In a specific embodiment, the additional modified moiety may be an additional modified moiety comprising a bioorthogonal functional group. The bioorthogonal functional group is the same as described above. The bioorthogonal functional group contained in the additional modified moiety may be the same as or different from the bioorthogonal functional group of (b), and is preferably different.

In a specific embodiment, the additional modified moiety comprising a bioorthogonal functional group may be introduced to the constant regions in the two heavy chains via modification of the amino group in the side chain of a lysine residue present at one or more positions in the constant regions in said two heavy chains. The antibody derivative or a salt thereof may comprise an additional modified moiety via modification of the amino group in the side chain of one or more (preferably one or two, and more preferably one) lysine residue in the constant regions (preferably the Fc regions or the CH2 domains) in the two heavy chains of the constituent unit (the immunoglobulin unit comprising two heavy chains and optionally two light chains) of an antibody. More specifically, the position of the one or more (preferably one or two, more preferably one) lysine residue may be the position 246/248, 288/290, or 317 in the human IgG heavy chain according to the EU numbering (see, for example, WO2016/186206, WO2018/199337, WO2019/240287, WO2019/240288, WO2020/009165, and WO2020/090979). The position at which the additional modified moiety comprising a bioorthogonal functional group is introduced is preferably different from the position at which the bioorthogonal functional group of (b) is introduced. For example, when the position at which the bioorthogonal functional group of (b) is introduced is the lysine residue at the position 246/248, the position at which the additional modified moiety is introduced is preferably the lysine residue at the position 288/290, or 317, and more preferably the lysine residue at the position 288/290. When the position at which the bioorthogonal functional group of (b) is introduced is the lysine residue at the position 288/290, the position at which the additional modified moiety is introduced is preferably the lysine residue at the position 246/248, or 317, and more preferably the lysine residue at the position 246/248. When the position at which the bioorthogonal functional group of (b) is introduced is the lysine residue at the position 317, the position at which the additional modified moiety is introduced is preferably the lysine residue at the position 246/248 or 288/290.

### 5-3. Antibody derivative comprising at least two bioorthogonal functional group or a salt thereof

The present invention also provides an antibody derivative or a salt thereof comprising first and second modified moieties, comprising (a) an immunoglobulin unit comprising two heavy chains composed of first and second heavy chains and optionally two light chains, and (b) a first modified moiety comprising a first bioorthogonal functional group and a second modified moiety comprising a second bioorthogonal functional group;
(c) wherein the first modified moiety has been introduced to the constant region in said first heavy chain;
(d) wherein the second modified moiety has been introduced to the constant region in said second heavy chain; and
(e) wherein the first and second modified moieties are different. The definitions, examples, and preferred examples of the antibody, the immunoglobulin unit, and the bioorthogonal functional group, and the components constituting them (e.g., the constant region) are as described above.

The antibody derivative or a salt thereof can comprise the first modified moiety comprising a first bioorthogonal functional group and the second modified moiety comprising a second bioorthogonal functional group via modification of the functional group in the side chain of any one or two or more (e.g., 2, 3, or 4) of 14 amino acid residues consisting of asparagine, glutamine, methionine, proline, serine, threonine, tryptophan, tyrosine, aspartic acid, glutamic acid, arginine, histidine, and lysine present in the constant region (preferably the Fc region or the CH2 domain). The antibody derivative or a salt thereof can preferably comprise the first modified moiety comprising a first bioorthogonal functional group and the second modified moiety comprising a second bioorthogonal functional group via modification of the functional group in the side chain of any one of lysine, tyrosine, tryptophan, or cysteine present in the constant region (preferably the Fc region or the CH2 domain), more preferably via modification of the functional group in the side chain of any one of lysine, tyrosine, or tryptophan, even more preferably via modification of the functional group in the side chain of lysine or tyrosine, and particularly preferably via modification of the amino group in the side chain of lysine. The positions of these amino acid residues in the constant region are as described above. The position of modification in the antibody or a salt thereof by the bioorthogonal functional group can be determined by peptide mapping. The modification may be regioselective as described above. Thus, the immunoglobulin unit in the formula described below may regioselectively have a corresponding modification unit via a functional group in the side chain of the amino acid residue described above.

Preferably, the antibody derivative may comprise a first modified moiety comprising a first bioorthogonal functional group and a second modified moiety comprising a second bioorthogonal functional group each via modification of the amino group in the side chain of one or more (preferably one or two, and more preferably one) lysine residue in the constant region (preferably the Fc region or the CH2 domain) in one heavy chain of the constituent unit (the immunoglobulin unit comprising two heavy chains and optionally two light chains) of an antibody. More specifically, the position of the one or more (preferably one or two, more preferably one) lysine residue may be the position 246/248, 288/290, or 317 in the human IgG heavy chain according to the EU numbering. The modification may be regioselective as described above. Thus, the immunoglobulin unit in the formula described below may regioselectively have a corresponding modification unit via the amino group in the side chain of the lysine residue described above.

**In** a specific embodiment, the antibody derivative or a salt thereof may be an antibody or a salt thereof comprising a structural unit represented by the following formula (VIa): 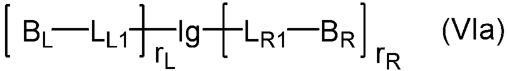 wherein
Ig indicates said immunoglobulin unit;
L_{L1} and L_{R1} each independently indicate a first linker;
B_{L} indicates a first group comprising a first bioorthogonal functional group;
B_{R} indicates a second group comprising a second bioorthogonal functional group; and
the average percent modification r_{L} of said immunoglobulin unit with the first modified moiety and the average percent modification r_{R} of said immunoglobulin unit with the second modified moiety are individually 65 to 135%.

The antibody or a salt thereof comprising a structural unit represented by the formula (VIa) can be produced by cleaving two cleavable moieties represented by CLE(B)_{L} and CLE(B)_{R} in an affinity substance-modified antibody or a salt thereof comprising a structural unit represented by the formula (Va). When the two cleavable moieties are the same, the cleavage reactions can be achieved by one cleavage reaction. When the two cleavable moieties are different from each other, the cleavage reactions can be achieved by one cleavage reaction (e.g., the case where the two different cleavable moieties can be cleaved with the same cleavage treatment or cleaving agent), or by two cleavage reactions (e.g., the case where the two different cleavable moieties can be cleaved with different cleavage treatments or cleaving agents). The details of the cleavage reaction are as described above.

In a specific embodiment, the antibody derivative or a salt thereof may be an antibody or a salt thereof comprising a structural unit represented by the following formula (VIa-1): 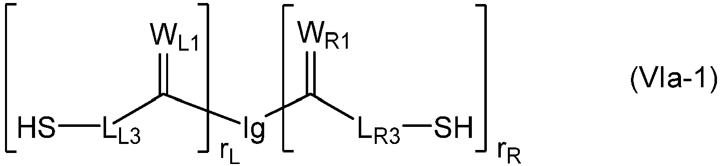 wherein
Ig indicates said immunoglobulin unit;
W_{L1} and W_{R1} each independently indicate an oxygen atom or a sulfur atom;
L_{L3} and L_{R3} each independently indicate a third linker;
SH indicates a thiol group that is a bioorthogonal functional group; and
the average percent modification r_{L} of said immunoglobulin unit with the first modified moiety and the average percent modification r_{R} of said immunoglobulin unit with the second modified moiety are individually 65 to 135%.

The antibody or a salt thereof comprising a structural unit represented by the formula (VIa-1) can be produced by cleaving two cleavable moieties represented by C-S in an affinity substance-modified antibody or a salt thereof comprising a structural unit represented by the formula (Va-1). The cleavage reactions can be achieved by one cleavage reaction. The details of the cleavage reaction are as described above.

In a specific embodiment, the antibody derivative or a salt thereof may be an antibody or a salt thereof comprising a structural unit represented by the following formula (VIb): 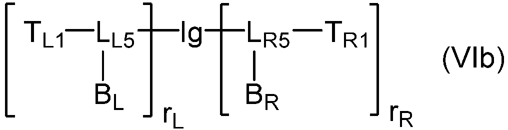 wherein
Ig indicates said immunoglobulin unit;
L_{L5} and L_{R5} each independently indicate a fifth linker;
B_{L} indicates a first group comprising a first bioorthogonal functional group;
B_{R} indicates a second group comprising a second bioorthogonal functional group;
T_{L1} and T_{R1} each independently indicate a monovalent group; and
the average percent modification r_{L} of said immunoglobulin unit with the first modified moiety and the average percent modification r_{R} of said immunoglobulin unit with the second modified moiety are individually 65 to 135%.

The antibody or a salt thereof comprising a structural unit represented by the formula (VIb) can be produced by cleaving two cleavable moieties represented by CLE_{L} and CLE_{R} in an affinity substance-modified antibody or a salt thereof comprising a structural unit represented by the formula (Vb). When the two cleavable moieties are the same, the cleavage reactions can be achieved by one cleavage reaction. When the two cleavable moieties are different from each other, the cleavage reactions can be achieved by one cleavage reaction (e.g., the case where the two different cleavable moieties can be cleaved with the same cleavage treatment or cleaving agent), or by two cleavage reactions (e.g., the case where the two different cleavable moieties can be cleaved with different cleavage treatments or cleaving agents). The details of the cleavage reaction are as described above.

In a specific embodiment, the antibody derivative or a salt thereof may be an antibody or a salt thereof comprising a structural unit represented by the following formula (VIb-1): 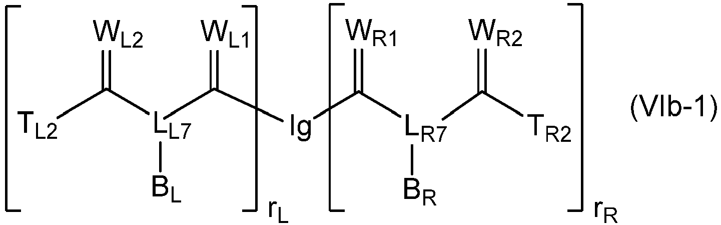 wherein
Ig indicates said immunoglobulin unit;
W_{L1} and W_{L2}, and W_{R1} and W_{R2} each independently indicate an oxygen atom or a sulfur atom;
L_{L7} and L_{R7} each independently indicate a seventh linker;
B_{L} indicates a first group comprising a first bioorthogonal functional group;
B_{R} indicates a second group comprising a second bioorthogonal functional group;
T_{L2} and T_{R2} each independently indicate a monovalent group; and
the average percent modification r_{L} of said immunoglobulin unit with the first modified moiety and the average percent modification r_{R} of said immunoglobulin unit with the second modified moiety are individually 65 to 135%.

The antibody or a salt thereof comprising a structural unit represented by the formula (VIb-1) can be produced by cleaving two cleavable moieties represented by C-V_{L} and C-V_{R} in an affinity substance-modified antibody or a salt thereof comprising a structural unit represented by the formula (Vb-1). When the two cleavable moieties are the same, the cleavage reactions can be achieved by one cleavage reaction. When the two cleavable moieties are different from each other, the cleavage reactions can be achieved by one cleavage reaction (e.g., the case where the two different cleavable moieties can be cleaved with the same cleavage treatment or cleaving agent), or by two cleavage reactions (e.g., the case where the two different cleavable moieties can be cleaved with different cleavage treatments or cleaving agents). The details of the cleavage reaction are as described above.

In a specific embodiment, the antibody derivative or a salt thereof may be an antibody or a salt thereof comprising a structural unit represented by the following formula (VIc): 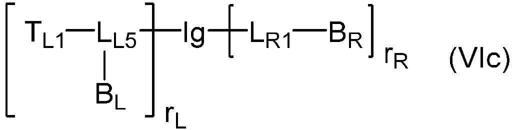 wherein
Ig indicates said immunoglobulin unit;
L_{R1} indicates a first linker;
L_{L5} indicates a fifth linker;
B_{L} indicates a first group comprising a first bioorthogonal functional group;
B_{R} indicates a second group comprising a second bioorthogonal functional group;
T_{L1} indicates a monovalent group; and
the average percent modification r_{L} of said immunoglobulin unit with the first modified moiety and the average percent modification r_{R} of said immunoglobulin unit with the second modified moiety are individually 65 to 135%.

The antibody or a salt thereof comprising a structural unit represented by the formula (VIc) can be produced by cleaving two cleavable moieties represented by CLE_{L} and CLE(B)_{R} in an affinity substance-modified antibody or a salt thereof comprising a structural unit represented by the formula (Vc). When the two cleavable moieties are the same, the cleavage reactions can be achieved by one cleavage reaction. When the two cleavable moieties are different from each other, the cleavage reactions can be achieved by one cleavage reaction (e.g., the case where the two different cleavable moieties can be cleaved with the same cleavage treatment or cleaving agent), or by two cleavage reactions (e.g., the case where the two different cleavable moieties can be cleaved with different cleavage treatments or cleaving agents). The details of the cleavage reaction are as described above.

In a specific embodiment, the antibody derivative or a salt thereof may be an antibody or a salt thereof comprising a structural unit represented by the following formula (VIc-1): 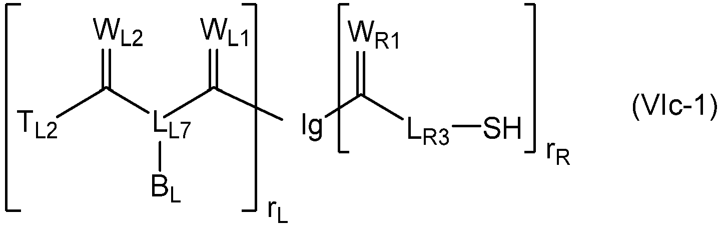 wherein
Ig indicates said immunoglobulin unit;
W_{L1} and W_{L2}, and W_{R1} each independently indicate an oxygen atom or a sulfur atom;
L_{R3} indicates a third linker;
L_{L7} indicates a seventh linker;
B_{L} indicates a first group comprising a first bioorthogonal functional group;
SH indicates a thiol group that is a second bioorthogonal functional group;
T_{L2} indicates a monovalent group; and
the average percent modification r_{L} of said immunoglobulin unit with the first modified moiety and the average percent modification r_{R} of said immunoglobulin unit with the second modified moiety are individually 65 to 135%.

The antibody or a salt thereof comprising a structural unit represented by the formula (VIc-1) can be produced by cleaving two cleavable moieties represented by C-V_{L} and C-S in an affinity substance-modified antibody or a salt thereof comprising a structural unit represented by the formula (Vc-1). When the two cleavable moieties are the same, the cleavage reactions can be achieved by one cleavage reaction. When the two cleavable moieties are different from each other, the cleavage reactions can be achieved by one cleavage reaction (e.g., the case where the two different cleavable moieties can be cleaved with the same cleavage treatment or cleaving agent), or by two cleavage reactions (e.g., the case where the two different cleavable moieties can be cleaved with different cleavage treatments or cleaving agents). The details of the cleavage reaction are as described above.

In the formulae (VIa), (VIa-1), (VIb), (VIb-1), (VIc), and (VIc-1), the definitions, examples, and preferred examples of the immunoglobulin unit represented by Ig, and the antibody are as described above.

In the formulae (VIa), (VIa-1), (VIb), (VIb-1), (VIc), and (VIc-1), the symbols with the subscripts "L" and "R" are as described below.

W_{L1} and W_{R1} are the same as W₁, and are each preferably an oxygen atom.

W_{L2} and W_{R2} are the same as W₂, and are each preferably an oxygen atom.

The first linkers represented by L_{L1} and L_{R1} are the same as the first linker represented by L₁. The first linkers represented by L_{L1} and L_{R1} may be the same or different from each other, and are preferably different from each other.

The third linkers represented by L_{L3} and L_{R3} are the same as the third linker represented by L₃. The third linkers represented by L_{L3} and L_{R3} may be the same or different from each other, and are preferably different from each other.

The fifth linkers represented by L_{L5} and L_{R5} are the same as the fifth linker represented by L₅. The first linkers represented by L_{L5} and L_{R5} may be the same or different from each other, and are preferably different from each other.

The seventh linkers represented by L_{L7} and L_{R7} are the same as the seventh linker represented by L₇. The seventh linkers represented by L_{L7} and L_{R7} may be the same or different from each other, and are preferably different from each other.

The first group comprising a first bioorthogonal functional group represented by B_{L} and the second group comprising a second bioorthogonal functional group represented by B_{R} are the same as the group containing a bioorthogonal functional group represented by B. The first group comprising a first bioorthogonal functional group represented by B_{L} and the second group comprising a second bioorthogonal functional group represented by B_{R} may be the same or different from each other, and are preferably different from each other.

The monovalent groups represented by T_{L1} and T_{R1} are the same as the monovalent group represented by T₁. The monovalent groups represented by T_{L1} and T_{R1} may the same or different from each other.

The monovalent groups represented by T_{L2} and T_{R2} are the same as the monovalent group represented by T₂. The monovalent groups represented by T_{L2} and T_{R2} may be the same or different from each other.

The extents of the average percent modifications represented by r_{L} and r_{R}, and the method for determining them are the same as the average percent modification represented by r. The average percent modification represented by r_{L} and r_{R} may be the same or different from each other, and are preferably different from each other.

In a specific embodiment, in the formulae (VIa) and (VIc), the molecular weight of the partial structure represented by L_{L1}-B_{L} and/or L_{R1}-B_{R} may be the same as the molecular weight of the partial structure represented by L₁-B in the formula (IIIa).

In a specific embodiment, in the formulae (VIa-1) and (VIc-1), the molecular weight of the partial structure represented by C(=W_{L1})-L_{L3}-SH and/or C(=W_{R1})-L_{R3}-SH may be the same as the molecular weight of the partial structure represented by C(=W₁)-L₃-SH in the formula (IIIa-1).

In a specific embodiment, in the formulae (VIa-1) and (VIc-1), the third linker represented by L_{L3} and/or L_{R3} may be the same as (CH₂)ₙ₁ that is the third linker represented by L₃ in the formula (IIIa-1).

In a specific embodiment, in the formula (VIb), (VIc), (VIb-1) and (VIc-1), the monovalent group represented by T_{L1}, T_{R1}, T_{L2}, and/or T_{R2} may be a hydroxyamino group that may be substituted, as represented by T₁ and/or T₂ in the formula (IIIb).

In a specific embodiment, in the formulae (VIb) and (VIc), the molecular weight of the partial structure represented by L_{L5}(-B_{L})-T_{L1} and/or L_{R5}(-B_{R})-T_{R1} may be the same as the molecular weight of the partial structure represented by L₅(-B)-T₁ in the formula (IIIb).

In a specific embodiment, in the formulae (VIb-1) and (VIc-1), the molecular weight of the partial structure represented by C(=W_{L1})-L_{L7}(-B_{L})-C(=W_{L2})-T_{L2} and/or C(=W_{R1})-L_{R7}(-B_{R})-C(=W_{R2})-T_{R2} may be the same as the molecular weight of the partial structure represented by C(=W₁)-L₇(-B)-C(=W₂)-T₂ in the formula (IIIb-1).

In a specific embodiment, in the formulae (VIb-1) and (VIc-1), the seventh linker represented by L_{L7} and/or L_{R7} may be the same as (CH₂)ₙ₂ that is the seventh linker represented by L₇ in the formula (IIIb-1).

In a specific embodiment, in the formulae (VIb-1) and (VIc-1), the first group comprising a first bioorthogonal functional group and/or the second group comprising a second bioorthogonal functional group may be NH-C(=O)-(CH₂)ₙ₃-N₃, as represented as the group comprising a bioorthogonal functional group in the formula (IIIb-1).

The antibody derivative or a salt thereof may further comprise an additional modified moiety. Various methods are known as methods for modifying antibodies. Thus, in the present invention, the antibody derivative or a salt thereof can be modified to further comprise an additional modified moiety. The additional modified moiety may be introduced to a heavy chain or a light chain of an antibody, and preferably in a heavy chain of an antibody (particularly in the constant region in a heavy chain).

In a specific embodiment, the additional modified moiety may be an additional modified moiety comprising a bioorthogonal functional group. The bioorthogonal functional group is the same as described above. The bioorthogonal functional group contained in the additional modified moiety may be the same as or different from the first bioorthogonal functional group and the second bioorthogonal functional group in (b), and is preferably different.

In a specific embodiment, the additional modified moiety comprising a bioorthogonal functional group may be introduced to the constant regions in the two heavy chains via modification of the amino group in the side chain of a lysine residue present at one or more positions in the constant regions in said two heavy chains. The antibody derivative or a salt thereof may comprise an additional modified moiety via modification of the amino group in the side chain of one or more (preferably one or two, and more preferably one) lysine residue in the constant regions (preferably the Fc regions or the CH2 domains) in the two heavy chains of the constituent unit (the immunoglobulin unit comprising two heavy chains and optionally two light chains) of an antibody. More specifically, the position of the one or more (preferably one or two, more preferably one) lysine residue may be the position 246/248, 288/290, or 317 in the human IgG heavy chain according to the EU numbering (see, for example, WO2016/186206, WO2018/199337, WO2019/240287, WO2019/240288, WO2020/009165, and WO2020/090979). The position at which the additional modified moiety comprising a bioorthogonal functional group is introduced is preferably different from the positions at which the first modified moiety comprising a first bioorthogonal functional group and the second modified moiety comprising a second bioorthogonal functional group in (b) are introduced. For example, when the position at which both the first modified moiety and the second modified moiety in (b) are introduced is the lysine residue at the position 246/248, the position at which the additional modified moiety is introduced is preferably the lysine residue at the position 288/290, or 317, and more preferably the lysine residue at the position 288/290. When the position at which both the first modified moiety and the second modified moiety in (b) are introduced is the lysine residue at the position 288/290, the position at which the additional modified moiety is introduced is preferably the lysine residue at the position 246/248, or 317, and more preferably the lysine residue at the position 246/248. When the position at which both the first modified moiety and the second modified moiety in (b) are introduced is the lysine residue at the position 317, the position at which the additional modified moiety is introduced is preferably the lysine residue at the position 246/248 or 288/290.

The determination of the formation of the antibody derivative or a salt thereof comprising a bioorthogonal functional group, which depends on its specific raw materials and the molecular weight of a product, can be performed by electrophoresis, chromatography (e.g., gel permutation chromatography, ion-exchange chromatography, reversed phase column chromatography, and HPLC), or mass spectrometry, for example. Determination of regioselectivity can be performed by peptide mapping as described above. The determination of the number of the bioorthogonal functional group can be performed by using mass spectrometry (DAR calculator (Agilent software)) in combination. The antibody derivative or a salt thereof comprising a bioorthogonal functional group can be purified as appropriate by any method such as chromatography (e.g., chromatography described above and affinity chromatography).

### 5-4. Conjugate of antibody and at least one functional substance or salt thereof

The present invention provides a conjugate of an antibody and a functional substance, or a salt thereof, comprising (a) a constituent unit (immunoglobulin unit comprising two heavy chains and optionally two light chains) of an antibody, and (b) a functional substance, and wherein (c) the functional substance is introduced to the constant region in only one heavy chain of the immunoglobulin unit (that is, a functional substance is introduced to the constant region in one heavy chain of the immunoglobulin unit, while no functional substance is introduced to the constant region of the other heavy chain). The definitions, examples, and preferred examples of the antibody, the immunoglobulin unit, and the conjugate of an antibody and a functional substance, and the components constituting them (e.g., constant region) are as described above.

The conjugate or a salt thereof can comprise a functional substance via modification of the functional group in the side chain of any one or two or more (e.g., 2, 3, or 4) of 14 amino acid residues consisting of asparagine, glutamine, methionine, proline, serine, threonine, tryptophan, tyrosine, aspartic acid, glutamic acid, arginine, histidine, and lysine present in the constant region (preferably the Fc region or the CH2 domain). The conjugate or a salt thereof can preferably comprise a functional substance via modification of the functional group in the side chain of any one of lysine, tyrosine, tryptophan, or cysteine present in the constant region (preferably the Fc region or the CH2 domain), more preferably via modification of the functional group in the side chain of any one of lysine, tyrosine, or tryptophan, even more preferably via modification of the functional group in the side chain of lysine or tyrosine, and particularly preferably via modification of the amino group in the side chain of lysine. The positions of these amino acid residues in the constant region are as described above. The position of modification in the antibody or a salt thereof by the functional substance can be determined by peptide mapping. The modification may be regioselective as described above. Thus, the immunoglobulin unit in the formulae (IVa), (IVa-1), (IVb), and (IVb-1) described below may regioselectively have a corresponding modification unit via a functional group in the side chain of the amino acid residue described above.

Preferably, the conjugate can comprise the functional substance via modification of the amino group in the side chain of one or more (preferably one or two, more preferably one) lysine residue in the constant region (preferably the Fc region or the CH2 domain) in one heavy chain of the constituent unit (the immunoglobulin unit comprising two heavy chains and optionally two light chains) of an antibody (in other words, comprises the functional substance via the amino group in the side chain of a lysine residue in the constant region in one heavy chain of the immunoglobulin unit, but does not comprise the functional substance via the amino group in the side chain of a lysine residue in the constant region in the other heavy chain). More specifically, the position of the one or more (preferably one or two, more preferably one) lysine residue may be the position 246/248, 288/290, or 317 in the human IgG heavy chain according to the EU numbering (see, for example, WO2016/186206, WO2018/199337, WO2019/240287, WO2019/240288, WO2020/009165, and WO2020/090979). The modification may be regioselective as described above. Thus, the immunoglobulin unit in the formulae (IVa), (IVa-1), (IVb), and (IVb-1) described below may regioselectively have a corresponding modification unit via the amino group in the side chain of the lysine residue described above.

In a specific embodiment, the conjugate or a salt thereof may be an antibody or a salt thereof comprising a structural unit represented by the following formula (IVa): 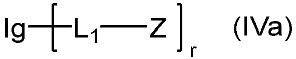 wherein
Ig indicates an immunoglobulin unit comprising two heavy chains and optionally two light chains;
L₁ indicates a first linker;
Z indicates a functional substance; and
the average percent modification r of the immunoglobulin unit with the functional substance is 65 to 135%. The definitions, examples, and preferred examples of the immunoglobulin unit represented by Ig, the first linker represented by L₁, the functional substance represented by Z, and the average percent modification represented by r, and the antibody are as described above. A particularly preferred bioorthogonal functional group is a thiol group.

In the formula (IVa), the molecular weight of the partial structure represented by L₁-Z may be 700 or less. When the molecular weight of the partial structure represented by L₁-Z is 700 or less, the conjugate or a salt thereof has a very small ratio of the molecular weight of the partial structure to the molecular weight of the entire antibody and thus is relatively difficult to be purified based on the difference in the molecular weight. However, according to the present invention, which allows for a high degree of control of DAR, conjugates exhibiting the desired DAR can be obtained with high purity without necessarily requiring purification based on the difference in the molecular weight. The molecular weight of the partial structure represented by L₁-Z may be preferably 600 or less, more preferably 500 or less, even more preferably 400 or less, and particularly preferably 300 or less, 250 or less, 200 or less, or 100 or less.

In another specific embodiment, the conjugate or a salt thereof may be an antibody or a salt thereof comprising a structural unit represented by the following formula (IVa-1): 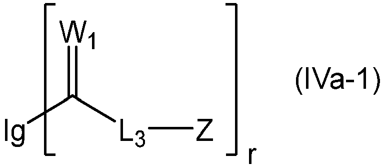 wherein
Ig indicates an immunoglobulin unit comprising two heavy chains and optionally two light chains;
W₁ indicates an oxygen atom or a sulfur atom;
L₃ indicates a third linker;
Z indicates a functional substance; and
the average percent modification r of the immunoglobulin unit with the functional substance is 65 to 135%. The definitions, examples, and preferred examples of the immunoglobulin unit represented by Ig, the atom represented by W₁, the third linker represented by L₃, the functional substance represented by Z, and the average percent modification represented by r, and the antibody are as described above.

In the formula (IVa-1), the molecular weight of the partial structure represented by C(=W₁)-L₃-Z may be 700 or less. The molecular weight of the partial structure represented by C(=W₁)-L₃-Z may be preferably 600 or less, more preferably 500 or less, even more preferably 400 or less, and particularly preferably 300 or less, 250 or less, 200 or less, 150 or less, or 100 or less.

In the formula (IVa-1), the partial structure represented by L₃ may be (CH₂)ₙ₁. The definition, examples, and preferred examples of n1 are as described above.

In still another specific embodiment, the conjugate or a salt thereof may be an antibody or a salt thereof comprising a structural unit represented by the following formula (IVb): 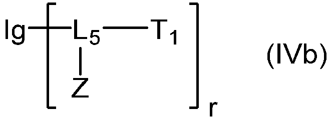 wherein
Ig indicates an immunoglobulin unit comprising two heavy chains and optionally two light chains;
L₅ indicates a fifth linker;
Z indicates a functional substance;
T₁ indicates a monovalent group; and
the average percent modification r of the immunoglobulin unit with the functional substance is 65 to 135%. The definitions, examples, and preferred examples of the immunoglobulin unit represented by Ig, the fifth linker represented by L₅, the functional substance represented by Z, the monovalent group represented by T₁, and the average percent modification represented by r, and the antibody are as described above. A particularly preferred bioorthogonal functional group is an azide group.

In the formula (IVb), the molecular weight of the partial structure represented by L₅(-Z)-T₁ may be 700 or less. The molecular weight of the partial structure represented by L₅(-Z)-T₁ may be preferably 600 or less, more preferably 500 or less, even more preferably 400 or less, and particularly preferably 300 or less, 250 or less, 200 or less, or 100 or less.

In still another specific embodiment, the conjugate or a salt thereof may be an antibody or a salt thereof comprising a structural unit represented by the following formula (IVb-1): 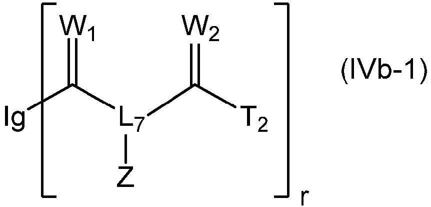 wherein
Ig indicates an immunoglobulin unit comprising two heavy chains and optionally two light chains;
W₁ and W₂ each independently indicate an oxygen atom or a sulfur atom;
L₇ indicates a seventh linker;
Z indicates a functional substance;
T₂ indicates a monovalent group; and
the percent modification r of the immunoglobulin unit with the functional substance is 65 to 135%. The definitions, examples, and preferred examples of the immunoglobulin unit represented by Ig, the atoms represented by W₁ and W₂, the seventh linker represented by L₇, the functional substance represented by Z, the monovalent group represented by T₂, and the average percent modification represented by r, and the antibody are as described above. A particularly preferred bioorthogonal functional group is an azide group.

In the formula (IVb-1), the molecular weight of the partial structure represented by C(=W₁)-L₇(-Z)-C(=W₂)-T₂ may be 700 or less. The molecular weight of the partial structure represented by C(=W₁)-L₇(-Z)-C(=W₂)-T₂ may be preferably 600 or less, more preferably 500 or less, even more preferably 400 or less, and particularly preferably 300 or less, 250 or less, 200 or less, or 100 or less.

In the formula (IVb-1), the partial structure represented by L₇ may be (CH₂)ₙ₂. The definition, examples, and preferred examples of n2 are as described above.

In the formula (IVb-1), a group comprising a bioorthogonal functional group may be NH-C(=O)-(CH₂)ₙ₃-N₃. The definition, examples, and preferred examples of n3 are as described above.

The method of producing a conjugate or a salt thereof may be performed by reacting an antibody derivative or a salt thereof comprising a bioorthogonal functional group with a functional substance to produce a conjugate or a salt thereof comprising the antibody and the functional substance.

Alternatively, the method of producing a conjugate or a salt thereof may be performed by a method comprising the following (1) and (2):
(1) producing an antibody derivative or a salt thereof comprising a bioorthogonal functional group according to the method described above; and
(2) reacting the antibody derivative or a salt thereof comprising a bioorthogonal functional group with a functional substance to produce a conjugate or a salt thereof comprising the antibody and the functional substance.

More specifically, examples of the method of producing a conjugate or a salt thereof include (3-1) to (3-12) described below (FIGS. 2 to 6).

(Method 3-1) A method of producing a conjugate of an antibody and a functional substance or a salt thereof, the method comprising reacting an antibody derivative or a salt thereof comprising a structural unit represented by the formula (IIIa) with a functional substance to produce a conjugate or a salt thereof comprising a structural unit represented by the formula (IVa).
(Method 3-2) A method of producing a conjugate of an antibody and a functional substance, or a salt thereof, the method comprising (1) and (2) below:
   (1) cleaving an antibody or a salt thereof comprising a structural unit represented by the formula (IIa) at the cleavable moiety to produce an antibody derivative or a salt thereof comprising a structural unit represented by the formula (IIIa); and
   (2) reacting the antibody derivative or a salt thereof comprising a structural unit represented by the formula (IIIa) with a functional substance to produce a conjugate or a salt thereof comprising a structural unit represented by the formula (IVa).
(Method 3-3) A method of producing a conjugate of an antibody and a functional substance, or a salt thereof, the method comprising (1) to (3) below:
   (1) reacting the compound or a salt thereof represented by the formula (Ia) with an antibody or a salt thereof comprising an immunoglobulin unit comprising two heavy chains and optionally two light chains to produce an antibody or a salt thereof comprising a structural unit represented by the formula (IIa); and
   (2) cleaving an antibody or a salt thereof comprising a structural unit represented by the formula (IIa) at the cleavable moiety to produce an antibody derivative or a salt thereof comprising a structural unit represented by the formula (IIIa); and
   (3) reacting the antibody derivative or a salt thereof comprising a structural unit represented by the formula (IIIa) with a functional substance to produce a conjugate or a salt thereof comprising a structural unit represented by the formula (IVa).
(Method 3-4) A method of producing a conjugate of an antibody and a functional substance or a salt thereof, the method comprising reacting an antibody derivative or a salt thereof comprising a structural unit represented by the formula (IIIa-1) with a functional substance to produce a conjugate or a salt thereof comprising a structural unit represented by the formula (IVa-1).
(Method 3-5) A method of producing a conjugate of an antibody and a functional substance, or a salt thereof, the method comprising (1) and (2) below:
   (1) the formula (IIa-1) at the cleavable moiety to produce an antibody derivative or a salt thereof comprising a structural unit represented by the formula (IIIa-1);
   (2) reacting the antibody derivative or a salt thereof comprising a structural unit represented by the formula (IIIa-1) with a functional substance to produce a conjugate or a salt thereof comprising a structural unit represented by the formula (IVa-1).
(Method 3-6) A method of producing a conjugate of an antibody and a functional substance, or a salt thereof, the method comprising (1) to (3) below:
   (1) reacting the compound or a salt thereof represented by the formula (Ia-1) with an antibody or a salt thereof comprising an immunoglobulin unit comprising two heavy chains and optionally two light chains to produce an antibody or a salt thereof comprising a structural unit represented by the formula (IIa-1);
   (2) cleaving the antibody or a salt thereof comprising a structural unit represented by the formula (IIa-1) at the cleavable moiety to produce an antibody derivative or a salt thereof comprising a structural unit represented by the formula (IIIa-1);
   (3) reacting the antibody derivative or a salt thereof comprising a structural unit represented by the formula (IIIa-1) with a functional substance to produce a conjugate or a salt thereof comprising a structural unit represented by the formula (IVa-1).
(Method 3-7) A method of producing a conjugate of an antibody and a functional substance or a salt thereof, the method comprising reacting an antibody derivative or a salt thereof comprising a structural unit represented by the formula (IIIb) with a functional substance to produce a conjugate or a salt thereof comprising a structural unit represented by the formula (IVb).
(Method 3-8) A method of producing a conjugate of an antibody and a functional substance, or a salt thereof, the method comprising (1) and (2) below:
   (1) cleaving an antibody or a salt thereof comprising a structural unit represented by the formula (IIb) at the cleavable moiety to produce an antibody derivative or a salt thereof comprising a structural unit represented by the formula (IIIb); and
   (2) reacting the antibody derivative or a salt thereof comprising a structural unit represented by the formula (IIIb) with a functional substance to produce a conjugate or a salt thereof comprising a structural unit represented by the formula (IVb).
(Method 3-9) A method of producing a conjugate of an antibody and a functional substance, or a salt thereof, the method comprising (1) to (3) below:
   (1) reacting the compound or a salt thereof represented by the formula (Ib) with an antibody or a salt thereof comprising an immunoglobulin unit comprising two heavy chains and optionally two light chains to produce an antibody or a salt thereof comprising a structural unit represented by the formula (IIb); and
   (2) cleaving the antibody or a salt thereof comprising a structural unit represented by the formula (IIb) at the cleavable moiety to produce an antibody derivative or a salt thereof comprising a structural unit represented by the formula (IIIb); and
   (3) reacting the antibody derivative or a salt thereof comprising a structural unit represented by the formula (IIIb) with a functional substance to produce a conjugate or a salt thereof comprising a structural unit represented by the formula (IVb).
(Method 3-10) A method of producing a conjugate of an antibody and a functional substance or a salt thereof, the method comprising reacting an antibody derivative or a salt thereof comprising a structural unit represented by the formula (IIIb-1) with a functional substance to produce a conjugate or a salt thereof comprising a structural unit represented by the formula (IVb-1).
(Method 3-11) A method of producing a conjugate of an antibody and a functional substance, or a salt thereof, the method comprising (1) and (2) below:
   (1) cleaving an antibody or a salt thereof comprising a structural unit represented by the formula (IIb-1) at the cleavable moiety to produce an antibody derivative or a salt thereof comprising a structural unit represented by the formula (IIIb-1);
   (2) reacting the antibody derivative or a salt thereof comprising a structural unit represented by the formula (IIIb-1) with a functional substance to produce a conjugate or a salt thereof comprising a structural unit represented by the formula (IVb-1).
(Method 3-12) A method of producing a conjugate of an antibody and a functional substance, or a salt thereof, the method comprising (1) to (3) below:
   (1) reacting the compound or a salt thereof represented by the formula (IB-1) with an antibody or a salt thereof comprising an immunoglobulin unit comprising two heavy chains and optionally two light chains to produce an antibody or a salt thereof comprising a structural unit represented by the formula (IIb-1); and
   (2) cleaving the antibody or a salt thereof comprising a structural unit represented by the formula (IIb-1) at the cleavable moiety to produce an antibody derivative or a salt thereof comprising a structural unit represented by the formula (IIIb-1); and
   (3) reacting the antibody derivative or a salt thereof comprising a structural unit represented by the formula (IIIb-1) with a functional substance to produce a conjugate or a salt thereof comprising a structural unit represented by the formula (IVb-1).

The methods 3-1 to 3-12 may further comprise reacting an affinity substance of the present invention with a moiety compound containing a reactive group for an antibody to produce a compound or a salt thereof of the present invention (FIGS 2 to 6).

The conjugate or a salt thereof may further comprise an additional modified moiety. Various methods are known as methods for modifying antibodies. Thus, in the present invention, the conjugate or a salt thereof can be modified to further comprise an additional modified moiety. The additional modified moiety may be introduced to a heavy chain or a light chain of an antibody, and preferably in a heavy chain of an antibody (particularly in the constant region in a heavy chain).

In a specific embodiment, the additional modified moiety may be an additional modified moiety comprising a functional substance. The functional substance is the same as described above. The functional substance contained in the additional modified moiety may be the same as or different from the functional substance of (b), and is preferably different.

In a specific embodiment, the additional modified moiety comprising a functional substance may be introduced to the constant regions in the two heavy chains via modification of the amino group in the side chain of a lysine residue present at one or more positions in the constant regions in said two heavy chains. The conjugate or a salt thereof may comprise an additional modified moiety via modification of the amino group in the side chain of one or more (preferably one or two, and more preferably one) lysine residue in the constant regions (preferably the Fc regions or the CH2 domains) in the two heavy chains of the constituent unit (the immunoglobulin unit comprising two heavy chains and optionally two light chains) of an antibody. More specifically, the position of the one or more (preferably one or two, more preferably one) lysine residue may be the position 246/248, 288/290, or 317 in the human IgG heavy chain according to the EU numbering (see, for example, WO2016/186206, WO2018/199337, WO2019/240287, WO2019/240288, WO2020/009165, and WO2020/090979). The position at which the additional modified moiety comprising a functional substance is introduced is preferably different from the position at which the functional substance of (b) is introduced. For example, when the position at which the functional substance of (b) is introduced is the lysine residue at the position 246/248, the position at which the additional modified moiety is introduced is preferably the lysine residue at the position 288/290, or 317, and more preferably the lysine residue at the position 288/290. When the position at which the functional substance of (b) is introduced is the lysine residue at the position 288/290, the position at which the additional modified moiety is introduced is preferably the lysine residue at the position 246/248, or 317, and more preferably the lysine residue at the position 246/248. When the position at which the functional substance of (b) is introduced is the lysine residue at the position 317, the position at which the additional modified moiety is introduced is preferably the lysine residue at the position 246/248 or 288/290.

### 5-5. Conjugate of antibody and at least two functional substances or salt thereof

The present invention also provides a conjugate of an antibody and first and second modified moieties, or a salt thereof, comprising (a) an immunoglobulin unit comprising two heavy chains composed of first and second heavy chains and optionally two light chains, and (b) a first modified moiety comprising a first functional substance and a second modified moiety comprising a second functional substance;
(c) wherein the first modified moiety has been introduced to the constant region in said first heavy chain;
(d) wherein the second modified moiety has been introduced to the constant region in said second heavy chain; and
(e) wherein the first and second modified moieties are different from each other. The definitions, examples, and preferred examples of the antibody, the immunoglobulin unit, and the bioorthogonal functional group, and the components constituting them (e.g., the constant region) are as described above.

The conjugate or a salt thereof can comprise the first modified moiety comprising a first functional substance and the second modified moiety comprising a second functional substance via modification of the functional group in the side chain of any one or two or more (e.g., 2, 3, or 4) of 14 amino acid residues consisting of asparagine, glutamine, methionine, proline, serine, threonine, tryptophan, tyrosine, aspartic acid, glutamic acid, arginine, histidine, and lysine present in the constant region (preferably the Fc region or the CH2 domain). The antibody derivative or a salt thereof can preferably comprise the first modified moiety comprising a first functional substance and the second modified moiety comprising the second functional substance via modification of the functional group in the side chain of any one of lysine, tyrosine, tryptophan, or cysteine present in the constant region (preferably the Fc region or the CH2 domain), more preferably via modification of the functional group in the side chain of any one of lysine, tyrosine, or tryptophan, even more preferably via modification of the functional group in the side chain of lysine or tyrosine, and particularly preferably via modification of the amino group in the side chain of lysine. The positions of these amino acid residues in the constant region are as described above. The position of modification in the antibody or a salt thereof by the functional substance can be determined by peptide mapping. The modification may be regioselective as described above. Thus, the immunoglobulin unit in the formula described below may regioselectively have a corresponding modification unit via a functional group in the side chain of the amino acid residue described above.

Preferably, the conjugate or a salt thereof may comprise a first modified moiety comprising a first functional substance and a second modified moiety comprising a second functional substance each via modification of the amino group in the side chain of one or more (preferably one or two, and more preferably one) lysine residue in the constant region (preferably the Fc region or the CH2 domain) in the first or second heavy chain of the constituent unit (the immunoglobulin unit comprising two heavy chains and optionally two light chains) of an antibody. More specifically, the position of the one or more (preferably one or two, more preferably one) lysine residue may be the position 246/248, 288/290, or 317 in the human IgG heavy chain according to the EU numbering. The modification may be regioselective as described above. Thus, the immunoglobulin unit in the formula described below may regioselectively have a corresponding modification unit via the amino group in the side chain of the lysine residue described above.

In a specific embodiment, the conjugate or a salt thereof may be a conjugate or a salt thereof comprising a structural unit represented by the following formula (VIIa): 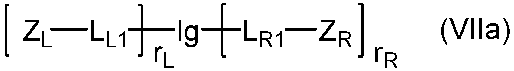 wherein
Ig indicates said immunoglobulin unit;
L_{L1} and L_{R1} each independently indicate a first linker;
Z_{L} indicates a first functional substance;
Z_{R} indicates a second functional substance; and
the average percent modification r_{L} of said immunoglobulin unit with the first modified moiety and the average percent modification r_{R} of said immunoglobulin unit with the second modified moiety are individually 65 to 135%.

The conjugate or a salt thereof comprising a structural unit represented by the formula (VIIa) can be produced by reacting an antibody derivative or a salt thereof comprising a structural unit represented by the formula (VIa) with a functional substance. The two bioorthogonal functional groups in the antibody derivative or a salt thereof comprising a structural unit represented by the formula (VIa) are the same, the reactions can be achieved by one reaction. When the two bioorthogonal functional groups are different from each other, the reactions can be achieved by one reaction (e.g., the case where the two different bioorthogonal functional groups can react under the same reaction conditions), or by two reactions (e.g., the case where the two bioorthogonal functional groups can react under different reaction conditions). The details of the reaction are as described above.

In a specific embodiment, the antibody derivative or a salt thereof may be an antibody or a salt thereof comprising a structural unit represented by the following formula (VIIa-1): 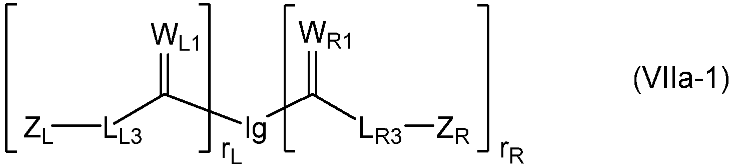 wherein
Ig indicates said immunoglobulin unit;
W_{L1} and W_{R1} each independently indicate an oxygen atom or a sulfur atom;
L_{L3} and L_{R3} each independently indicate a third linker;
Z_{L} indicates a first functional substance;
Z_{R} indicates a second functional substance; and
the average percent modification r_{L} of said immunoglobulin unit with the first modified moiety and the average percent modification r_{R} of said immunoglobulin unit with the second modified moiety are individually 65 to 135%.

The conjugate or a salt thereof comprising a structural unit represented by the formula (VIIa-1) can be produced by reacting an antibody derivative or a salt thereof comprising a structural unit represented by the formula (VIa-1) with a functional substance. The cleavage reactions can be achieved by one reaction. The details of the reaction are as described above.

In a specific embodiment, the conjugate or a salt thereof may be a conjugate or a salt thereof comprising a structural unit represented by the following formula (VIIb): 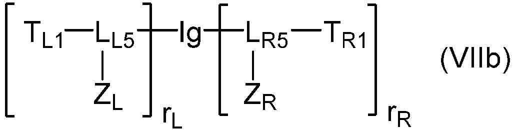 wherein
Ig indicates said immunoglobulin unit;
L_{L5} and L_{R5} each independently indicate a fifth linker;
Z_{L} indicates a first functional substance;
Z_{R} indicates a second functional substance;
T_{L1} and T_{R1} each independently indicate a monovalent group; and
the average percent modification r_{L} of said immunoglobulin unit with the first modified moiety and the average percent modification r_{R} of said immunoglobulin unit with the second modified moiety are individually 65 to 135%.

The conjugate or a salt thereof comprising a structural unit represented by the formula (VIIb) can be produced by reacting an antibody derivative or a salt thereof comprising a structural unit represented by the formula (VIb) with a functional substance. The two bioorthogonal functional groups in the antibody derivative or a salt thereof comprising a structural unit represented by the formula (VIb) are the same, the reactions can be achieved by one reaction. When the two bioorthogonal functional groups are different from each other, the reactions can be achieved by one reaction (e.g., the case where the two different bioorthogonal functional groups can react under the same reaction conditions), or by two reactions (e.g., the case where the two bioorthogonal functional groups can react under different reaction conditions). The details of the reaction are as described above.

In a specific embodiment, the conjugate or a salt thereof may be a conjugate or a salt thereof comprising a structural unit represented by the following formula (VIIb-1): 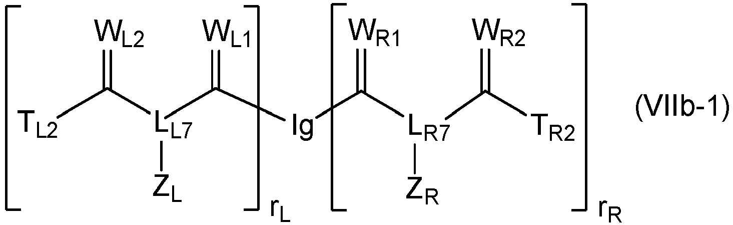 wherein
Ig indicates said immunoglobulin unit;
W_{L1} and W_{L2}, and W_{R1} and W_{R2} each independently indicate an oxygen atom or a sulfur atom;
L_{L7} and L_{R7} each independently indicate a seventh linker;
Z_{L} indicates a first functional substance;
Z_{R} indicates a second functional substance;
T_{L2} and T_{R2} each independently indicate a monovalent group; and
the average percent modification r_{L} of said immunoglobulin unit with the first modified moiety and the average percent modification r_{R} of said immunoglobulin unit with the second modified moiety are individually 65 to 135%.

The conjugate or a salt thereof comprising a structural unit represented by the formula (VIIb-1) can be produced by reacting an antibody derivative or a salt thereof comprising a structural unit represented by the formula (VIb-1) with a functional substance. The two bioorthogonal functional groups in the antibody derivative or a salt thereof comprising a structural unit represented by the formula (VIb-1) are the same, the reactions can be achieved by one reaction. When the two bioorthogonal functional groups are different from each other, the reactions can be achieved by one reaction (e.g., the case where the two different bioorthogonal functional groups can react under the same reaction conditions), or by two reactions (e.g., the case where the two bioorthogonal functional groups can react under different reaction conditions). The details of the reaction are as described above.

In a specific embodiment, the conjugate or a salt thereof may be a conjugate or a salt thereof comprising a structural unit represented by the following formula (VIIc): 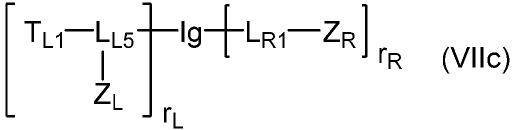 wherein
Ig indicates said immunoglobulin unit;
L_{R1} indicates a first linker;
L_{L5} indicates a fifth linker;
Z_{L} indicates a first functional substance;
Z_{R} indicates a second functional substance;
T_{L1} indicates a monovalent group; and
the average percent modification r_{L} of said immunoglobulin unit with the first modified moiety and the average percent modification r_{R} of said immunoglobulin unit with the second modified moiety are individually 65 to 135%.

The conjugate or a salt thereof comprising a structural unit represented by the formula (VIIc) can be produced by reacting an antibody derivative or a salt thereof comprising a structural unit represented by the formula (VIc) with a functional substance. The two bioorthogonal functional groups in the antibody derivative or a salt thereof comprising a structural unit represented by the formula (VIc) are the same, the reactions can be achieved by one reaction. When the two bioorthogonal functional groups are different from each other, the reactions can be achieved by one reaction (e.g., the case where the two different bioorthogonal functional groups can react under the same reaction conditions), or by two reactions (e.g., the case where the two bioorthogonal functional groups can react under different reaction conditions). The details of the reaction are as described above.

In a specific embodiment, the conjugate or a salt thereof may be a conjugate or a salt thereof comprising a structural unit represented by the following formula (VIIc-1): 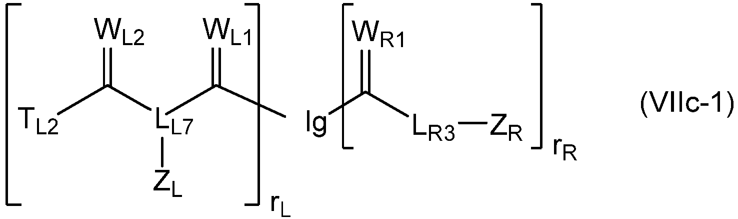 wherein
Ig indicates said immunoglobulin unit;
W_{L1}, W_{L2}, and W_{R1} each independently indicate an oxygen atom or a sulfur atom;
L_{R3} indicates a third linker;
L_{L7} indicates a seventh linker;
Z_{L} indicates a first functional substance;
Z_{R} indicates a second functional substance;
T_{L2} indicates a monovalent group; and
the average percent modification r_{L} of said immunoglobulin unit with the first modified moiety and the average percent modification r_{R} of said immunoglobulin unit with the second modified moiety are individually 65 to 135%.

The conjugate or a salt thereof comprising a structural unit represented by the formula (VIIc-1) can be produced by reacting an antibody derivative or a salt thereof comprising a structural unit represented by the formula (VIc-1) with a functional substance. The two bioorthogonal functional groups in the antibody derivative or a salt thereof comprising a structural unit represented by the formula (VIc) are the same, the reactions can be achieved by one reaction. When the two bioorthogonal functional groups are different from each other, the reactions can be achieved by one reaction (e.g., the case where the two different bioorthogonal functional groups can react under the same reaction conditions), or by two reactions (e.g., the case where the two bioorthogonal functional groups can react under different reaction conditions). The details of the reaction are as described above.

In the formulae (VIa), (VIa-1), (VIb), (VIb-1), (VIc), and (VIc-1), the definitions, examples, and preferred examples of the immunoglobulin unit represented by Ig, and the antibody are as described above.

In the formulae (VIa), (VIa-1), (VIb), (VIb-1), (VIc), and (VIc-1), the symbols with the subscripts "L" and "R" are as described below.

W_{L1} and W_{R1} are the same as W₁, and are each preferably an oxygen atom.

W_{L2} and W_{R2} are the same as W₂, and are each preferably an oxygen atom.

The first linkers represented by L_{L1} and L_{R1} are the same as the first linker represented by L₁. The first linkers represented by L_{L1} and L_{R1} may be the same or different from each other, and are preferably different from each other.

The third linkers represented by L_{L3} and L_{R3} are the same as the third linker represented by L₃. The third linkers represented by L_{L3} and L_{R3} may be the same or different from each other, and are preferably different from each other.

The fifth linkers represented by L_{L5} and L_{R5} are the same as the fifth linker represented by L₅. The first linkers represented by L_{L5} and L_{R5} may be the same or different from each other, and are preferably different from each other.

The seventh linkers represented by L_{L7} and L_{R7} are the same as the seventh linker represented by L₇. The seventh linkers represented by L_{L7} and L_{R7} may be the same or different from each other, and are preferably different from each other.

The first group comprising a first bioorthogonal functional group represented by B_{L} and the second group comprising a second bioorthogonal functional group represented by B_{R} are the same as the group containing a bioorthogonal functional group represented by B. The first group comprising a first bioorthogonal functional group represented by B_{L} and the second group comprising a second bioorthogonal functional group represented by B_{R} may be the same or different from each other, and are preferably different from each other.

The monovalent groups represented by T_{L1} and T_{R1} are the same as the monovalent group represented by T₁. The monovalent groups represented by T_{L1} and T_{R1} may the same or different from each other.

The monovalent groups represented by T_{L2} and T_{R2} are the same as the monovalent group represented by T₂. The monovalent groups represented by T_{L2} and T_{R2} may be the same or different from each other.

The extents of the average percent modifications represented by r_{L} and r_{R}, and the method for determining them are the same as the average percent modification represented by r. The average percent modification represented by r_{L} and r_{R} may be the same or different from each other, and are preferably different from each other.

In a specific embodiment, in the formulae (VIIa) and (VIIc), the molecular weight of the partial structure represented by L_{L1}-Z_{L} and/or L_{R1}-Z_{R} may be the same as the molecular weight of the partial structure represented by L₁-Z in the formula (IVa).

In a specific embodiment, in the formulae (VIIa-1) and (VIIc-1), the molecular weight of the partial structure represented by C(=W_{L1})-L_{L3}-Z_{L} and/or C(=W_{R1})-L_{R3}-Z_{R} may be the same as the molecular weight of the partial structure represented by C(=W₁)-L₃-Z in the formula (IVa-1).

In a specific embodiment, in the formulae (VIIa-1) and (VIIc-1), the third linker represented by L_{L3} and/or L_{R3} may be the same as (CH₂)ₙ₁ that is the third linker represented by L₃ in the formula (IVa-1).

In a specific embodiment, in the formula (VIIb), (VIIc), (VIIb-1), and (VIIc-1), the monovalent group represented by T_{L1}, T_{R1}, T_{L2}, and/or T_{R2} may be a hydroxyamino group that may be substituted, as represented by T₁ and/or T₂ in the formula (IVb).

In a specific embodiment, in the formulae (VIIb) and (VIIc), the molecular weight of the partial structure represented by L_{L5}(-Z_{L})-T_{L1} and/or L_{R5}(-Z_{R})-T_{R1} may be the same as the molecular weight of the partial structure represented by L₅(-Z)-T₁ in the formula (IVb).

In a specific embodiment, in the formulae (VIIb-1) and (VIIc-1), the molecular weight of the partial structure represented by C(=W_{L1})-L_{L7}(-Z_{L})-C(=W_{L2})-T_{L2} and/or C(=W_{R1})-L_{R7}(-Z_{R})-C(=W_{R2})-T_{R2} may be the same as the molecular weight of the partial structure represented by C(=W₁)-L₇(-Z)-C(=W₂)-T₂ in the formula (IVb-1).

In a specific embodiment, in the formulae (VIIb-1) and (VIIc-1), the seventh linker represented by L_{L7} and/or L_{R7} may be the same as (CH₂)ₙ₂ that is the seventh linker represented by L₇ in the formula (IVb-1).

In a specific embodiment, in the formulae (VIIb-1) and (VIIc-1), the first group comprising a first bioorthogonal functional group and/or the second group comprising a second bioorthogonal functional group may be NH-C(=O)-(CH₂)ₙ₃-N₃, as represented as the group comprising a bioorthogonal functional group in the formula (IVb-1).

The conjugate or a salt thereof may further comprise an additional modified moiety. Various methods are known as methods for modifying antibodies. Thus, in the present invention, the conjugate or a salt thereof can be modified to further comprise an additional modified moiety. The additional modified moiety may be introduced to a heavy chain or a light chain of an antibody, and preferably in a heavy chain of an antibody (particularly in the constant region in a heavy chain).

In a specific embodiment, the additional modified moiety may be an additional modified moiety comprising a functional substance. The functional substance is the same as described above. The functional substance contained in the additional modified moiety may be the same as or different from the first and second functional substances of (b), and is preferably different.

In a specific embodiment, the additional modified moiety comprising a functional substance may be introduced to the constant regions in the two heavy chains via modification of the amino group in the side chain of a lysine residue present at one or more positions in the constant regions in said two heavy chains. The conjugate or a salt thereof may comprise an additional modified moiety via modification of the amino group in the side chain of one or more (preferably one or two, and more preferably one) lysine residue in the constant regions (preferably the Fc regions or the CH2 domains) in the two heavy chains of the constituent unit (the immunoglobulin unit comprising two heavy chains and optionally two light chains) of an antibody. More specifically, the position of the one or more (preferably one or two, more preferably one) lysine residue may be the position 246/248, 288/290, or 317 in the human IgG heavy chain according to the EU numbering (see, for example, WO2016/186206, WO2018/199337, WO2019/240287, WO2019/240288, WO2020/009165, and WO2020/090979). The position at which the additional modified moiety comprising a functional substance is introduced is preferably different from the positions at which the first modified moiety comprising a first functional substance and the second modified moiety comprising a second functional substance in (b) are introduced. For example, when the position at which both the first modified moiety and the second modified moiety in (b) are introduced is the lysine residue at the position 246/248, the position at which the additional modified moiety is introduced is preferably the lysine residue at the position 288/290, or 317, and more preferably the lysine residue at the position 288/290. When the position at which both the first modified moiety and the second modified moiety in (b) are introduced is the lysine residue at the position 288/290, the position at which the additional modified moiety is introduced is preferably the lysine residue at the position 246/248, or 317, and more preferably the lysine residue at the position 246/248. When the position at which both the first modified moiety and the second modified moiety in (b) are introduced is the lysine residue at the position 317, the position at which the additional modified moiety is introduced is preferably the lysine residue at the position 246/248 or 288/290.

The determination of the formation of the conjugate or salt thereof, which depends on its specific raw materials and the molecular weight of a product, can be performed by electrophoresis, chromatography (e.g., gel permutation chromatography, ion-exchange chromatography, reversed phase column chromatography, and high-performance liquid chromatography (HPLC)), or mass spectrometry, for example. Determination of regioselectivity can be performed by peptide mapping as described above. The determination of the number of the introduced functional substances can be performed by using mass spectrometry (DAR calculator (Agilent software)) in combination. The conjugate or salt thereof can be purified as appropriate by any method such as chromatography (e.g., chromatography described above and affinity chromatography).

### 6. Uses

The compound or a salt thereof of the present invention can easily modify only one heavy chain of the constituent unit (the immunoglobulin unit comprising two heavy chains and optionally two light chains) of an antibody (the average percent modification r of the immunoglobulin unit is 65 to 135%). The compound or a salt thereof of the present invention can also regioselectively modify a specific amino acid residue (preferably a lysine residue) in a heavy chain of the immunoglobulin unit. Consequently, the present invention provides a reagent for derivatizing an antibody, comprising the compound or a salt thereof of the present invention.

The reagent of the present invention may be provided in a form of a composition further comprising other components. Examples of such other compounds include solutions and stabilizers (e.g., antioxidants and preservatives). Among solutions, aqueous solutions are preferred. Examples of aqueous solutions include water (e.g., distilled water, sterilized distilled water, purified water, and a physiological saline solution) and buffers (e.g., an aqueous phosphoric acid solution, a Tris-hydrochloric acid buffer, a carbonic acid-bicarbonic acid buffer, an aqueous boric acid solution, a glycine-sodium hydroxide buffer, and a citric acid buffer); and buffers are preferred. The pH of solutions is e.g., 5.0 to 9.0 and preferably 5.5 to 8.5. The reagent of the present invention can be provided in a liquid form or a powder form (e.g., freeze-dried powder).

The affinity substance-modified antibody and the antibody derivative, or a salt thereof of the present invention are useful, for example, as intermediates for preparation of the conjugate or a salt thereof of the present invention.

The conjugate or salt thereof of the present invention is useful as pharmaceuticals or reagents (e.g., diagnostic reagents and reagents for research), for example. In particular, the conjugate or a salt thereof of the present invention, which not only has modification in only one heavy chain of the constituent unit of an antibody (the average percent modification r of the immunoglobulin unit is 65 to 135%), but also has a regioselective modification with a functional substance is useful as pharmaceuticals. It is reported that when the number of bonds and the bond positions of a drug of an antibody drug conjugate (ADC) are changed, pharmacokinetics, a releasing rate of the drug, and effects change. Given these circumstances, next-generation ADCs are required to control the number and positions of a drug to be conjugated. It is believed that when the number and positions are constant, the problems of expected efficacy, variations in conjugate medicines, and lot difference, or what is called regulation, will be solved. Therefore, the conjugate or salt thereof of the present invention can solve such a problem of regulation.

The conjugate or salt thereof of the present invention may be provided in the form of a pharmaceutical composition. Such a pharmaceutical composition may comprise a pharmaceutically acceptable carrier in addition to the conjugate or salt thereof of the present invention. Examples of the pharmaceutically acceptable carrier include, but are not limited to, excipients such as sucrose, starch, mannitol, sorbitol, lactose, glucose, cellulose, talc, calcium phosphate, and calcium carbonate; binders such as cellulose, methylcellulose, hydroxypropylcellulose, polypropylpyrrolidone, gelatin, gum arabic, polyethylene glycol, sucrose, and starch; disintegrators such as starch, carboxymethylcellulose, hydroxypropyl starch, sodium hydrogencarbonate, calcium phosphate, and calcium citrate; lubricants such as magnesium stearate, Aerosil, talc, sodium lauryl sulfate; aromatics such as citric acid, menthol, glycyl lysine ammonium salts, glycine, and orange powder; preservatives such as sodium benzoate, sodium hydrogen sulfite, methylparaben, and propylparaben; stabilizers such as citric acid, sodium citrate, and acetic acid, suspensions such as methylcellulose, polyvinylpyrrolidone, and aluminum stearate; dispersants such as surfactants; diluents such as water, a physiological saline solution, and orange juice; and base waxes such as cacao butter, polyethylene glycol, and refined kerosene. The conjugate or salt thereof of the present invention may also have any modification (e.g., PEGylation) for achieving stability.

Examples of preparations suitable for oral administration include liquid medicines in which an effective amount of a ligand is dissolved in a diluent such as water, a physiological saline solution; capsules, sachets, and tablets comprising an effective amount of a ligand as a solid or granules; suspension medicines in which an effective amount of an active ingredient is suspended in an appropriate dispersion medium; and emulsions in which a solution dissolving an effective amount of an active ingredient in an appropriate dispersion medium is dispersed and emulsified.

The pharmaceutical composition is suitable for nonoral administration (e.g., intravenous injection, hypodermic injection, intramuscular injection, local injection, and intraperitoneal administration). Examples of the pharmaceutical composition suitable for such nonoral administration include aqueous or nonaqueous, isotonic, sterile injection medicines, which may comprise an antioxidant, a buffer, an antimicrobial agent, a tonicity agent, or the like. Examples thereof also include aqueous or nonaqueous, sterile suspension medicines, which may comprise a suspending agent, a solubilizing agent, a thickener, a stabilizer, an antiseptic, or the like.

The dose of the pharmaceutical composition, which varies depending on the type and activity of the active ingredient, the severity of the diseases, the species of the animal to be dosed, the drug receptivity, body weight, and age of the subject to be dosed, or the like, can be set as appropriate.

### EXAMPLES

The present invention will be described in more details with reference to the following Examples, but are not intended to be limited thereto.

### (Example 1) Design of Affinity Substance and Secretory Expression of Affinity Substance in C. glutamicum

### (1-1) Outline of Design of Affinity Substance

In the present invention, it is required to design an affinity substance containing in its molecule two or more sites that can bind to an antibody. That is, it is necessary to design an affinity substance such that
A) a molecule containing two or more sites capable of binding to an antibody (and, as necessary, a linker to link the sites) is obtained.

It is preferable in the present invention, in order to allow for modification of an immunoglobulin unit, to attach a compound having a reactive group for the immunoglobulin unit to the affinity substance.

Additionally, it is preferable, in order to remove the affinity substance after modifying the immunoglobulin unit, to comprise a cleavable moiety between the reactive group and the affinity substance.

Furthermore, it is preferable, in order to achieve a specific binding of a compound to a specific site in the affinity substance, to design the affinity substance such that the affinity substance has only one site capable of reacting with the compound. Therefore, when an affinity polypeptide is used as the affinity substance, and the affinity polypeptide and a compound are linked via the amino group in the side chain of a lysine residue (K) in the affinity polypeptide, it is preferable to design the affinity polypeptide such that only one lysine residue is present in the affinity polypeptide. Therefore, the affinity polypeptide is designed as follows.
B) The polypeptide contains only one K residue.
C) By comprising Q (glutamine) as an N-terminal amino acid, Q (glutamine) is pyroglutamylated, and the N-terminal amino group is converted into an amide.

A polypeptidic affinity substance was designed as following (a) to (h) according to the rules of A), B), and C) above.

### (1-2) Preparation of Affinity Substance

The following affinity substances were prepared.
(a) QET-Z34CM-PA32-Fc3K 
QET-FNMQCQRRFYEALHDPNLNEEQRNARIRSIREEC-
GGAAPAAPAPAAPAAPAPAAPAAPAPAAPAAPAPGG-RGNCAYHKGQIIWCTYH-
NH2 (SEQ ID NO: 1)
(b) QET-Z34CM-PA48-Fc3K 
QET-FNMQCQRRFYEALHDPNLNEEQRNARIRSIREEC-
GGAAPAAPAPAAPAAPAPAAPAAPAPAAPAAPAPAAPAAPAPAAPAAPAPGG-
RGNCAYHKGQIIWCTYH-NH2 (SEQ ID NO: 2)
(c) QET-Fc3K-PA32-Z34CM 
QETRGNCAYHKGQIIWCTYH-
GGAAPAAPAPAAPAAPAPAAPAAPAPAAPAAPAPGG-
FNMQCQRRFYEALHDPNLNEEQRNARIRSIREEC-NH2 (SEQ ID NO: 3)
(d) QET-Fc3K-PA48-Z34CM 
QETRGNCAYHKGQIIWCTYH-
GGAAPAAPAPAAPAAPAPAAPAAPAPAAPAAPAPAAPAAPAPAAPAAPAPAAPAAPAPGG-
FNMQCQRRFYEALHDPNLNEEQRNARIRSIREEC-NH2 (SEQ ID NO: 4)
(e) QET-Fc3K-PA32-ProAR 
QETRGNCAYHKGQIIWCTYH-
GGAAPAAPAPAAPAAPAPAAPAAPAPAAPAAPAPGG-
FNREQQNAFYEILHLPNLNEEQRNGFIQSLRDDPSQSANLLAEA-NH2 (SEQ ID
NO: 5)
(f) QET-Fc3K-PA48-ProAR 
QETRGNCAYHKGQIIWCTYH-
GGAAPAAPAPAAPAAPAPAAPAAPAPAAPAAPAPAAPAAPAPAAPAAPAPAAPAAPAPGG-
FNREQQNAFYEILHLPNLNEEQRNGFIQSLRDDPSQSANLLAEA-NH2 (SEQ ID
NO: 6)
(g) QET-ProAR-PA32-Z34CK 
QET-FNREQQNAFYEILHLPNLNEEQRNGFIQSLRDDPSQSANLLAEA-
GGAAPAAPAPAAPAAPAPAAPAAPAPAAPAAPAPGG-
FNKQCQRRFYEALHDPNLNEEQRNARIRSIREEC-NH2 (SEQ ID NO: 7)
(h) QET-ProAR-PA48-Z34CK

QET-FNREQQNAFYEILHLPNLNEEQRNGFIQSLRDDPSQSANLLAEA-
GGAAPAAPAPAAPAAPAPAAPAAPAPAAPAAPAPAAPAAPAPAAPAAPAPGG-
FNKQCQRRFYEALHDPNLNEEQRNARIRSIREEC-NH2 (SEQ ID NO: 8)

### (1-3) Expression of Affinity Substance

Expression of these polypeptidic affinity substances was examined using Corynex^{®}. In expression using Corynex^{®}, a CspB fusion method (WO 2013/062029), that is, a technique capable of improving the secretory production amount of a target polypeptide by inserting a base sequence encoding an amino acid sequence comprising N-terminal three residues Gln-Glu-Thr (QET) of a CspB mature protein between a base sequence encoding a signal peptide and a base sequence encoding a target polypeptide was utilized. In addition, since an N-terminal of a CspB tag is Q, there is an advantage that Q at the first residue can be pyroglutamylated to protect the N-terminal amino group after cleavage of the signal sequence. That is, in addition to the rules A), B) and C) above, it is effective to consider the rule D) below for improvement of the expression of an affinity polypeptide using Corynex^{®}.
D) Three residues of QET are added to an N-terminal to improve secretory efficiency using Corynex^{®}.

Hereinafter, Examples of expression studies using Corynex^{®} will be described.

### (1-4) Construction of Secretory Expression Plasmids of QET-Z34CM-PA32-Fc3K, QET-Z34CM-PA48-Fc3K, QET-Fc3K-PA32-Z34CM, QET-Fc3K-PA48-Z34CM, QET-Fc3K-PA32-ProAR, QET-Fc3K-PA48-ProAR, QET-ProAR-PA32-Z34CK, and QET-ProAR-PA48-Z34CK

As affinity polypeptides, the above eight types of amino acid sequences of QET-Z34CM-PA32-Fc3K, QET-Z34CM-PA48-Fc3K, QET-Fc3K-PA32-Z34CM, QET-Fc3K-PA48-Z34CM, QET-Fc3K-PA32-ProAR, QET-Fc3K-PA48-ProAR, QET-ProAR-PA32-Z34CK, and QET-ProAR-PA48-Z34CK were designed, and base sequences encoding these polypeptides were designated in consideration of codon usage frequency of *C*. *glutamicum.* Furthermore, the following expression cassettes were designed so as to make secretory expression by *C*. *glutamicum* possible.

QET-Z34CM-PA32-Fc3K underwent secretory expression as a fusion protein of a signal peptide 30 amino acid residue of CspB derived from *C*. *glutamicum* ATCC13869 strain, an N-terminal 3 amino acid residue QET of CspB mature protein derived from *C*. *glutamicum* ATCC13869 strain, and Z34CM-PA32-Fc3K (hereinafter referred to as "CspBss-QET-Z34CM-PA32-Fc3K"). The designed base sequence encoding CspBss-QET-Z34CM-PA32-Fc3K and the amino acid sequence thereof are illustrated in SEQ ID NOs: 9 and 10, respectively.

Base sequence encoding CspBss-QET-Z34CM-PA32-Fc3K 
atgtttaacaaccgtatccgcactgcagctctcgctggtgcaatcgcaatctccaccgcagcttccggcgtagctatcccagcat
tcgctcaggagaccttcaacatgcagtgccaacggcgcttttacgaagcgctccacgatcccaacctgaacgaggaacagcg
taacgcgcgcattcgctccatccgtgaggaatgtggcggtgctgcccctgctgccccagcaccggcagcacctgcggcacc
agctccggcagctccagctgcaccggcccctgccgctccagcagcgccagctcccggaggtcgaggcaattgcgcctatca
caaggggcagatcatctggtgcacttaccattaa (SEQ ID NO: 9)
Amino acid sequence of CspBss-QET-Z34CM-PA32-Fc3K

MFNNRIRTAALAGAIAISTAASGVAIPAFAQETFNMQCQRRFYEALHDPNLNEEQ
RNARIRSIREECGGAAPAAPAPAAPAAPAPAAPAAPAPAAPAAPAPGGRGNCAYH
KGQIIWCTYH (SEQ ID NO: 10)

QET-Z34CM-PA48-Fc3K underwent secretory expression as a fusion protein of a signal peptide 30 amino acid residue of CspB derived from *C*. *glutamicum* ATCC13869 strain, an N-terminal 3 amino acid residue QET of CspB mature protein derived from *C*. *glutamicum* ATCC13869 strain, and Z34CM-PA48-Fc3K (hereinafter referred to as "CspBss-QET-Z34CM-PA48-Fc3K"). The designed base sequence encoding CspBss-QET-Z34CM-PA48-Fc3K and the amino acid sequence thereof are illustrated in SEQ ID NOs: 11 and 12, respectively.

Base sequence encoding CspBss-QET-Z34CM-PA48-Fc3K 
atgtttaacaaccgtatccgcactgcagctctcgctggtgcaatcgcaatctccaccgcagcttccggcgtagctatcccagcat
tcgctcaggagacctttaacatgcagtgccaacgtcgtttttacgaagcgctgcacgatcccaacctgaacgaggaacagcgc
aatgcgcgaatacgctccattcgcgaggaatgtggcggagcagctcccgcggctcctgctccagcagcgcctgctgctcctg
cccctgcggcacctgcagcacctgcaccagcagctccggcagcaccagctccagctgcacccgccgccccagctcctgca
gcgccagccgctccggcccccggaggtaggggcaactgcgcatatcacaaggggcagataatctggtgcacttatcactaa
(SEQ ID NO: 11)
Amino acid sequence of CspBss-QET-Z34CM-PA48-Fc3K

MFNNRIRTAALAGAIAISTAASGVAIPAFAQETFNMQCQRRFYEALHDPNLNEEQ
RNARIRSIREECGGAAPAAPAPAAPAAPAPAAPAAPAPAAPAAPAPAAPAAPAPAA
PAAPAPGGRGNCAYHKGQIIWCTYH (SEQ ID NO: 12)

QET-Fc3K-PA32-Z34CM underwent secretory expression as a fusion protein of a signal peptide 30 amino acid residue of CspB derived from *C*. *glutamicum* ATCC13869 strain, an N-terminal 3 amino acid residue QET of CspB mature protein derived from *C*. *glutamicum* ATCC13869 strain, and Fc3K-PA32-Z34CM (hereinafter referred to as "CspBss-QET-Fc3K-PA32-Z34CM"). The designed base sequence encoding CspBss-QET-Fc3K-PA32-Z34CM and the amino acid sequence thereof are illustrated in SEQ ID NOs: 13 and 14, respectively.

Base sequence encoding CspBss-QET-Fc3K-PA32-Z34CM 
atgtttaacaaccgtatccgcactgcagctctcgctggtgcaatcgcaatctccaccgcagcttccggcgtagctatcccagcat
tcgctcaggagacccgtggcaactgtgcctaccacaagggccagatcatctggtgcacgtaccacggaggtgccgcgcctg
ccgcacctgctccagctgctcccgctgcccctgcaccagctgcgccagcagcaccagctccagcggcacctgcagcaccg
gcacctggtggcttcaacatgcagtgccaacgacgcttctacgaggctctgcacgatccgaacctcaacgaggaacagcgca
atgcccgtattcggtccatcagggaggaatgctaa (SEQ ID NO: 13)
Amino acid sequence of CspBss-QET-Fc3K-PA32-Z34CM

MFNNRIRTAALAGAIAISTAASGVAIPAFAQETRGNCAYHKGQIIWCTYHGGAAP
AAPAPAAPAAPAPAAPAAPAPAAPAAPAPGGFNMQCQRRFYEALHDPNLNEEQR

NARIRSIREEC (SEQ ID NO: 14)

QET-Fc3K-PA48-Z34CM underwent secretory expression as a fusion protein of a signal peptide 30 amino acid residue of CspB derived from *C*. *glutamicum* ATCC13869 strain, an N-terminal 3 amino acid residue QET of CspB mature protein derived from *C*. *glutamicum* ATCC13869 strain, and Fc3K-PA48-Z34CM (hereinafter referred to as "CspBss-QET-Fc3K-PA48-Z34CM"). The designed base sequence encoding CspBss-QET-Fc3K-PA48-Z34CM and the amino acid sequence thereof are illustrated in SEQ ID NOs: 15 and 16, respectively.

Base sequence encoding CspBss-QET-Fc3K-PA48-Z34CM 
atgtttaacaaccgtatccgcactgcagctctcgctggtgcaatcgcaatctccaccgcagcttccggcgtagctatcccagcat
tcgctcaggagacccgtggcaattgcgcctatcacaagggtcagatcatttggtgcacctaccatggtggtgcagcaccagct
gccccggctccggctgcgcctgctgcacctgcgccagctgcacctgcagcaccggcacctgctgctccagcagcacccgct
cctgcagctcccgctgccccagctccagcggcgcctgcggcgccagcacctggagggttcaacatgcagtgccaacgtcg
cttttacgaggctttgcatgatcctaacttgaacgaggaacagcggaatgcgcggatacgctccatacgcgaagaatgttaa
(SEQ ID NO: 15)
Amino acid sequence of CspBss-QET-Fc3K-PA48-Z34CM

MFNNRIRTAALAGAIAISTAASGVAIPAFAQETRGNCAYHKGQIIWCTYHGGAAP
AAPAPAAPAAPAPAAPAAPAPAAPAAPAPAAPAAPAPAAPAAPAPGGFNMQCQRR
FYEALHDPNLNEEQRNARIRSIREEC (SEQ ID NO: 16)

QET-Fc3K-PA32-ProAR underwent secretory expression as a fusion protein of a signal peptide 30 amino acid residue of CspB derived from *C*. *glutamicum* ATCC13869 strain, an N-terminal 3 amino acid residue QET of CspB mature protein derived from *C*. *glutamicum* ATCC13869 strain, and Fc3K-PA32-ProAR (hereinafter referred to as "CspBss-QET-Fc3K-PA32-ProAR"). The designed base sequence encoding CspBss-QET-Fc3K-PA32-ProAR and the amino acid sequence thereof are illustrated in SEQ ID NOs: 17 and 18, respectively.

Base sequence encoding CspBss-QET-Fc3K-PA32-ProAR 
atgtttaacaaccgtatccgcactgcagctctcgctggtgcaatcgcaatctccaccgcagcttccggcgtagctatcccagcat
tcgctcaggagacccgcggcaactgtgcctaccacaaggggcagatcatctggtgcacctatcacggtggtgcggctccgg
ctgctccagcgccagcagctcccgctgcaccagccccagcggcacctgccgcacctgcacctgcagctccagcggctccc
gcaccgggaggctttaaccgcgaacagcagaacgccttctacgagattctccatctgcccaacctcaacgaggagcaacgg
aatggcttcatccagagcttgcgtgacgatccgtctcaatccgccaatctgcttgcggaagcctaa (SEQ ID NO: 17)
Amino acid sequence of CspBss-QET-Fc3K-PA32-ProAR

MFNNRIRTAALAGAIAISTAASGVAIPAFAQETRGNCAYHKGQIIWCTYHGGAAP
AAPAPAAPAAPAPAAPAAPAPAAPAAPAPGGFNREQQNAFYEILHLPNLNEEQRN
GFIQSLRDDPSQSANLLAEA (SEQ ID NO: 18)

QET-Fc3K-PA48-ProAR underwent secretory expression as a fusion protein of a signal peptide 30 amino acid residue of CspB derived from *C*. *glutamicum* ATCC13869 strain, an N-terminal 3 amino acid residue QET of CspB mature protein derived from *C*. *glutamicum* ATCC13869 strain, and Fc3K-PA48-ProAR (hereinafter referred to as "CspBss-QET-Fc3K-PA48-ProAR"). The designed base sequence encoding CspBss-QET-Fc3K-PA48-ProAR and the amino acid sequence thereof are illustrated in SEQ ID NOs: 19 and 20, respectively.

Base sequence encoding CspBss-QET-Fc3K-PA48-ProAR 
atgtttaacaaccgtatccgcactgcagctctcgctggtgcaatcgcaatctccaccgcagcttccggcgtagctatcccagcat
tcgctcaggagacccgtgggaactgcgcgtaccataagggccagatcatctggtgcacctatcatggcggtgctgcgcctgc
agctccggctcctgccgctccagccgcgccagccccagcagctccagcagcacctgcacctgctgcaccagcggctccag
ctcctgcggcacccgctgcaccggcaccagccgctccggctgccccagctccgggtggctttaaccgcgaacagcagaac
gccttctacgagatactccaccttcccaacctgaacgaggagcagcgaaacggcttcatccagtctcttcgcgatgatccatcc
cagagcgctaacttgcttgcagaagcgtaa (SEQ ID NO: 19)
Amino acid sequence of CspBss-QET-Fc3K-PA32-ProAR

MFNNRIRTAALAGAIAISTAASGVAIPAFAQETRGNCAYHKGQIIWCTYHGGAAP
AAPAPAAPAAPAPAAPAAPAPAAPAAPAPAAPAAPAPAAPAAPAPGGFNREQQNA
FYEILHLPNLNEEQRNGFIQSLRDDPSQSANLLAEA (SEQ ID NO: 20)

QET-ProAR-PA32-Z34CK underwent secretory expression as a fusion protein of a signal peptide 30 amino acid residue of CspB derived from *C*. *glutamicum* ATCC13869 strain, an N-terminal 3 amino acid residue QET of CspB mature protein derived from *C*. *glutamicum* ATCC13869 strain, and ProAR-PA32-Z34CK (hereinafter referred to as "CspBss-QET-ProAR-PA32-Z34CK"). The designed base sequence encoding CspBss-QET-ProAR-PA32-Z34CK and the amino acid sequence thereof are illustrated in SEQ ID NOs: 21 and 22, respectively.

Base sequence encoding CspBss-QET-ProAR-PA32-Z34CK 
atgtttaacaaccgtatccgcactgcagctctcgctggtgcaatcgcaatctccaccgcagcttccggcgtagctatcccagcat
tcgctcaggagacctttaaccgcgaacagcagaacgccttctacgagatcctgcatctccccaatctgaacgaagagcagag
gaatggctttatccagagccttcgtgacgatccgtcgcaatctgccaacctgttggcggaagctggaggtgccgcgccagcg
gcaccagcaccagctgcaccggctgctcccgcacctgccgcaccagccgctcctgccccagcagctccggcagctcctgc
acctggtggcttcaacaagcagtgccaacgtcgcttctatgaagcgctacacgatcccaacctcaacgaggaacagcgcaat
gcgcgaatccggtccattcgcgaagagtgttaa (SEQ ID NO: 21)
Amino acid sequence of CspBss-QET-ProAR-PA32-Z34CK

MFNNRIRTAALAGAIAISTAASGVAIPAFAQETFNREQQNAFYEILHLPNLNEEQR
NGFIQSLRDDPSQSANLLAEAGGAAPAAPAPAAPAAPAPAAPAAPAPAAPAAPAP
GGFNKQCQRRFYEALHDPNLNEEQRNARIRSIREEC (SEQ ID NO: 22)

QET-ProAR-PA48-Z34CK underwent secretory expression as a fusion protein of a signal peptide 30 amino acid residue of CspB derived from *C*. *glutamicum* ATCC13869 strain, an N-terminal 3 amino acid residue QET of CspB mature protein derived from *C*. *glutamicum* ATCC13869 strain, and ProAR-PA48-Z34CK (hereinafter referred to as "CspBss-QET-ProAR-PA48-Z34CK"). The designed base sequence encoding CspBss-QET-ProAR-PA48-Z34CK and the amino acid sequence thereof are illustrated in SEQ ID NOs: 23 and 24, respectively.

Base sequence encoding CspBss-QET-ProAR-PA48-Z34CK 
atgtttaacaaccgtatccgcactgcagctctcgctggtgcaatcgcaatctccaccgcagcttccggcgtagctatcccagcat
tcgctcaggagacctttaaccgcgaacagcagaacgccttctacgagatcctccatctgccaaacctgaacgaagaacagcg
 
caacggctttatccagtccctcagggacgatccgtctcagtcggccaatttgctagccgaagcgggaggtgctgctccggcag
cacccgcaccagccgcgcccgctgctccagcgccggcagctcctgcggcaccagctccagcagcaccagcggctccagc
acctgccgcacctgcagctccggctcctgcagcccccgccgctcctgcgccgggtggcttcaacaagcagtgccaacgacg
cttctatgaggcgcttcacgatcccaacctgaatgaggagcaacggaatgcccgtatccgtagcattcgcgaagaatgttaa
(SEQ ID NO: 23)
Amino acid sequence of CspBss-QET-ProAR-PA48-Z34CK

MFNNRIRTAALAGAIAISTAASGVAIPAFAQETFNREQQNAFYEILHLPNLNEEQR
NGFIQSLRDDPSQSANLLAEAGGAAPAAPAPAAPAAPAPAAPAAPAPAAPAAPAP
AAPAAPAPAAPAAPAPGGFNKQCQRRFYEALHDPNLNEEQRNARIRSIREEC
(SEQ ID NO: 24)

Expression cassettes of eight types of affinity polypeptides were designed in each of which a promoter of a cspB gene derived from *C*. *glutamicum* ATCC13869 strain was coupled with each of upstream sides of the base sequences described in CspBss-QET-Z34CM-PA32-Fc3K, CspBss-QET-Z34CM-PA48-Fc3K, CspBss-QET-Fc3K-PA32-Z34CM, CspBss-QET-Fc3K-PA48-Z34CM, CspBss-QET-Fc3K-PA32-ProAR, CspBss-QET-Fc3K-PA48-ProAR, CspBss-QET-ProAR-PA32-Z34CK, and CspBss-QET-ProAR-PA48-Z34CK, and a KpnI site was added to the 5'-side and a BamHI site was added to the 3'-side, and total synthesis was performed. By inserting the totally synthesized DNA fragments (expression cassettes of affinity polypeptides) into the KpnI-BamHI site of pPK4 described in JP 9-322774 A, pPK4_CspBss-QET-Z34CM-PA32-Fc3K, pPK4_CspBss-QET-Z34CM-PA48-Fc3K, pPK4_CspBss-QET-Fc3K-PA32-Z34CM, pPK4_CspBss-QET-Fc3K-PA48-Z34CM, pPK4_CspBss-QET-Fc3K-PA32-ProAR, pPK4_CspBss-QET-Fc3K-PA48-ProAR, pPK4_CspBss-QET-ProAR-PA32-Z34CK, and pPK4_CspBss-QET-ProAR-PA48-Z34CK, which are secretory expression plasmids of affinity polypeptides, were constructed, respectively. As a result of determining the base sequences of the inserted fragments, it was determined that the expression cassettes of affinity polypeptides as designed were constructed, respectively. The base sequence was determined using BigDye (R) Terminator v3.1 Cycle Sequencing Kit (Applied Biosystems) and 3500xL Genetic Analyzer (Applied Biosystems).

### (1-5) Secretory Expression of Affinity Polypeptide in C. glutamicum

Using pPK4_CspBss-QET-Z34CM-PA32-Fc3K, pPK4_CspBss-QET-Z34CM-PA48-Fc3K, pPK4_CspBss-QET-Fc3K-PA32-Z34CM, pPK4_CspBss-QET-Fc3K-PA48-Z34CM, pPK4_CspBss-QET-Fc3K-PA32-ProAR, pPK4_CspBss-QET-Fc3K-PA48-ProAR, pPK4_CspBss-QET-ProAR-PA32-Z34CK, and pPK4_CspBss-QET-ProAR-PA48-Z34CK constructed above, *C. glutamicum* YDK0107 strain described in WO2016/171224 was transformed to obtain YDK0107/pPK4_CspBss-QET-Z34CM-PA32-Fc3K strain, YDK0107/pPK4_CspBss-QET-Z34CM-PA48-Fc3K strain, YDK0107/pPK4_CspBss-QET-Fc3K-PA32-Z34CM strain, YDK0107/pPK4_CspBss-QET-Fc3K-PA48-Z34CM strain, YDK0107/pPK4_CspBss-QET-Fc3K-PA32-ProAR strain, YDK0107/pPK4_CspBss-QET-Fc3K-PA48-ProAR strain, YDK0107/pPK4_CspBss-QET-ProAR-PA32-Z34CK strain, and YDK0107/pPK4_CspBss-QET-ProAR-PA48-Z34CK strain.

Each of the obtained transformants was cultured at 30°C for 72 hours in an MMTG liquid medium containing 25 mg/L kanamycin (120 g of glucose, 3 g of magnesium sulfate heptahydrate, 30 g of ammonium sulfate, 1.5 g of potassium dihydrogen phosphate, 0.03 g of iron (II) sulfate heptahydrate, 0.03 g of manganese (II) sulfate pentahydrate, 0.45 mg of thiamine hydrochloride, 0.45 mg of biotin, 0.15 g of DL-methionine, soybean hydrochloric acid hydrolyzate (total nitrogen amount 0.2 g), and 50 g of calcium carbonate, the total volume of which was set to 1 L with water, and the pH of which was adjusted to pH 7.0).

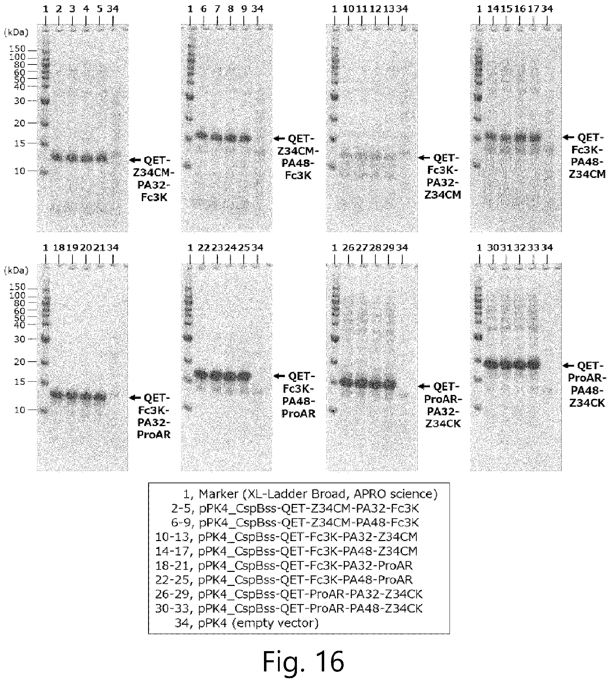

After completion of the culture, 6.5 µL of the culture supernatant obtained by centrifuging each of the culture solutions was subjected to reducing SDS-PAGE using NuPAGE^{®} 12% Bis-Tris Gel (Thermo Fisher Scientific, Inc.), and then stained with Quick-CBB (Wako). As a result, protein bands estimated to be YDK0107/pPK4_CspBss-QET-Z34CM-PA32-Fc3K (FIG. 16, Lanes 2-5) were detected in the culture supernatant of QET-Z34CM-PA32-Fc3K strain, protein bands estimated to be YDK0107/pPK4_CspBss-QET-Z34CM-PA32-Fc3K (FIG. 16, Lanes 6-9) were detected in the culture supernatant of QET-Z34CM-PA32-Fc3K strain, protein bands estimated to be YDK0107/pPK4_CspBss-QET-Fc3K-PA32-Z34CM (FIG. 16, Lanes 10-13) were detected in the culture supernatant of QET-Fc3K-PA32-Z34CM strain, protein bands estimated to be YDK0107/pPK4_CspBss-QET-Fc3K-PA48-Z34CM (FIG. 16, Lanes 14-17) were detected in the culture supernatant of QET-Fc3K-PA48-Z34CM strain, protein bands estimated to be YDK0107/pPK4_CspBss-QET-Fc3K-PA32-ProAR (FIG. 16, Lanes 18-21) were detected in the culture supernatant of QET-Fc3K-PA32-ProAR strain, protein bands estimated to be YDK0107/pPK4_CspBss-QET-Fc3K-PA48-ProAR (FIG. 16, Lanes 22-25) were detected in the culture supernatant of QET-Fc3K-PA48-ProAR strain, protein bands estimated to be YDK0107/pPK4_CspBss-QET-ProAR-PA32-Z34CK (FIG. 16, Lanes 26-29) were detected in the culture supernatant of QET-ProAR-PA32-Z34CK strain, and protein bands estimated to be QET-ProAR-PA48-Z34CK (FIG. 16, Lanes 30-33) were detected in the culture supernatant of YDK0107/pPK4_CspBss-QET-ProAR-PA48-Z34CK strain.

### (Example 2) Purification of Affinity Substance, and Measurement of Affinity between Affinity Substance and Fc Region of IgG1 Antibody

The strains obtained in Example 1 (1-5) in a fermenter for mass culture were cultured according to a known method (Appl Microbiol Biotechnol (2008) 78:621-625). The centrifugal supernatants of the cultures were subjected to sterile filtration through a 0.2-µm PVDF membrane filter. The expressed polypeptides were purified by preparative HPLC using a cation-exchange column in order to remove contaminants in the culture solutions. Then, the affinity of the obtained polypeptides was measured. Specifically, the above was performed as follows.

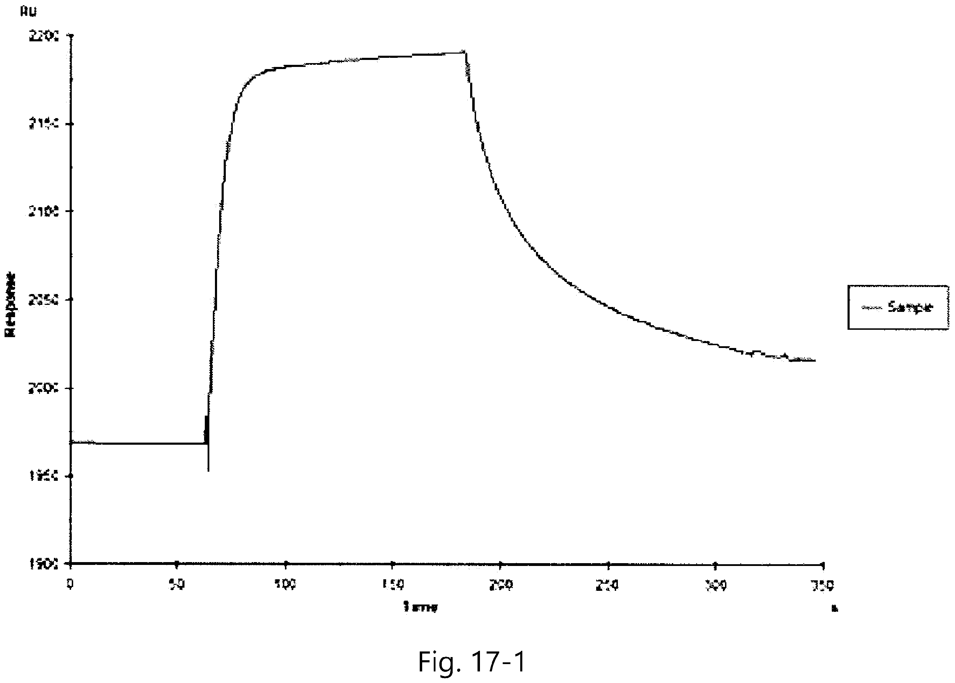

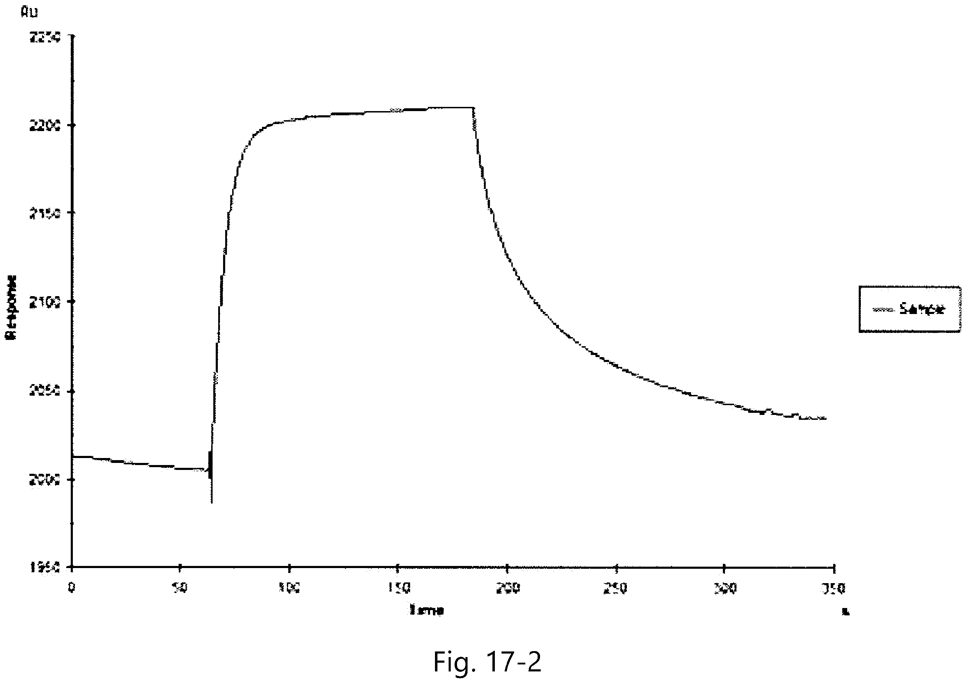

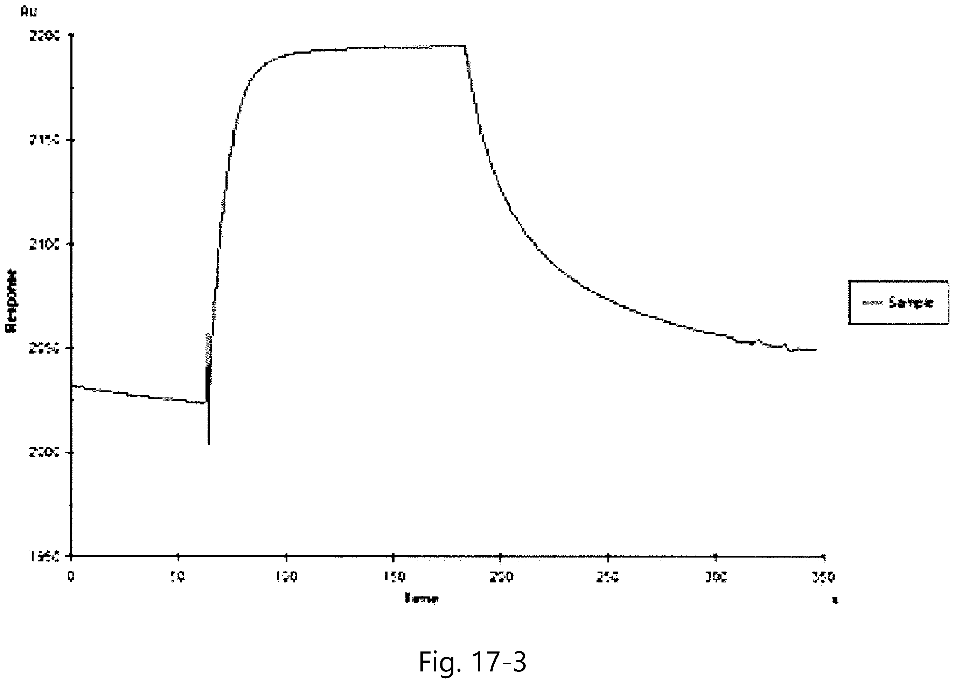

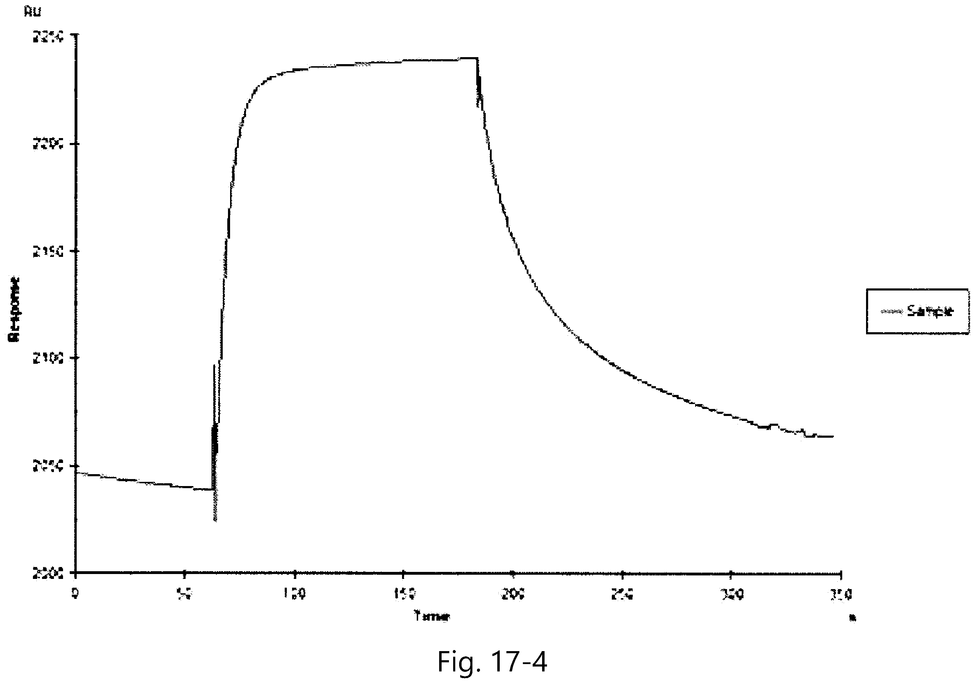

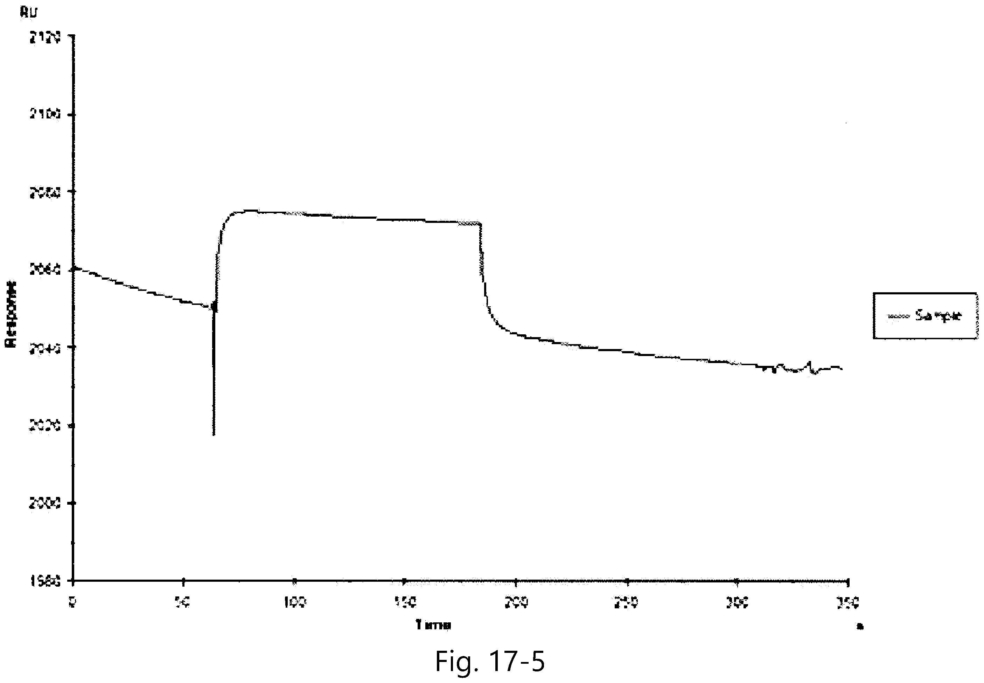

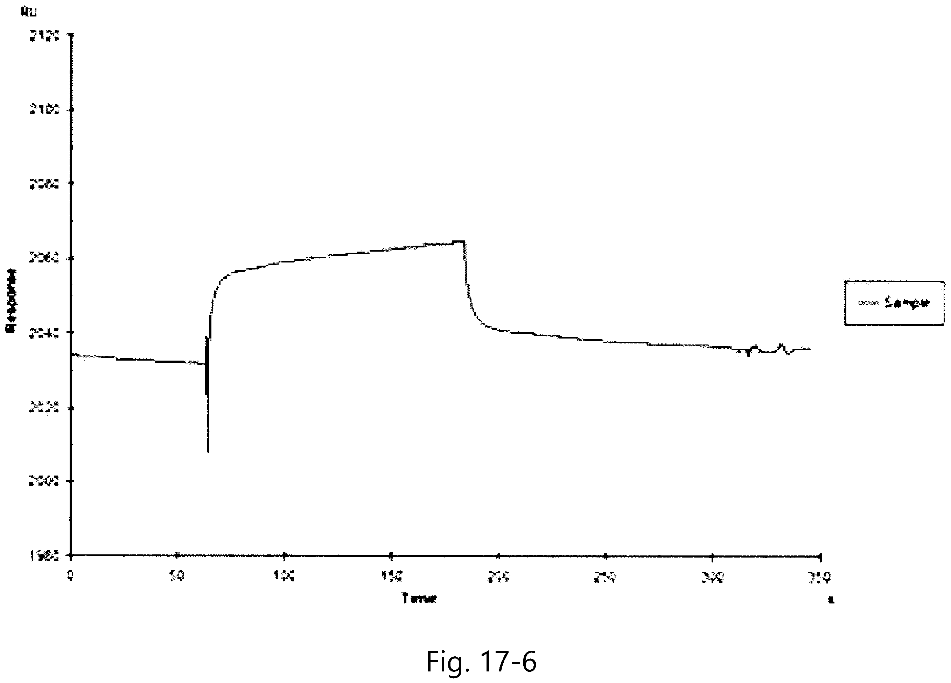

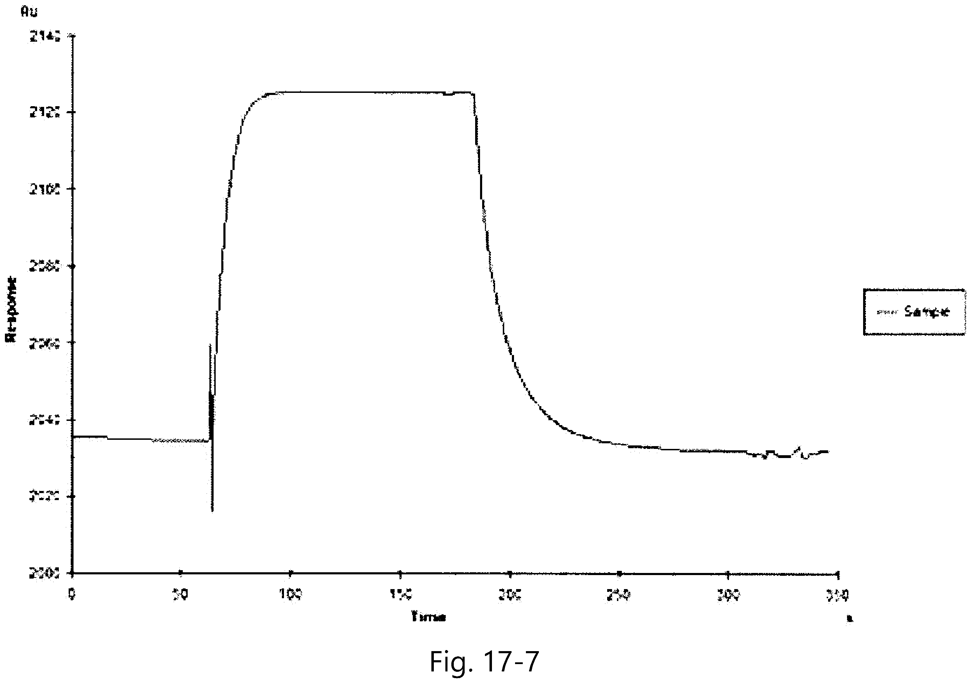

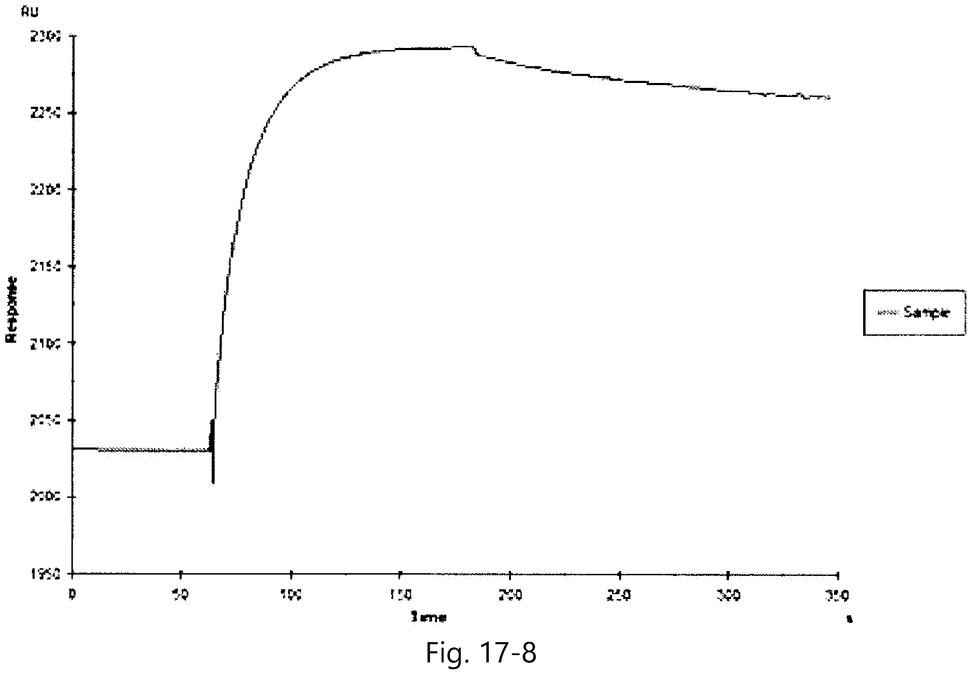

The affinity between each polypeptide and the Fc region of an IgG1 antibody was evaluated by SPR measurement (Biacore T200 (GE Healthcare)). The measurement was performed at 25°C, and HBS-EP+ was used as the running buffer. The Fc protein of human IgG1 antibody, as the ligand, was diluted to 30 µg/mL in sodium acetate buffer, pH 4.5 (GE Healthcare) and immobilized on the surface of a CM5 Sensor Chip (GE Healthcare) at approximately 1400 RU using the amine coupling method. The peptides were diluted to 100 nM in HBS-EP+ buffer containing 0.1% DMSO. The measurement was performed using the single cycle kinetics method, with an analyte injection time of 120 seconds, a flow rate of 30 µL/min, and a dissociation time of 120 seconds. The obtained sensorgrams were fitted based on the affinity to calculate the dissociation constant (KD). As a result of the measurements, all expressed polypeptides were confirmed to bind to the Fc protein of human IgG1 antibody (Table 1 and FIGS 17-1 to 17-8).

**Table 1. KD values of various affinity substances with respect to the antibody**

| | KD (M) |
|---|---|
| SEQ ID NO: 1 | 7.91 × 10⁻⁹ |
| SEQ ID NO: 2 | 8.98 × 10⁻⁹ |
| SEQ ID NO: 3 | 1.27 × 10⁻⁸ |
| SEQ ID NO: 4 | 8.48 × 10⁻⁹ |
| SEQ ID NO: 5 | 2.41 × 10⁻⁷ |
| SEQ ID NO: 6 | 2.69 × 10⁻⁷ |
| SEQ ID NO: 7 | 9.43 × 10⁻⁸ |
| SEQ ID NO: 8 | 1.08 × 10⁻⁹ |

### Example 3. Preparation of Compound Having Affinity Substance for Antibody, Cleavable Moiety, and Reactive Group

A compound having a cleavable moiety and reactive group was conjugated to the purified polypeptides.

Following the previous report (WO2019/0240287), the affinity substance QET-Z34CM-PA32-Fc3K prepared in Example 1-2 was amidated to prepare affinity reagent (1).

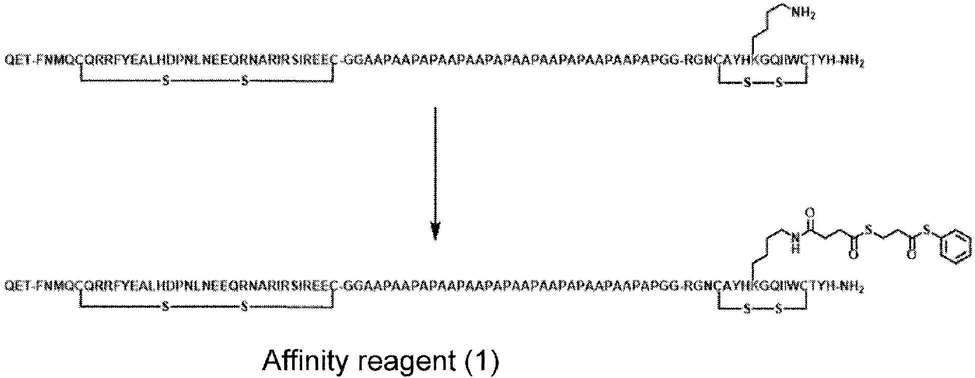

The aforementioned amino acid sequences are the amino acid sequence of SEQ ID NO: 1.
MS (ESI) m/z: z = 11 885.80[M+3H]³⁺

Similarly, Affinity reagent (2) was prepared from the affinity substance prepared in Example 1-2, QET-Z34CM-PA48-Fc3K.
MS (ESI) m/z: z = 13 849.35 [M+3H]³⁺

Similarly, Affinity reagent (3) was prepared from the affinity substance prepared in Example 1-2, QET-Fc3K-PA32-Z34CM.
MS (ESI) m/z: z = 11 886.15[M+3H]³⁺

Similarly, Affinity reagent (4) was prepared from the affinity substance prepared in Example 1-2, QET-Fc3K-PA48-Z34CM.
MS (ESI) m/z: z = 13 849.20[M+3H]³⁺

Similarly, Affinity reagent (5) was prepared from the affinity substance prepared in Example 1-2, QET-Fc3K-PA32-ProAR.
MS (ESI) m/z: z = 12 885.1[M+3H]³⁺

Similarly, Affinity reagent (6) was prepared from the affinity substance prepared in Example 1-2, QET-Fc3K-PA48-ProAR.
MS (ESI) m/z: z = 11 1082.00[M+3H]³⁺

Similarly, Affinity reagent (7) was prepared from the affinity substance prepared in Example 1-2, QET-ProAR-PA32-Z34CK.
MS (ESI) m/z: z = 15 855.20[M+3H]³⁺

Similarly, Affinity reagent (8) was prepared from the affinity substance prepared in Example 1-2, QET-ProAR-PA48-Z34CK.
MS (ESI) m/z: z = 17 830.75[M+3H]³⁺

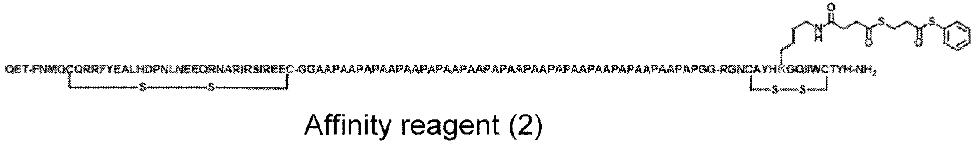

The aforementioned amino acid sequences is the amino acid sequence of SEQ ID NO: 2.

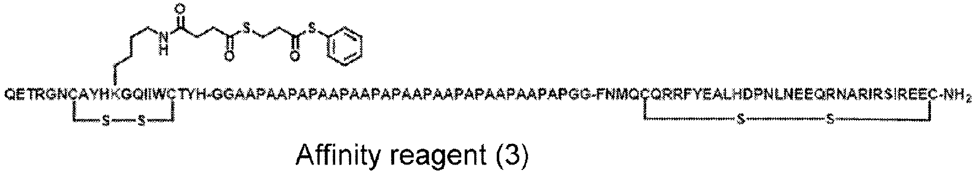

The aforementioned amino acid sequences is the amino acid sequence of SEQ ID NO: 3.

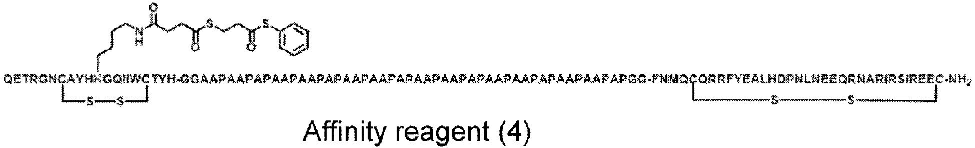

The aforementioned amino acid sequences is the amino acid sequence of SEQ ID NO: 4.

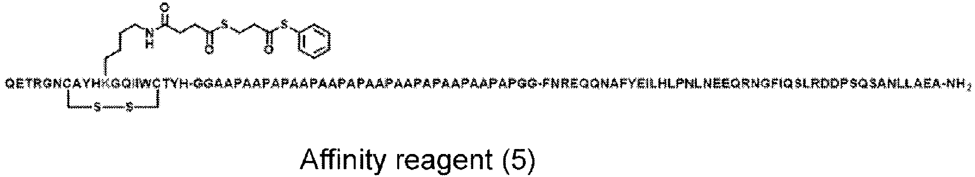

The aforementioned amino acid sequences is the amino acid sequence of SEQ ID NO: 5.

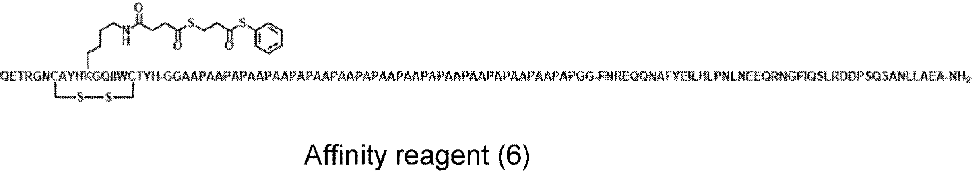

The aforementioned amino acid sequences is the amino acid sequence of SEQ ID NO: 6.

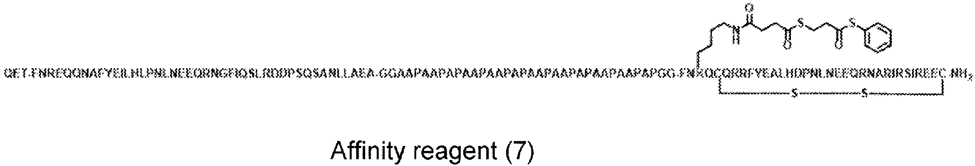

The afore mentioned amino acid sequences is the amino acid sequence of SEQ ID NO: 7.

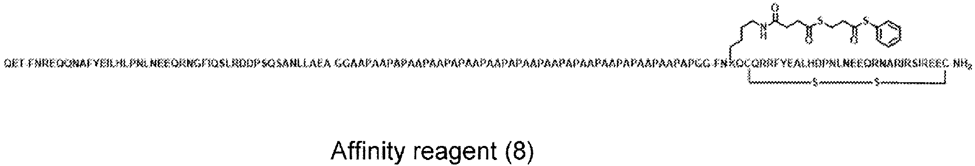

The aforementioned amino acid sequences is the amino acid sequence of SEQ ID NO: 8.

### (Example 4) Specific Modification of the Anti-HER2 IgG Antibody Trastuzumab and Synthesis of ADC Mimic

### (4-1) Specific Modification of Anti-HER2 IgG Antibody Trastuzumab Using Affinity Reagent (4)

500 µg of the anti-HER2 antibody trastuzumab (Chugai Pharmaceutical Co., Ltd.) was dissolved in 171 µL of 50 mM sodium acetate buffer (pH 5.5). A solution of the affinity reagent (4) synthesized in Example 3 in N,N'-dimethylformamide (10 equivalents) was added to the trastuzumab solution, and stirred at room temperature for 1 hour. After removing excess affinity reagent (4) by ultrafiltration, the mass was measured by ESI-TOFMS; for the raw material trastuzumab, a peak was observed at 148400, for a product with one binding peptide introduced, a peak was observed at 159300, and for a product with two binding peptides introduced, a peak was observed at 170390.

### (4-2) Confirmation of the Peptide/Antibody Bonding Ratio of the Specific Modified Form of Trastuzumab by DAR Calculator

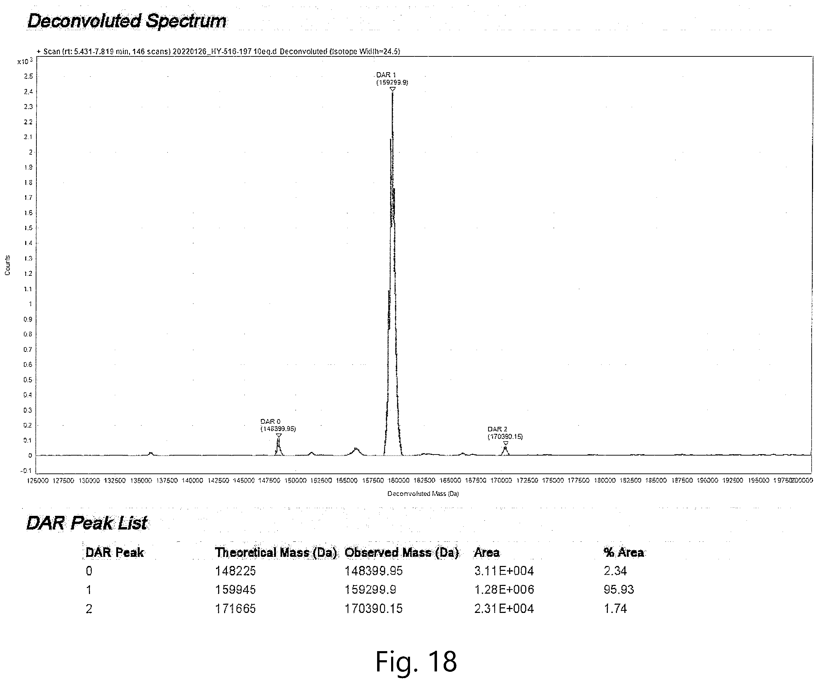

For MS data analyzed in (4-1), the peptide/antibody bonding ratio was confirmed by DAR calculator (Agilent Software), and the results are shown in Table 2 and FIG. 18. The average peptide/antibody bonding ratio calculated from DAR peak and %Area in Table 2 was 1.0. Therefore, the production of an antibody intermediate (average peptide/antibody bonding ratio: 1.0) was confirmed. In addition, based on the %Area ratio, it was found that a compound with one peptide introduced to the antibody was obtained with approximately 96% selectivity.

**Table 2.**

| DAR Peak | Observed mass (Da) | Peak Area | %Area |
|---|---|---|---|
| 0 | 148400 | 3.11E+004 | 2.34 |
| 1 | 159300 | 1.28E+006 | 95.93 |
| 2 | 170390 | 2.31E+004 | 1.74 |

### (4-3) Synthesis of Thiol Group-Introduced Antibody Derivative (T-1-SH) by Cleavage of Thioester Group

The antibody obtained above was subjected to the cleavage reaction (treatment with hydroxylamine) of the thioester group according to the previous report (WO2019/240287) to obtain the thiol group-introduced antibody derivative (T-1-SH) with one thiol group introduced, of the structural formula below. The masses were measured by ESI-TOFMS. The peak was confirmed at 148489 at which the cleavage reaction proceeded.

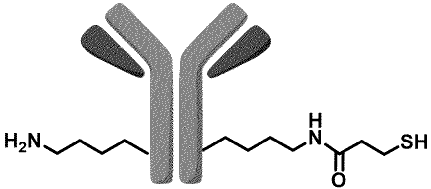

### (4-4) Synthesis of Thiol-group-Introduced Antibody Derivatives by Specific Modification of the Anti-HER2 Antibody Trastuzumab using Affinity Reagent (6) and Cleavage of the Thioester Group

Similarly, the modification reaction of trastuzumab was performed using Affinity reagent (6) synthesized in Example 3. After removing excess affinity reagent (6), the mass was measured by ESI-TOFMS; for the raw material trastuzumab, a peak was observed at 148227, for a product with one binding peptide introduced, a peak was observed at 160165, and for a product with two binding peptides introduced, a peak was observed at 171953. The peptide/antibody bonding ratio was confirmed by DAR calculator (Agilent Software). The results are shown in Table 3.

**Table 3.**

| DAR Peak | Observed mass (Da) | Peak Area | %Area |
|---|---|---|---|
| 0 | 148227 | 9.64E+004 | 14.38 |
| 1 | 160165 | 5.24E+005 | 78.12 |
| 2 | 171953 | 4.99E+004 | 7.44 |

The antibody obtained above was subjected to the cleavage reaction of the thioester group according to the previous report (WO2019/0240287) to obtain the thiol group-introduced antibody derivative (T-1-SH) with one thiol group introduced. The masses were measured by ESI-TOFMS. The peak was confirmed at 148489 at which the cleavage reaction proceeded.

### (4-5) Peptide Mapping by Trypsinization

The thiol-introduced trastuzumab (T-1-SH) obtained in (4-3) was subjected to peptide mapping in the following step.

### (4-5-1) Trypsinization of the Thiol-Introduced Trastuzumab

To 1.5 mL low-adsorption microtest tube, 10 µL of the sample solution, 150 mM tris-hydrochloric acid buffer (pH8.0), and 10 µL of 20 mM dithiothreitol aqueous solution dissolved in 40% trifluoroethanol were added, and after heating at 65°C for 1 hour, 10 µL of 50 mM iodoacetamide aqueous solution was added, and the mixture was allowed to react for 30 minutes at room temperature under light shielding. After the reaction, 40 µL of 150mM tris-hydrochloric acid buffer (pH8.0) was added and stirred, and 10 µL of 20 ng/µL of trypsin aqueous solution was added and enzymatically digested at 37°C for 16 hours. After digestion, 2 µL of 20% aqueous trifluoroacetic acid solution was added to stop the reaction. Then, the sample solution was diluted 10-fold with an aqueous solution containing 0.1% formic acid and 2% acetonitrile, and subjected to LC-MS/MS measurement.

### (4-5-2) LC-MS/MS Determination of Trastuzumab

### (Analyzer)

Nano HPLC: EASY-nLC 1000 (Thermo Fisher Scientific)
Mass Spectrometer: Tribrid Mass Spectrometer Orbitrap Fusion (Thermo Fisher Scientific)

### (HPLC Analysis Conditions)

Trap Column: Acclaim PepMap^{®} 100, 75 µm × 2 cm (Thermo Fisher Scientific)
Analytical columns: ESI-column (NTCC-360/75-3-125, 75 µm × 12.5 cm, 3 µm
(Nikkyo Technos Co., Ltd.)
Mobile phase A: 0.1% formic acid in water
Mobile phase B: 0.1% formic acid, acetonitrile solution
Loading solution: 0.1% aqueous trifluoroacetic acid solution
Flow rate: 300 nL/min
Sample injection volume: 0.6 µL
Gradient conditions (B %): 2% (0.0-0.5 min), 2% to 50% (0.5-50 min), 50% (50-55.5 min), 50% to 95% (55.5-56.5 min), 95% (56.5-60 min)

### (Mass Spectrometer Analysis Conditions)

Ionization method: ESI, Positive
Scan Type: Data Dependent Aquisition
Activation Type: Collision Induced Dissociation (CID)

Data were acquired using the attached software Xcalibur 4.3 (Thermo Fisher Scientific) and Thermo Orbitrap Fusion Tune Application 3.3 (Thermo Fisher Scientific).

### (4-5-3) Analysis of Trastuzumab Modification Sites

Modification site analysis on the LC-MS/MS measurement result was performed using BioPharma Finder 3.2 (Thermo Fisher Scientific).

Analysis with BioPharma Finder was performed with S/N Threshold set to 64 and MS Noise Level set to 4000. The digestive enzyme was set to Trypsin and Specificity to High. For Static Modification, Carbamidomethyl (+57.021 Da) was set as modification of a cysteine residue with iodoacetamide. For Dynamic Modifications, oxidation of a methionine residue and a tryptophan residue (+15.995 Da), deamidation of a glutamic acid residue and an aspartic acid residue (+0.984Da), and a modified compound to a lysine residue (thiol-introduced compound subjected to carbamidomethylation with iodoacetamide (+145.019 Da)) were set. In addition, for Dynamic Modification on the N-terminus of the protein, pyroglutamylation of a glutamic acid residue (-18.011Da) was set, and for Dynamic Modification on the C-terminus of the protein, addition of lysine (+128.095Da) was set. In addition, a filter was set such that only those with a Confidence Score of 80 or higher, Mass Accuracy at the time of peptide identification of 5 ppm or less, and with observed MS/MS were obtained. The residue number of the lysine residue on the heavy chain VH domain and the light chain was indicated by the number in the sequence (i.e., the first amino acid at the N-terminal end; the same shall apply hereinafter), and those on the heavy chain CH1, CH2, and CH3 domains were indicated according to the EU numbering.

### (4-5-4) Analysis Result of Modified Site of Trastuzumab by LC-MS/MS

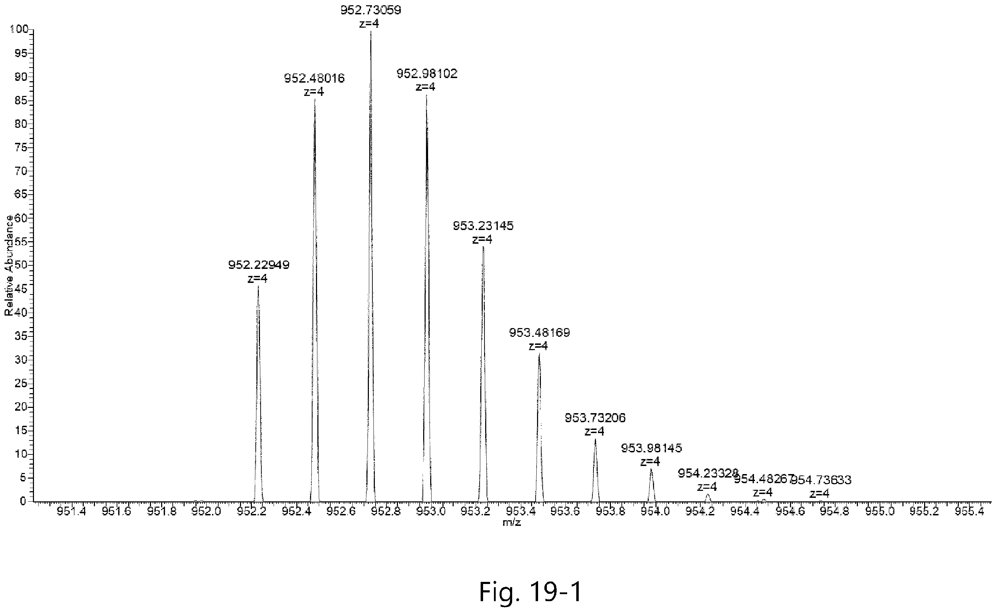

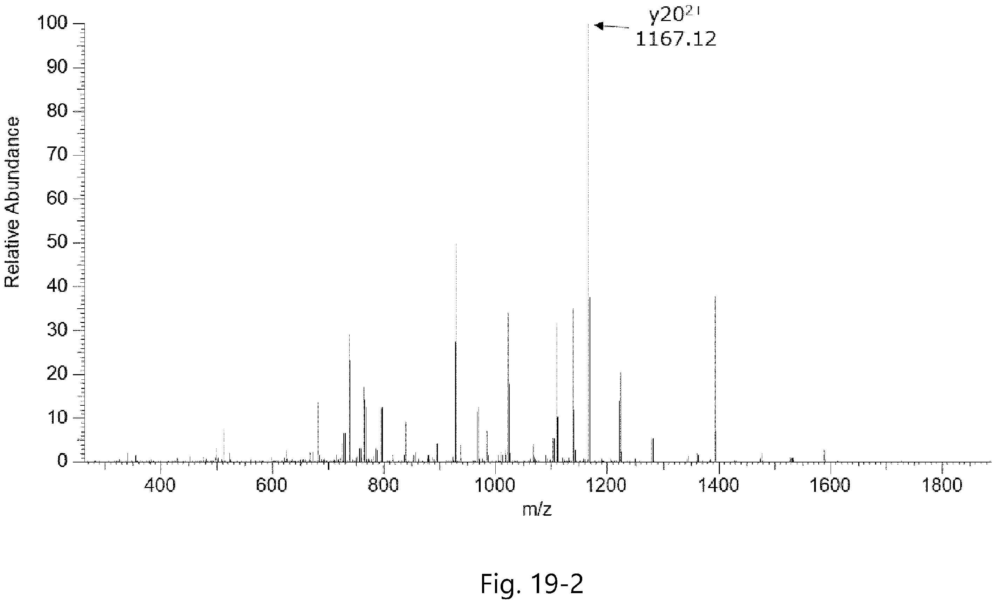

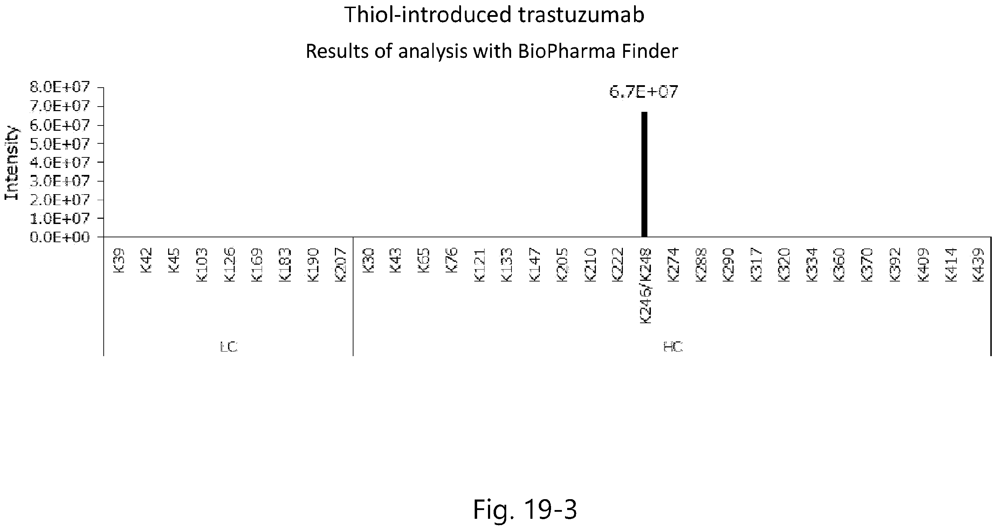

As a result of analysis using LC-MS/MS, an MS spectrum of a peptide fragment of THTCPPCPAPELLGGPSVFLFPPKPKDTLMISR (SEQ ID NO: 25), which is a peptide composed of 23 amino acid residues comprising a modified site to a lysine residue by trypsin digestion of trastuzumab (thiol-introduced compound (+145.019 Da) subjected to carbamidomethylation with iodoacetamide) (measured value: m/z 952.22949; theoretical value: 952.22942; tetravalent) was observed (FIG. 19-1); and from a CID spectrum, a product ion of m/z 1167.12 (theoretical value: 1166.61) corresponding to divalent y20 indicating modification of a lysine residue at the position 246 or 248 of a heavy chain according to the EU numbering was determined (FIG. 19-2). Analysis with BioPharma Finder also showed that the modification to the lysine residue at the position 248 occurred highly selectively (FIG. 19-3).

From this result, it was found that in the thiol-introduced trastuzumab (T-1-SH) obtained in (4-3) above, conjugation progressed regioselectively at Lys246 or Lys248 according to the EU numbering on the heavy chain of the antibody.

### (4-6) Synthesis of ADC Mimic

The thiol-introduced antibody (T-1-SH, PBS solution) obtained in Example (4-3) was allowed to react to m-dPEG-maleimide (quanta biodesign, Compound 10). After the reaction, the mass was measured by ESI-TOFMS. As a result, a peak was confirmed at 149735. DAR was 0.9.

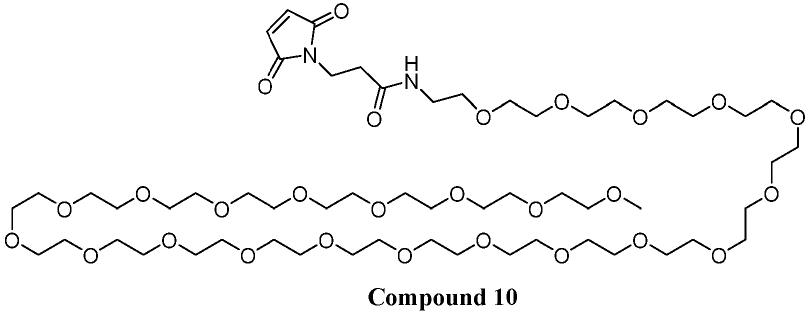

### (Example 5) Specific modification of Other IgG Antibody

The modifying reagent (4) was reacted with various antibodies under the conditions in Example (4-1). Similarly as in Example 4-1, ESI-TOFMS was measured, and average peptide/antibody bonding ratio was calculated using DAR calculator. The antibodies and the average peptide/antibody bonding ratios are listed in Table 4.

**Table 4.**

| Antibody | Peptide/Antibody Bonding Ratio |
|---|---|
| Rituximab | 0.9 |
| Infliximab | 0.9 |
| Cetuximab | 1.0 |

From the above, it was found that the peptide/antibody bonding ratio can be controlled regardless of differences in antibodies.

### (Example 6) Specific Introduction of Azide Group to Anti-HER2 IgG Antibody Trastuzumab and Synthesis of ADC Mimic

### (6-1) Synthesis of Small Linker Having Azide Group

### (6-1-1) Synthesis of Linker Intermediate (11)

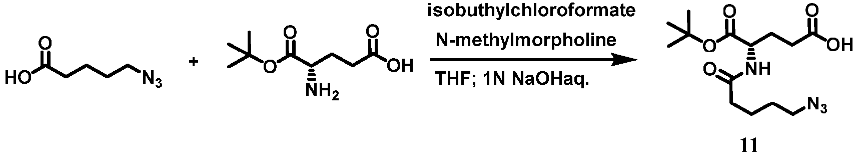

5-azidovaleric acid (800 mg, 5.59 mmol) was dissolved in THF (14 mL), to which isobutyl chloroformate (808 µL, 6.15 mmol) and N-methylmorpholine (873 µL, 8.39 mmol) were added followed by stirring at 0°C for 30 minutes, and then hydrazine hydrate (1.36 g, 6.71 mmol) dissolved in 1 M NaOH aqueous solution (4 mL) was added followed by stirring at room temperature for 3 hours. After concentration under reduced pressure, 1 M NaOH aqueous solution was added to adjust the pH in the system to pH10. After washing with ethyl acetate, 1M HCl aqueous solution was added to the aqueous layer to adjust the pH in the system to 3.0. After washing with addition of ethyl acetate, sodium sulfate was added to the resulting ethyl acetate solution. Sodium sulfate was removed by filtration, and the resulting solution was concentrated under reduced pressure, purified by column chromatography (dichloromethane: methanol = 10:1). Fractions containing the product were collected and concentrated under reduced pressure to obtain a linker intermediate (11).

¹H NMR (400 MHz, Chloroform-d) δ6.29 (d, J = 7.7 Hz, 1 H), 4.56 (td, J = 8.0, 4.9 Hz, 1H), 3.32 (t,J = 6.6 Hz, 2H), 2.53-2.38 (m, 3H), 2.36-2.16 (m, 3H), 2.12 (s, 2H), 1.96 (dq, J = 14.7, 7.6 Hz, 1H), 1.84-1.59 (m, 4H), 1.50 (s, 9H).

MS (ESI) m/z: 329 [M+H]⁺

### (6-1-2) Synthesis of Linker Intermediate (12)

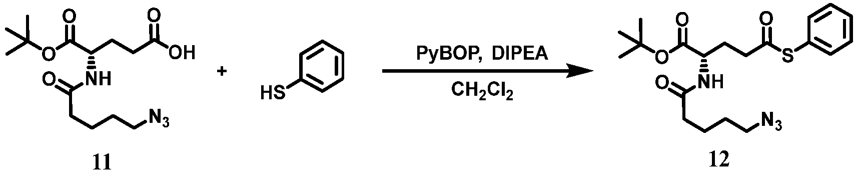

The linker intermediate (11) (2.41 g, 5.59 mmol) was dissolved in dichloromethane (28 mL), and thiophenol (627 µL, 6.15 mmol), benzotriazole-1-yloxy (3.49 g, 6.71 mmol), and DIPEA (1.42 mL, 8.39 mmol) were added and stirred at room temperature for 2 hours. Thereafter, the resulting product was concentrated under reduced pressure and purified by column chromatography (hexane: ethyl acetate = 4:1). Fractions containing the product were collected and concentrated under reduced pressure to obtain a linker intermediate (12) (2.20 g, 5.23 mmol).

¹H NMR (400 MHz, Chloroform-d)δ7.43 (s, 5H), 6.10 (d,J = 7.8 Hz, 1H), 4.55 (td, J = 7.7, 4.9 Hz, 1H), 3.31 (t, J = 6.7 Hz, 2H), 2.87-2.63 (m, 2H), 2.28 (dd, J = 8.7, 5.9 Hz, 2H), 2.16-1.98 (m, 1H), 1.83-1.58 (m, 4H), 1.50 (s, 9H), 1.37-1.22 (m, 2H), 0.91 (t, J = 6.7 Hz, 1H).

MS (ESI) m/z: 421 [M+H]⁺

### (6-1-3) Synthesis of Linker Intermediate (13)

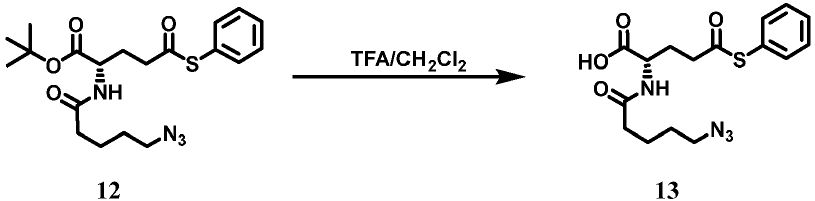

The linker intermediate (12) (2.20 g, 5.23 mmol) was dissolved in dichloromethane (10 mL), to which trifluoroacetic acid (10 mL) was added and stirred at room temperature for 1 hour. Then, the resulting product was concentrated under reduced pressure to remove dichloromethane, and water was added for freeze-drying to obtain a linker intermediate (13) (1.98 g, 5.43 mmo).

¹H NMR (400 MHz, Chloroform-d)δ7.44 (s, J = 6.3, 4.6, 2.4 Hz, 5H), 6.76 (s, 1H), 4.62 (td, J = 7.5, 4.9 Hz, 1H), 3.31 (t, J = 6.6 Hz, 2H), 2.88 (qt, J = 16.8, 6.8 Hz, 2H), 2.33 (dt, J = 12.4, 6.8 Hz, 3H), 2.18 (dq, J = 14.4, 7.4 Hz, 1H), 1.74 (dq, J = 11.8, 7.5, 6.9 Hz, 2H), 1.63 (ddd, J = 17.7, 10.5, 4.8 Hz, 2H).

MS (ESI) m/z: 365 [M+H]⁺

### (6-1-4) Synthesis of Linker Intermediate (14)

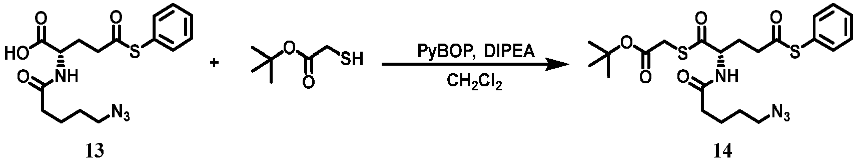

The linker intermediate (13) (100 mg, 0.274 mmo) was dissolved in dichloromethane (3 mL), and (40.6 µL, 0.280 mmol), benzotriazole-1-yloxy (150 mg, 0.288 mmol), DIPEA (70.1 µL, 0.412 mmol) were added and stirred at room temperature for 2 hours. 1 M HCl aqueous solution was added to adjust the pH of the system to 3, followed by dilution with dichloromethane. The mixture was then washed with water and brine, and sodium sulfate was added. Sodium sulfate was removed by filtration, and the resulting solution was concentrated under reduced pressure and purified by column chromatography (hexane: ethyl acetate = 4:1). Fractions containing the product were collected and concentrated under reduced pressure to obtain a linker intermediate (14) (84.7 mg, 0.171 mmol).

¹H NMR (400 MHz, Chloroform-d)δ7.50-7.38 (m, 5H), 6.33 (d, J = 8.4 Hz, 1H), 4.78 (tdd, J = 7.8, 4.6, 3.0 Hz, 1H), 3.70-3.54 (m, 2H), 3.32 (dt, J = 9.1, 6.7 Hz, 2H), 2.96-2.67 (m, 2H), 2.30 (pd, J = 7.1, 4.5 Hz, 2H), 1.85-1.60 (m, 6H), 1.49 (d, J = 2.8 Hz, 9H).

MS (ESI) m/z: 495 [M+H]⁺

### (6-1-5) Synthesis of Linker Intermediate (15)

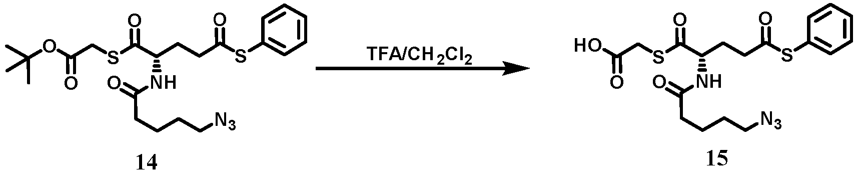

The linker intermediate (14) (84.7 mg, 0.171 mmol) was dissolved in dichloromethane (5 mL), to which trifluoroacetic acid (5 mL) was added and stirred at room temperature for 1 hour. Then, the resulting product was concentrated under reduced pressure to remove dichloromethane, and water was added for freeze-drying and purified by column chromatography (dichloromethane: methanol = 10: 1). Fractions containing the product were collected and concentrated under reduced pressure to obtain a linker intermediate (15) (46.8 mg, 0.107 mmo).

¹H NMR (400 MHz, Methanol-d4)δ7.44 (dq, J = 2.3, 1.5 Hz, 5H), 4.69-4.57 (m, 1H), 3.79-3.67 (m, 2H), 3.40-3.30 (m, 2H), 2.89-2.71 (m, 2H), 2.44-2.23 (m, 4H), 2.08-1.95 (m, 1H), 1.82-1.61 (m, 4H).

MS (ESI) m/z: 439 [M+H]⁺

### (6-2) Synthesis of Modifying Reagent (9) Having Azide Group

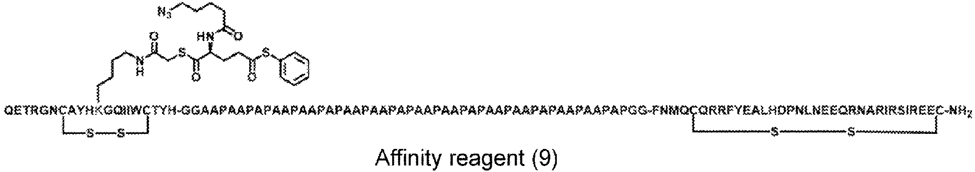

The aforementioned amino acid sequences is the amino acid sequence of SEQ ID NO: 4.

Amidation of affinity substance QET-Fc3K-PA48-Z34CM and a linker intermediate (16) was performed according to Example 3 to prepare an affinity reagent (9).

MS (ESI) m/z: z = 14 798.65 [M+3H]³⁺

### (6-3) Specific Modification of Anti-HER2 Antibody Trastuzumab

500 µg of the anti-HER2 IgG antibody trastuzumab (Chugai Pharmaceutical Co., Ltd.) was dissolved in 171 µL of 50 mM sodium acetate buffer (pH 5.5). A solution of the affinity reagent (9) synthesized in Example 7-2 in N,N'-dimethylformamide (10 equivalents) was added to the trastuzumab solution, and stirred at room temperature for 16 hours. After removing excess affinity reagent (9) by ultrafiltration, the mass was measured by ESI-TOFMS; for the raw material trastuzumab, a peak was observed at 148400, and for a product with one binding peptide introduced, a peak was observed at 159442. In addition, the production of an antibody intermediate (average peptide/antibody bonding ratio: 1.0) was confirmed by DAR calculator.

### (6-4) Synthesis of Azide Group-introduced Antibody Derivative (T-1-N3) by Cleavage of Thioester Group

A thiol group-introduced antibody derivative was subjected to cleavage reaction of a thioester group with methoxyamine according to the previous report (WO2019/0240287) to obtain an antibody with one azide group introduced corresponding to the structural formula below. The masses were measured by ESI-TOFMS. The peak was confirmed at 148672 at which the cleavage reaction proceeded.

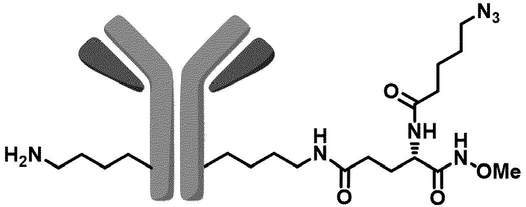

### (6-5) Synthesis of ADC Mimic

The azide-introduced antibody (T-1-N3) (PBS solution) obtained in Example (6-4) was allowed to react to m-dPEG-DBCO (quanta biodesign, Compound 16). After the reaction, the mass was measured by ESI-TOFMS. As a result, a peak was confirmed at 149893. DAR was 0.9.

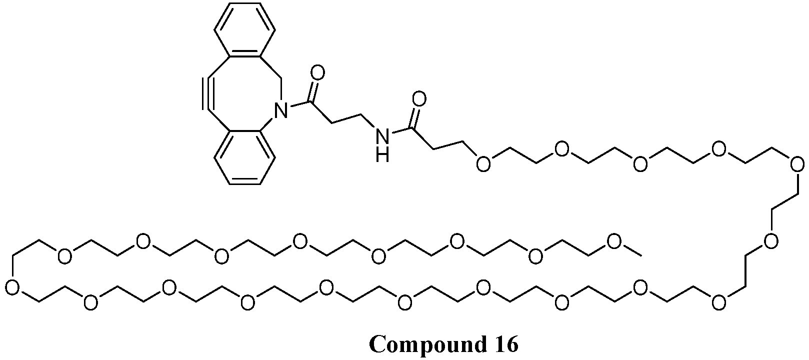

### (Comparative Example 1) Specific Modification of Anti-HER2 Antibody Trastuzumab Using Antibody Modifying Reagent for Obtaining DAR = 2

Specific modification of trastuzumab was performed using affinity reagent (17) that has been reported to give ADC with DAR = 2 in a previous report (WO2019/0240287). In this process, in order to enhance the selectivity of the compound with one peptide introduced into an antibody by examining the equivalents of the reagent, the equivalents of the affinity reagent (17) were examined.

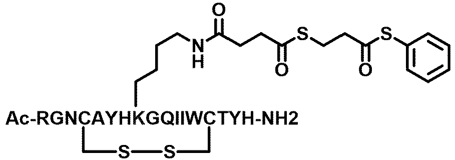

### Modifying reagent (17)

**The aforementioned amino acid sequence is the amino acid sequence of SEQ ID NO: 26.**

**Table 5.**

| Equivalents of affinity reagent (17) | Selectivity of compound in which one peptide is introduced to antibody |
|---|---|
| 0.2 | 5.7% |
| 0.5 | 26.6% |
| 0.8 | 40.4% |
| 1.2 | 59.8% |
| 1.5 | 44.3% |
| 1.8 | 23.5% |
| 2.0 | 20.8% |

The selectivity was determined using %Area from the DAR calculator, as in Example 4-2. As shown in Table 5, the modification reagent (17) showed a selectivity of only less than 60%, even when the equivalents were changed. On the other hand, as shown in Examples 4-1 and 4-4, the affinity reagents (4) and (6) showed selectivity of 65% or more, successfully yielding a compound with one peptide introduced into the antibody.

### (Example 7) Synthesis of Antibody with One Thiol Group Molecule and One Azide Group Molecule Introduced

### (7-1) Synthesis of Trastuzumab with One Thiol Group Molecule and One Azide Group Molecule Introduced (T-2-N3/SH)

### (7-1-1) Single-molecule Specific Modification of Anti-HER2 Antibody Trastuzumab using Affinity Reagent Through Conjugation and HPLC Purification

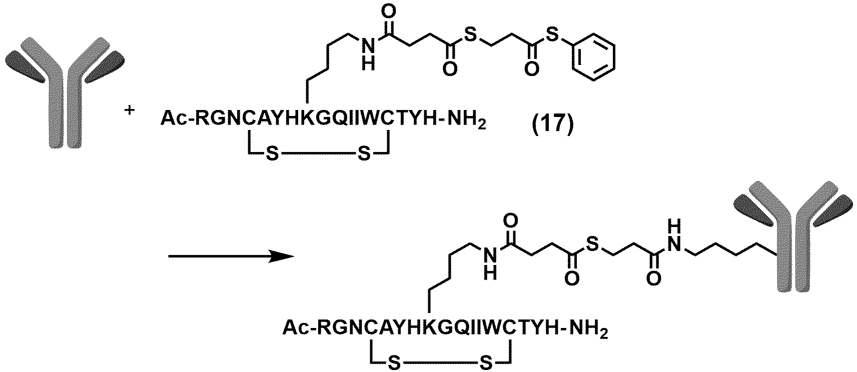

**The aforementioned amino acid sequence is the amino acid sequence of SEQ ID NO: 26.**

To a solution of 10 mg/mL anti-human HER2 monoclonal antibody Trastuzumab (Chugai Pharmaceutical Co., Ltd.) in an acetate buffer (50 mM Sodium acetate, pH5.5), a solution of a modifying reagent (17) described in the previous report (WO2019/240287A1) in dimethylformamide (1 equivalent relative to the antibody, dimethylformamide (DMF) (8%v/v) solution) was added, and then shaken at room temperature for 1 hour. The reaction solution was purified using a NAP-25 desalting column (Cytiva) and then using AKTA pure25 (Cytiva) to obtain an antibody with one peptide reagent molecule introduced. For DAR analysis of the antibody with one peptide reagent molecule introduced, HIC-HPLC analysis was performed according to the previous report (Anal. Chem., 2019, 91, 20, 12724-12732) to confirm that one peptide reagent was introduced.

The purification with AKTA pure25 (Cytiva) was performed under the following conditions:
Column: RESOURCE S (Cytiva)
Eluent A: 50 mM Sodium acetate, 0.1% tween 20 (pH5.0)
Eluent B: 50 mM Sodium acetate, 1 M NaCl, 0.1% tween 20 (pH5.0)
Flow rate: 5.0 ml/min
Detector: Detection was performed at wavelengths of 215 and 280 nm.

### (7-1-2) Preparation of Affinity Reagent (18) Having Azide Group

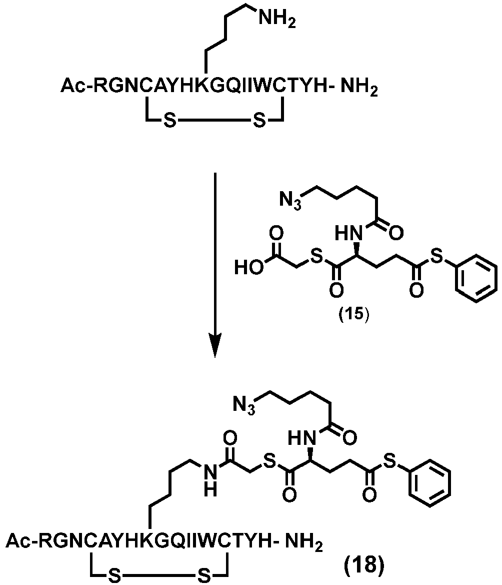

**Both the above amino acid sequences are the amino acid sequence of SEQ ID NO: 26.**

Ac-RGNCAYHKGQIIWCTYH-NH2 described in the previous report (WO2019/240287A1) (SEQ ID NO: 26, 30.9 mg, 14.9 µmol, where the two cysteines at positions 4 and 14 each form a disulfide bond in the molecule) was dissolved in dimethylformamide (468 µL), and then the linker intermediate (15) synthesized in Example 6-1-5 (46.8 mg, 0.107 mmol) and WSC·HCl (29.7 mg, 0.155 mmol) were added, and the mixture was stirred at room temperature for 5 hours and eluted by reversed phase preparative chromatography. The fraction containing the product was collected, and the acetonitrile was removed by concentration under reduced pressure, followed by lyophilization to obtain the modifying reagent (18) (15.1 mg, 6.02 µmol).

### (7-1-3) Introduction of Two Peptide Reagent Molecules to Trastuzumab

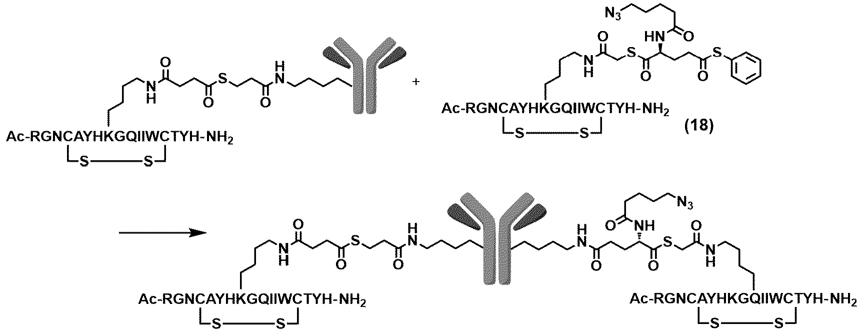

**Both the above amino acid sequences are the amino acid sequence of SEQ ID NO: 26.**

Thereafter, a conjugation reaction was performed on the antibody with one peptide reagent molecule introduced obtained in Example 7-1-1 using the peptide reagent (18) prepared in Example 7-1-2 according to the method in the previous report (WO2019/240287A1). As a result, an antibody with the modifying reagents (17) and (18) introduced was obtained. For DAR analysis of the antibody with the peptide reagents (17) and (18) introduced, HIC-HPLC analysis was performed according to the previous report *(*Anal. Chem., 2019, 91, 20, 12724-12732*)* to confirm that two peptide reagents were introduced.

### (7-1-4) Synthesis of Trastuzumab (T-1) with one thiol group molecule and one azide group molecule introduced

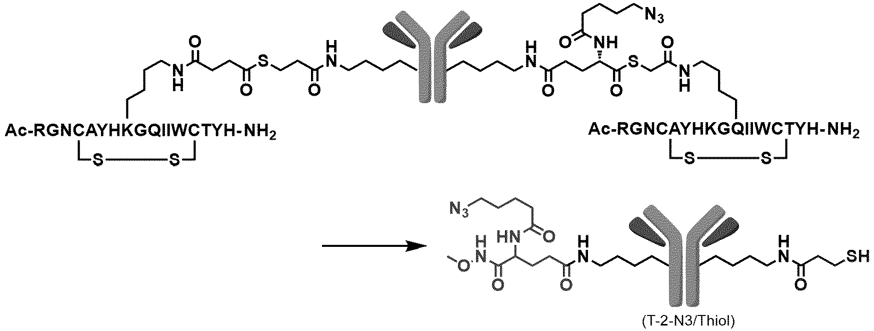

**Both the above amino acid sequences are the amino acid sequence of SEQ ID NO: 26.**

A cleavage reaction was performed on the antibody with the modifying reagents (17) and (18) introduced obtained in Example 7-1-2, with reference to the technique in the previous report (WO2019/240287A1), by adding a methoxyamine solution and shaking the mixture at room temperature for 3 hours. This resulted in obtaining an antibody with one thiol group molecule and one azide group molecule (T-2-N3/SH). For DAR analysis of the antibody with one thiol group molecule and one azide group molecule (T-2-N3/SH) introduced, HIC-HPLC analysis was performed according to the previous report (Anal. Chem., 2019, 91, 20, 12724-12732) to confirm that one thiol group molecule and one azide group molecule were introduced.

### (7-1-5) Introduction of Two Peptide Reagent Molecules to Trastuzumab

A conjugation reaction was performed on the antibody with one affinity reagent (4) molecule introduced obtained in Example 4-1 using the peptide reagent (18) prepared in Example 7-1-2 according to the method in the previous report (WO2019/240287A1). As a result, an antibody with the affinity reagent (4) and the affinity reagent (18) introduced was obtained. For DAR analysis of the antibody with the affinity reagent (4) and the modifying reagent (11) introduced, HIC-HPLC analysis was performed according to the previous report (Anal. Chem., 2019, 91, 20, 12724-12732) to confirm that two peptide reagents were introduced.

### (7-1-6) Synthesis of Trastuzumab with One Thiol Group Molecule and One Azide Group Molecule Introduced (T-1)

A cleavage reaction was performed on the antibody with the modifying reagents (4) and (18) introduced obtained in Example 7-1-5, according to Example 7-1-4, by adding a methoxyamine solution and shaking the mixture at room temperature for 3 hours. This resulted in obtaining an antibody with one thiol group molecule and one azide group molecule (T-2-N3/SH). For DAR analysis of the antibody with one thiol group molecule and one azide group molecule (T-2-N3/SH) introduced, HIC-HPLC analysis was performed according to the previous report (Anal. Chem., 2019, 91, 20, 12724-12732) to confirm that one thiol group molecule and one azide group molecule were introduced.

### (7-1-7) Introduction of Two Peptide Reagent Molecules to Trastuzumab

A conjugation reaction was performed on the antibody with one affinity reagent (9) molecule introduced obtained in Example 6-3 using the modifying reagent (17) according to the method in the previous report (WO2019/240287A1). As a result, an antibody with the affinity reagent (9) and the modifying reagent (17) introduced was obtained. For DAR analysis of the antibody with the affinity reagent (9) and the modifying reagent (17) introduced, HIC-HPLC analysis was performed according to the previous report (Anal. Chem., 2019, 91, 20, 12724-12732) to confirm that two peptide reagents were introduced.

### (7-1-8) Synthesis of Trastuzumab with One Thiol Group Molecule and One Azide Group Molecule Introduced (T-1)

A cleavage reaction was performed on the antibody with the modifying reagents (9) and (17) introduced obtained in Example 7-1-7, according to Example 7-1-4, by adding a methoxyamine solution and shaking the mixture at room temperature for 3 hours. This resulted in obtaining an antibody with one thiol group molecule and one azide group molecule (T-2-N3/SH). For DAR analysis of the antibody with one thiol group molecule and one azide group molecule (T-2-N3/SH) introduced, HIC-HPLC analysis was performed according to the previous report (Anal. Chem., 2019, 91, 20, 12724-12732) to confirm that one thiol group molecule and one azide group molecule were introduced.

### (7-2) Synthesis of Cetuximab with One Thiol Group Molecule and One Azide Group Molecule Introduced (C-2-N3/SH)

An antibody with one thiol group molecule and one azide group molecule introduced (C-2-N3/SH) was obtained according to Example 7-1, by using an anti-human EGFR monoclonal antibody Cetuximab (Merck Biopharma). For DAR analysis of the antibody with one thiol group molecule and one azide group molecule introduced, HIC-HPLC analysis was performed according to the previous report (Anal. Chem., 2019, 91, 20, 12724-12732) to confirm that one thiol group molecule and one azide group molecule were introduced.

### (7-3) Synthesis of Cetuximab with One Azide Group Molecule Introduced (C-1-N3)

An antibody with one modifying reagent (9) introduced (C-1-N3) was obtained according to Example 6-3, by using an anti-human EGFR monoclonal antibody Cetuximab (Merck Biopharma) and the modifying reagent (9).

Subsequently, a methoxyamine solution was added according to the previous report (WO2019/240287A1) and shaken at room temperature for 3 hours to obtain an antibody (C-1-N3) with only one azide group introduced. For DAR analysis of the antibody with only one azide group molecule and one azide group molecule introduced, HIC-HPLC analysis was performed according to the previous report (Anal. Chem., 2019, 91, 20, 12724-12732) to confirm that only one azide group molecule was introduced.

### (7-4) Synthesis of Cetuximab with Two Azide Group Molecules Introduced (C-2-N3)

To a solution of an anti-human EGFR monoclonal antibody, Cetuximab (Merck Biopharma) in an acetate buffer (50 mM Sodium acetate, pH5.5), the peptide reagent (18) prepared in Example 7-1-2 was added and shaken at room temperature for 1 hour according to the previous report (WO2019/240287A1).

Subsequently, a methoxyamine solution was added according to the previous report (WO2019/240287A1) and shaken at room temperature for 3 hours to obtain an azide group-introduced antibody (C-2-N3). For DAR analysis of the antibody with two azide group molecules introduced, HIC-HPLC analysis was performed according to the previous report (Anal. Chem., 2019, 91, 20, 12724-12732) to confirm that two azide group molecules were introduced.

### (Example 8) Synthesis of Fab Molecule with Linker Introduced

### (8-1) Introduction of Linker to Cetuximab

### (8-1-1) Synthesis of Cetuximab F(ab)₂

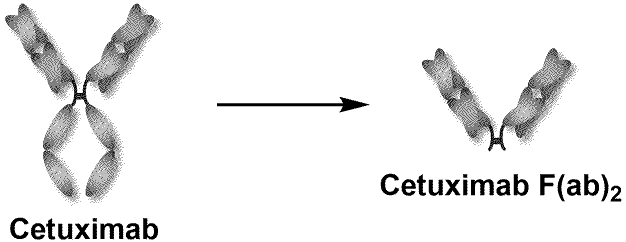

To a solution of 10 mg/mL anti-human EGFR monoclonal antibody, Cetuximab (Merck Biopharma) in an acetate buffer (50 mM Sodium acetate, pH4.5), Immobilized Pepsin (Thermo Fisher) was added and shaken at 37°C for 4 hours. The reaction solution was purified using a NAP-25 desalting column (Cytiva) and then using AKTA pure25 (Cytiva) to obtain a Cetuximab F(ab)₂ molecule. For Fab analysis, ESI-TOFMS analysis was performed according to the previous report (Anal. Chem., 2019, 91, 20, 12724-12732) to observe a peak at 101581.

The purification with AKTA pure25 (Cytiva) was performed under the following conditions:
Column: RESOURCE S (Cytiva)
Eluent A: 50 mM Sodium acetate, 0.1% tween 20 (pH5.0)
Eluent B: 50 mM Sodium acetate, 1 M NaCl, 0.1% tween 20 (pH5.0)
Flow rate: 5.0 ml/min
Detector: Detection was performed at wavelengths of 215 and 280 nm.

### (8-1-2) Synthesis of Cetuximab Fab

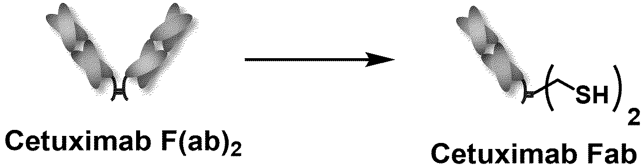

To a solution of F(ab)₂ obtained in Example 8-1-1 in a PBSE buffer (10 mM Phosphate Buffered Saline (PBS), 10 mM EDTA, pH7.4), a solution of 2-Mercaptoethylamine in a PBSE buffer (10 mM) was added and shaken at 37°C for 1 hour. The reaction solution was purified using a NAP-25 desalting column (Cytiva) to obtain Fab. For Fab analysis, ESI-TOFMS analysis was performed according to the previous report (Anal. Chem., 2019, 91, 20, 12724-12732) to observe a peak at 50793.

### (8-1-3) Introduction of PEG4-DBCO Group to Cetuximab Fab (C-F-1)

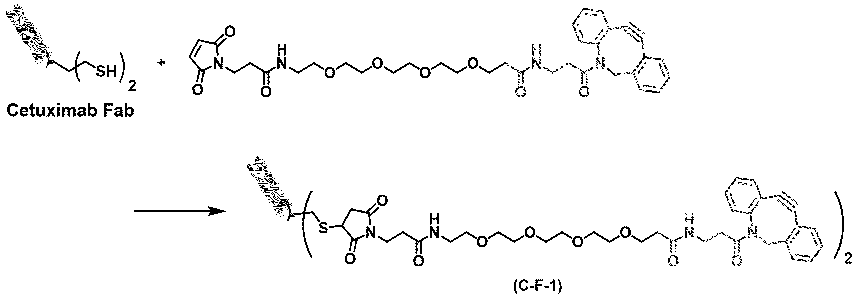

To a solution of Cetuximab Fab obtained in Example 8-1-2 in an acetate buffer (50 mM Sodium acetate, pH5.5), a solution of a commercially-available DBCO-PEG4-Maleimide in dimethylformamide (3 equivalents relative to Fab, dimethylformamide (DMF) (8%v/v) solution) was added, and then shaken at room temperature for 1 hour. The reaction solution was purified using a NAP-25 desalting column (Cytiva) to obtain Fab with a PEG4-DBCO group introduced. ESI-TOFMS analysis was performed according to the previous report (Anal. Chem., 2019, 91, 20, 12724-12732) to observe a peak at 52142.

### (8-1-4) Introduction of PEG12-DBCO Group to Cetuximab Fab (C-F-2)

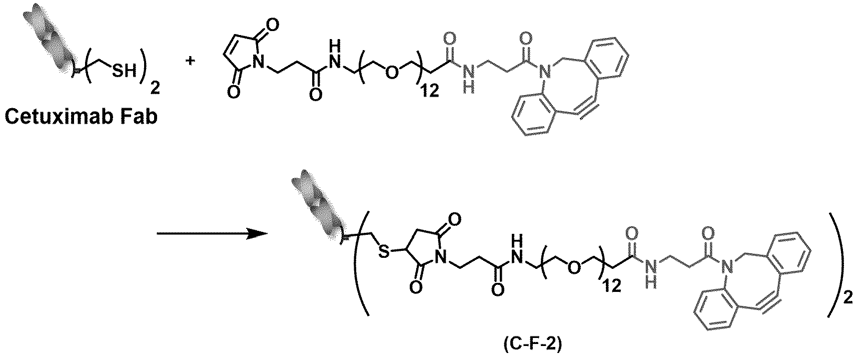

Cetuximab Fab obtained in Example 8-1-2 and a commercially-available DBCO-PEG12-Maleimide were used according to Example 8-1-3 to obtain Fab with a PEG12- DBCO group introduced. ESI-TOFMS analysis was performed according to the previous report (Anal. Chem., 2019, 91, 20, 12724-12732) to observe a peak at 52846.

### (8-2) Introduction of Linker to Trastuzumab

### (8-2-1) Synthesis of Trastuzumab F(ab)₂

An anti-HER2 monoclonal antibody, Trastuzumab (Chugai Pharmaceutical Co., Ltd.) was used according to Example 8-1-1 to obtain a Trastuzumab F(ab)₂ molecule. For Fab analysis, ESI-TOFMS analysis was performed according to the previous report (Anal. Chem., 2019, 91, 20, 12724-12732) to observe a peak at 97286.

### (8-2-2) Synthesis of Trastuzumab Fab

F(ab)₂ obtained in Example 2-2-1 was used according to Example 8-1-2 to obtain Fab. For Fab analysis, ESI-TOFMS analysis was performed according to the previous report (Anal. Chem., 2019, 91, 20, 12724-12732) to observe a peak at 48644.

### (8-2-3) Introduction of PEG4-DBCO Group to Trastuzumab Fab (T-F-1)

Trastuzumab Fab obtained in Example 8-2-2 was used according to Example 8-1-3 to obtain Fab with a PEG4-DBCO group introduced. ESI-TOFMS analysis was performed according to the previous report (Anal. Chem., 2019, 91, 20, 12724-12732) to observe a peak at 49994.

### (8-2-4) Introduction of Azide Group to Trastuzumab Fab (T-F-2)

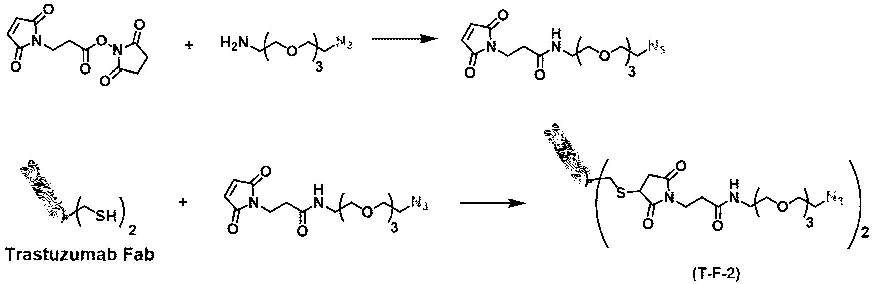

To a solution of a commercially available N-Succinimidyl 3-Maleimidopropionate in dimethylformamide (20 mM), an equal volume of solution of 3-Azidopropylamine in dimethylformamide (20 mM) was added and shaken for 30 minutes to prepare a solution of Azido-PEG3-Maleimide in dimethylformamide (10 mM). To a solution of Trastuzumab Fab obtained in Example 8-2-2 in an acetate buffer (50 mM Sodium acetate (PBS), pH5.5), a solution of a prepared Azido-PEG3-Maleimide in dimethylformamide (3 equivalents relative to Fab, dimethylformamide (DMF) (8%v/v) solution) was added, and then shaken for 1 hour. The reaction solution was purified using a NAP-25 desalting column (Cytiva) to obtain Fab with an azide group introduced. ESI-TOFMS analysis was performed according to the previous report (Anal. Chem., 2019, 91, 20, 12724-12732) to observe a peak at 49383.

### (8-2-5) Introduction of Maleimide Group to Trastuzumab Fab (T-F-3)

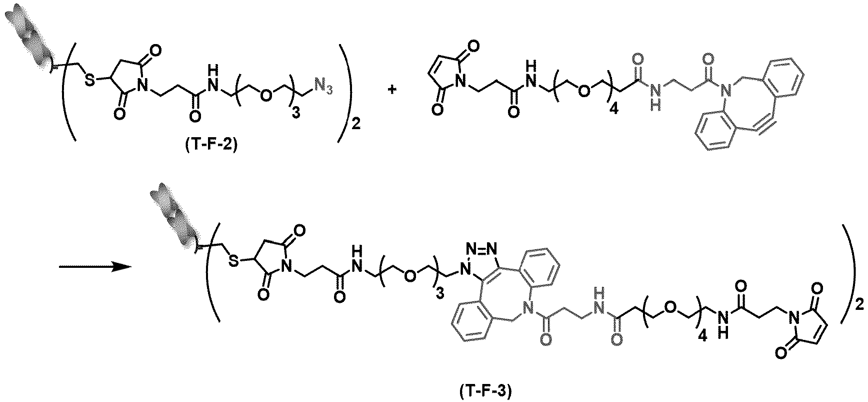

To a solution of Trastuzumab Fab with an azide group introduced obtained in Example 8-2-4 in an acetate buffer (50 mM Sodium acetate, pH5.5), a solution of a commercially available DBCO-PEG4-Maleimide in dimethylformamide (3 equivalents relative to Fab, dimethylformamide (DMF) (8%v/v) solution) was added, and then shaken for 20 hour. The reaction solution was purified using a NAP-25 desalting column (Cytiva) to obtain Tastuzumab Fab with a maleimide group introduced. ESI-TOFMS analysis was performed according to the previous report (Anal. Chem., 2019, 91, 20, 12724-12732) to observe a peak at 50733.

### (8-3) Introduced of Linker to Pembrolizumab

### (8-3-1) Synthesis of Pembrolizumab F(ab)₂

An anti-human PD-1 monoclonal antibody, Pembrolizumab (MSD) was used according to Example 8-1-1 to obtain a Pembrolizumab F(ab)₂ molecule. For Fab analysis, ESI-TOFMS analysis was performed according to the previous report (Anal. Chem., 2019, 91, 20, 12724-12732) to observe a peak at 98151.

### (8-3-2) Synthesis of Pembrolizumab Fab

F(ab)₂ obtained in Example 8-3-1 was used according to Example 8-1-2 to obtain Fab. For Fab analysis, ESI-TOFMS analysis was performed according to the previous report (Anal. Chem., 2019, 91, 20, 12724-12732) to observe a peak at 49076.

### (8-3-3) Introduction of PEG4-DBCO Group to Pembrolizumab Fab (P-F-1)

Pembrolizumab Fab obtained in Example 8-3-2 was used according to Example 8-1-3 to obtain Fab with a PEG4-DBCO group introduced. ESI-TOFMS analysis was performed according to the previous report (Anal. Chem., 2019, 91, 20, 12724-12732) to observe a peak at 50426.

### (8-3-4) Introduction of PEG12-DBCO Group to Pembrolizumab Fab (P-F-2)

Pembrolizumab Fab obtained in Example 8-3-2 was used according to Example 8-1-3 to obtain Fab with a PEG12-DBCO group introduced. ESI-TOFMS analysis was performed according to the previous report (Anal. Chem., 2019, 91, 20, 12724-12732) to observe a peak at 51130.

### (Example 9) Synthesis of Multispecific Antibody

### (9-1) Synthesis of Tri-specific Antibody (M-1)

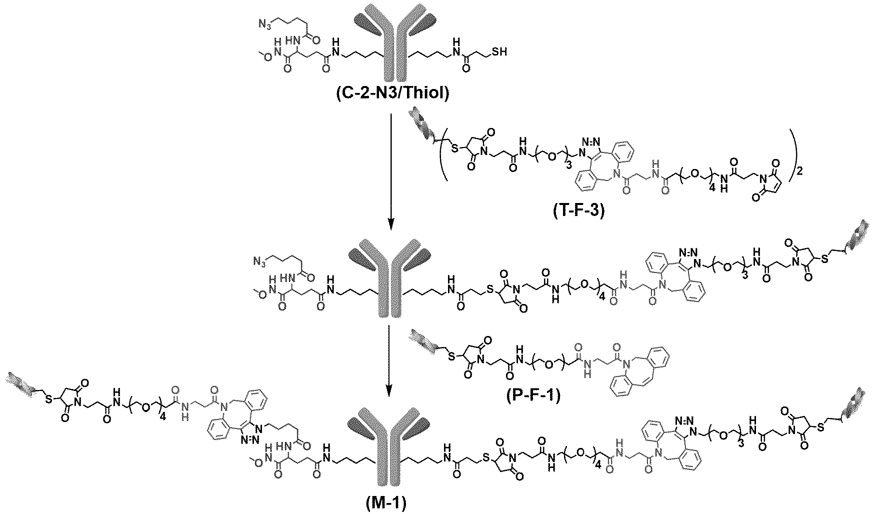

To a solution of the Cetuximab with one thiol group molecule and one azide group molecule introduced synthesized in Example 7-2 in a PBSE buffer (10 mM Phosphate Buffered Saline (PBS), 10 mM EDTA, pH7.4), a solution of the Trastuzumab Fab with a maleimide group introduced synthesized in Example 8-2-5 in a PBSE buffer (10 mM Phosphate Buffered Saline (PBS), 10 mM EDTA, pH7.4) (1.5 equivalents relative to the antibody) was added and shaken for 1 hour. To this reaction solution, a solution of the Pembrolizumab Fab with a PEG4-DBCO group introduced synthesized in Example 8-3-3 in a PBSE buffer (10 mM Phosphate Buffered Saline (PBS), 10 mM EDTA, pH7.4) (2.0 equivalents relative to the antibody) was added and shaken for 20 hours. The reaction solution was purified using AKTA pure25 (Cytiva) to obtain a Tri-specific antibody (M-1). For Tri-specific antibody (M-1) analysis, ESI-TOFMS analysis was performed according to the previous report (Anal. Chem., 2019, 91, 20, 12724-12732) to observe a peak at 254362.

The purification with AKTA pure25 (Cytiva) was performed under the following conditions:
Column: Superdex200 Increase 10/300GL (Cytiva)
Eluent: PBS buffer (10 mM Phosphate Buffered Saline (PBS), pH7.4)
Flow rate: 0.6 ml/min
Detector: Detection was performed at wavelengths of 215 and 280 nm.

### (9-2) Synthesis of Tri-specific Antibody (M-2)

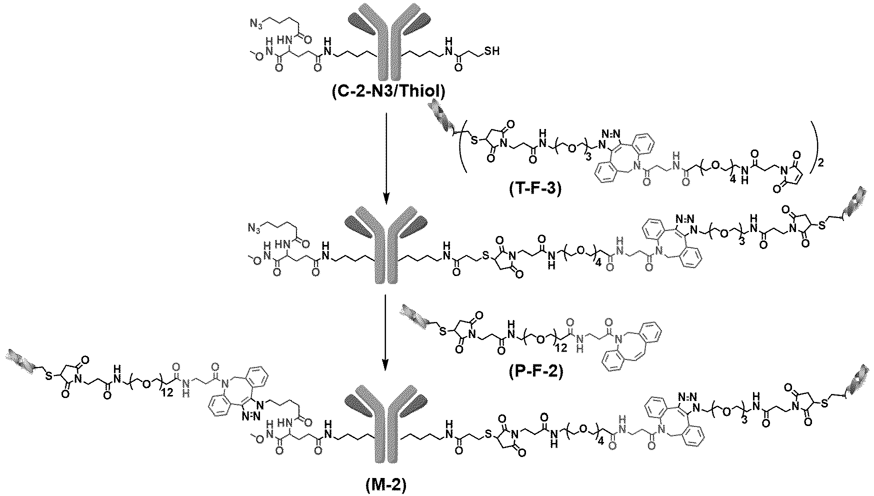

The antibody with one thiol group molecule and one azide group molecule introduced synthesized in Example 7-2, the Trastuzumab Fab with a maleimide group introduced synthesized in Example 8-2-5, and Pembrolizumab Fab with a PEG12-DBCO group introduced synthesized in Example 8-3-4 were used according to Example 9-1 to obtain a Tri-specific antibody. For Tri-specific antibody analysis, ESI-TOFMS analysis was performed according to the previous report (Anal. Chem., 2019, 91, 20, 12724-12732) to observe a peak at 255090.

### (9-3) Synthesis of DAR1 Bi-specific Antibody (Cetuximab-Trastuzumab Fab) (M-3)

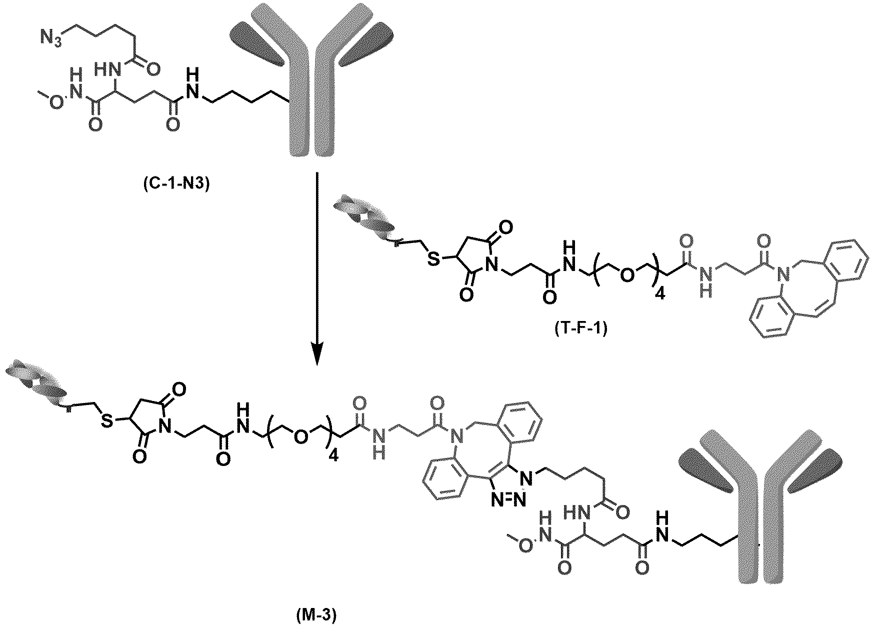

To a solution of the antibody with only one azide group introduced (C-1-N3) synthesized in Example 7-3 in an acetate buffer (50 mM Sodium acetate, pH5.5), a solution of the Trastuzumab Fab with a PEG4-DBCO group introduced synthesized in Example 8-2-3 in an acetate buffer (50 mM Sodium acetate, pH5.5) (2.0 equivalents relative to the antibody) was added and shaken for 20 hours. The reaction solution was purified using AKTA pure25 (Cytiva) to obtain a Bi-specific antibody. The purification conditions followed Example 9-1. For Bi-specific antibody analysis, ESI-TOFMS analysis was performed according to the previous report (Anal. Chem., 2019, 91, 20, 12724-12732) to observe a peak at 202941.

### (9-4) Synthesis of DAR1 Bi-specific Antibody (Trastuzumab-Cetuximab Fab) (M-4)

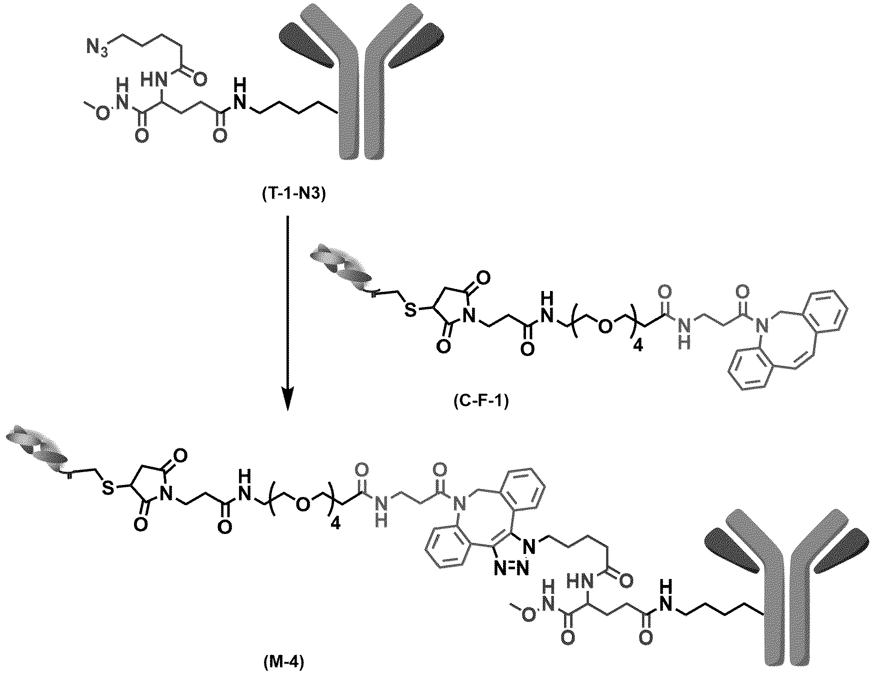

The antibody with only one azide group introduced (T-1-N3) synthesized in Example 6-4, and a PEG12-DBCO group synthesized in Example 8-1-4 were used according to Example 9-3 to obtain a Bi-specific antibody. For Bi-specific antibody analysis, ESI-TOFMS analysis was performed according to the previous report (Anal. Chem., 2019, 91, 20, 12724-12732) to observe a peak at 201356.

### (9-5) Synthesis of DAR2 Bi-specific Antibody (Cetuximab-Trastuzumab Fab) (M-5)

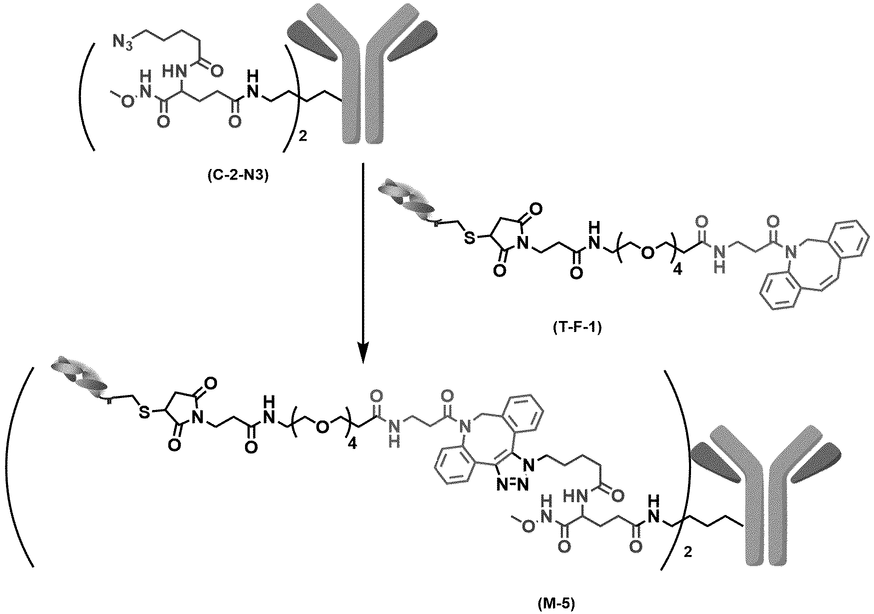

To a solution of the antibody with two azide groups introduced (C-2-N3) synthesized in Example 7-4 in an acetate buffer (50 mM Sodium acetate, pH5.5), a solution of the Trastuzumab Fab with a PEG4-DBCO group introduced synthesized in Example 8-2-3 in an acetate buffer (50 mM Sodium acetate, pH5.5) (2.5 equivalents relative to the antibody) was added and shaken for 20 hours. The reaction solution was purified using AKTA pure25 (Cytiva) to obtain a Bi-specific antibody. The purification conditions followed Example 9-1. For Bi-specific antibody analysis, ESI-TOFMS analysis was performed according to the previous report (Anal. Chem., 2019, 91, 20, 12724-12732) to observe a peak at 253224.

### (Comparative Example 2) Affinity Evaluation of Antibody by Surface Plasmon Resonance (SPR) Method

The association constants of trastuzumab, cetuximab, and pembrolizumab to antigens (HER2, EGFR, and PD-1) and neonatal Fc receptor (FcRn) were determined by SPR analysis using Biacore T200 (Cytiva).

Recombinant human HER2-Fc (R&D Systems), recombinant human EGFR-Fc (R&D Systems), recombinant human PD-1-Fc (R&D Systems) were biotinylated using Biotin Labeling Kit (Dojindo), and immobilized on sensor chips using Biotin CAPture Kit (Cytiva). For FcRn, a biotinylated recombinant human FcRn (Immunitrack) was used and immobilized on a sensor chip SA (Cytiva). As a running buffer for evaluation of the affinity to antigens, HBS-EP+ buffer (Cytiva) was used. In evaluation of the affinity to FcRn, HBS-EP+ buffer with the pH adjusted to 6.0 as a running buffer for association and HBS-EP+ buffer at pH 7.4 as a running buffer for dissociation were used.

Association of the antibodies to the antigens and FcRn was evaluated by the Single cycle kinetics method in which the antibodies were added at five concentration points. Fitting was performed on the obtained sensorgram using a 1:1 binding model, and the association constant was calculated. After binding of the antibodies to FcRn, the buffer at pH7.4 was added for determination of the antibody dissociation.

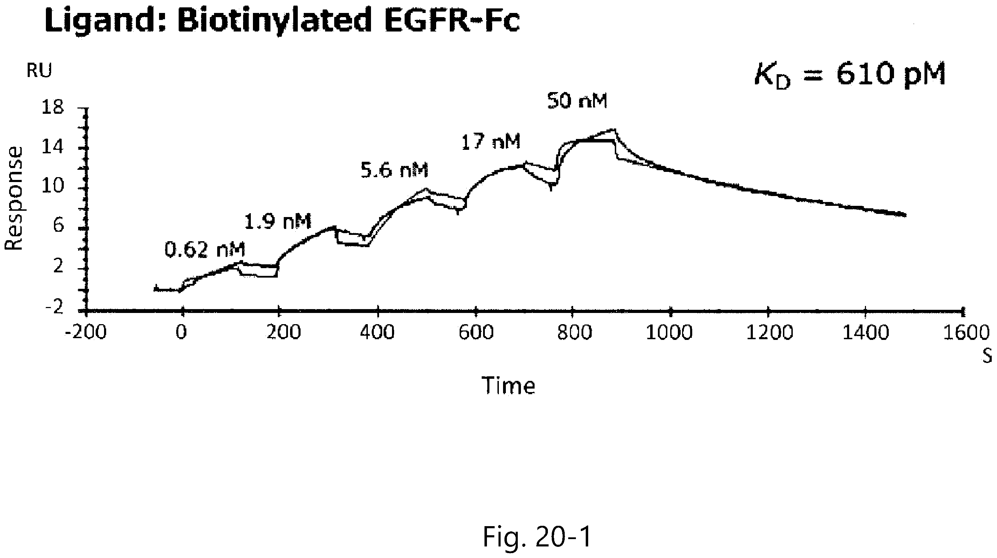

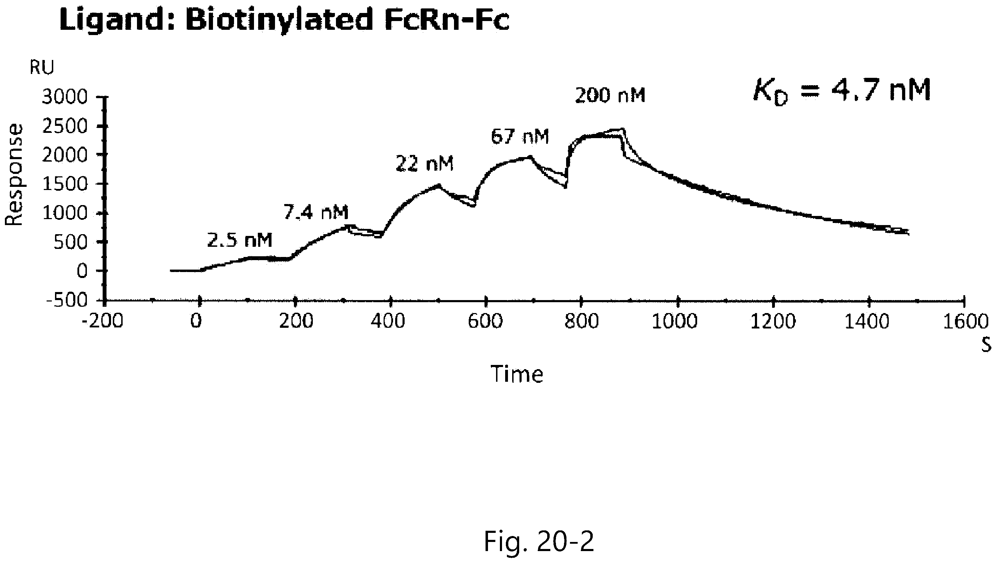

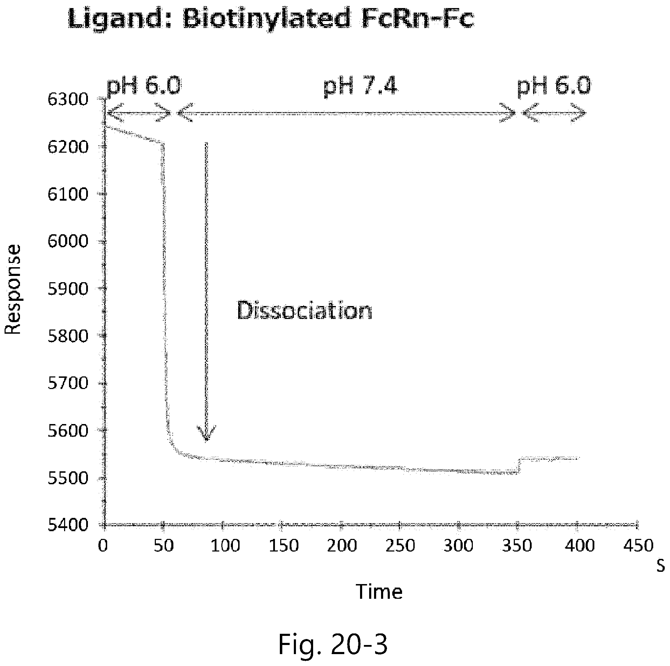

The sensorgrams that are the results of the evaluation of the cetuximab affinity are shown in FIGS. 20-1 to 20-3. It was demonstrated that cetuximab bound to EGFR, with a dissociation constant of K_{D} = 610 pM. It was also demonstrated that cetuximab bound to FcRn at pH6.0 with K_{D} = 4.7 nM and dissociated at pH7.4.

### (Example 10) Affinity Evaluation of Multispecific Antibody by Surface Plasmon Resonance (SPR) Method

### (10-1) Affinity Evaluation of Bi-specific Antibody (Cetuximab-Trastuzumab Fab) (M-3)

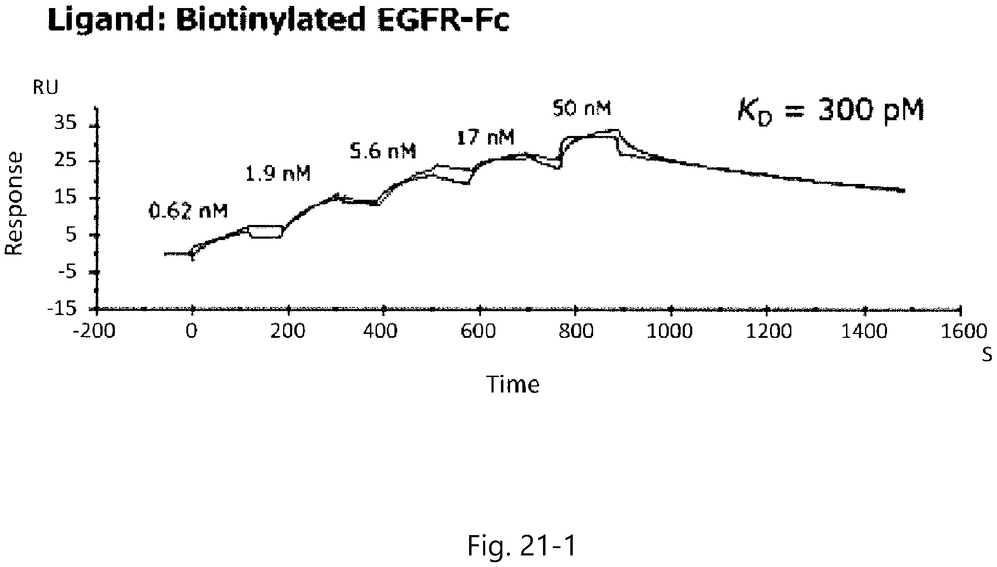

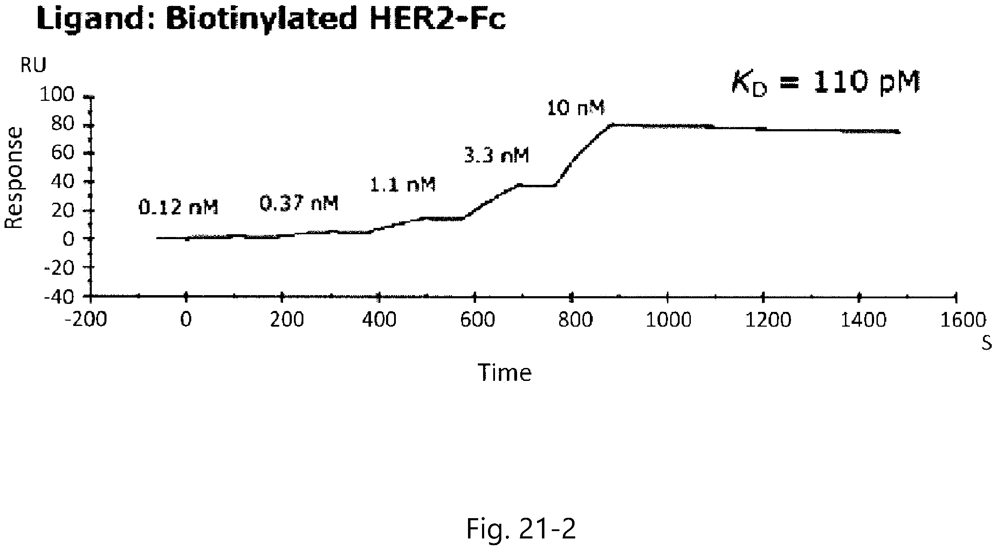

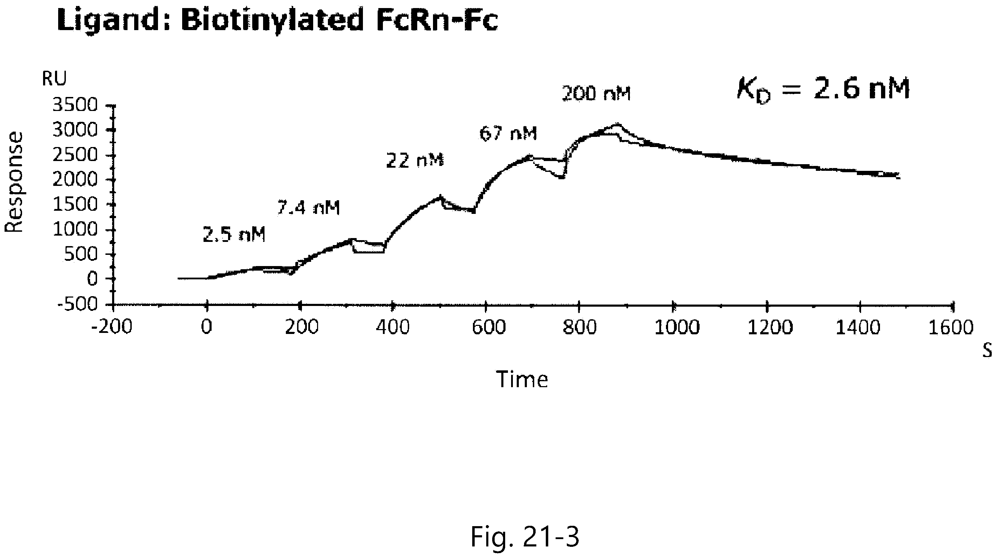

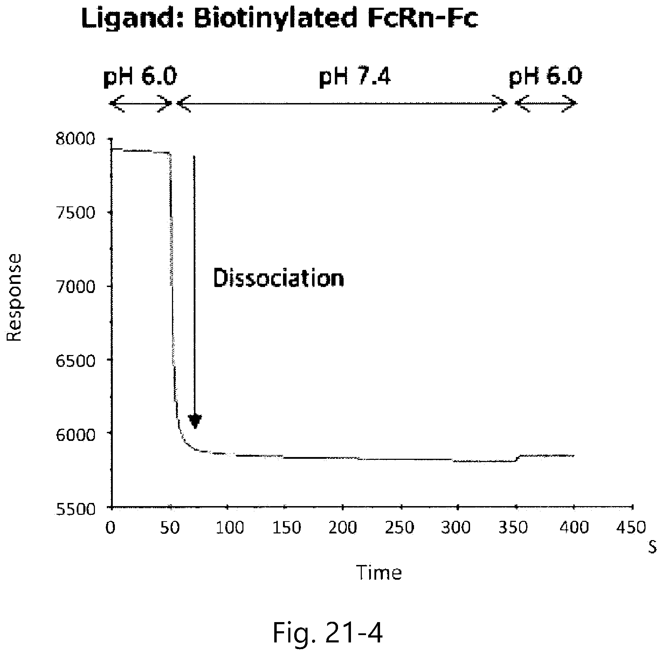

The affinity of the bi-specific antibody (Cetuximab-Trastuzumab Fab) (M-3) synthesized in Example 9-3 was evaluated according to the method in Comparative Example 2. The resulting sensorgrams are shown in FIGS. 21-1 to 21-4. It was demonstrated that the Bi-specific antibody (Cetuximab-Trastuzumab Fab) (M-3) bound to EGFR that is an antigen of the parent antibody cetuximab, with a dissociation constant of K_{D} = 300 pM. It was also demonstrated that the Bi-specific antibody also bound to HER2 that is an antigen of trastuzumab, with a dissociation constant of K_{D} = 110 pM. It was also demonstrated that the Bi-specific antibody bound to FcRn at pH6.0 with K_{D} = 2.6 nM and dissociated at pH7.4.

### (10-2) Affinity Evaluation of Bi-specific Antibody (Cetuximab-Trastuzumab Fab) (M-4)

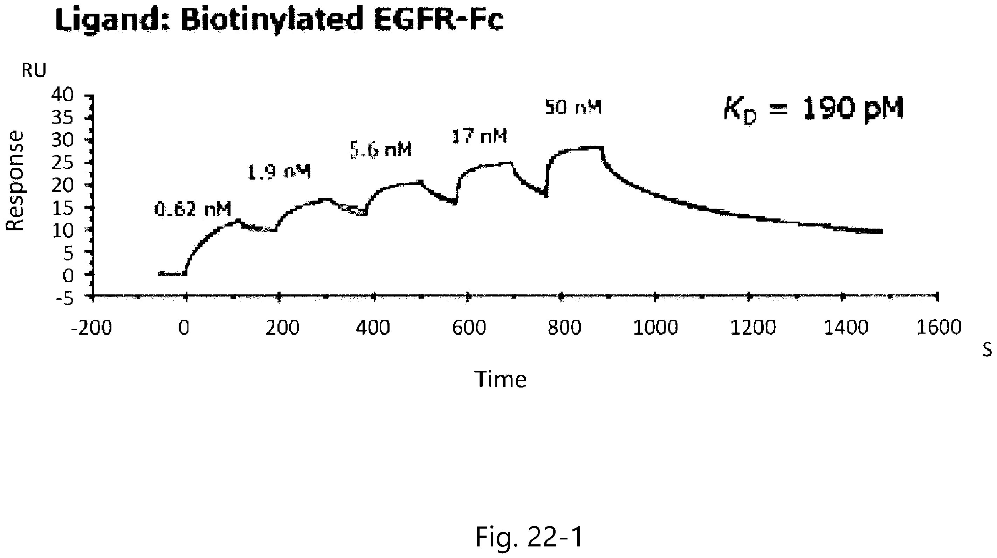

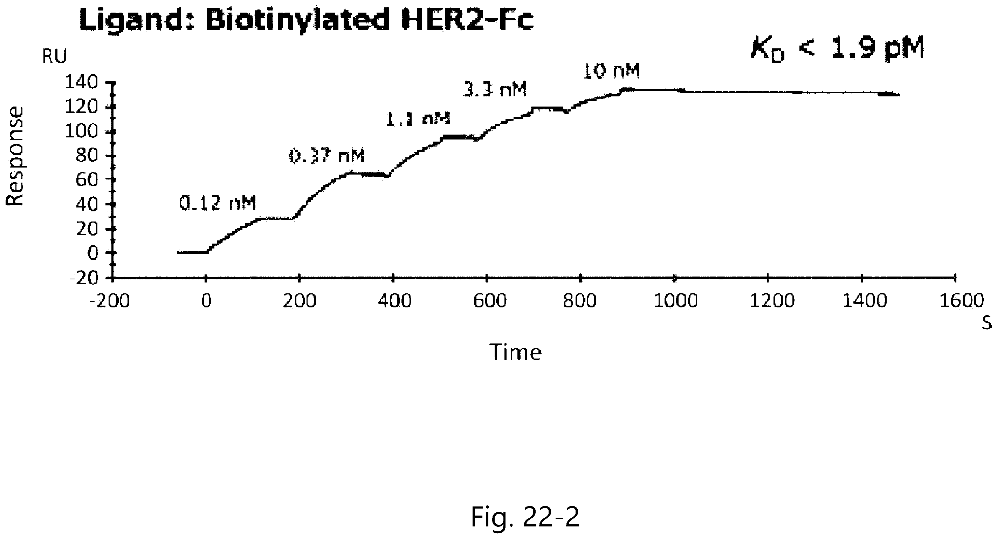

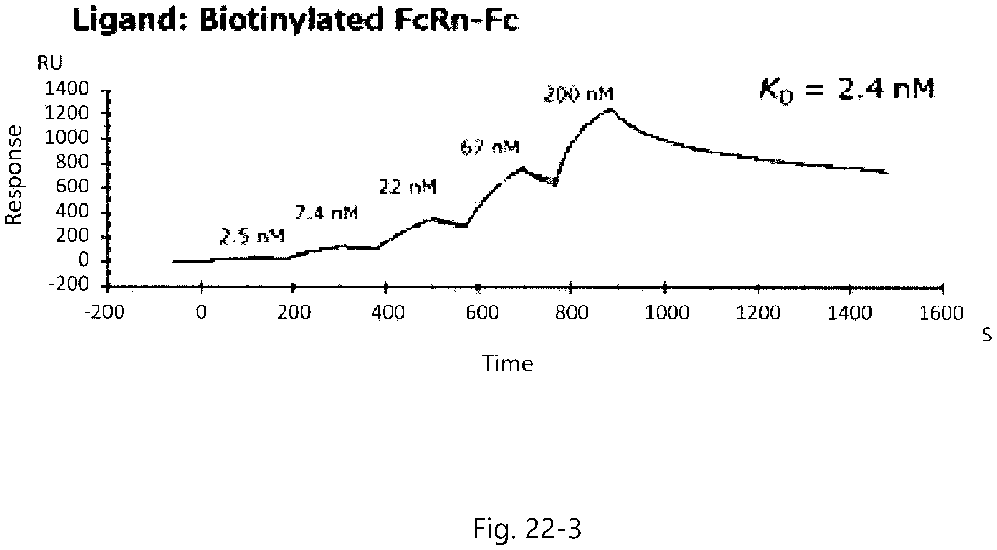

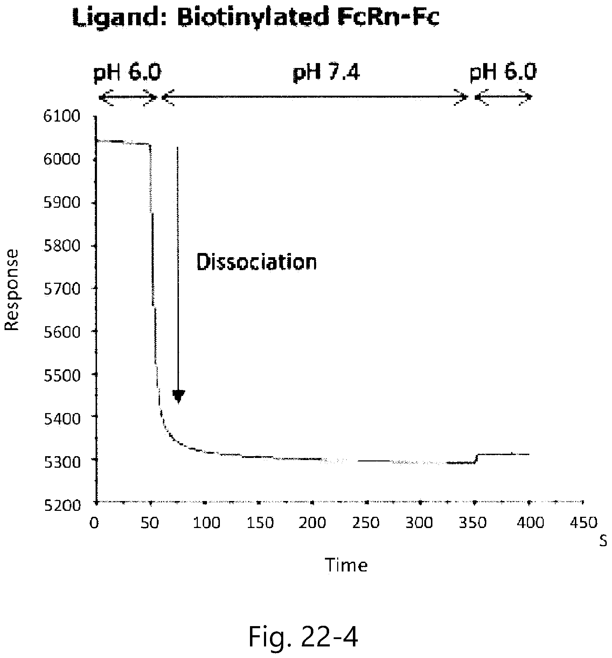

The affinity of the bi-specific antibody (Trastuzumab-Cetuximab Fab) (M-4) synthesized in Example 9-4 was evaluated according to the method in Comparative Example 2. The resulting sensorgrams are shown in FIGS. 22-1 to 22-4. It was demonstrated that the Bi-specific antibody (Trastuzumab-Cetuximab Fab) (M-4) bound to HER2 that is an antigen of the parent antibody trastuzumab, with a dissociation constant of K_{D} = 1.9 pM or less. It was also demonstrated that the Bi-specific antibody also bound to EGFR that is an antigen of cetuximab, with a dissociation constant of K_{D} = 190 pM. It was also demonstrated that the Bi-specific antibody also bound to FcRn at pH6.0 with K_{D} = 2.4 nM and dissociated at pH7.4.

### (10-3) Affinity Evaluation of Tri-specific antibody (M-2)

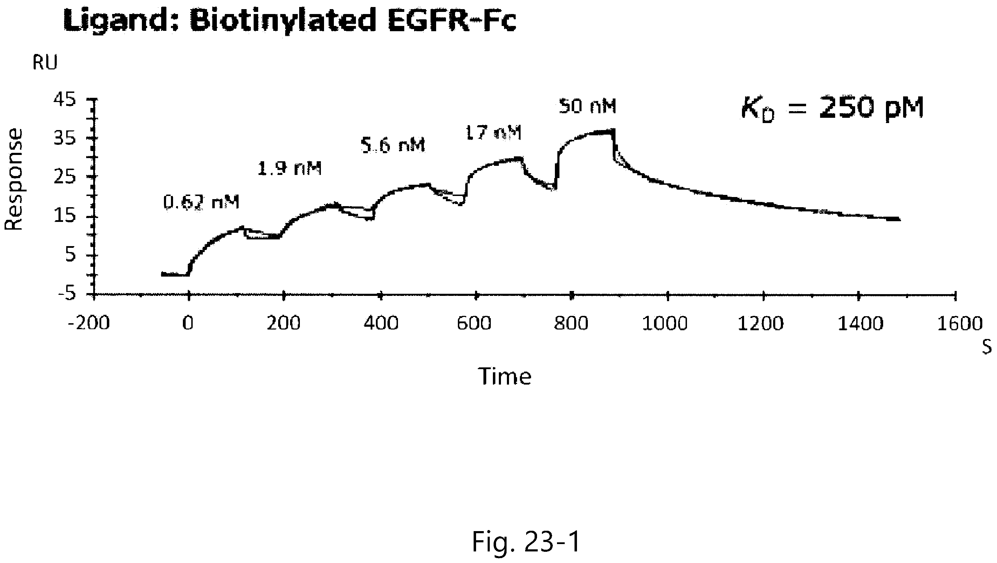

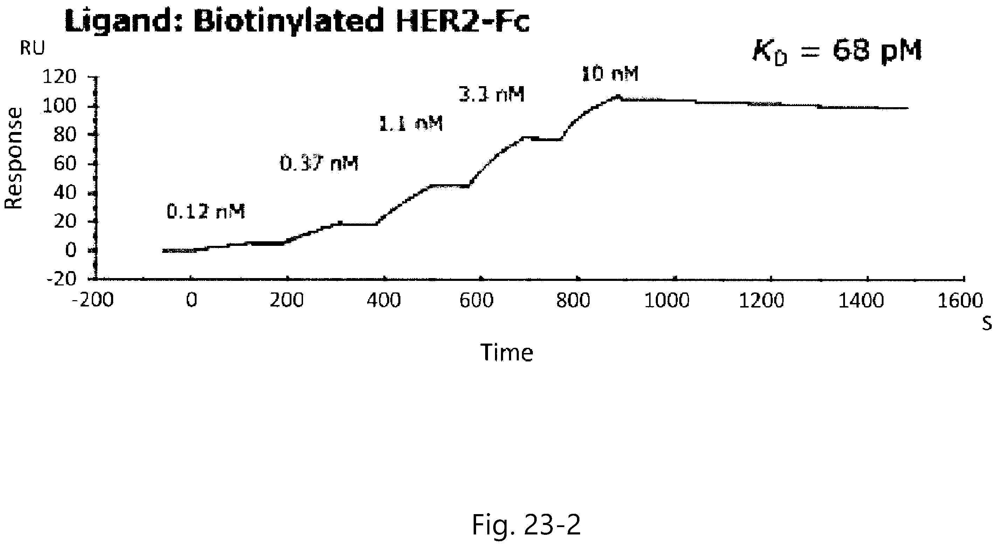

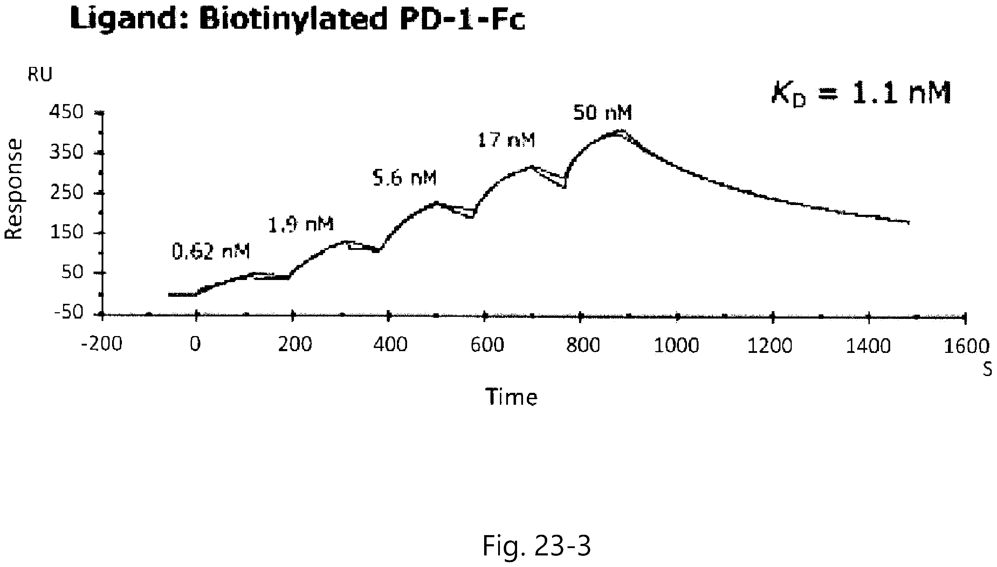

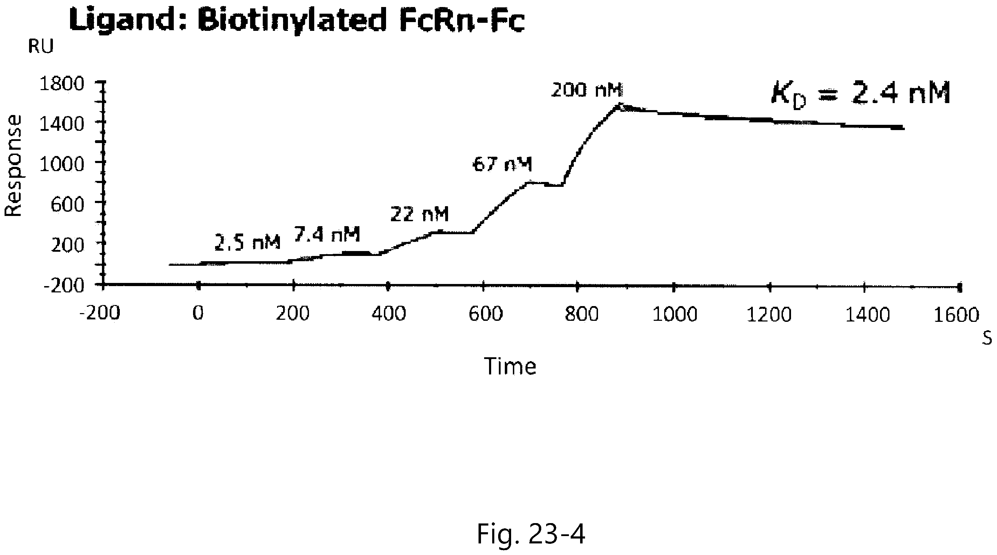

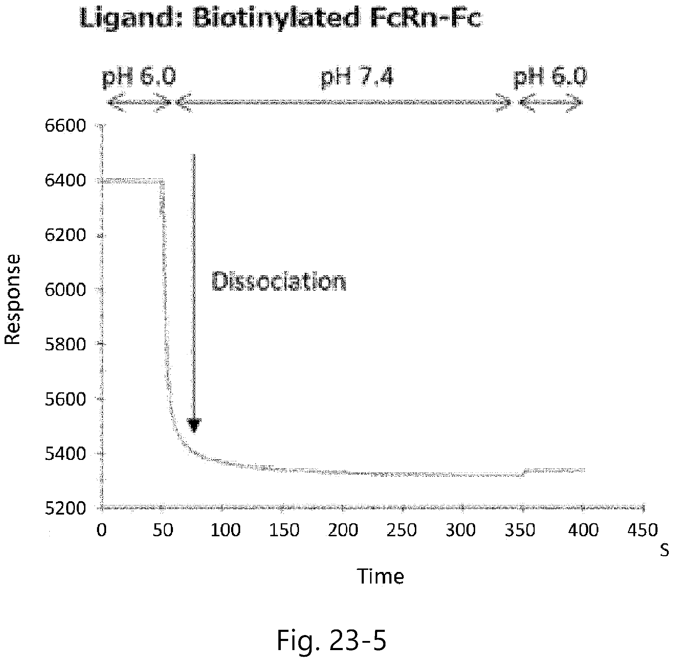

The sensorgrams that are the results of the affinity evaluation of the Tri-specific antibody (M-2) synthesized in Example 9-2 are shown in FIGS. 23-1 to 23-5. It was demonstrated that the Tri-specific antibody (M-2) bound to EGFR that is an antigen of the parent antibody cetuximab, with a dissociation constant of K_{D} = 250 pM. It was also demonstrated that the Tri-specific antibody also bound to HER2 that is an antigen of trastuzumab and to PD-1 that is an antigen of pembrolizumab, with dissociation constants of K_{D} = 6.8 pM and 1.1 nM, respectively. It was also demonstrated that the tri-specific antibody also bound to FcRn at pH 6.0 with KD = 2.4 nM and dissociated at pH 7.4.

These results demonstrated that the bi-specific antibody (Cetuximab-Trastuzumab Fab) (M-3) and the bi-specific antibody (Trastuzumab-Cetuximab Fab) (M-4) are bi-specific antibodies having affinities to EGFR and HER2, and the Tri-specific antibody (M-2) is a tri-specific antibody having affinities to EGFR, HER2, and PD-1. It was also demonstrated that the affinity and dissociation ability with FcRn involved in antibody recycling *in vivo* are also maintained.

### (Example 11) Evaluation of Binding of Multispecific Antibodies to Antigens on Cells by Flow Cytometry

The cells used were CD3-activated human T cells containing HER2-positive SKBR-3 cells, EGFR-positive A-431 cells, and PD-1-positive cells. The evaluation of binding of cetuximab, trastuzumab, pembrolizumab, the bi-specific antibody (Cetuximab-Trastuzumab Fab) (M-3), the bi-specific antibody (Trastuzumab-Cetuximab Fab) (M-4), and the Tri-specific antibody (M-2) to the cells was performed by the flow cytometry method.

200,000 cells cultured in D-MEM medium (Thermo Fisher Scientific, Inc.) supplemented with 10% FBS (Thermo Fisher Scientific, Inc.) were collected, and 20 µL of PBS containing Human TruStain FcX (Biolegend) was added, followed by blocking of Fc receptors on ice for 10 minutes. The primary antibody reaction was performed by adding the above-described antibodies as primary antibodies to a cell staining buffer (PBS containing 1% FBS) to a final concentration of 20 µg/mL, adding 20 µL of the mixtures to the cells, and incubating them for 20 minutes on ice. The cells after the primary antibody reaction were washed twice with the cell staining buffer. The second antibody reaction was performed by adding 40 µL of Goat anti-Human IgG (H+L) Cross-Adsorbed Secondary Antibody, Alexa Fluor^{™}488 (Thermo Fisher Scientific, Inc.) diluted with the cell staining buffer to 10 µg/mL as the second antibody to the cells, and incubating the cells on ice for 20 minutes. The cells were washed twice with the cell staining buffer, and then subjected to analysis using Attune NxT Flow Cytometer (Thermo Fisher Scientific, Inc.).

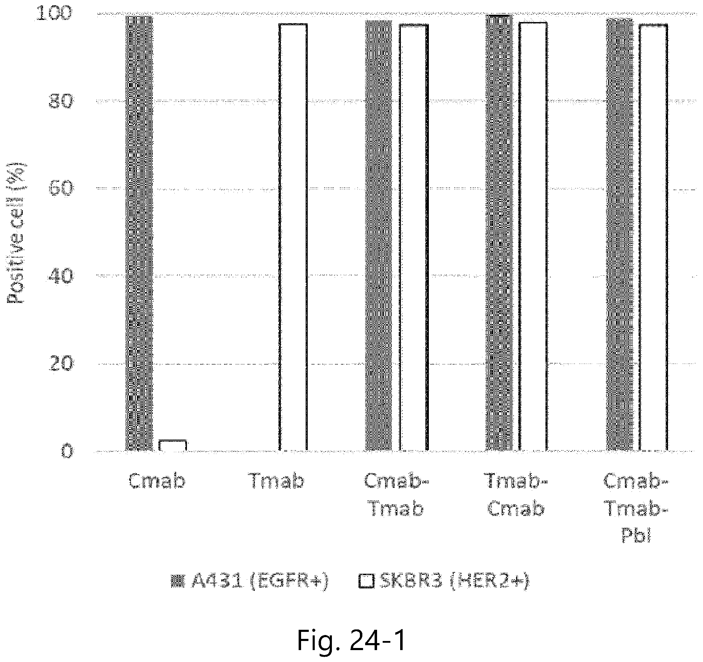

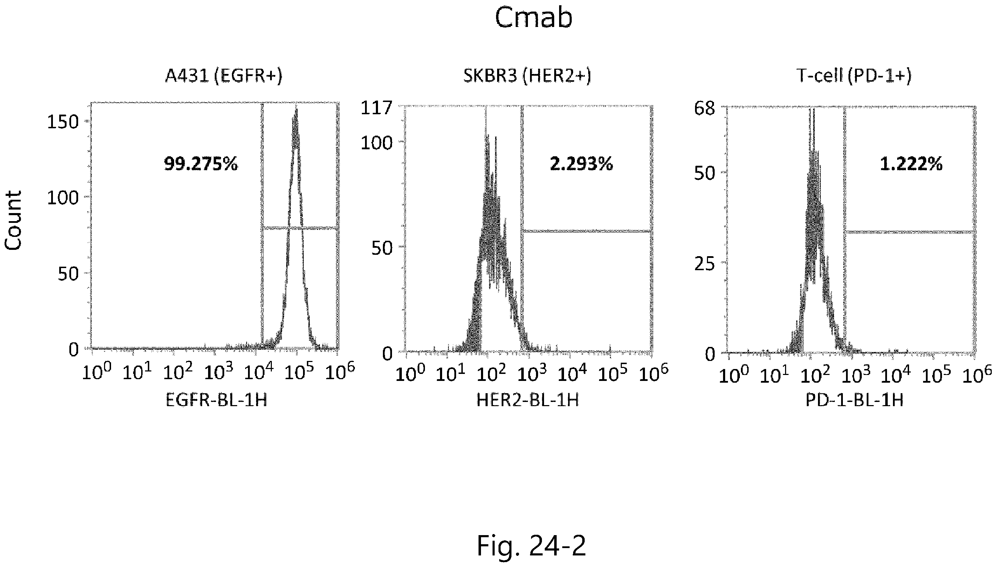

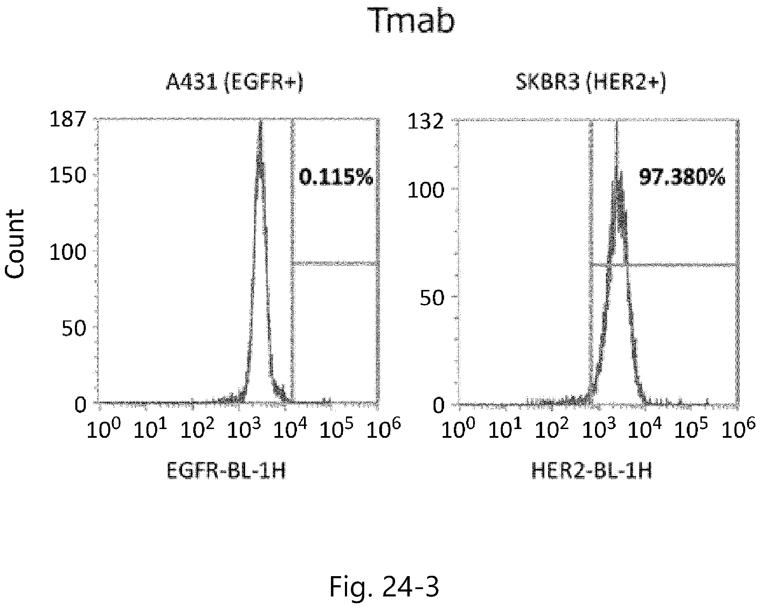

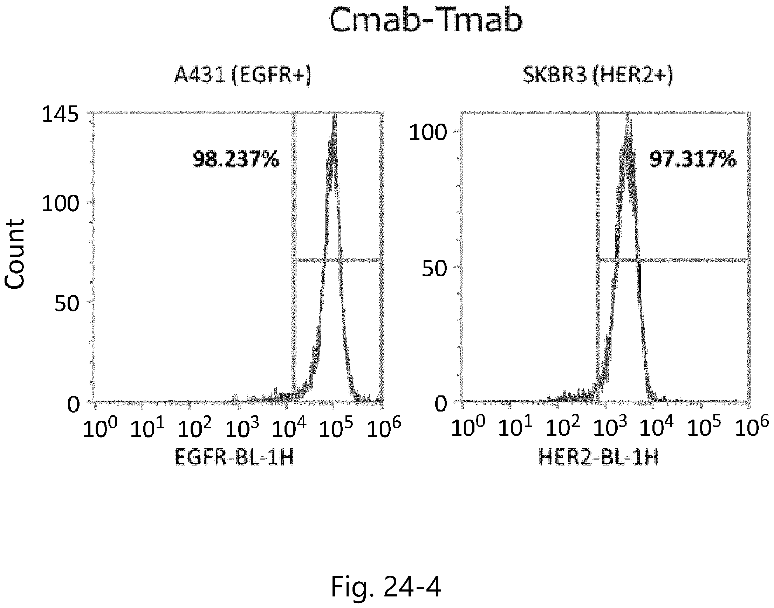

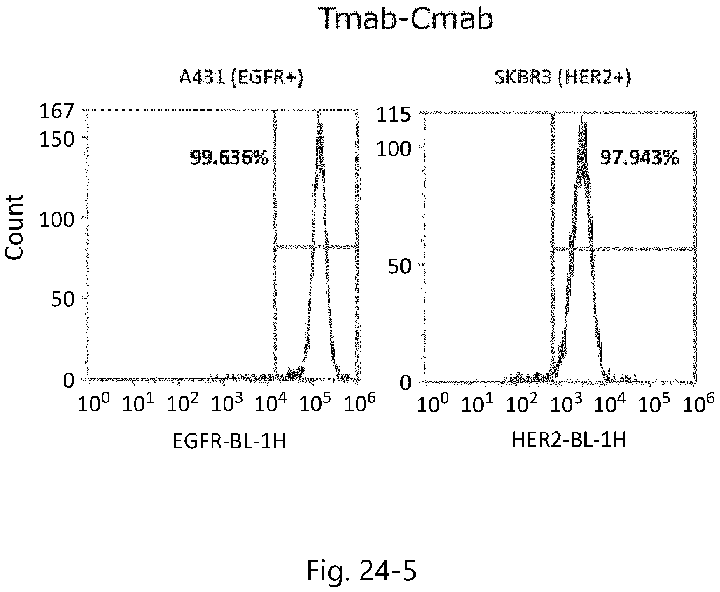

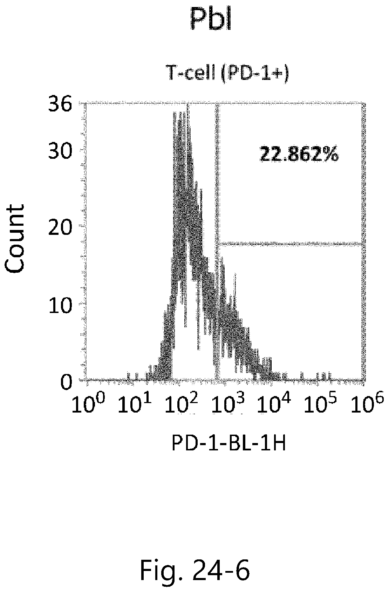

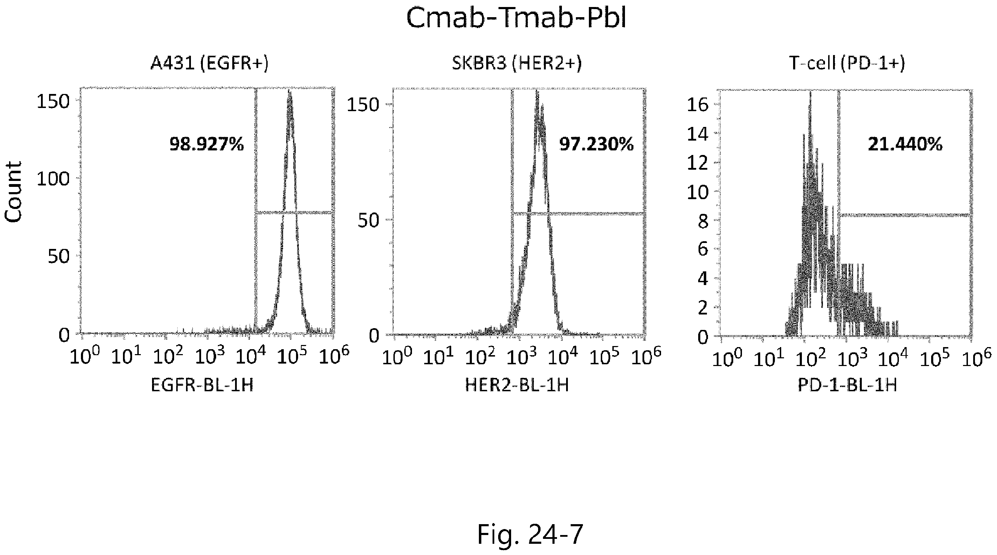

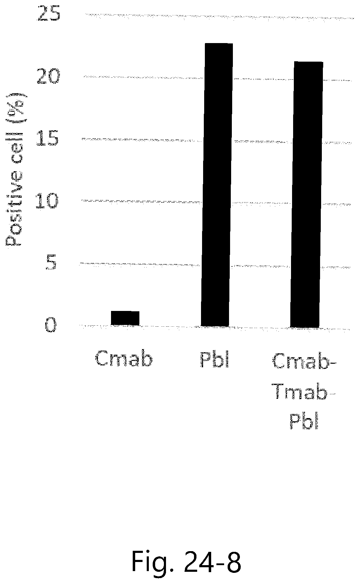

The analysis results for the cells are shown in FIGS. 24-1 to 24-8. For SKBR-3 cells, fluorescence was observed in 97% or more of the cells with trastuzumab, the bi-specific antibody (Cetuximab-Trastuzumab Fab) (M-3), the bi-specific antibody (Trastuzumab-Cetuximab Fab) (M-4), and the Tri-specific antibody (M-2). For A-431 cells, fluorescence was observed in 98% or more of the cells with cetuximab, the bi-specific antibody (Cetuximab-Trastuzumab Fab) (M-3), the bi-specific antibody (Trastuzumab-Cetuximab Fab) (M-4), and the Tri-specific antibody (M-2). That is, it was demonstrated that trastuzumab only bound to SKBR3 cells that are HER2-positive cells, and cetuximab only bound to A-431 cells that are EGFR-positive cells, while the bi-specific antibodies, the bi-specific antibody (Cetuximab-Trastuzumab Fab) (M-3) and the bi-specific antibody (Trastuzumab-Cetuximab Fab) (M-4), and the Tri-specific antibody (M-2) successfully bound to both SKBR3 cells and A-431 cells. Little or no fluorescence was observed in CD3-activated human T cells with cetuximab that does not recognize PD-1 while fluorescence was observed in about 23% cells with PD-1-specific pembrolizumab, demonstrating that about 23% PD-1-expressing cells were contained. Fluorescence was observed in almost equal amount of cells with the Tri-specific antibody (M-2), demonstrating that the Tri-specific antibody (M-2) can bind to PD-1. These results demonstrate that the Tri-specific antibody (M-2) can bind to all of the antigens, EGFR, HER2, and PD-1.

### (Example 12) Expression of VHH Antibody with Site-specifically Introduced Azide Phenylalanine (AzF) (CD3-VHH, V-1)

### (12-1) Construction of plasmid vector (pPK14e) that is pPK5 vector having incorporated AzFN3 DNA cassette

It is known that secretory production of heterologous proteins in C. *glutamicum* allows for introduction of site-specific azido-phenylalanine (AzF) into target proteins (WO2023/282315).

An AzFN3 DNA cassette (SEQ ID NO: 43) was obtained by total synthesis with reference to WO2023/282315. The AzFN3 DNA cassette comprises the azido-phenylalanyl-tRNA synthetase (AzFRS) gene, an ApaI site downstream of it, a F1 promoter further downstream of it, the suppressor tRNA (tRNA_{CTA}) gene capable of translating a UAG codon into azido-phenylalanine (AzF) linked downstream of the F1 promoter, and a rrnC terminator further downstream of it, and further comprises a KpnI site at 5'-terminus and a XbaI site at 3'-terminus. AzFRS is a modified tyrosyl-tRNA synthetase that has the mutations of Y32T/E107N/D158P/I159L/L162Q, is modified to use azido-phenylalanine as a substrate, and is derived from archaebacterium *Methanococcus jannaschii* (J.AM.CHEM.SOC.2002,124,9026-9027). The base sequence of the AzFRS gene and the amino acid sequence of AzFRS are illustrated in SEQ ID NOs: 44 and 45, respectively. tRNA_{CTA} is a modified tyrosyl-tRNA that is modified to have an anticodon (CTA) corresponding to UAG (amber) and is derived from archaebacterium *Methanococcus jannaschii.* The base sequence of the tRNA_{CTA} gene and the base sequence of tRNA(Tyr) encoded by the gene are illustrated in SEQ ID NOs: 46 and 47, respectively. When the AzFN3 DNA cassette is introduced into coryneform bacteria and the bacteria are cultured in a medium supplemented with azido-phenylalanine, azido-phenylalanine is taken up by the coryneform bacteria and binds to the terminus of tRNACTA via AzFRS, resulting in the formation of azido-phenylalanyl-tRNA. During protein translation, the azido-phenylalanyl-tRNA binds to the UAG codon in mRNA, leading to the translation of the UAG codon as azido-phenylalanine and the synthesis of a protein with azido-phenylalanine incorporated at the UAG codon position.

The fully synthesized AzFN3 DNA cassette (Sequence No. 43) was incorporated into the KpnI-XbaI site of the pPK5 vector described in WO2016/171224 by infusion reaction, resulting in the construction of the vector pPK14e with the AzFN3 DNA cassette incorporated. The infusion reaction was carried out using In-Fusion^{®} HD Cloning Kit (Takara Bio), and the reaction conditions followed the manufacturer-recommended protocol. As a result of determining the base sequences of the inserted fragments, it was determined that a vector carrying an AzFN3 DNA cassette as designed was constructed. The base sequence was determined using BigDye^{®} Terminator v3.1 Cycle Sequencing Kit (Applied Biosystems) and 3500xL Genetic Analyzer (Applied Biosystems).

SEQ ID NO: 43 
ggtaccggagaccacaacggtttccctctagaaataattttgtttaactttaagaaggagatataccaatggatgagttcgagatg
atcaaacgtaacacttccgagatcatttccgaggaagaactgcgcgaagtgcttaagaaagatgagaagtctgcgacgattgg
cttcgaaccatcagggaagattcaccttggccactacttgcagatcaagaagatgatcgacttgcagaatgctggcttcgacat
catcattctgctggcggatctgcatgcctacctcaatcagaagggagaactggacgaaatccgcaagatcggcgactacaac
aagaaagtctttgaagcaatgggtctgaaggcgaaatacgtctatgggagcaacttccagcttgacaaagactacaccttgaac
gtgtatcggcttgctctgaaaaccacactcaaacgtgcacgccgatcgatggaactcatcgcacgggaggacgagaatccga
aggttgctgaagtcatctatcccatcatgcaagttaacccgcttcattaccaaggagtggatgttgcagttggtggtatggaaca
gcgcaagattcacatgttggcacgcgaactgctgccgaagaaagtggtctgcatccacaatcccgtgctcactggtttggacg
gtgaaggcaagatgagctcctctaaaggcaactttatcgctgtcgatgattcgcctgaagagattcgtgccaagattaagaagg
cttactgtccagcgggagtagtggaaggcaacccaatcatggagattgccaagtacttcctggagtatccattgaccatcaagc
 
gtcctgagaagtttggcggtgatctcaccgtaaactcctacgaggaactggaatccctcttcaagaacaaagagctgcaccca
atggatctcaagaacgcagttgccgaggagctgatcaagatccttgagcctattcgcaaacgcctgtaagggcccggcatgg
atgaactatacgatctactcgttactcaaggcaagctcgagcgggacggtcgaaccagcttcaagcgaccggatgagtatgtt
acagtagatagcgacggggtctcatagtaaagtatgaggcgcaccaaacgaggtgccccccgccttagttcagcagggcag
aacggcggactctaaatccgcatggcacgggttcaaatcccgtaggcgggaccactgcagatccttagcgaaagctaaggat
ttttttttgatatctctaga
SEQ ID NO: 44 
atggatgagttcgagatgatcaaacgtaacacttccgagatcatttccgaggaagaactgcgcgaagtgcttaagaaagatga
gaagtctgcgacgattggcttcgaaccatcagggaagattcaccttggccactacttgcagatcaagaagatgatcgacttgca
gaatgctggcttcgacatcatcattctgctggcggatctgcatgcctacctcaatcagaagggagaactggacgaaatccgca
agatcggcgactacaacaagaaagtctttgaagcaatgggtctgaaggcgaaatacgtctatgggagcaacttccagcttgac
aaagactacaccttgaacgtgtatcggcttgctctgaaaaccacactcaaacgtgcacgccgatcgatggaactcatcgcacg
ggaggacgagaatccgaaggttgctgaagtcatctatcccatcatgcaagttaacccgcttcattaccaaggagtggatgttgc
agttggtggtatggaacagcgcaagattcacatgttggcacgcgaactgctgccgaagaaagtggtctgcatccacaatcccg
tgctcactggtttggacggtgaaggcaagatgagctcctctaaaggcaactttatcgctgtcgatgattcgcctgaagagattcg
tgccaagattaagaaggcttactgtccagcgggagtagtggaaggcaacccaatcatggagattgccaagtacttcctggagt
atccattgaccatcaagcgtcctgagaagtttggcggtgatctcaccgtaaactcctacgaggaactggaatccctcttcaagaa
caaagagctgcacccaatggatctcaagaacgcagttgccgaggagctgatcaagatccttgagcctattcgcaaacgcctgt
aa
SEQ ID NO: 45 
MDEFEMIKRNTSEIISEEELREVLKKDEKSATIGFEPSGKIHLGHYLQIKKMIDLQ
NAGFDIIILLADLHAYLNQKGELDEIRKIGDYNKKVFEAMGLKAKYVYGSNFQL
DKDYTLNVYRLALKTTLKRARRSMELIAREDENPKVAEVIYPIMQVNPLHYQG
VDVAVGGMEQRKIHMLARELLPKKVVCIHNPVLTGLDGEGKMSSSKGNFIAVD
DSPEEIRAKIKKAYCPAGVVEGNPIMEIAKYFLEYPLTIKRPEKFGGDLTVNSYEE
LESLFKNKELHPMDLKNAVAEELIKILEPIRKRL
SEQ ID NO: 46
   cccgccttagttcagcagggcagaacggcggactctaaatccgcatggcacgggttcaaatcccgtaggcgggacca
SEQ ID NO: 47
   cccgccuuaguucagcagggcagaacggcggacucuaaauccgcauggcacggguucaaaucccguaggcgggac ca

The pPK14e vector can be used as a basic vector for the expression of AzF-introduced proteins. That is, by subcloning the expression cassette of the target protein using the ApaI site of pPK14e, it is possible to co-express the AzFN3 DNA cassette (azido-phenylalanyl-tRNA synthetase (AzFRS) gene and the suppressor tRNA (tRNA_{CTA}) gene that allows translation of UAG codon into azido-phenylalanine (AzF)) and the expression cassette of the AzF-introduced protein on a single plasmid.

### (12-2) Construction of Secretory Expression Plasmids of CD3-VHH-PA24H6-WT and CD3-VHH-PA24H6-AzF

As the VHH antibody to which azido-phenylalanine was introduced, an anti-CD3-VHH antibody (CD3-VHH) described in WO2015/095412 was used. In order to introduce azido-phenylalanine without modifying the inside of the CD3-VHH sequence, a modified CD3-VHH with a PA24 linker and a 6x His tag attached at the C-terminus of CD3-VHH was designed (CD3-VHH-PA24H6-WT). In addition, a variant CD3-VHH-PA24H6 was designed such that AzF was introduced within the PA24 linker of CD3-VHH-PA24H6-WT (between the positions 141 and 142) (CD3-VHH-PA24H6-AzF). The amino acid sequences of the designed CD3-VHH-PA24H6-WT and CD3-VHH-PA24H6-AzF are illustrated in SEQ ID NOs: 48 and 49, respectively.

Amino acid sequence of CD3-VHH-PA24H6-WT 
QVQLQESGGGLVQAGGSLRLSCAASGRTFSNYHMGWFRQAPGKERELVAAISG
SGGSTYYTDSVKGRFTISRNNAKNTMSLQMSNLKPEDTGVYYCTTPTEKGSSID
YWGQGTQVTVSSGRYPYDVPDYAAPAAPAPAAPAAPAPAAPAAPAPHHHHHH
(SEQ ID NO: 48)
Amino acid sequence of CD3-VHH-PA24H6-AzF 
QVQLQESGGGLVQAGGSLRLSCAASGRTFSNYHMGWFRQAPGKERELVAAISG
SGGSTYYTDSVKGRFTISRNNAKNTMSLQMSNLKPEDTGVYYCTTPTEKGSSID
 YWGQGTQVTVSSGRYPYDVPDYAAPAAPAPAAPAFAPAPAAPAAPAPHHHHHH (SEQ ID NO: 49; F at position 142 is azidated)

Base sequences encoding these polypeptides were designed in consideration of codon usage frequency of *C*. *glutamicum.* Furthermore, the following expression cassettes were designed so as to make secretory expression by *C*. *glutamicum* possible.

CD3-VHH-PA24H6-WT was expressed in a secretory manner as a fusion protein of a signal peptide 25 amino acid residue of CspA derived from C. stationis and CD3-VHH-PA24H6-WT (hereinafter referred to as "CspAss-CD3-VHH-PA24H6-WT"). The designed base sequence encoding CspAss-CD3-VHH-PA24H6-WT and the amino acid sequence thereof are illustrated in SEQ ID NOs: 50 and 51, respectively.

SEQ ID NO: 50 
atgaaacgcatgaaatcgctggctgcggcgctcaccgtcgctggggccatgctggccgcacctgtggcaacggcacaggta
cagctacaggaatccggaggcggtcttgtacaggcaggaggcagtttgcgtctgtcctgtgctgcgtctggtcgtacgttctcc
aactatcacatggggtggtttcgccaagcaccaggcaaagaacgcgagttggttgccgccattagcgggtctggaggaagca
cgtactacaccgatagcgtcaaaggccgtttcacgatcagccgcaataacgctaagaacaccatgtctctgcagatgtcgaac
ctcaagcctgaagacactggcgtgtactactgcactacacctaccgagaaaggttcctcgatcgactattggggtcaaggcact
caagtcaccgtctcgagtgggaggtatccctatgatgtgccggattatgctgcacccgcagcacccgcgccagcagcgccag
ctgcgcctgctccagcagcaccagccgctcctgctccgcaccatcaccaccatcactaa
SEQ ID NO: 51

MKRMKSLAAALTVAGAMLAAPVATAQVQLQESGGGLVQAGGSLRLSCAASGR
TFSNYHMGWFRQAPGKERELVAAISGSGGSTYYTDSVKGRFTISRNNAKNTMS
LQMSNLKPEDTGVYYCTTPTEKGSSIDYWGQGTQVTVSSGRYPYDVPDYAAPA
APAPAAPAAPAPAAPAAPAPHHHHHH

CD3-VHH-PA24H6-AzF was expressed in a secretory manner as a fusion protein of a signal peptide 25 amino acid residue of CspA derived from C. stationis and CD3-VHH-PA24H6-AzF (hereinafter referred to as "CspAss-CD3-VHH-PA24H6-Amber"). Specifically, the CspAss-CD3-VHH-PA24H6-Amber gene comprises TAG triplet corresponding to UAG codon (amber), one of the stop codons, inserted in the base sequence of the CspAss-CD3-VHH-PA24H6-WT gene, and the TAG triplet can be translated into AzF. The designed base sequence encoding CspAss-CD3- VHH-PA24H6-Amber and the amino acid sequence thereof are illustrated in SEQ ID NOs: 52 and 53, respectively.

SEQ ID NO: 52 
atgaaacgcatgaaatcgctggctgcggcgctcaccgtcgctggggccatgctggccgcacctgtggcaacggcacaggta
cagctacaggaatccggaggcggtcttgtacaggcaggaggcagtttgcgtctgtcctgtgctgcgtctggtcgtacgttctcc
aactatcacatggggtggtttcgccaagcaccaggcaaagaacgcgagttggttgccgccattagcgggtctggaggaagca
cgtactacaccgatagcgtcaaaggccgtttcacgatcagccgcaataacgctaagaacaccatgtctctgcagatgtcgaac
ctcaagcctgaagacactggcgtgtactactgcactacacctaccgagaaaggttcctcgatcgactattggggtcaaggcact
caagtcaccgtctcgagtgggaggtatccctatgatgtgccggattatgctgcacccgcagcacccgcgccagcagcgccag
ctTAGgcgcctgctccagcagcaccagccgctcctgctccgcaccatcaccaccatcactaa
SEQ ID NO: 53 (F at position 167 is azidated)

MKRMKSLAAALTVAGAMLAAPVATAQVQLQESGGGLVQAGGSLRLSCAASGR
TFSNYHMGWFRQAPGKERELVAAISGSGGSTYYTDSVKGRFTISRNNAKNTMS
LQMSNLKPEDTGVYYCTTPTEKGSSIDYWGQGTQVTVSSGRYPYDVPDYAAPA
APAPAAPAFAPAPAAPAAPAPHHHHHH

Expression cassettes of wild and variant CD3-VHH-PA24H6 were designed in each of which a promoter of the cspB gene derived from *C*. *glutamicum* ATCC13869 strain was coupled with each of upstream sides of the base sequences described in CspAss-CD3-VHH-PA24H6-WT and CspAss-CD3-VHH-PA24H6-Amber, and a ApaI sites were added to the 5'- and 3'-sides, and total synthesis was performed. By inserting the totally synthesized DNA fragments (expression cassettes of CD3-VHH-PA24H6) into the ApaI site of the pPK14e vector constructed in Example (12-1), pPK14e_CspAss-CD3-VHH-PA24H6-WT and pPK14e_CspAss-CD3-VHH-PA24H6-Amber, which are secretory expression plasmids of wild and variant CD3-VHH-PA24H6, were constructed. As a result of determining the base sequences of the inserted fragments, it was determined that a vector carrying CD3-VHH-PA24H6+AzFN3 as designed was constructed.

### (12-3) Secretory Expression of CD3-VHH-PA24H6 with Azido-phenylalanine Introduced (CD3-VHH-PA24H6-AzF, V-1) in C. glutamicum

The *C. glutamicum* YDK0107 strain was transformed with pPK14e_CspAss-CD3-VHH-PA24H6-Amber that is the expression vector of CD3-VHH-PA24H6-Amber+AzFN3 constructed as described above to obtain a strain expressing CD3-VHH-PA24H6-Amber. As a control, the *C. glutamicum* YDK0107 strain was transformed with pPK14e_CspAss-CD3-VHH-PA24H6-WT that is the expression vector of CD3-VHH-PA24H6-WT+AzFN3 to obtain a strain expressing CD3-VHH-PA24H6-WT.

Each of the obtained transformants was cultured at 25°C for 120 hours in an MMTG liquid medium comprising 0.3 mM azido-phenylalanine and 25 mg/L kanamycin. After completion of the culture, 1.0 µL of the culture supernatant obtained by centrifuging each of the culture solutions was subjected to reducing SDS-PAGE using NuPAGE^{®} 12% Bis-Tris Gel (Thermo Fisher Scientific, Inc.), and then stained with Quick-CBB (Wako).

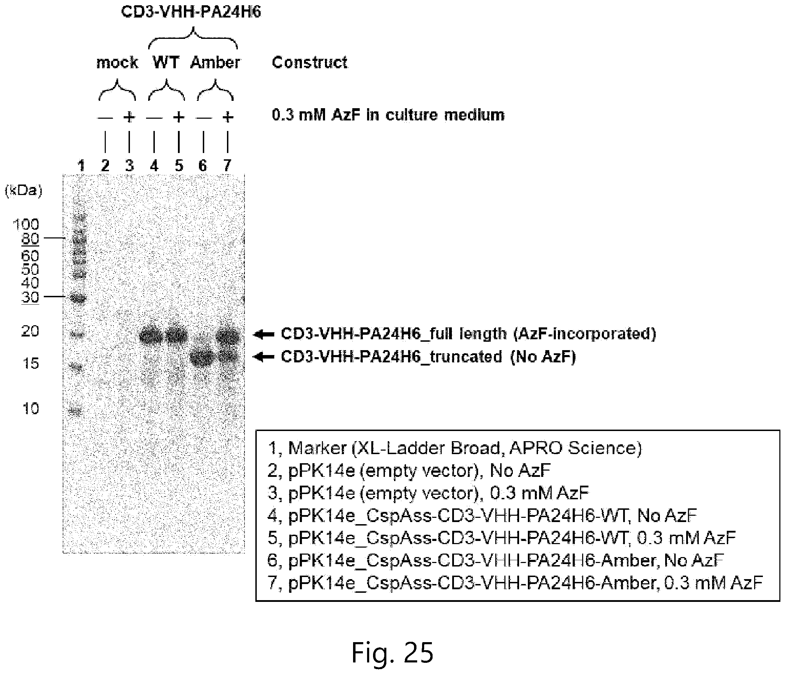

As a result, protein bands estimated to be CD3-VHH-PA24H6-WT were detected in the culture supernatant of the strain expressing CD3-VHH-PA24H6-WT regardless of the presence of azido-phenylalanine (FIG. 25, Lanes 4-5). In the culture supernatant of the strain expressing CD3-VHH-PA24H6-Amber, the absence of azido-phenylalanine resulted in detection of the protein band mainly on the smaller molecule side than CD3-VHH-PA24H6-WT, suggesting that a protein whose translation was terminated at the Amber codon site (position 142) in the PA24 linker was mainly secreted (FIG. 25, Lane 6), while the presence of azido-phenylalanine resulted in detection of a protein band mainly at the same position as CD3-VHH-PA24H6-WT, suggesting that full-length CD3-VHH-PA24H6-AzF was expressed whose translation was not terminated at the Amber codon site (position 142) in the PA24 linker, resulting in introduction of azido-phenylalanine (FIG. 25, Lane 7).

### (12-4) Purification of CD3-VHH-PA24H6-AzF (V-1)

The centrifugal supernatants of the culture of the CD3-VHH-PA24H6-AzF-expressing strain obtained in Example (12-3) were sterilized by filtration through a 0.2-µm PVDF membrane filter. The expressed CD3-VHH-PA24H6-AzF was purified through an affinity column by utilizing the C-terminal 6xHis-tag in order to remove contaminants and analogs without AzF introduced in the culture solution. Purification of the 6xHis-tag-added proteins was carried out using Ni-NTA Spin Kin (QIAGEN), and the purification conditions followed the manufacturer recommended protocol.

### (Example 13) Synthesis of Cetuximab with one VHH (V-1) molecule bound (D-4)

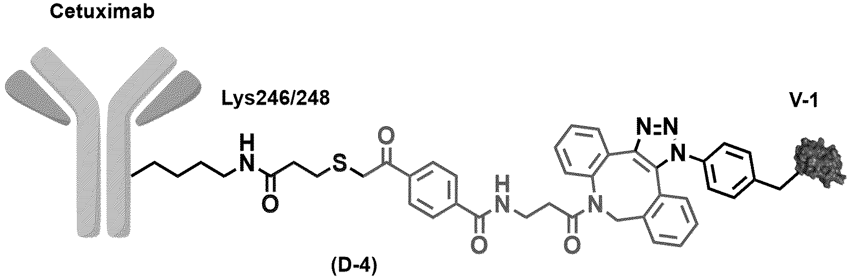

### (13-1) Modification of Cetuximab Using Affinity Reagent (4) (D-1)

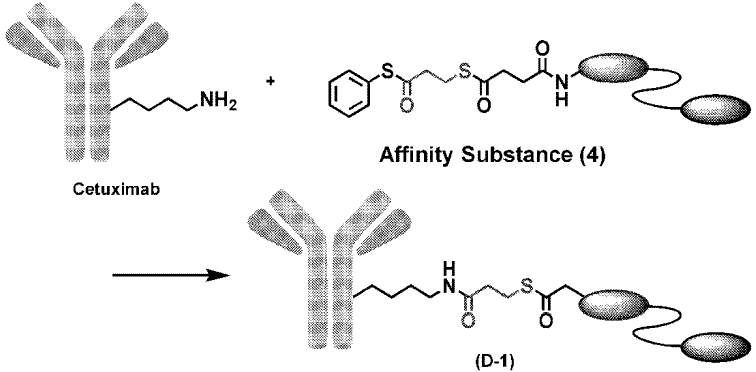

To a solution of the anti-human EGFR monoclonal antibody, Cetuximab (Merck Biopharma) in an acetate buffer (50 mM Sodium acetate, pH5.5), a solution of the affinity reagent (4) synthesized in Example 3 in DMF (5 equivalents relative to the antibody) was added, and then shaken for 1 hour. The reaction solution was purified using NAP-25 desalting column (Cytiva) to obtain Cetuximab with one affinity reagent (4) molecule bound (D-1). HIC-HPLC analysis was performed according to the previous report *(*Anal. Chem., 2019, 91, 20, 12724-12732*)* to confirm that Cetuximab had one affinity reagent (4) molecule bound.

### (13-2) Synthesis of Cetuximab with One Thiol Group Molecule (D-2)

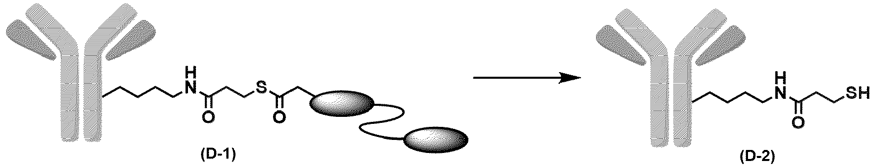

Subsequently, a hydroxylamine solution was added according to Example (4-3) and shaken at room temperature for 1 hour to obtain a thiol group-introduced antibody (D-2). HIC-HPLC analysis was performed according to the previous report (Anal. Chem., 2019, 91, 20, 12724-12732) to confirm that one thiol group molecule was introduced.

### (13-3) Synthesis of Cetuximab Having One DBCO Group Molecule (D-3)

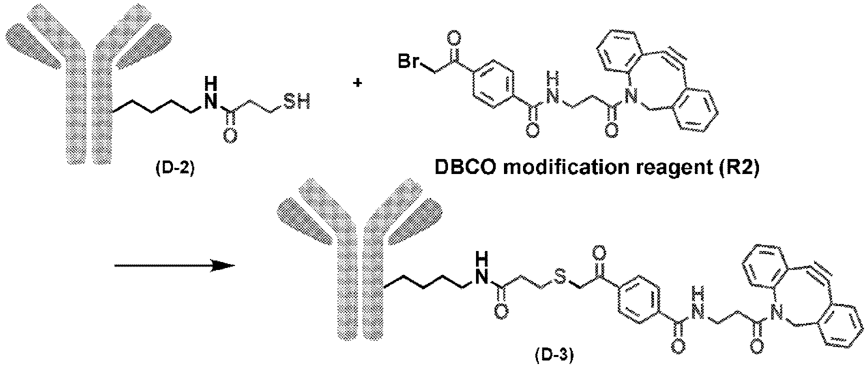

Cetuximab with one thiol group molecule introduced (D-2) synthesized in Example 13-2 and a DBCO group reagent (R2) described in WO2022154127 were used following the method described in WO2022154127 to obtain an antibody with one DBCO group molecule introduced (D-3). HIC-HPLC analysis was performed according to the previous report (Anal. Chem., 2019, 91, 20, 12724-12732) to confirm that Cetuximab had one DBCO group molecule bound.

### (13-4) Synthesis of Cetuximab with one VHH (V-1) molecule bound (D-4)

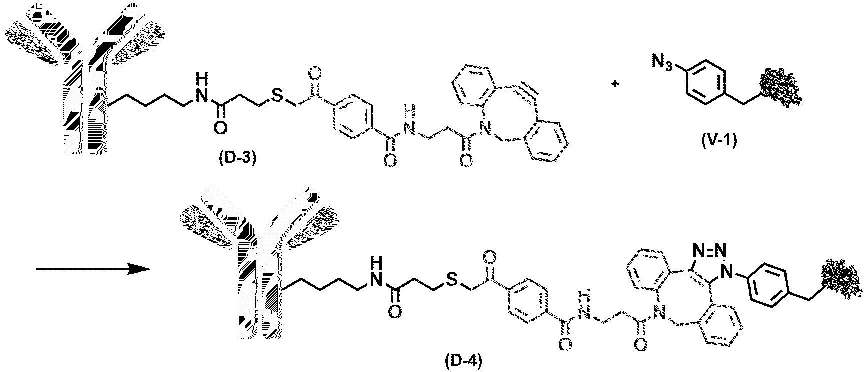

To a solution of Cetuximab with one DBCO group molecule bound (D-3) synthesized in Example (13-3) in an acetate buffer (50 mM Sodium acetate, pH5.5), the azido-phenylalanine-introduced VHH (3 equivalents relative to the antibody) synthesized in Example Z was added, and then shaken for 20 hour. The reaction solution was purified using AKTA pure25 (Cytiva) to obtain an antibody with one VHH molecule bound (D-4). ESI-TOFMS analysis was performed according to the previous report (Anal. Chem., 2019, 91, 20, 12724-12732) to observe a peak at 170492.

The purification with AKTA pure25 (Cytiva) was performed under the following conditions:
Column: Superdex200 Increase 10/300GL (Cytiva)
Eluent: PBS buffer (10 mM Phosphate Buffered Saline (PBS), pH7.4)
Flow rate: 0.6 ml/min
Detector: Detection was performed at wavelengths of 215 and 280 nm.

### (13-5) Affinity Evaluation of Cetuximab with One VHH (V-1) Molecule Bound (D-4) by Surface Plasmon (SPR) Method

The association constant of Cetuximab with one VHH (V-1) molecule bound (D-4) synthesized in Example (13-4) to antigens (EGFR and CD3) was determined by SPR analysis using Biacore T200 (Cytiva).

Recombinant human EGFR-Fc (R&D Systems) was biotinylated using Biotin Labeling Kit (Dojindo), and immobilized on sensor chips using Biotin CAPture Kit (Cytiva). For CD3, biotinylated recombinant human CD3ε/δ-Fc (ACRO Biosystems) was used, and immobilized on sensor chips using Biotin CAPture Kit (Cytiva). As a running buffer for evaluation of the affinity to antigens, HBS-EP+ buffer (Cytiva) was used.

Association of Cetuximab with one VHH (V-1) molecule bound (D-4) to the antigens and FcRn was evaluated by the Single cycle kinetics method in which the antibody was added at five concentration points. Fitting was performed on the obtained sensorgram using a 1:1 binding model, and the association constant was calculated.

As shown in Comparative Example 2, Cetuximab binds to EGFR that is an antigen with a dissociation constant of *K*_{D} = 610 pM.

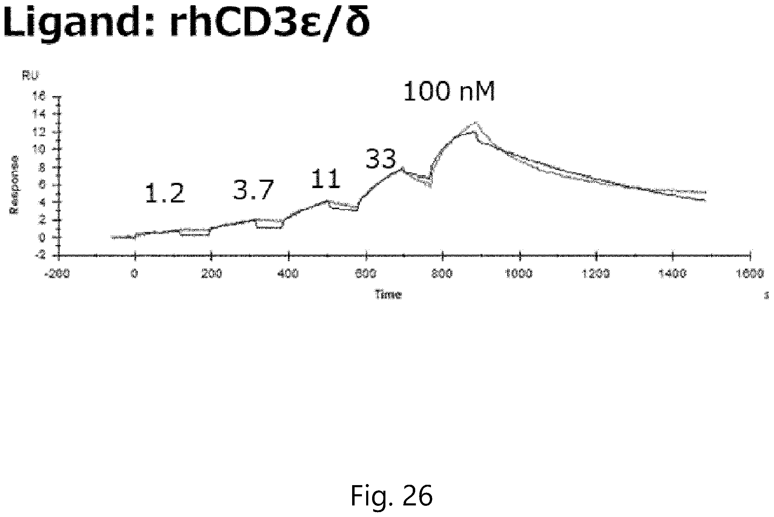

The sensorgrams that are the results of the evaluation of the CD3-VHH (V-1) affinity are shown in FIG. 26. It was demonstrated that CD3-VHH(V-1) bound to CD3ε/δ, with a dissociation constant of *K*_{D} = 7.6nM.

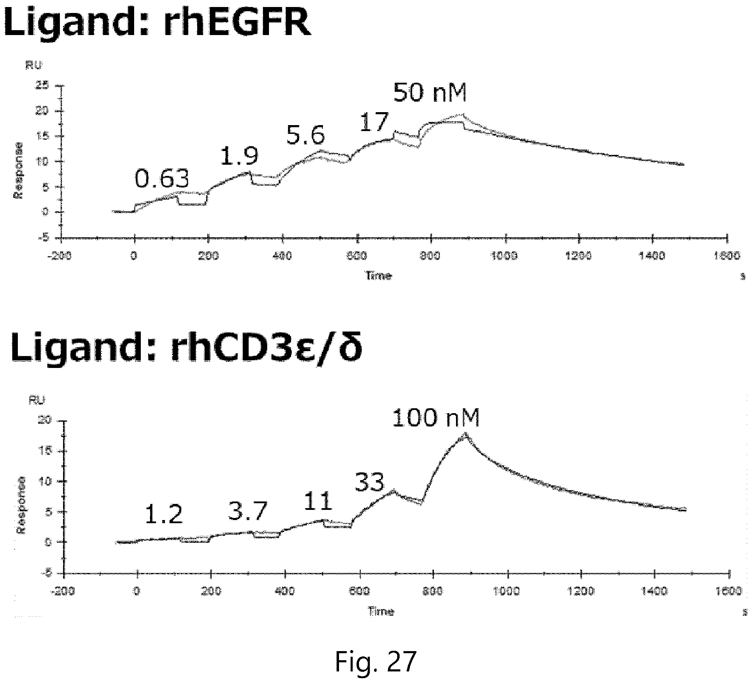

The sensorgram that is the result of the evaluation of the affinity of Cetuximab with one VHH (V-1) molecule bound (D-4) is shown in FIG. 27. It was demonstrated that (D-4) bound to EGFR that is an antigen of the parent antibody Cetuximab, with a dissociation constant of K_{D} = 630 pM. It was demonstrated that (D-4) also bound to CD3ε/δ that is an antigen of CD3-VHH (V-1), with a dissociation constant of K_{D} = 13 nM.

These results demonstrated that Cetuximab with one VHH (V-1) molecule bound (D-4) is a bi-specific antibody that can bind to EGFR and CD3 without impairing the binding affinity of the antibody and VHH that are binding molecules.

### (Example 14)

### (14-1) Synthesis of Trastuzumab with Two Thiol Group Molecules Bound to Lys288/290 of Antibody (E-1)

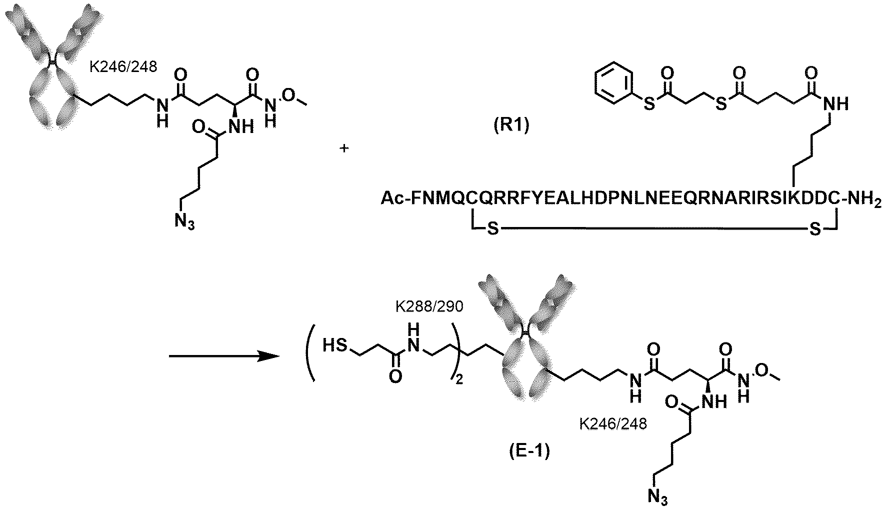

Trastuzumab with one azide group molecule introduced synthesized in Example (6-4) and a peptide reagent (R1) described in the previous report (WO2022/154116A1) was used following the method described in the previous report (WO2022/154116A1) to obtain an antibody with two thiol group molecules introduced (E-1). HIC-HPLC analysis was performed according to the previous report (Anal. Chem., 2019, 91, 20, 12724-12732) to confirm that one azide group molecule and two thiol group molecules were introduced.

### (14-2) Synthesis of DAR2 ADC (E-2)

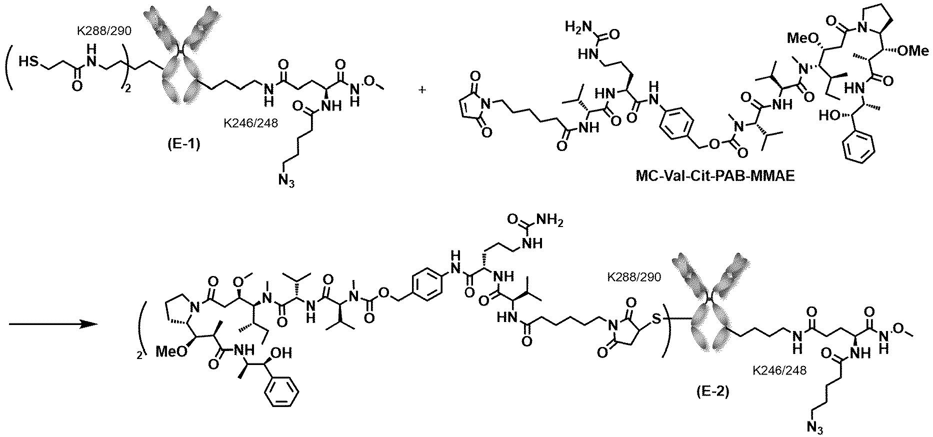

Using the antibody having one azide group molecule and two thiol group molecules (E-1) obtained in Example 14-1 and the known product MC-Val-Cit-PABA-MMAE (Organic & Biomolecular Chemistry, 2016, 14, 9501-9518), an antibody with two Val-Cit-PABA-MMAE molecules introduced (E-2) was obtained. HIC-HPLC analysis was performed according to the previous report (WO2019/240287A1) to confirm that two Val-Cit-PABA-MMAE molecules were introduced.

### (14-3) Synthesis of Cetuximab Fab Having DBCO Group (D-5)

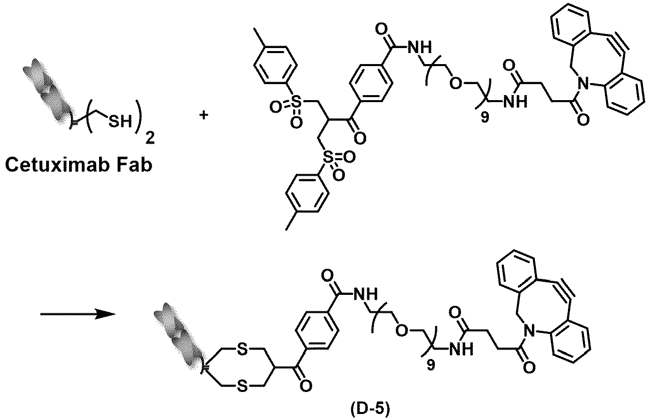

To a solution of Cetuximab Fab synthesized in Example (8-1-2) in a PBSE buffer (10 mM Phosphate Buffered Saline (PBS), 10 mM EDTA, pH7.4), a solution of a commercially-available Bis-Sulfone-PEG9-DBCO (Broadpharma) in dimethylformamide (3 equivalents relative to Fab, dimethylformamide (DMF) (8%v/v) solution) was added and shaken for 2 hours. The reaction solution was purified using a NAP-25 desalting column (Cytiva) to obtain synthesized Cetuximab Fab with a DBCO group introduced (D-5). ESI-TOFMS analysis was performed according to the previous report (Anal. Chem., 2019, 91, 20, 12724-12732) to observe a peak at 51706.

### (14-4) Synthesis of DAR2 ADC with one Cetuximab Fab molecule bound (E-3)

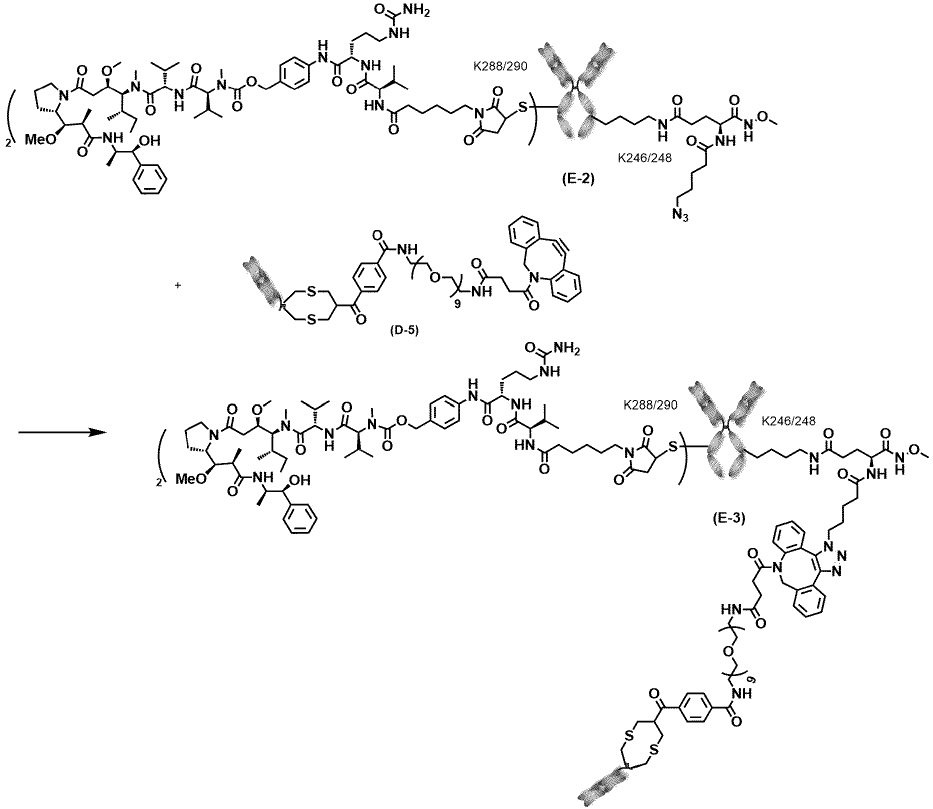

To a solution of the DAR2 ADC having one azide group molecule (E-2) synthesized in Example (14-2) in an acetate buffer (50 mM Sodium acetate, pH5.5), a solution of Cetuximab Fab having a DBCO group synthesized in Example (B-3) in an acetate buffer (50 mM Sodium acetate, pH5.5) (3 equivalents relative to the antibody) was added and shaken for 40 hours. The reaction solution was purified using AKTA pure25 (Cytiva) to obtain ADC with one Fab molecule bound (E-3). ESI-TOFMS analysis was performed according to the previous report (Anal. Chem., 2019, 91, 20, 12724-12732) to observe a peak at 203036.

The purification with AKTA pure25 (Cytiva) was performed under the following conditions:
Column: Superdex200 Increase 10/300GL (Cytiva)
Eluent: PBS buffer (10 mM Phosphate Buffered Saline (PBS), pH7.4)
Flow rate: 0.6 ml/min
Detector: Detection was performed at wavelengths of 215 and 280 nm.

### Example 15: Design of Affinity Substance and Secretory Expression Thereof in C. glutamicum

### (15-1) Outline of Design of Affinity Substance

In the present invention, it is required to design an affinity substance containing in its molecule two or more sites that can bind to an antibody. That is, it is necessary to design an affinity substance such that
A) a molecule in which two or more sites capable of binding to an antibody are linked via a linker to link the sites capable of binding to an antibody.

In the present invention, it is also required that a linker is linked to the affinity substance. That is, in order to achieve a specific binding of a linker to a specific site in the affinity substance, it is necessary to design the affinity substance such that the affinity substance has only one site capable of reacting with the linker.

When the affinity substance is polypeptide, and a linker is linked to the amino group of K (lysine) in the polypeptide, it is necessary to design the polypeptide such that only one amino group that reacts with the linker exists in the polypeptide. Based on this, the following design was made.
B) The polypeptide contains only one K residue.
C) By comprising Q (glutamine) as an N-terminal amino acid, Q (glutamine) is pyroglutamylated, and the N-terminal amino group is converted into an amide.

A polypeptidic affinity substance was designed as following (i) to (h) according to the rules of A), B), and C) above.

### (15-2) Preparation of Affinity Substance

The following affinity substances were prepared.
(i) QET-Fc3K-PA48G-Fc3R 
QET-RGNCAYHKGQIIWCTYH-
GGGGSGGAAPAAPAPAAPAAPAPAAPAAPAPAAPAAPAPAAPAAPAPAAPAAPAP
GGSGGGGSGG-RGNCAYHRGQIIWCTYH-NH2 (SEQ ID NO: 54)
(j) QET-Fc3K-PAS56-Fc3R 
QET-RGNCAYHKGQIIWCTYH-
GGGAAPASPAPAAPASPAPAAPASPAPAAPASPAPAAPASPAPAAPASPAPAAPASP
APGGG-RGNCAYHRGQIIWCTYH-NH2 (SEQ ID NO: 55)
(k) QET-Fc3K-PAS60-Fc3R 
QET-RGNCAYHKGQIIWCTYH-
GGAPSSPSPSAPSSPSPASPSSAPSSPSPSAPSSPSPASPSSAPSSPSPSAPSSPSPASP
SSGG-RGNCAYHRGQIIWCTYH-NH2 (SEQ ID NO: 56)
(l) QET-Fc3K-PAS64-Fc3R 
QET-RGNCAYHKGQIIWCTYH-
GGASPSAPAPASPSAPAPASPSAPAPASPSAPAPASPSAPAPASPSAPAPASPSAPAP
ASPSAPAPGG-RGNCAYHRGQIIWCTYH-NH2 (SEQ ID NO: 57)
(m) QET-Fc3K-PAS68-Fc3R 
QET-RGNCAYHKGQIIWCTYH-
GGAPSSPSPSAPSSPSPASPSSPAPAPAPAPAPAPAPAPAPAPAPAPAPAAPSSPSPSA
PSSPSPASPSSGG-RGNCAYHRGQIIWCTYH-NH2 (SEQ ID NO: 585)
(n) QET-Fc3K-PAS80-Fc3R

QET-RGNCAYHKGQIIWCTYH-
GGAPSSPSPSAPSSPSPASPSSAPSSPSPSAPSSPSPASPSSAPSSPSPSAPSSPSPASP

SSAPSSPSPSAPSSPSPASPSSGG-RGNCAYHRGQIIWCTYH-NH2 (SEQ ID NO: 59)

### (15-3) Expression of Affinity Substance

Expression of these polypeptidic affinity substances was examined using Corynex^{®}. In expression using Corynex^{®}, a CspB fusion method (WO 2013/062029), that is, a technique capable of improving the secretory production amount of a target polypeptide by inserting a base sequence encoding an amino acid sequence comprising N-terminal three residues Gln-Glu-Thr (QET) of a CspB mature protein between a base sequence encoding a signal peptide and a base sequence encoding a target polypeptide was utilized. In addition, since an N-terminal of a CspB tag is Q, there is an advantage that Q at the first residue can be pyroglutamylated to protect the N-terminal amino group after cleavage of the signal sequence. That is, in addition to the rules A), B) and C) above, it is effective to consider the rule D) below in expression of an affinity polypeptide using Corynex^{®}.
D) Three residues of QET are added to an N-terminal to improve secretory efficiency using Corynex^{®}.

Hereinafter, Examples of expression studies using Corynex^{®} will be described.

### (15-4) Construction of secretory expression plasmids QET-Fc3K-PA48G-Fc3R, QET-Fc3K-PAS56-Fc3R, QET-Fc3K-PAS60-Fc3R, QET-Fc3K-PAS64-Fc3R, QET-Fc3K-PAS68-Fc3R, and QET-Fc3K-PAS80-Fc3R

As affinity polypeptides, the above six types of amino acid sequences of QET-Fc3K-PA48G-Fc3R, QET-Fc3K-PAS56-Fc3R, QET-Fc3K-PAS60-Fc3R, QET-Fc3K-PAS64-Fc3R, QET-Fc3K-PAS68-Fc3R, and QET-Fc3K-PAS80-Fc3R were designed, and base sequences encoding these polypeptides were designated in consideration of codon usage frequency of *C. glutamicum.* Furthermore, the following expression cassettes were designed so as to make secretory expression by *C. glutamicum* possible.

QET-Fc3K-PA48G-Fc3R underwent secretory expression as a fusion protein of a signal peptide 30 amino acid residue of CspB derived from *C. glutamicum* ATCC13869 strain, an N-terminal 3 amino acid residue QET of CspB mature protein derived from *C. glutamicum* ATCC13869 strain, and Fc3K-PA48G-Fc3R (hereinafter referred to as "CspBss-QET-Fc3KR-PA48G"). The designed base sequence encoding CspBss-QET-Fc3KR-PA48G and the amino acid sequence thereof are illustrated in SEQ ID NOs: 60 and 61, respectively.

Base sequence encoding CspBss-QET-Fc3KR-PA48G 
atgtttaacaaccgtatccgcactgcagctctcgctggtgcaatcgcaatctccaccgcagcttccggcgtagctatcccagcat
tcgctcaggagacccgtggcaattgcgcctatcacaagggtcagatcatttggtgcacctaccatggtggtggaggttctggtg
gtgccgccccggctgcaccagcgccagcggccccagcggctcctgcaccggctgctccagctgcgccagctcctgcagct
cccgcagcaccggcgcctgcagcaccagcggcaccagcacccgccgctccggccgcccctgcccccggcggctccggg
ggaggcggcagcggaggacgcggtaactgcgcataccatcgcggccaaattatctggtgcacttatcactaa (SEQ ID
NO: 60)
Amino acid sequence of CspBss-QET-Fc3KR-PA48G

MFNNRIRTAALAGAIAISTAASGVAIPAFAQETRGNCAYHKGQIIWCTYHGGGGS
GGAAPAAPAPAAPAAPAPAAPAAPAPAAPAAPAPAAPAAPAPAAPAAPAPGGSGG
GGSGGRGNCAYHRGQIIWCTYH (SEQ ID NO: 61)

QET-Fc3K-PAS56-Fc3R underwent secretory expression as a fusion protein of a signal peptide 30 amino acid residue of CspB derived from *C. glutamicum* ATCC13869 strain, an N-terminal 3 amino acid residue QET of CspB mature protein derived from *C. glutamicum* ATCC13869 strain, and Fc3K-PAS56-Fc3R (hereinafter referred to as "CspBss-QET-Fc3KR-PAS56"). The designed base sequence encoding CspBss-QET-Fc3KR-PAS56 and the amino acid sequence thereof are illustrated in SEQ ID NOs: 62 and 63, respectively.

Base sequence encoding CspBss-QET-Fc3KR-PAS56 
atgtttaacaaccgtatccgcactgcagctctcgctggtgcaatcgcaatctccaccgcagcttccggcgtagctatcccagcat
tcgctcaggagacccgtggcaattgcgcctatcacaagggtcagatcatttggtgcacctaccatggtggtggtgccgctcca
gcatccccagccccggccgcacctgcaagccctgctcccgctgctccggcttcaccagcgccagcagcacctgcctctccc
gctcctgcggcaccagcgagtcctgcgccagccgctccggcatcgcccgcaccagcggctccggcctccccggcccccg
gcggaggacgcggtaactgcgcataccatcgcggccaaattatctggtgcacttatcactaa (SEQ ID NO: 62)
Amino acid sequence of CspBss-QET-Fc3KR-PAS56

MFNNRIRTAALAGAIAISTAASGVAIPAFAQETRGNCAYHKGQIIWCTYHGGGAA
PASPAPAAPASPAPAAPASPAPAAPASPAPAAPASPAPAAPASPAPAAPASPAPGGGR
GNCAYHRGQIIWCTYH (SEQ ID NO: 63)

QET-Fc3K-PAS60-Fc3R underwent secretory expression as a fusion protein of a signal peptide 30 amino acid residue of CspB derived from *C. glutamicum* ATCC13869 strain, an N-terminal 3 amino acid residue QET of CspB mature protein derived from *C. glutamicum* ATCC13869 strain, and Fc3K-PAS60-Fc3R (hereinafter referred to as "CspBss-QET-Fc3KR-PAS60"). The designed base sequence encoding CspBss-QET-Fc3KR-PAS60 and the amino acid sequence thereof are illustrated in SEQ ID NOs: 64 and 65, respectively.

Base sequence encoding CspBss-QET-Fc3KR-PAS60 
atgtttaacaaccgtatccgcactgcagctctcgctggtgcaatcgcaatctccaccgcagcttccggcgtagctatcccagcat
tcgctcaggagacccgtggcaattgcgcctatcacaagggtcagatcatttggtgcacctaccatggtggtgccccatcctctc
cctcaccctcggcaccgagctccccatctccggcgtcaccatcctccgcaccctcttccccttctccatcggctccttcgtcccc
ttccccagcttcccctagctccgcaccgtccagcccctctccatcagcgccttcctcgccatcaccggcctctccgagctccgg
aggacgcggtaactgcgcataccatcgcggccaaattatctggtgcacttatcactaa (SEQ ID NO: 64)
Amino acid sequence of CspBss-QET-Fc3KR-PAS60

MFNNRIRTAALAGAIAISTAASGVAIPAFAQETRGNCAYHKGQIIWCTYHGGAPS
SPSPSAPSSPSPASPSSAPSSPSPSAPSSPSPASPSSAPSSPSPSAPSSPSPASPSSGGR
GNCAYHRGQIIWCTYH (SEQ ID NO: 65)

QET-Fc3K-PAS64-Fc3R underwent secretory expression as a fusion protein of a signal peptide 30 amino acid residue of CspB derived from *C. glutamicum* ATCC13869 strain, an N-terminal 3 amino acid residue QET of CspB mature protein derived from *C. glutamicum* ATCC13869 strain, and Fc3K-PAS64-Fc3R (hereinafter referred to as "CspBss-QET-Fc3KR-PAS64"). The designed base sequence encoding CspBss-QET-Fc3KR-PAS64 and the amino acid sequence thereof are illustrated in SEQ ID NOs: 66 and 67, respectively.

Base sequence encoding CspBss-QET-Fc3KR-PAS64 
atgtttaacaaccgtatccgcactgcagctctcgctggtgcaatcgcaatctccaccgcagcttccggcgtagctatcccagcat
tcgctcaggagacccgtggcaattgcgcctatcacaagggtcagatcatttggtgcacctaccatggtggtgcgtccccctcag
ctcccgcacctgcgagtcctagcgcgccagcacctgcatccccttccgctccggcacctgcctctccgtcagcaccggctcc
agcaagcccatccgccccagctccagcttctccgtccgcaccagcccccgcctctccttcggcgccagctccggcgtcgcca
agcgctccagcccccggaggacgcggtaactgcgcataccatcgcggccaaattatctggtgcacttatcactaa (SEQ
ID NO: 66)
Amino acid sequence of CspBss-QET-Fc3KR-PAS64

MFNNRIRTAALAGAIAISTAASGVAIPAFAQETRGNCAYHKGQIIWCTYHGGASP
SAPAPASPSAPAPASPSAPAPASPSAPAPASPSAPAPASPSAPAPASPSAPAPASPSAP
APGGRGNCAYHRGQIIWCTYH (SEQ ID NO: 67)

QET-Fc3K-PAS68-Fc3R underwent secretory expression as a fusion protein of a signal peptide 30 amino acid residue of CspB derived from *C. glutamicum* ATCC13869 strain, an N-terminal 3 amino acid residue QET of CspB mature protein derived from *C. glutamicum* ATCC13869 strain, and Fc3K-PAS68-Fc3R (hereinafter referred to as "CspBss-QET-Fc3KR-PAS68"). The designed base sequence encoding CspBss-QET-Fc3KR-PAS68 and the amino acid sequence thereof are illustrated in SEQ ID NOs: 68 and 69, respectively.

Base sequence encoding CspBss-QET-Fc3KR-PAS68 
atgtttaacaaccgtatccgcactgcagctctcgctggtgcaatcgcaatctccaccgcagcttccggcgtagctatcccagcat
tcgctcaggagacccgtggcaattgcgcctatcacaagggtcagatcatttggtgcacctaccatggtggtgcaccgagctctc
cctccccctctgccccgtcttcgccgtcaccagcttccccatccagtccagctccggcgcctgcccctgccccagcaccggct
cctgctcctgcgccagctccagcaccagcgcctgcaccagcccctgcagcaccctcctcaccatcgccaagtgcgccctcct
ccccttccccggcttctcccagcagcggaggacgcggtaactgcgcataccatcgcggccaaattatctggtgcacttatcact
aa (SEQ ID NO: 68)
Amino acid sequence of CspBss-QET-Fc3KR-PAS68

MFNNRIRTAALAGAIAISTAASGVAIPAFAQETRGNCAYHKGQIIWCTYHGGAPS
SPSPSAPSSPSPASPSSPAPAPAPAPAPAPAPAPAPAPAPAPAAPSSPSPSAPSSPSP
ASPSSGGRGNCAYHRGQIIWCTYH (SEQ ID NO: 69)

QET-Fc3K-PAS80-Fc3R underwent secretory expression as a fusion protein of a signal peptide 30 amino acid residue of CspB derived from *C. glutamicum* ATCC13869 strain, an N-terminal 3 amino acid residue QET of CspB mature protein derived from *C. glutamicum* ATCC13869 strain, and Fc3K-PAS80-Fc3R (hereinafter referred to as "CspBss-QET-Fc3KR-PAS80"). The designed base sequence encoding CspBss-QET-Fc3KR-PAS80 and the amino acid sequence thereof are illustrated in SEQ ID NOs: 70 and 71, respectively.

Base sequence encoding CspBss-QET-Fc3KR-PAS80 
atgtttaacaaccgtatccgcactgcagctctcgctggtgcaatcgcaatctccaccgcagcttccggcgtagctatcccagcat
tcgctcaggagacccgtggcaattgcgcctatcacaagggtcagatcatttggtgcacctaccatggtggtgccccgtcctccc
catccccatctgccccttcgagcccatcccctgcatcaccctcctcggctccttcctcaccctctccatccgcgccatctagccc
atctccagcctccccttcttccgctccttcatctccttcgccctccgcacccagctccccaagccctgcgtccccgtctagcgca
ccgtcgtcgccgtccccatccgcaccgtcatccccgtcaccagctagcccctcctccggaggacgcggtaactgcgcatacc
atcgcggccaaattatctggtgcacttatcactaa (SEQ ID NO: 70)
Amino acid sequence of CspBss-QET-Fc3KR-PAS80

MFNNRIRTAALAGAIAISTAASGVAIPAFAQETRGNCAYHKGQIIWCTYHGGAPS
SPSPSAPSSPSPASPSSAPSSPSPSAPSSPSPASPSSAPSSPSPSAPSSPSPASPSSAPSS
PSPSAPSSPSPASPSSGGRGNCAYHRGQIIWCTYH (SEQ ID NO: 71)

Expression cassettes of six types of affinity polypeptides were designed in each of which a promoter of the cspB gene derived from *C. glutamicum* ATCC13869 strain was coupled with each of upstream sides of the base sequences described in CspBss-QET-Fc3KR-PA48G, CspBss-QET-Fc3KR-PAS56, CspBss-QET-Fc3KR-PAS60, CspBss-QET-Fc3KR-PAS64, CspBss-QET-Fc3KR-PAS68, and CspBss-QET-Fc3KR-PAS80, and a KpnI site was added to the 5'-side and a BamHI site was added to the 3'-side, and total synthesis was performed. By inserting the totally synthesized DNA fragments (expression cassettes of affinity polypeptides) into the KpnI-BamHI site of pPK4 described in JP 9-322774 A, pPK4_CspBss-QET-Fc3KR-PA48G, pPK4_CspBss-QET-Fc3KR-PAS56, pPK4_CspBss-QET-Fc3KR-PAS60, pPK4_CspBss-QET-Fc3KR-PAS64, pPK4_CspBss-QET-Fc3KR-PAS68, and pPK4_CspBss-QET-Fc3KR-PAS80, which are secretory expression plasmids of affinity polypeptides, were constructed. As a result of determining the base sequences of the inserted fragments, it was determined that the expression cassettes of affinity polypeptides as designed were constructed. The base sequence was determined using BigDye (R) Terminator v3.1 Cycle Sequencing Kit (Applied Biosystems) and 3500xL Genetic Analyzer (Applied Biosystems).

### (15-5) Secretory Expression of Affinity Polypeptide in C. glutamicum

Using pPK4_CspBss-QET-Fc3KR-PA48G, pPK4_CspBss-QET-Fc3KR-PAS56, pPK4_CspBss-QET-Fc3KR-PAS60, pPK4_CspBss-QET-Fc3KR-PAS64, pPK4_CspBss-QET-Fc3KR-PAS68, and pPK4_CspBss-QET-Fc3KR-PAS80 constructed in Example (15-4), *C. glutamicum* YDK0107 strain described in WO2016/171224 was transformed to obtain YDK0107/pPK4_CspBss-QET-Fc3KR-PA48G strain, YDK0107/pPK4_CspBss-QET-Fc3KR-PAS56 strain, YDK0107/pPK4_CspBss-QET-Fc3KR-PAS60 strain, YDK0107/pPK4_CspBss-QET-Fc3KR-PAS64 strain, YDK0107/pPK4_CspBss-QET-Fc3KR-PAS68 strain, and YDK0107/pPK4_CspBss-QET-Fc3KR-PAS80 strain.

Similarly, using the plasmids, the YDK0107Δpmt1 strain designed in (Reference Example 1) described below was transformed to obtain
YDK0107Δpmt1/pPK4_CspBss-QET-Fc3KR-PA48G strain,
YDK0107Δpmt1/pPK4_CspBss-QET-Fc3KR-PAS56 strain,
YDK0107Δpmt1/pPK4_CspBss-QET-Fc3KR-PAS60 strain,
YDK0107Δpmt1/pPK4_CspBss-QET-Fc3KR-PAS64 strain,
YDK0107Δpmt1/pPK4_CspBss-QET-Fc3KR-PAS68 strain, and
YDK0107Δpmt1/pPK4_CspBss-QET-Fc3KR-PAS80 strain.

Each of the obtained transformants was cultured at 30°C for 72 hours in an MMTG liquid medium comprising 25 mg/L kanamycin.

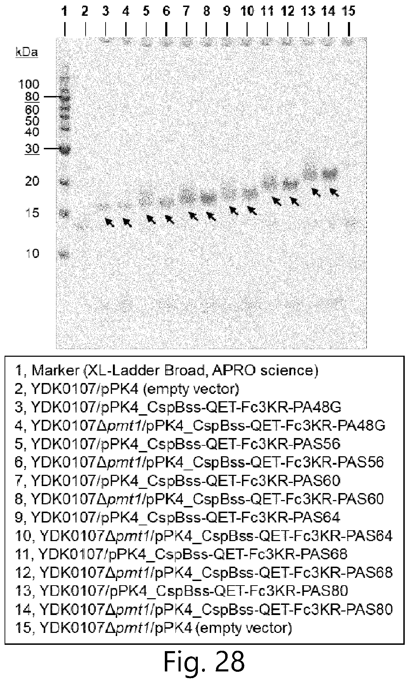

After completion of the culture, 6.5 µL of the culture supernatant obtained by centrifuging each of the culture solutions was subjected to reducing SDS-PAGE using NuPAGE^{®} 12% Bis-Tris Gel (Thermo Fisher Scientific, Inc.), and then stained with Quick-CBB (Wako). As a result, polypeptide bands estimated to be QET-Fc3KR-PA48G were detected in culture supernatant of YDK0107/pPK4_CspBss-QET-Fc3KR-PA48G strain and YDK0107Δpmt1/pPK4_CspBss-QET-Fc3KR-PA48G strain (FIG. 28, Lanes 3-4), polypeptide bands estimated to be QET-Fc3KR-PAS56 were detected in culture supernatant of YDK0107/pPK4_CspBss-QET-Fc3KR-PAS56 strain and YDK0107Δpmt1/pPK4_CspBss-QET-Fc3KR-PAS56 strain (FIG. 28, Lanes 5-6), polypeptide bands estimated to be QET-Fc3KR-PAS60 were detected in culture supernatant of YDK0107/pPK4_CspBss-QET-Fc3KR-PAS60 strain and YDK0107Δpmt1/pPK4_CspBss-QET-Fc3KR-PAS60 strain (FIG. 28, Lanes 7-8), polypeptide bands estimated to be QET-Fc3KR-PAS64 were detected in culture supernatant of YDK0107/pPK4_CspBss-QET-Fc3KR-PAS64 strain and YDK0107Δpmt1/pPK4_CspBss-QET-Fc3KR-PAS64 strain (FIG. 28, Lanes 9-10), polypeptide bands estimated to be QET-Fc3KR-PAS68 were detected in culture supernatant of YDK0107/pPK4_CspBss-QET-Fc3KR-PAS68 strain and YDK0107Δpmt1/pPK4_CspBss-QET-Fc3KR-PAS68 strain (FIG. 28, Lanes 11-12), and polypeptide bands estimated to be QET-Fc3KR-PAS80 were detected in culture supernatant of YDK0107/pPK4_CspBss-QET-Fc3KR-PAS80 strain and YDK0107Δpmt1/pPK4_CspBss-QET-Fc3KR-PAS80 strain (FIG. 28, Lanes 13-14).

When the polypeptides QET-Fc3KR-PAS56, QET-Fc3KR-PAS60, QET-Fc3KR-PAS64, QET-Fc3KR-PAS68, and QET-Fc3KR-PAS80 were expressed using the YDK0107 strain as the host, smear-like bands were detected, suggesting that O-glycosylation had occurred at the Ser residues within the PAS linker (FIG. 28, Lanes 5, 7, 9, 11, and 13).

On the other hand, when these polypeptides were expressed using the YDK0107Δpmt1 strain as the host, sharp bands were detected, suggesting that the O-glycosylation was eliminated (FIG. 28, Lanes 6, 8, 10, 12, and 14).

### (Reference Example 1) Construction of Corynebacterium glutamicum with Deletion of O-mannosyltransferase Gene pmt1

### (1) Construction of pmt1 Gene Deleting Vector pBSSTΔpmt1

It is known that O-glycosylation occurs in secretory proteins expressed using *C. glutamicum* as the host, and deletion of Protein O-mannosyltransferase PMT1 can result in the loss of the ability to modify secreted proteins with O-glycosylation (Martina Mahne et al., The Corynebacterium glutamicum gene pmt encoding a glycosyltransferase related to eukaryotic protein-O-mannosyltransferases is essential for glycosylation of the resuscitation promoting factor (Rpf2) and other secreted proteins. FEMS Microbiol Lett. 2006 Jun; 259(2): 226-33.).

It is also known that when Proline/Alanine/Serine (PAS) biopolymer is expressed in a secretory manner using the *C. glutamicum* YDK0107 as the host, O-glycosylation occurs at the Ser residues within the PAS sequence, and use of the YDK0107Δpmt1 strain that is deficient in the PMT1 gene as the host can result in elimination of the O-glycosylation (L. Friedrich1 et al., Efficient secretory production of proline/alanine/serine (PAS) biopolymers in Corynebacterium glutamicum yielding a monodisperse biological alternative to polyethylene glycol (PEG). Microb Cell Fact. 2022 Oct 28; 21(1): 227*).*

The genome sequence of *C. glutamicum* ATCC13869 strain and the base sequence of the pmt1 gene encoding Protein O-mannosyltransferase PMT1 have already been determined (GenBank Accession No. AP017557, and NCBI locus_tag CGBL_0109730). The base sequence of the pmt1 gene of *C. glutamicum* ATCC 13869 strain and the amino acid sequence of PMT1 encoded by the gene are illustrated in SEQ ID NOs: 72 and 73, respectively.

SEQ ID NO: 72 
gtgagccaagccctacctgttcgtgaccaggggcgggaccaggggattttcgctggtactctcccgcccgcaccgccaaagt
tcaagtggacccgcctggatacctatacctgggcgatcatcgcggtgtttgctttagtcacgcgttttacaggtttgagcagtgca
accgcctcaggcaccccagtttttgatgaaaaacactacgtcccacaagcctgggacatggtccgctcctggatcaaccccat
 
caccggcggcattgaatccaaccccggatacggactcgtcgttcacccaccgttggccaaacaacttgaagcactcggtgaat
gggtcttcggatacacccccctcggctggcgcatcatggtcgccattttcggaacactcaccattttcgccatcatggcaatcgc
acggcgcctcagcggatccaccattgtgactttcatcgccggaattctcgcgcttgccgacggcgtcctcctcgtctcatcacg
cttcggcatgctggacatcttccttgttttcttcatcaccgcagcagcatgggcattgatccgagatcaccaacaaatgcaccaac
gcctcaacgatttactcctcaccaacggccaaatcaccaaagactttggcccccgattcggcttccgatggtggcgtttcacca
caggtgtattccttggtctcgcgctctccgtcaaatggtccggactgtactacatcgcattcttcggcctgaccagcgtgttccta
gacttgtggctgcgcaaacgctacggcgtccgacgctacgtcaccggcaccctaaaaaatgacgtcatcccagcgctcggct
ccttggtgatcatccctgccctgctttatatctggagctggcgagcctggttcgcctccgaaacctccgtctaccgccacgccaa
aacagacggcaccatcacagaagactccatcctgcagctcttccctgaatcaattgcgggctggatccactatcacatcagcgt
cctggaattccacggctcactgaccacatcctcaggtcacagccacccctgggattccaagccgtgggcctggcttgtctccg
gtcgacccatcctctacttctcctccaccgacatctcctgcgacgttggcggcacctgccgacgcatgatctacctcttcggcac
gcccgccatttggtggctgacagtcccagtcattctgtgggcgctgtggtctttcttcgcacgtcggagtcgtggctatattgtcc
cactagtggccttcgctgcaggatttttgccgtggcttgcagcatacgaccgccaaatgtacttcttctacgccaccgcattggtg
ccattcaccatcatcatgttggcactcgcctgcggtgaactgtggggccgtggaaaaatgacccccactggcttaacccgcgg
atcaatggcagtggtcacctacatttccctcgtggtgatgatgtttctcgcgttttcgccactgttttacggatttgtcatcccagatt
atgtctacgaatccttgatgtggttcccaagctggcgctaa
SEQ ID NO: 73

MSQALPVRDQGRDQGIFAGTLPPAPPKFKWTRLDTYTWAIIAVFALVTRFTGLSS
ATASGTPVFDEKHYVPQAWDMVRSWINPITGGIESNPGYGLVVHPPLAKQLEAL
GEWVFGYTPLGWRIMVAIFGTLTIFAIMAIARRLSGSTIVTFIAGILALADGVLLV
SSRFGMLDIFLVFFITAAAWALIRDHQQMHQRLNDLLLTNGQITKDFGPRFGFRW
WRFTTGVFLGLALSVKWSGLYYIAFFGLTSVFLDLWLRKRYGVRRYVTGTLKN
DVIPALGSLVIIPALLYIWSWRAWFASETSVYRHAKTDGTITEDSILQLFPESIAGW
IHYHISVLEFHGSLTTSSGHSHPWDSKPWAWLVSGRPILYFSSTDISCDVGGTCRR
MIYLFGTPAIWWLTVPVILWALWSFFARRSRGYIVPLVAFAAGFLPWLAAYDRQ
MYFFYATALVPFTIIMLALACGELWGRGKMTPTGLTRGSMAVVTYISLVVMMFL
AFSPLFYGFVIPDYVVYESLMWFPSWR

Using the chromosomal DNA of *C. glutamicum* ATCC13869 strain, prepared using PurElute^{™} Genomic DNA Kit (EdgeBio) as a template, about 1 kbp upstream of the 5' side of the pmt1 gene was amplified using primers of SEQ ID NOs: 74 and 75, and about 1 kbp region downstream of the 3' side of the pmt1 gene was amplified using primers of SEQ ID NOs: 76 and 77, by the PCR method. Next, the PCR method using both the amplified DNA fragments as templates and the DNAs illustrated in SEQ ID NOs: 74 and 77 as primers gave about 2-kbp DNA fragment in which both the DNA fragments were fused. The PCR used Pyrobest^{®} DNA polymerase (Takara Bio), and the reaction conditions followed the manufacturer recommended protocol. The DNA fragment was inserted in the SmaI site of pBS5T described in WO2006/057450 to obtain pBSSTΔpmt1 vector for pmt1 gene deletion. The ligation reaction was carried out using DNA Ligation Kit <Mighty Mix> (Takara Bio), and the reaction conditions followed the manufacturer recommended protocol.

### Sequence of Primer for pmt1 Gene Knockout

SEQ ID NO: 74
   gtttcgtacctaaaccgacccgaacc
SEQ ID NO: 75
   tcggttgattgtagagccttggcg
SEQ ID NO: 76
   gccaaggctctacaatcaaccgatggccgaaccaagtgaccaatgc
SEQ ID NO: 77
   aagctccggcattgattccgttacc

### (2) Establishment of pmt1 Gene-deficient Strain from C. glutamicum YDK0107 Strain

pBSSTΔpmt1 constructed in (1) was used to transform the *C. glutamicum* YDK0107 strain described in WO2016/171224. The *C. glutamicum* YDK0107 strain is a strain with natural mutation, derived from the *C. glutamicum* YDK010 strain as the parent strain, in which the phoS gene has introduced W302C mutation (WO2016/171224). The *C. glutamicum* YDK010 strain is a strain lacking a cell surface protein CspB derived from *C. glutamicum* AJ12036 (FERM BP-734) (WO2002/081694). Strain selection was performed from the obtained transformants according to the method described in WO2006/057450 to obtain YDK0107Δpmt1 strain with pmt1 gene deficiency.

### Example 16

### (16-1) Synthesis of Affinity Reagent

The peptides (SEQ ID NOs: 54 to 59) synthesized in Example 15 were amidated according to Example 3 to prepare affinity substances (W-1, -2, -3, -4, -5, -6).

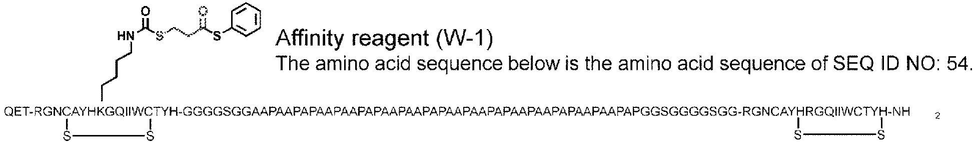

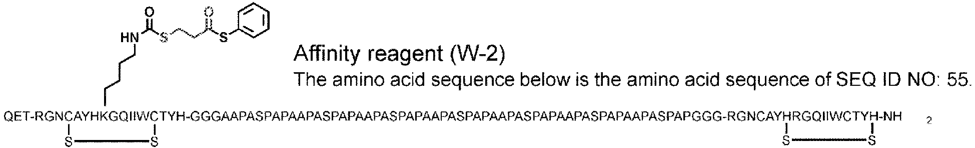

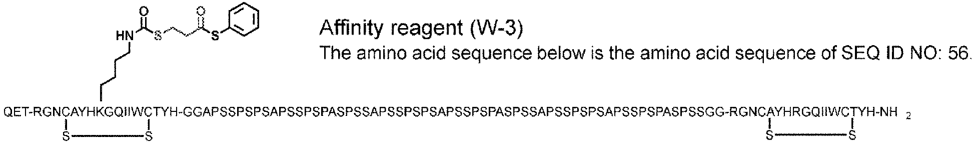

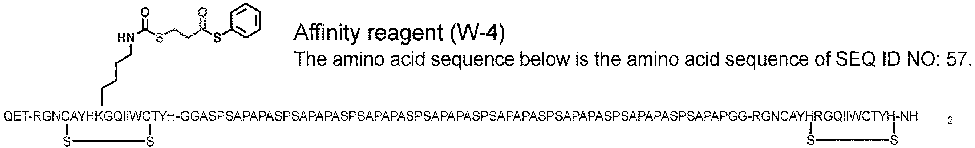

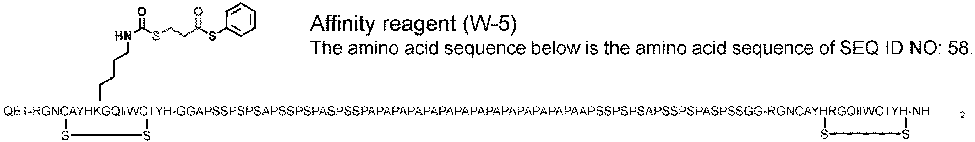

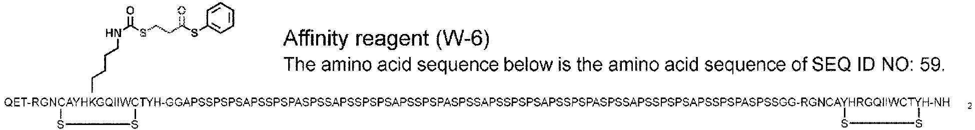

### (16-2) Modification of Trastuzumab Using Affinity Reagent

According to Example (4-1), to a solution of the anti-human HER2 monoclonal antibody Trastuzumab (Chugai Pharmaceutical Co., Ltd.) in an acetate buffer (50 mM Sodium acetate, pH5.5), a solution of the affinity reagent (W-3, -4, or -5) synthesized in Example (U-1) in DMF (5 equivalents relative to the antibody) was added, and then shaken for 1 hour. The reaction solution was purified using AKTA Nap25 (Cytiva) to obtain Trastuzumab with one affinity reagent molecule bound. ESI-TOFMS analysis was performed according to the previous report (Anal. Chem., 2019, 91, 20, 12724-12732) to confirm that Trastuzumab had one affinity reagent molecule bound.

### (16-3) Confirmation of the Peptide/Antibody Bonding Ratio of the Specific Modified Form of Trastuzumab by DAR Calculator

For MS data analyzed in Example (16-2), the peptide/antibody bonding ratio was confirmed by DAR calculator (Agilent software). The results are shown in Table 6.

**Table 6.**

| | DAR peak | Observed mass (Da) | Peak area | %area |
|---|---|---|---|---|
| W-3 | 0 | 148224 | 2.73 E+04 | 4.6 |
| | 1 | 158484 | 5.67 E+05 | 95.4 |
| W-4 | 1 | 158506 | 8.65 E+05 | 90.98 |
| | 2 | 168788 | 8.57 E+04 | 9.02 |
| W-5 | 1 | 158957 | 5.29 E+05 | 65.35 |
| | 2 | 169690 | 2.81 E+05 | 34.65 |

The results show that when the affinity reagent (W-3) was reacted, Trastuzumab with one affinity reagent molecule bound with one affinity reagent molecule bound was obtained with approximately 95% selectivity.

The results also show that when the affinity reagent (W-4) was reacted, Trastuzumab with one affinity reagent molecule bound with one affinity reagent molecule bound was obtained with approximately 91% selectivity.

The results also show that when the affinity reagent (W-5) was reacted, Trastuzumab with one affinity reagent molecule bound with one affinity reagent molecule bound was obtained with approximately 65% selectivity.

### (16-4) Synthesis of Trastzumab with One Thiol Group Molecule

The antibody with one affinity reagent molecule bound synthesized in Example (16-3) was used according to Example (4-3) to obtain a thiol group-introduced antibody. HIC-HPLC analysis was performed according to the previous report (Anal. Chem., 2019, 91, 20, 12724-12732) to confirm that one thiol group molecule was introduced.

### Example 17 Synthesis of Affinity Reagent

### (17-1) Synthesis of Compound (PL-1)

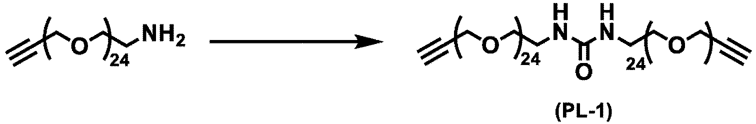

To a solution of commercially-available Propargyl-PEG24-amine (BroadPharma, 51.7 mg) in DMF (1 mL), 1,1'-Carbodiimidazole (3.3 mg) and triethylamine (8.5 µL) were added and stirred at room temperature for 3 hours. After completion of the reaction, reversed phase preparative chromatography was performed for elution. The fraction containing the product was collected, and the acetonitrile was removed by concentration under reduced pressure, followed by lyophilization to obtain the modifying reagent (PL-1) (34.7 mg).

### (17-2) Synthesis of Compound (PL-2)

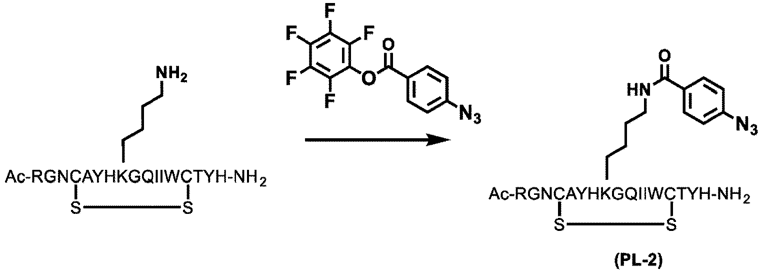

**The aforementioned amino acid sequence is the amino acid sequence of SEQ ID NO: 38.**

Ac-RGNCAYHKGQIIWCTYH-NH2 (SEQ ID NO: 38, 51.7 mg, where the two cysteines at positions 4 and 14 each form a disulfide bond in the molecule) described in the previous report (WO2019/240287A1) was dissolved in dimethylformamide (1 mL), to which known 2,3,4,5,6-Pentafluorophenyl 4-azidobenzoate (40.7 mg) and triethylamine (17.2 µL) were added, and the mixture was stirred at room temperature for 1 hour. After completion of the reaction, reversed phase preparative chromatography was performed for elution. The fraction containing the product was collected, and the acetonitrile was removed by concentration under reduced pressure, followed by lyophilization to obtain the modifying reagent (PL-2) (37.8 mg).

### (17-3) Synthesis of Compound (PL-3)

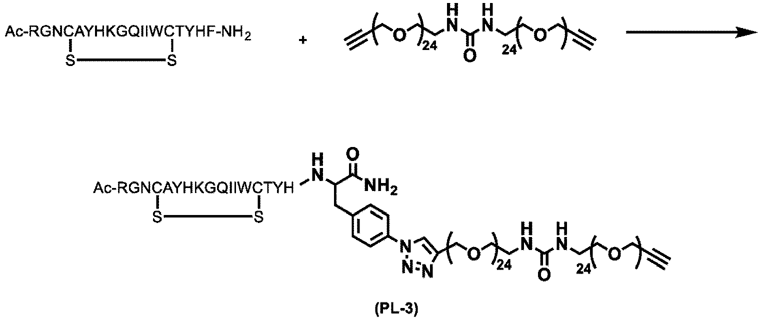

**The amino acid sequence in the upper row is the amino acid sequence of SEQ ID NO: 79.**
**The amino acid sequence in the lower row is the amino acid sequence of SEQ ID NO: 38.**

Ac-RGNCAYHKGQIIWCTYHF-NH2 (SEQ ID NO: 79, 6.7 mg, where the two cysteines at positions 4 and 14 each forms a disulfide bond in the molecule, and F is azidated) synthesized according to the method described in the previous report (WO2019/240287A1) and the compound (PL-1, 10mg) synthesized in (17-1) were dissolved in water (0.3 mL), to which copper(II) sulfate pentahydrate (3.0 µmol), L(+)-ascorbic acid sodium salt (7.4 µmol), and Tris(3-hydroxypropyltriazolylmethyl)amine (3.0 µmol) were added, and the mixture was stirred at room temperature for 20 minutes. After completion of the reaction, reversed phase preparative chromatography was performed for elution. The fraction containing the product was collected, and the acetonitrile was removed by concentration under reduced pressure, followed by lyophilization to obtain the modifying reagent (PL-3) (1.5 mg).

### (17-4) Synthesis of Compound (PL-4)

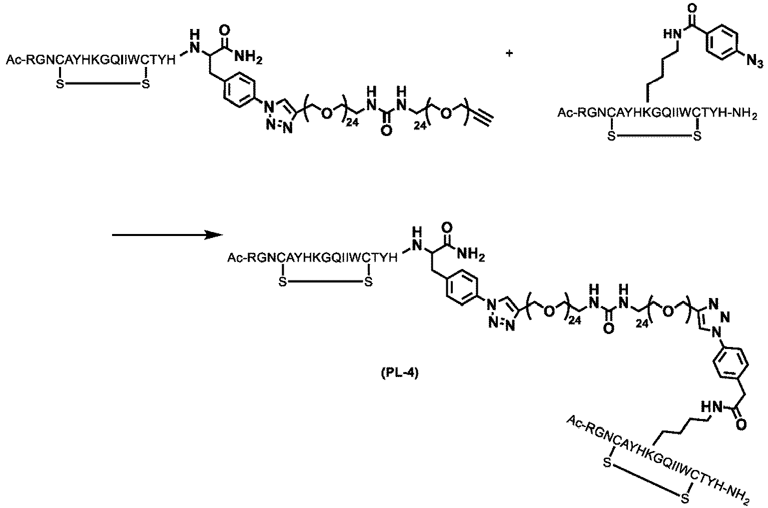

**The aforementioned amino acid sequence is the amino acid sequence of SEQ ID NO: 38.**

PL-3 (1.5 mg) synthesized in (17-3) and PL-2 (1.1 mg) synthesized in (17-2) were dissolved in water (1.0 mL), to which copper(II) sulfate pentahydrate (0.33 µmol), L(+)-ascorbic acid sodium salt (0.83 µmol), and Tris(3-hydroxypropyltriazolylmethyl)amine (0.33 µmol) were added, and the mixture was stirred at room temperature for 20 minutes. After completion of the reaction, reversed phase preparative chromatography was performed for elution. The fraction containing the product was collected, and the acetonitrile was removed by concentration under reduced pressure, followed by lyophilization to obtain the modifying reagent (PL-4)

### (2.0 mg).

### (17-5) Synthesis of Affinity Substance (PL-5)

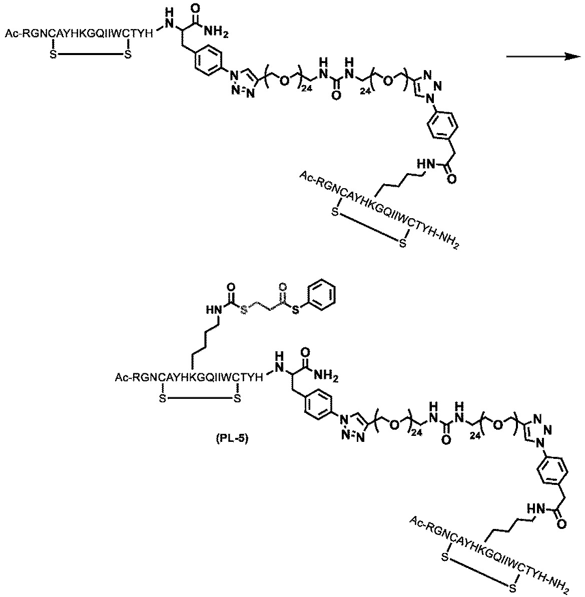

**The aforementioned amino acid sequence is the amino acid sequence of SEQ ID NO: 38.**

Following the previous report (WO2019/0240287), PL-4 prepared in (17-4) was amidated to prepare an affinity reagent (PL-5).
MS (ESI) m/z: z = 17 1174[M+6H]6+

### Example 18 Modification of Trastuzumab Using Affinity Reagent (PL-5)

### (18-1) Modification of Trastuzumab Using Affinity Reagent (PL-5)

According to Example (4-1), to a solution of the anti-human HER2 monoclonal antibody Trastuzumab (Chugai Pharmaceutical Co., Ltd.) in an acetate buffer (50 mM Sodium acetate, pH6.5), a solution of the affinity reagent (PL-5) synthesized in Example (17-5) in DMF (5 equivalents relative to the antibody) was added, and then shaken for 1 hour. The reaction solution was purified using AKTA Nap25 (Cytiva) to obtain Trastuzumab with one affinity reagent molecule bound. ESI-TOFMS analysis was performed according to the previous report *(*Anal. Chem., 2019, 91, 20, 12724-12732) to confirm that Trastuzumab had one affinity reagent molecule bound.

### (18-2) Confirmation of the Peptide/Antibody Bonding Ratio of the Specific Modified Form of Trastuzumab by DAR Calculator

For MS data analyzed in Example (18-1), the peptide/antibody bonding ratio was confirmed by DAR calculator (Agilent software). The results are shown in Table 7.

**Table 7.**

| DAR peak | Observed mass (Da) | Peak area | %area |
|---|---|---|---|
| 1 | 155271 | 3.52E+05 | 66.8 |
| 2 | 162314 | 1.75E+05 | 33.2 |

The results show that when the affinity reagent (PL-5) was reacted, Trastuzumab with one affinity reagent molecule bound with one affinity reagent molecule bound was obtained with approximately 67% selectivity.

### (18-3) Synthesis of Trastzumab with One Thiol Group Molecule

The antibody with one affinity reagent molecule bound synthesized in Example (18-1) was used according to Example (4-3) to obtain a thiol group-introduced antibody. HIC-HPLC analysis was performed according to the previous report (Anal. Chem., 2019, 91, 20, 12724-12732*)* to confirm that one thiol group molecule was introduced.

## Claims

1. A compound or a salt thereof, comprising (A) an affinity substance comprising first and second affinity moieties each having an affinity to the constant region in a heavy chain of an antibody; and (B) a reactive group for the antibody.

2. The compound or a salt thereof according to claim 1, wherein the constant region is a Fc region.

3. The compound or a salt thereof according to claim 1, wherein the constant region is a CH2 domain.

4. The compound or a salt thereof according to claim 1, wherein the constant region is a human constant region.

5. The compound or a salt thereof according to claim 1, wherein the antibody is IgG.

6. The compound or a salt thereof according to claim 1, wherein the first and second affinity moieties are different affinity moieties.

7. The compound or a salt thereof according to claim 1, wherein the affinity substance is represented by the following formula (A):
AP1-L_{A}-AP2 (A)
wherein
AP1 indicates a first affinity peptide having an affinity to the constant region in a heavy chain of the antibody;
AP2 indicates a second affinity peptide having an affinity to the constant region in a heavy chain of the antibody; and
L_{A} indicates a linker.

8. The compound or a salt thereof according to claim 7, wherein the affinity substance (i) (i-1) comprises only a single specific reactive group, and (i-2) is linked via said specific reactive group, to the reactive group for the antibody.

9. The compound or a salt thereof according to claim 1, wherein the affinity substance is an affinity polypeptide comprising first and second affinity peptides each having an affinity to the constant region in a heavy chain of the antibody.

10. The compound or a salt thereof according to claim 9, wherein the affinity polypeptide is represented by the following formula (A'):
API-PL_{A}-AP2 (A')
wherein
AP1 indicates a first affinity peptide that has an affinity to the constant region in a heavy chain of the antibody and is present on the N-terminal side of the affinity polypeptide;
AP2 indicates a second affinity peptide that has an affinity to the constant region in a heavy chain of the antibody and is present on the C-terminal side of the affinity polypeptide; and
PL_{A} represents a peptide linker.

11. The compound or a salt thereof according to claim 10, wherein the affinity polypeptide (i) (i-1) comprises only a single amino acid residue having an amino group in its side chain, and (ii-2) is linked to the reactive group for the antibody via said amino group, or (ii) is linked to the reactive group for the antibody via the N-terminal amino group in the first affinity peptide.

12. The compound or a salt thereof according to claim 11, wherein the amino acid residue having an amino group in its side chain is a lysine residue.

13. The compound or a salt thereof according to claim 10, wherein the affinity polypeptide further comprises a tripeptide composed of Gln-Glu-Thr (QET) at the N-terminus.

14. The compound or a salt thereof according to claim 10, wherein the peptide linker has a length of 20 or more amino acid residues.

15. The compound or a salt thereof according to claim 1, wherein
one of the first and second affinity peptides is an affinity peptide having an affinity to the constant region in a heavy chain of the antibody and having a single lysine residue; and
the other of the first and second affinity peptides is an affinity peptide having an affinity to the constant region in a heavy chain of the antibody and having no lysine residue.

16. The compound or a salt thereof according to claim 15,
wherein the affinity peptide having an affinity to the constant region in a heavy chain of the antibody and having a single lysine residue is any one of the following (1) to (4):
(1) an affinity peptide comprising the amino acid sequence (Fc3K) of RGNCAYHKGQIIWCTYH (SEQ ID NO: 38);
(2) an affinity peptide comprising an amino acid sequence in which one or two amino acid residues other than lysine or cysteine residue in the amino acid sequence of RGNCAYHKGQIIWCTYH (SEQ ID NO: 38) are replaced by other amino acid residues other than lysine or cysteine residue, and having an affinity to the constant region in a heavy chain of the antibody;
(3) an affinity peptide comprising the amino acid sequence (Z34CK) of FNKQCQRRFYEALHDPNLNEEQRNARIRSIREEC (SEQ ID NO: 39); and
(4) an affinity peptide comprising an amino acid sequence in which one or two amino acid residues other than lysine or cysteine residue in the amino acid sequence of FNKQCQRRFYEALHDPNLNEEQRNARIRSIREEC (SEQ ID NO: 39) are replaced by other amino acid residues other than lysine or cysteine residue, and having an affinity to the constant region in a heavy chain of the antibody;
wherein the two cysteine residues comprised in said amino acid sequences may be cross-linked by a disulfide bond, and/or
wherein the affinity peptide having an affinity to the constant region in a heavy chain of the antibody and having no lysine residue is any one of the following (5) to (10):
(5) an affinity peptide comprising the amino acid sequence (Z34CM) of FNMQCQRRFYEALHDPNLNEEQRNARIRSIREEC (SEQ ID NO: 40);
(6) an affinity peptide comprising an amino acid sequence in which one or two amino acid residues other than cysteine residue in the amino acid sequence of FNMQCQRRFYEALHDPNLNEEQRNARIRSIREEC (SEQ ID NO: 40) are replaced by other amino acid residues other than lysine or cysteine residue, and having an affinity to the constant region in a heavy chain of the antibody;
(7) an affinity peptide comprising the amino acid sequence (ProAR) of FNREQQNAFYEILHLPNLNEEQRNGFIQSLRDDPSQSANLLAEA (SEQ ID NO: 41);
(8) an affinity peptide comprising an amino acid sequence in which one or two amino acid residues other than cysteine residue in the amino acid sequence of FNREQQNAFYEILHLPNLNEEQRNGFIQSLRDDPSQSANLLAEA (SEQ ID NO: 41) are replaced by other amino acid residues other than lysine or cysteine residue, and having an affinity to the constant region in a heavy chain of the antibody;
(9) an affinity peptide comprising the amino acid sequence of RGNCAYHRGQIIWCTYH (SEQ ID NO: 78); and
(10) an affinity peptide comprising an amino acid sequence in which one or two amino acid residues other than cysteine residue in the amino acid sequence of RGNCAYHRGQIIWCTYH (SEQ ID NO: 78) are replaced by other amino acid residues other than lysine or cysteine residue, and having an affinity to the constant region in a heavy chain of the antibody;
wherein the two cysteine residues comprised in said amino acid sequences may be cross-linked by a disulfide bond.

17. The compound or a salt thereof according to claim 1,
wherein the compound is represented by the following formula (I):
R-L-A (I)
wherein
R indicates said reactive group;
L indicates a linker; and
A indicates said affinity substance.

18. The compound or a salt thereof according to claim 1, wherein the compound or a salt thereof further comprises (iii) a cleavable moiety between (i) said affinity substance and (ii) said reactive group.

19. The compound or a salt thereof according to claim 18, wherein the cleavable moiety is a cleavable moiety that can be cleaved to generate a bioorthogonal functional group on said reactive group side.

20. The compound or a salt thereof according to claim 19,
wherein the compound is represented by the following formula (Ia):
R-L₁-CLE(B)-L₂-A (Ia)
wherein
R indicates said reactive group;
L₁ indicates a first linker;
L₂ indicates a second linker;
CLE(B) represents a cleavable moiety that can be cleaved to generate a bioorthogonal functional group on said reactive group side; and
A indicates said affinity substance.

21. The compound or a salt thereof according to claim 19,
wherein the compound is represented by the following formula (Ia-1): 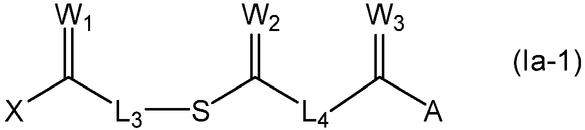 wherein
X indicates a leaving group;
W₁, W₂ and W₃ each independently represent an oxygen atom or a sulfur atom;
L₃ indicates a third linker;
L₄ represents a fourth linker;
S represents a sulfur atom; and
A indicates said affinity substance.

22. The compound or a salt thereof according to claim 21, wherein the leaving group is selected from the following:
(a) R_{A}-S, wherein R_{A} indicates a hydrogen atom, a monovalent hydrocarbon group which may have a substituent, or a monovalent heterocyclic group which may have a substituent, and S indicates a sulfur atom;
(b) R_{A}-O, wherein R_{A} indicates a hydrogen atom, a monovalent hydrocarbon group which may have a substituent, or a monovalent heterocyclic group which may have a substituent, and O indicates an oxygen atom;
(c) R_{A}-(R_{B}-)N, wherein R_{A} and R_{B} each independently indicate a hydrogen atom, a monovalent hydrocarbon group which may have a substituent, or a monovalent heterocyclic group which may have a substituent, and N indicates a nitrogen atom; or
(d) a halogen atom.

23. The compound or a salt thereof according to claim 1, wherein the compound or a salt thereof further comprises (iv) a bioorthogonal functional group between (ii) said reactive group and (iii) the cleavable moiety.

24. The compound or a salt thereof according to claim 23,
wherein the compound is represented by the following formula (Ib): 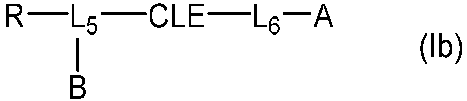 wherein
R indicates said reactive group;
L₅ indicates a fifth linker;
L₆ indicates a sixth linker;
B indicates a group comprising a bioorthogonal functional group;
CLE indicates a cleavable moiety; and
A indicates said affinity substance.

25. The compound or a salt thereof according to claim 23,
wherein the compound is represented by the following formula (Ib-1): 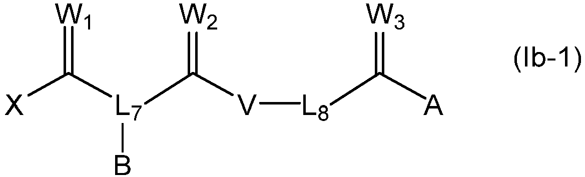 wherein
X indicates a leaving group;
W₁, W₂ and W₃ each independently indicate an oxygen atom or a sulfur atom;
L₇ indicates a seventh linker;
L₈ indicates an eighth linker;
B indicates a group comprising a bioorthogonal functional group;
V indicates an oxygen atom or a sulfur atom; and
A indicates said affinity substance.

26. The compound or a salt thereof according to claim 23, wherein the bioorthogonal functional group is an azide residue, an alkyne residue, a tetrazine residue, an alkene residue, a thiol residue, a maleimide residue, a thiol residue, a furan residue, or a halocarbonyl residue.

27. A reagent for derivatizing an antibody, comprising the compound or a salt thereof according to any one of claims 1 to 26.

28. An affinity substance-modified antibody or a salt thereof, comprising an affinity substance comprising first and second affinity moieties each having an affinity to the constant region in a heavy chain of the antibody in the constant region in a heavy chain of the antibody.

29. The affinity substance-modified antibody or a salt thereof according to claim 28, comprising: (a) an immunoglobulin unit comprising two heavy chains and optionally two light chains; and (b) said affinity substance, (c) wherein said affinity substance is introduced to the constant region in only one heavy chain of said immunoglobulin unit.

30. The affinity substance-modified antibody or a salt thereof according to claim 29, wherein said affinity substance is introduced to said constant region in only one heavy chain via modification of the amino group in the side chain of a lysine residue present at one or more positions in said constant region in one heavy chain.

31. The affinity substance-modified antibody or a salt thereof according to claim 30, wherein said one or more positions in the constant region in one heavy chain are positions 246/248, 288/290, or 317 in the human IgG heavy chain according to the EU numbering.

32. The affinity substance-modified antibody or a salt thereof according to claim 28,
wherein the affinity substance-modified antibody comprises a structural unit represented by the following formula (II): 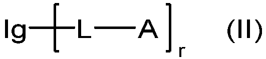 wherein
Ig indicates an immunoglobulin unit comprising two heavy chains and optionally two light chains;
L indicates a linker;
A indicates said affinity substance; and
the average percent modification r of said immunoglobulin unit with said affinity substance is 65 to 135%.

33. The affinity substance-modified antibody or a salt thereof according to claim 28, wherein said antibody or a salt thereof further comprises (iii') a cleavable moiety between (i') said affinity substance (ii') said antibody.

34. The affinity substance-modified antibody or a salt thereof according to claim 33, wherein the cleavable moiety is a cleavable moiety that can be cleaved to generate a bioorthogonal functional group on said immunoglobulin unit.

35. The affinity substance-modified antibody or a salt thereof according to claim 34,
wherein the affinity substance-modified antibody comprises a structural unit represented by the following formula (IIa): 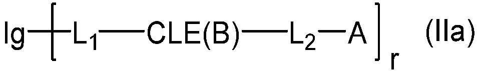 wherein
Ig indicates said immunoglobulin unit;
L₁ indicates a first linker;
L₂ indicates a second linker;
CLE(B) indicates a cleavable moiety that can be cleaved to generate a bioorthogonal functional group on said immunoglobulin unit side;
A indicates said affinity substance; and
the average percent modification r of said immunoglobulin unit with said affinity substance is 65 to 135%.

36. The affinity substance-modified antibody or a salt thereof according to claim 34,
wherein the affinity substance-modified antibody comprises a structural unit represented by the following formula (IIa-1): 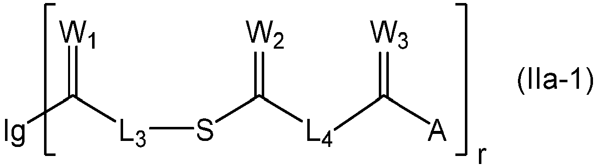 wherein
Ig indicates said immunoglobulin unit;
W₁, W₂ and W₃ each independently indicate an oxygen atom or a sulfur atom;
L₃ indicates a third linker;
L₄ represents a fourth linker;
S represents a sulfur atom;
A indicates said affinity substance; and
the average percent modification r of said immunoglobulin unit with said affinity substance is 65 to 135%.

37. The affinity substance-modified antibody or a salt thereof according to claim 33, wherein said antibody or a salt thereof further comprises (iv') a bioorthogonal functional group between (ii') said antibody and (iii') the cleavable moiety.

38. The affinity substance-modified antibody or a salt thereof according to claim 37,
wherein the affinity substance-modified antibody comprises a structural unit represented by the following formula (IIb): 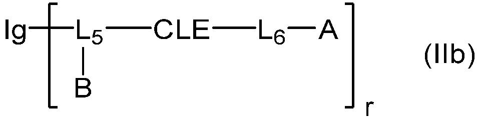 wherein
Ig indicates said immunoglobulin unit;
L₅ indicates a fifth linker;
L₆ indicates a sixth linker;
B indicates a group comprising a bioorthogonal functional group;
CLE indicates a cleavable moiety;
A indicates said affinity substance; and
the average percent modification r of said immunoglobulin unit with said affinity substance is 65 to 135%.

39. The affinity substance-modified antibody or a salt thereof according to claim 37,
wherein the affinity substance-modified antibody comprises a structural unit represented by the following formula (IIb-1): 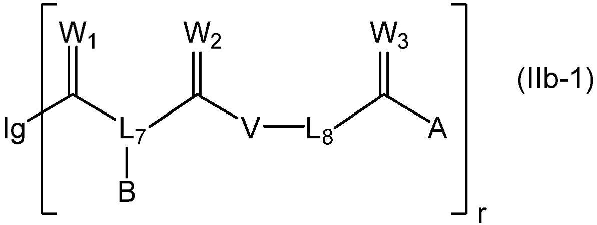 wherein
Ig indicates said immunoglobulin unit;
W₁, W₂ and W₃ each independently indicate an oxygen atom or a sulfur atom;
L₇ indicates a seventh linker;
L₈ indicates an eighth linker;
B indicates a group comprising a bioorthogonal functional group;
V indicates an oxygen atom or a sulfur atom;
A indicates said affinity substance; and
the average percent modification r of said immunoglobulin unit with said affinity substance is 65 to 135%.

40. The affinity substance-modified antibody or a salt thereof according to claim 28, wherein the affinity substance-modified antibody further comprises an additional modified moiety.

41. The affinity substance-modified antibody or a salt thereof according to claim 40, wherein the additional modified moiety is an additional affinity substance comprising a third affinity moiety having an affinity to the constant region in a heavy chain of the antibody, and said additional affinity substance is comprised in the constant region in a heavy chain of the antibody.

42. The affinity substance-modified antibody or a salt thereof according to claim 41, wherein said additional affinity substance is introduced to the constant regions in said two heavy chains via modification of the amino group in the side chain of a lysine residue present at one or more positions in the constant regions in said two heavy chains.

43. The affinity substance-modified antibody or a salt thereof according to claim 42, wherein said one or more positions in the constant regions in the two heavy chains are positions 246/248, 288/290, or 317 in the human IgG heavy chain according to the EU numbering.

44. A method of producing an affinity substance-modified antibody or a salt thereof, the method comprising
reacting a compound or a salt thereof with an antibody, wherein
the compound or a salt thereof comprises (A) an affinity substance comprising first and second affinity moieties each having an affinity to the constant region in a heavy chain of the antibody, and (B) a reactive group for the antibody; and
the antibody comprises an immunoglobulin unit comprising two heavy chains and optionally two light chains,
to produce an affinity substance-modified antibody or a salt thereof comprising said affinity substance in the constant region in a heavy chain of said immunoglobulin unit.

45. The method according to claim 44, wherein the compound or a salt thereof further comprises (iii) a cleavable moiety between (i) said affinity substance and (ii) said reactive group.

46. The method according to claim 45, wherein the cleavable moiety is a cleavable moiety that can be cleaved to generate a bioorthogonal functional group on said reactive group side.

47. The method according to claim 44, wherein the compound or a salt thereof further comprises (iv) a bioorthogonal functional group between (ii) said reactive group and (iii) the cleavable moiety.

48. The method according to claim 44, wherein the affinity substance-modified antibody or a salt thereof is the affinity substance-modified antibody or a salt thereof according to any one of claims 29 to 43.

49. An antibody derivative or a salt thereof comprising a bioorthogonal functional group, comprising: (a) an immunoglobulin unit comprising two heavy chains and optionally two light chains; and (b) the bioorthogonal functional group, (c) wherein the bioorthogonal functional group is introduced to the constant region in only one heavy chain of said immunoglobulin unit.

50. The antibody derivative or a salt thereof according to claim 49, wherein the bioorthogonal functional group is introduced to said constant region in only one heavy chain via modification of the amino group in the side chain of a lysine residue present at one or more positions in said constant region in one heavy chain.

51. The antibody derivative or a salt thereof according to claim 50, wherein said one or more positions in the constant region in one heavy chain are positions 246/248, 288/290, or 317 in the human IgG heavy chain according to the EU numbering.

52. The antibody derivative or a salt thereof according to claim 49,
wherein the antibody derivative or a salt thereof comprising a bioorthogonal functional group comprises a structural unit represented by the following formula (IIIa): 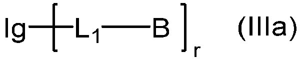 wherein
Ig indicates said immunoglobulin unit;
L₁ indicates a first linker;
B indicates a group comprising a bioorthogonal functional group; and
the average percent modification r of said immunoglobulin unit with the bioorthogonal functional group is 65 to 135%.

53. The antibody derivative or a salt thereof according to claim 49,
wherein the antibody derivative or a salt thereof comprising a bioorthogonal functional group comprises a structural unit represented by the following formula (IIIa-1): 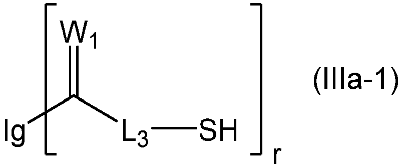 wherein
Ig indicates said immunoglobulin unit;
W₁ indicates an oxygen atom or a sulfur atom;
L₃ indicates a third linker;
SH indicates a thiol group; and
the average percent modification r of said immunoglobulin unit with the bioorthogonal functional group is 65 to 135%.

54. The antibody derivative or a salt thereof according to claim 49,
wherein the antibody derivative or a salt thereof comprising a bioorthogonal functional group comprises a structural unit represented by the following formula (IIIb): 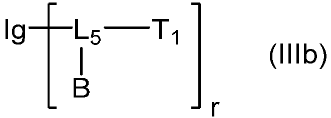 wherein
Ig indicates said immunoglobulin unit;
L₅ indicates a fifth linker;
B indicates a group comprising a bioorthogonal functional group;
T₁ indicates a monovalent group; and
the average percent modification r of said immunoglobulin unit with the bioorthogonal functional group is 65 to 135%.

55. The antibody derivative or a salt thereof according to claim 49,
wherein the antibody derivative or a salt thereof comprising a bioorthogonal functional group comprises a structural unit represented by the following formula (IIIb-1): 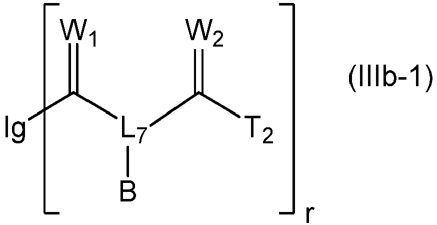 wherein
Ig indicates said immunoglobulin unit;
W₁ and W₂ each independently indicate an oxygen atom or a sulfur atom;
L₇ indicates a seventh linker;
B indicates a group comprising a bioorthogonal functional group;
T₂ indicates a monovalent group; and
the percent modification r of said immunoglobulin unit with the bioorthogonal functional group is 65 to 135%.

56. The antibody derivative or a salt thereof according to claim 49, wherein the antibody derivative further comprises an additional modified moiety.

57. The antibody derivative or a salt thereof according to claim 56, wherein the additional modified moiety is an additional modified moiety comprising a bioorthogonal functional group, and said additional affinity substance comprising a bioorthogonal functional group is comprised in the constant region in a heavy chain of the antibody.

58. The antibody derivative or a salt thereof according to claim 57, wherein said additional modified moiety comprising a bioorthogonal functional group is introduced to the constant regions in said two heavy chains via modification of the amino group in the side chain of a lysine residue present at one or more positions in the constant regions in said two heavy chains.

59. The antibody derivative or a salt thereof according to claim 58, wherein said one or more positions in the constant regions in the two heavy chains are positions 246/248, 288/290, or 317 in the human IgG heavy chain according to the EU numbering.

60. A conjugate of an antibody and a functional substance, or a salt thereof, comprising: (a) an immunoglobulin unit comprising two heavy chains and optionally two light chains; and (b) a functional substance, (c) wherein the functional substance is introduced to the constant region in only one heavy chain of said immunoglobulin unit.

61. The conjugate or a salt thereof according to claim 60, wherein the functional substance is introduced to said constant region in only one heavy chain via modification of the amino group in the side chain of a lysine residue present at one or more positions in said constant region in one heavy chain.

62. The conjugate or a salt thereof according to claim 61, wherein said one or more positions in the constant region in one heavy chain are positions 246/248, 288/290, or 317 in the human IgG heavy chain according to the EU numbering.

63. The conjugate or a salt thereof according to claim 60,
wherein the conjugate or a salt thereof comprises a structural unit represented by the following formula (IVa): 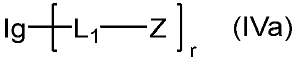 wherein
Ig indicates said immunoglobulin unit;
L₁ indicates a first linker;
Z indicates a functional substance; and
the average percent modification r of said immunoglobulin unit with the functional substance is 65 to 135%.

64. The conjugate or a salt thereof according to claim 60,
wherein the conjugate or a salt thereof comprises a structural unit represented by the following formula (IVa-1): 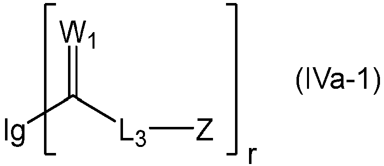 wherein
Ig indicates said immunoglobulin unit;
W₁ indicates an oxygen atom or a sulfur atom;
L₃ indicates a third linker;
Z indicates a functional substance; and
the average percent modification r of said immunoglobulin unit with the functional substance is 65 to 135%.

65. The conjugate or a salt thereof according to claim 60,
wherein the conjugate or a salt thereof comprises a structural unit represented by the following formula (IVb): 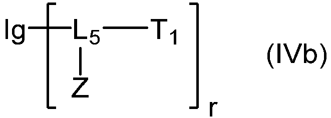 wherein
Ig indicates said immunoglobulin unit;
L₅ indicates a fifth linker;
Z indicates a functional substance;
T₁ indicates a monovalent group; and
the average percent modification r of said immunoglobulin unit with the functional substance is 65 to 135%.

66. The conjugate or a salt thereof according to claim 60,
wherein the conjugate or a salt thereof comprises a structural unit represented by the following formula (IVb-1): 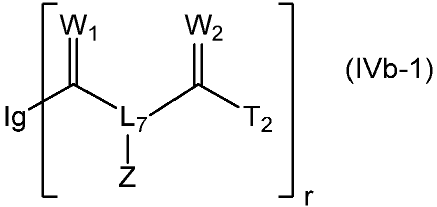 wherein
Ig indicates said immunoglobulin unit;
W₁ and W₂ each independently indicate an oxygen atom or a sulfur atom;
L₇ indicates a seventh linker;
Z indicates a functional substance;
T₂ indicates a monovalent group; and
the percent modification r of said immunoglobulin unit with the functional substance is 65 to 135%.

67. The conjugate or a salt thereof according to claim 60, wherein the functional substance is a drug, a labelling substance, an affinity substance, a transporting substance, or a stabilizer.

68. The conjugate or a salt thereof according to claim 67, wherein the affinity substance is a full-length antibody or a fragment thereof.

69. The conjugate or a salt thereof according to claim 60, wherein the conjugate further comprises an additional modified moiety.

70. The conjugate or a salt thereof according to claim 69, wherein the additional modified moiety is an additional modified moiety comprising a functional substance, and said additional modified moiety comprising a functional substance is comprised in a constant region in a heavy chain of the antibody.

71. The conjugate or a salt thereof according to claim 70, wherein the additional modified moiety comprising a functional substance is introduced into said constant regions in two heavy chains via modification of the amino group in the side chain of a lysine residue present at one or more positions in said constant regions in two heavy chains.

72. The conjugate or a salt thereof according to claim 71, wherein said one or more positions in the constant regions in two heavy chains are positions 246/248, 288/290, or 317 in the human IgG heavy chain according to the EU numbering.

73. A method of producing an antibody or a salt thereof without affinity substance, the method comprising cleaving an affinity substance-modified antibody or a salt thereof comprising (A) an affinity substance comprising first and second affinity moieties each having an affinity to the constant region in a heavy chain of an antibody, and (B) an antibody, and further comprising (C) a cleavable moiety between (A) the affinity substance and (B) the antibody at the cleavable moiety to produce an antibody or a salt thereof without affinity substance.

74. The method according to claim 73, wherein
the cleavable moiety is a cleavable moiety that can be cleaved to generate a bioorthogonal functional group on said antibody side; and
the antibody or a salt thereof without affinity substance is an antibody derivative or a salt thereof comprising a bioorthogonal functional group.

75. The method according to claim 74, wherein the antibody derivative or a salt thereof comprising a bioorthogonal functional group is an antibody derivative or a salt thereof according to any one of claims 49 to 53, or 56 to 59.

76. The method according to claim 73, wherein
the antibody or a salt thereof without affinity substance further comprises a bioorthogonal functional group between said antibody and said cleavable moiety;
the antibody or a salt thereof without affinity substance is an antibody derivative or a salt thereof comprising a bioorthogonal functional group.

77. The method according to claim 76, wherein the antibody derivative or a salt thereof comprising a bioorthogonal functional group is an antibody derivative or a salt thereof according to any one of claims 49 to 51, or 54 to 59.

78. A method of producing a conjugate or a salt thereof comprising an antibody and a functional substance, the method comprising (1) and (2) below:
(1) producing an antibody derivative or a salt thereof comprising a bioorthogonal functional group according to the method according to claim 74; and
(2) reacting the antibody derivative or a salt thereof comprising a bioorthogonal functional group with a functional substance to produce a conjugate or a salt thereof comprising the antibody and the functional substance.

79. The method according to claim 78, wherein the conjugate or a salt thereof is the conjugate or a salt thereof according to any one of claims 60 to 64.

80. A method of producing a conjugate or a salt thereof comprising an antibody and a functional substance, the method comprising (1) and (2) below:
(1) producing an antibody derivative or a salt thereof comprising a bioorthogonal functional group according to the method according to claim 76; and
(2) reacting the antibody derivative or a salt thereof comprising a bioorthogonal functional group with a functional substance to produce a conjugate or a salt thereof comprising the antibody and the functional substance.

81. The method according to claim 78, wherein the conjugate or a salt thereof is the conjugate or a salt thereof according to any one of claims 60 to 62, 65 and 66.

82. A method of producing a conjugate or a salt thereof comprising an antibody and a functional substance,
the method comprising reacting an antibody derivative or a salt thereof comprising a bioorthogonal functional group with a functional substance to produce a conjugate or a salt thereof comprising the antibody and the functional substance;
the antibody derivative or a salt thereof comprising a bioorthogonal functional group is an antibody derivative or a salt thereof comprising a bioorthogonal functional group, comprising: (a) an immunoglobulin unit comprising two heavy chains and optionally two light chains; and (b) the bioorthogonal functional group, (c) wherein the bioorthogonal functional group is introduced to the constant region in only one heavy chain of said immunoglobulin unit; and
the conjugate or a salt thereof comprising an antibody and a functional substance is a conjugate of an antibody and a functional substance, or a salt thereof, comprising: (a) an immunoglobulin unit comprising two heavy chains and optionally two light chains; and (b) a functional substance, (c) wherein the functional substance is introduced to the constant region in only one heavy chain of said immunoglobulin unit.

83. The method according to claim 80, wherein the antibody derivative or a salt thereof comprising a bioorthogonal functional group is an antibody derivative or a salt thereof according to any one of claims 49 to 59.

84. The method according to claim 80, wherein the conjugate or a salt thereof is the conjugate or a salt thereof according to any one of claims 60 to 72.

85. An affinity substance or a salt thereof, comprising first and second affinity moieties each having an affinity to the constant region in a heavy chain of an antibody.

86. The affinity substance or a salt thereof according to claim 85, wherein
the affinity substance is represented by the following formula (A):
AP1-L_{A}-AP2 (A)
wherein
AP1 indicates a first affinity peptide having an affinity to the constant region in a heavy chain of the antibody;
AP2 indicates a second affinity peptide having an affinity to the constant region in a heavy chain of the antibody; and
L_{A} indicates a linker.

87. The affinity substance or a salt thereof according to claim 85, wherein the affinity substance comprises only one specific reactive group.

88. The affinity substance or a salt thereof according to claim 85, wherein the affinity substance is an affinity polypeptide comprising first and second affinity peptides each having an affinity to a constant region in a heavy chain of the antibody.

89. The affinity substance or a salt thereof according to claim 88, wherein
the affinity polypeptide is represented by the following formula (A'):
AP1-PL_{A}-AP2 (A')
wherein
AP1 indicates a first affinity peptide that has an affinity to the constant region in a heavy chain of the antibody and is present on the N-terminal side of the affinity polypeptide;
AP2 indicates a second affinity peptide that has an affinity to the constant region in a heavy chain of the antibody and is present on the C-terminal side of the affinity polypeptide; and
PL_{A} indicates a peptide linker.

90. The affinity substance or a salt thereof according to claim 88, wherein the affinity polypeptide comprises (i) only one amino acid residue having an amino group in its side chain, or (ii) an unprotected N-terminal amino group.

91. The affinity substance or a salt thereof according to claim 90, wherein the amino acid residue having an amino group in its side chain is a lysine residue.

92. The affinity substance or a salt thereof according to claim 88, wherein the affinity polypeptide further comprises a tripeptide composed of Gln-Glu-Thr (QET) at the N-terminus.

93. The affinity substance or a salt thereof according to claim 89, wherein the peptide linker has a length of 20 or more amino acid residues.

94. The affinity substance or a salt thereof according to claim 85, wherein
one of the first and second affinity peptides is an affinity peptide having an affinity to the constant region in a heavy chain of the antibody and having a single lysine residue; and
the other of the first and second affinity peptides is an affinity peptide having an affinity to a constant region in a heavy chain of the antibody and having no lysine residue.

95. A polynucleotide encoding an affinity polypeptide comprising first and second affinity peptides each having an affinity to the constant region in a heavy chain of an antibody.

96. An expression vector comprising the polynucleotide according to claim 95 and a promoter operably linked thereto.

97. A host cell comprising an expression unit comprising the polynucleotide according to claim 95 and a promoter operably linked thereto.

98. An affinity substance-modified antibody or a salt thereof comprising first and second modified moieties wherein
the first modified moiety comprises a first affinity substance comprising first and second affinity moieties each having an affinity to the constant region in a heavy chain of an antibody;
the second modified moiety comprises a second affinity substance comprising third and fourth affinity moieties each having an affinity to the constant region in a heavy chain of the antibody; and
the first and second modified moieties are contained in the constant region in a heavy chain of the antibody.

99. The affinity substance-modified antibody or a salt thereof according to claim 98,
comprising (a) an immunoglobulin unit comprising two heavy chains composed of first and second heavy chains and optionally two light chains, and (b) said first and second modified moieties;
wherein (c) said first modified moiety has been introduced to the constant region in the first heavy chain of said immunoglobulin unit; and
(d) said second modified moiety has been introduced to the constant region in the second heavy chain of said immunoglobulin unit.

100. The affinity substance-modified antibody or a salt thereof according to claim 99, wherein said first modified moiety has been introduced to the constant region in said first heavy chain via modification of the amino group in the side chain of a lysine residue present at one or more positions in the constant region in said first heavy chain; and said second modified moiety has been introduced to the constant region in said second heavy chain via modification of the amino group in the side chain of a lysine residue present at one or more positions in the constant regions in said second heavy chain.

101. The affinity substance-modified antibody or a salt thereof according to claim 100, wherein said one or more positions in the constant region in the first heavy chain and said one or more positions in the constant region in the second heavy chain are positions selected from the group consisting of positions 246/248, 288/290, and 317 in the human IgG heavy chain according to the EU numbering, and combinations thereof.

102. The affinity substance-modified antibody or a salt thereof according to claim 98, wherein
the affinity substance-modified antibody comprising the first and second modified moieties comprises a structural unit represented by the following formula (V): 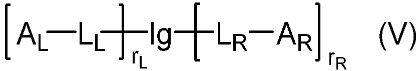 wherein
Ig indicates an immunoglobulin unit comprising two heavy chains composed of first and second heavy chains and optionally two light chains;
L_{L} and L_{R} each independently indicate a linker;
A_{L} indicates said first affinity substance;
A_{R} indicates said second affinity substance; and
the average percent modification r_{L} of said immunoglobulin unit with said first modified moiety and the average percent modification r_{R} of said immunoglobulin unit with said second modified moiety are individually 65 to 135%.

103. The affinity substance-modified antibody or a salt thereof according to claim 98, wherein said antibody or a salt thereof further comprises (iii') a first cleavable moiety between (i') said first affinity substance and (ii') said immunoglobulin unit, and/or further comprises (iii") a second cleavable moiety between (i") said second affinity substance and (ii") said immunoglobulin unit.

104. The affinity substance-modified antibody or a salt thereof according to claim 103, wherein said first and second cleavable moieties are each a cleavable moiety that can be cleaved to generate a bioorthogonal functional group on said immunoglobulin unit.

105. The affinity substance-modified antibody or a salt thereof according to claim 98, wherein
the affinity substance-modified antibody comprising the first and second modified moieties comprises a structural unit represented by the following formula (Va): 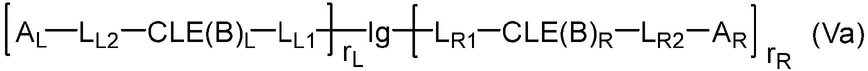 wherein
Ig indicates said immunoglobulin unit;
L_{L1} and L_{R1} each independently indicate a first linker;
L_{L2} and L_{R2} each independently indicate a second linker;
CLE(B)_{L} and CLE(B)_{R} each independently indicates a cleavable moiety that can be cleaved to generate a bioorthogonal functional group on said immunoglobulin unit side;
A_{L} indicates said first affinity substance;
A_{R} indicates said second affinity substance; and
the average percent modification r_{L} of said immunoglobulin unit with said first modified moiety and the average percent modification r_{R} of said immunoglobulin unit with said second modified moiety are individually 65 to 135%.

106. The affinity substance-modified antibody or a salt thereof according to claim 98, wherein
the affinity substance-modified antibody comprising the first and second modified moieties comprises a structural unit represented by the following formula (Va-1): 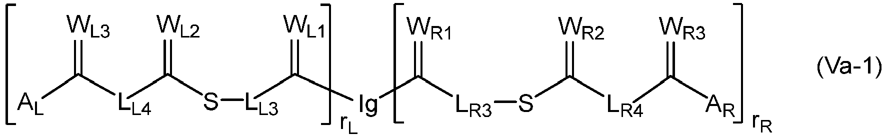 wherein
Ig indicates said immunoglobulin unit;
W_{L1}, W_{L2} and W_{L3}, and W_{R1}, W_{R2}, and W_{R3} each independently indicate an oxygen atom or a sulfur atom;
L_{L3} and L_{R3} each independently indicate a third linker;
L_{L4} and L_{R4} each independently indicate a fourth linker;
S represents a sulfur atom;
A_{L} indicates said first affinity substance;
A_{R} indicates said second affinity substance; and
the average percent modification r_{L} of said immunoglobulin unit with said first modified moiety and the average percent modification r_{R} of said immunoglobulin unit with said second modified moiety are individually 65 to 135%.

107. The affinity substance-modified antibody or a salt thereof according to claim 103, wherein said antibody or a salt thereof further comprises (iv') a first bioorthogonal functional group between (ii') said immunoglobulin unit and (iii') said first cleavable moiety, and/or further comprises (iv") a second bioorthogonal functional group between (ii") said immunoglobulin unit and (iii") said second cleavable moiety.

108. The affinity substance-modified antibody or a salt thereof according to claim 107, wherein
the affinity substance-modified antibody comprising the first and second modified moieties comprises a structural unit represented by the following formula (Vb): 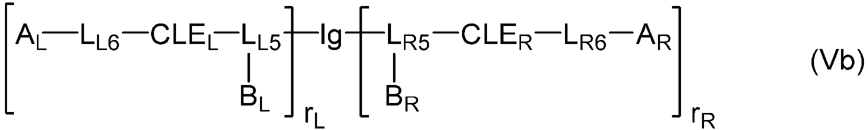 wherein
Ig indicates said immunoglobulin unit;
L_{L5} and L_{R5} each independently indicate a fifth linker;
L_{L6} and L_{R6} each independently indicate a sixth linker;
B_{L} indicates a first group comprising a first bioorthogonal functional group;
B_{R} indicates a second group comprising a second bioorthogonal functional group;
CLE_{L} and CLE_{R} each independently indicate a cleavable moiety;
A_{L} indicates said first affinity substance;
A_{R} indicates said second affinity substance; and
the average percent modification r_{L} of said immunoglobulin unit with said first modified moiety and the average percent modification r_{R} of said immunoglobulin unit with said second modified moiety are individually 65 to 135%.

109. The affinity substance-modified antibody or a salt thereof according to claim 108, wherein
the affinity substance-modified antibody comprising the first and second modified moieties comprises a structural unit represented by the following formula (Vb-1): 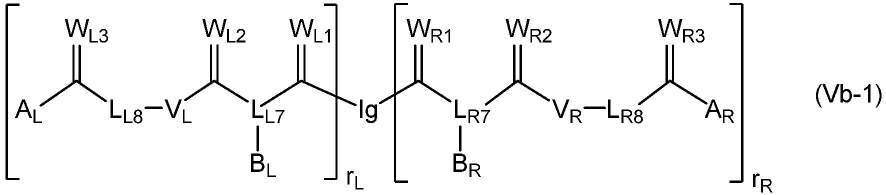 wherein
Ig indicates said immunoglobulin unit;
W_{L1}, W_{L2} and W_{L3}, and W_{R1}, W_{R2} and W_{R3} each independently indicate an oxygen atom or a sulfur atom;
L_{L7} and L_{R7} each independently indicate a seventh linker;
L_{L8} and L_{R8} each independently indicate an eighth linker;
B_{L} indicates a first group comprising a first bioorthogonal functional group;
B_{R} indicates a second group comprising a second bioorthogonal functional group;
V_{L} and V_{R} each independently indicate an oxygen atom or a sulfur atom;
A_{L} indicates said first affinity substance;
A_{R} indicates said second affinity substance; and
the average percent modification r_{L} of said immunoglobulin unit with said first modified moiety and the average percent modification r_{R} of said immunoglobulin unit with said second modified moiety are individually 65 to 135%.

110. The affinity substance-modified antibody or a salt thereof according to claim 103, wherein
said antibody or a salt thereof further comprises (iii') a first cleavable moiety between (i') said first affinity substance and (ii') said immunoglobulin unit, and further comprises (iv") a first bioorthogonal functional group between (ii") said immunoglobulin unit and (iii") the second cleavable moiety; and
said first cleavable moiety is a cleavable moiety that can be cleaved to generate a second bioorthogonal functional group on said immunoglobulin unit.

111. The affinity substance-modified antibody or a salt thereof according to claim 100, wherein
the affinity substance-modified antibody comprising the first and second modified moieties comprises a structural unit represented by the following formula (Vc): 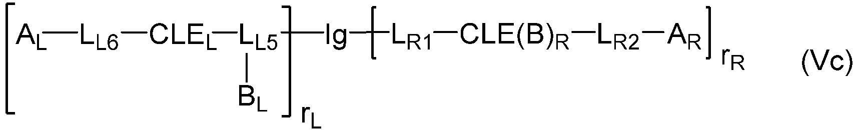 wherein
Ig indicates said immunoglobulin unit;
L_{R1} indicates a first linker;
L_{R2} indicates a second linker;
L_{L5} indicates a fifth linker;
L_{L6} indicates a sixth linker;
B₁ indicates a group comprising a first bioorthogonal functional group;
CLE_{L} indicates a first cleavable moiety;
CLE(B)_{R} indicates a second cleavable moiety that can be cleaved to generate a second bioorthogonal functional group on said immunoglobulin unit side;
A_{L} indicates said first affinity substance;
A_{R} indicates said second affinity substance; and
the average percent modification r_{L} of said immunoglobulin unit with said first modified moiety and the average percent modification r_{R} of said immunoglobulin unit with said second modified moiety are individually 65 to 135%.

112. The affinity substance-modified antibody or a salt thereof according to claim 111, wherein
the affinity substance-modified antibody comprising the first and second modified moieties comprises a structural unit represented by the following formula (V_{C}-1): 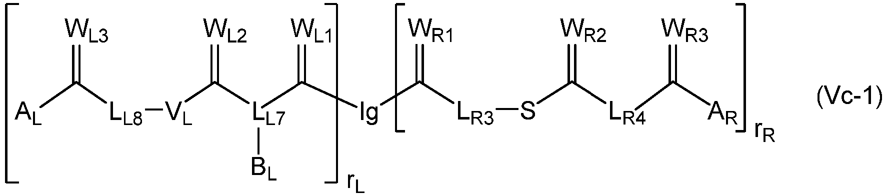 wherein
Ig indicates said immunoglobulin unit;
W_{L1}, W_{L2} and W_{L3}, and W_{R1}, W_{R2} and W_{R3} each independently indicate an oxygen atom or a sulfur atom;
L_{R3} each independently indicates a third linker;
L_{R4} each independently indicates a fourth linker;
L_{L7} each independently indicates a seventh linker;
L_{L5} each independently indicates an eighth linker;
B₁ indicates a group comprising a first bioorthogonal functional group;
V_{L} indicates an oxygen atom or a sulfur atom;
A_{L} indicates said first affinity substance;
A_{R} indicates said second affinity substance; and
the average percent modification r_{L} of said immunoglobulin unit with said first modified moiety and the average percent modification r_{R} of said immunoglobulin unit with said second modified moiety are individually 65 to 135%.

113. The affinity substance-modified antibody or a salt thereof according to claim 98, wherein the affinity substance-modified antibody further comprises an additional modified moiety.

114. The affinity substance-modified antibody or a salt thereof according to claim 113, wherein the additional modified moiety is an additional affinity substance comprising a fifth affinity moiety having an affinity to the constant region in a heavy chain of the antibody, and said additional affinity substance is comprised in the constant region in a heavy chain of the antibody.

115. The affinity substance-modified antibody or a salt thereof according to claim 114, wherein said additional affinity substance is introduced to the constant regions in said two heavy chains via modification of the amino group in the side chain of a lysine residue present at one or more positions in the constant regions in said two heavy chains.

116. The affinity substance-modified antibody or a salt thereof according to claim 115, wherein said one or more positions in the constant regions in the two heavy chains are positions 246/248, 288/290, or 317 in a human IgG heavy chain according to the EU numbering.

117. A method of producing an affinity substance-modified antibody or a salt thereof comprising first and second modified moieties, the method comprising: (1) reacting a compound or a salt thereof comprising (A) a first affinity substance comprising first and second affinity moieties each having an affinity to the constant region in a heavy chain of an antibody, and (B) a first reactive group for the antibody with the antibody comprising an immunoglobulin unit comprising two heavy chains and optionally two light chains to produce an affinity substance-modified antibody or a salt thereof comprising a first modified moiety in which said first affinity substance is contained in the constant region in a heavy chain of said immunoglobulin unit; and (2) reacting the affinity substance-modified antibody or a salt thereof comprising said first affinity substance in the constant region in a heavy chain of said immunoglobulin unit with a compound or a salt thereof comprising a second affinity substance comprising third and fourth affinity moieties each having an affinity to the constant region in a heavy chain of the antibody and (B) a second reactive group for the antibody to produce an affinity substance-modified antibody or a salt thereof comprising said first and second modified moieties in the constant region in the heavy chains of said immunoglobulin unit.

118. The method according to claim 117, wherein the affinity substance-modified antibody or a salt thereof comprising first and second modified moieties is the affinity substance-modified antibody or a salt thereof according to any one of claims 86 to 100.

119. An antibody derivative or a salt thereof comprising first and second modified moieties,
(a) comprising an immunoglobulin unit comprising two heavy chains composed of first and second heavy chains and optionally two light chains; and
(b) comprising a first modified moiety comprising a first bioorthogonal functional group and a second modified moiety comprising a second bioorthogonal functional group;
(c) wherein the first modified moiety has been introduced to the constant region in said first heavy chain;
(d) wherein the second modified moiety has been introduced to the constant region in said second heavy chain; and
(e) wherein the first and second modified moieties are different from each other.

120. The antibody derivative or a salt thereof according to claim 119, wherein said first modified moiety has been introduced to the constant region in said first heavy chain via modification of the amino group in the side chain of a lysine residue present at one or more positions in the constant region in said first heavy chain; and said second modified moiety has been introduced to the constant region in said second heavy chain via modification of the amino group in the side chain of a lysine residue present at one or more positions in the constant regions in said second heavy chain.

121. The antibody derivative or a salt thereof according to claim 120, wherein said one or more positions in the constant region in the first heavy chain and said one or more positions in the constant region in the second heavy chain are positions selected from the group consisting of positions 246/248, 288/290, and 317 in the human IgG heavy chain according to the EU numbering, and combinations thereof.

122. The antibody derivative or a salt thereof according to claim 119, wherein
the antibody derivative or a salt thereof comprising first and second modified moieties comprises a structural unit represented by the following formula (VIa): 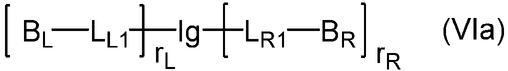 wherein
Ig indicates said immunoglobulin unit;
L_{L1} and L_{R1} each independently indicate a first linker;
B_{L} indicates a first group comprising a first bioorthogonal functional group;
B_{R} indicates a second group comprising a second bioorthogonal functional group; and
the average percent modification r_{L} of said immunoglobulin unit with the first modified moiety and the average percent modification r_{R} of said immunoglobulin unit with the second modified moiety are individually 65 to 135%.

123. The antibody derivative or a salt thereof according to claim 122, wherein
the antibody derivative or a salt thereof comprising first and second modified moieties comprises a structural unit represented by the following formula (VIa-1): 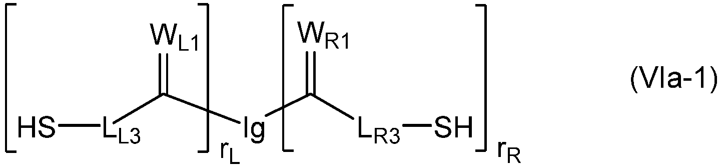 wherein
Ig indicates said immunoglobulin unit;
W_{L1} and W_{R1} each independently indicate an oxygen atom or a sulfur atom;
L_{L3} and L_{R3} each independently indicate a third linker;
SH indicates a thiol group that is a bioorthogonal functional group;
the average percent modification r_{L} of said immunoglobulin unit with the first modified moiety and the average percent modification r_{R} of said immunoglobulin unit with the second modified moiety are individually 65 to 135%.

124. The antibody derivative or a salt thereof according to claim 119, wherein
the antibody derivative or a salt thereof comprising first and second modified moieties comprises a structural unit represented by the following formula (VIb): 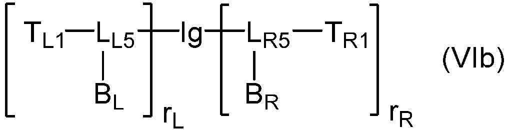 wherein
Ig indicates said immunoglobulin unit;
L_{L5} and L_{R5} each independently indicate a fifth linker;
B_{L} indicates a first group comprising a first bioorthogonal functional group;
B_{R} indicates a second group comprising a second bioorthogonal functional group;
T_{L1} and T_{R1} each independently indicate a monovalent group; and
the average percent modification r_{L} of said immunoglobulin unit with the first modified moiety and the average percent modification r_{R} of said immunoglobulin unit with the second modified moiety are individually 65 to 135%.

125. The antibody derivative or a salt thereof according to claim 124, wherein
the antibody derivative or a salt thereof comprising first and second modified moieties comprises a structural unit represented by the following formula (VIb-1): 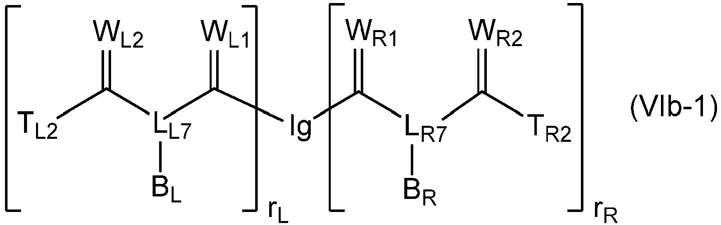 wherein
Ig indicates said immunoglobulin unit;
W_{L1} and W_{L2}, and W_{R1} and W_{R2} each independently indicate an oxygen atom or a sulfur atom;
L_{L7} and L_{R7} each independently indicate a seventh linker;
B_{L} indicates a first group comprising a first bioorthogonal functional group;
B_{R} indicates a second group comprising a second bioorthogonal functional group;
T_{L2} and T_{R2} each independently indicate a monovalent group; and
the average percent modification r_{L} of said immunoglobulin unit with the first modified moiety and the average percent modification r_{R} of said immunoglobulin unit with the second modified moiety are individually 65 to 135%.

126. The antibody derivative or a salt thereof according to claim 119, wherein
the antibody derivative or a salt thereof comprising first and second modified moieties comprises a structural unit represented by the following formula (VIc): 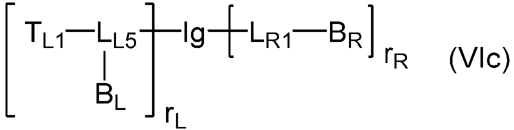 wherein
Ig indicates said immunoglobulin unit;
L_{R1} indicates a first linker;
L_{L5} indicates a fifth linker;
B_{L} indicates a first group comprising a first bioorthogonal functional group;
B_{R} indicates a second group comprising a second bioorthogonal functional group;
T_{L1} indicates a monovalent group; and
the average percent modification r_{L} of said immunoglobulin unit with the first modified moiety and the average percent modification r_{R} of said immunoglobulin unit with the second modified moiety are individually 65 to 135%.

127. The antibody derivative or a salt thereof according to claim 126, wherein
the antibody derivative or a salt thereof comprising first and second modified moieties comprises a structural unit represented by the following formula (VIc-1): 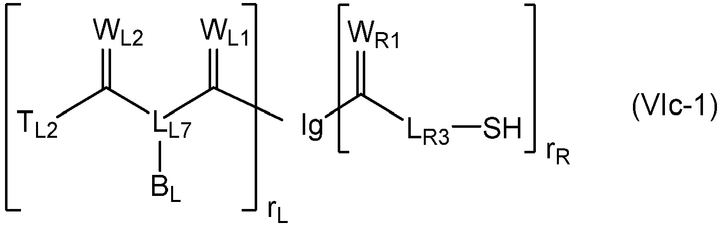 wherein
Ig indicates said immunoglobulin unit;
W_{L1} and W_{L2}, and W_{R1} each independently indicate an oxygen atom or a sulfur atom;
L_{R3} indicates a third linker;
L_{L7} indicates a seventh linker;
B_{L} indicates a first group comprising a first bioorthogonal functional group;
SH indicates a thiol group that is a second bioorthogonal functional group;
T_{L2} indicates a monovalent group; and
the average percent modification r_{L} of said immunoglobulin unit with the first modified moiety and the average percent modification r_{R} of said immunoglobulin unit with the second modified moiety are individually 65 to 135%.

128. The antibody derivative or a salt thereof according to claim 119, wherein the antibody derivative further comprises an additional modified moiety.

129. The antibody derivative or a salt thereof according to claim 128, wherein the additional modified moiety is an additional modified moiety comprising a bioorthogonal functional group, and said additional modified moiety comprising a bioorthogonal functional group is comprised in the constant region in a heavy chain of the antibody.

130. The antibody derivative or a salt thereof according to claim 129, wherein said additional modified moiety comprising a bioorthogonal functional group is introduced to the constant regions in said two heavy chains via modification of the amino group in the side chain of a lysine residue present at one or more positions in the constant regions in said two heavy chains.

131. The antibody derivative or a salt thereof according to claim 130, wherein said one or more positions in the constant regions in the two heavy chains are positions 246/248, 288/290, or 317 in a human IgG heavy chain according to the EU numbering.

132. A conjugate of an antibody and first and second modified moieties, or a salt thereof, wherein
comprising (a) an immunoglobulin unit comprising two heavy chains composed of first and second heavy chains and optionally two light chains, and (b) a first modified moiety comprising a first functional substance and a second modified moiety comprising a second functional substance;
(c) wherein the first modified moiety has been introduced to the constant region in said first heavy chain;
(d) wherein the second modified moiety has been introduced to the constant region in said second heavy chain; and
(e) wherein the first and second modified moieties are different from each other.

133. The conjugate or a salt thereof according to claim 132, wherein said first modified moiety has been introduced to the constant region in said first heavy chain via modification of the amino group in the side chain of a lysine residue present at one or more positions in the constant region in said first heavy chain; and said second modified moiety has been introduced to the constant region in said second heavy chain via modification of the amino group in the side chain of a lysine residue present at one or more positions in the constant regions in said second heavy chain.

134. The conjugate or a salt thereof according to claim 133, wherein said one or more positions in the constant region in the first heavy chain and said one or more positions in the constant region in the second heavy chain are positions selected from the group consisting of positions 246/248, 288/290, and 317 in the human IgG heavy chain according to the EU numbering, and combinations thereof.

135. The conjugate or a salt thereof according to claim 132, wherein
said conjugate or a salt thereof comprises a structural unit represented by the following formula (VIIa): 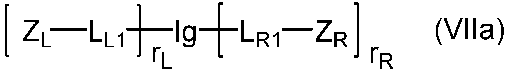 wherein
Ig indicates said immunoglobulin unit;
L_{L1} and L_{R1} each independently indicate a first linker;
Z_{L} indicates a first functional substance;
Z_{R} indicates a second functional substance; and
the average percent modification r_{L} of said immunoglobulin unit with the first modified moiety and the average percent modification r_{R} of said immunoglobulin unit with the second modified moiety are individually 65 to 135%.

136. The conjugate or a salt thereof according to claim 135, wherein
said conjugate or a salt thereof comprises a structural unit represented by the following formula (VIIa-1): 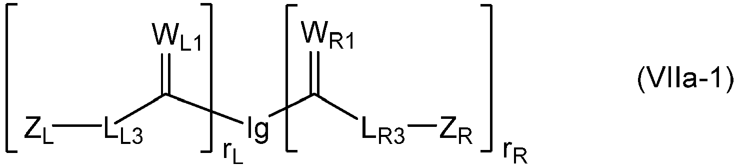 wherein
Ig indicates said immunoglobulin unit;
W_{L1} and W_{R1} each independently indicate an oxygen atom or a sulfur atom;
L_{L3} and L_{R3} each independently indicate a third linker;
Z_{L} indicates a first functional substance;
Z_{R} indicates a second functional substance; and
the average percent modification r_{L} of said immunoglobulin unit with the first modified moiety and the average percent modification r_{R} of said immunoglobulin unit with the second modified moiety are individually 65 to 135%.

137. The conjugate or a salt thereof according to claim 132, wherein
said conjugate or a salt thereof comprises a structural unit represented by the following formula (VIIb): 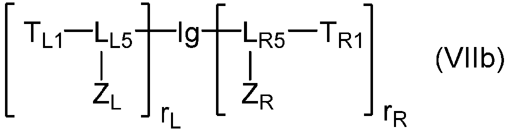 wherein
Ig indicates said immunoglobulin unit;
L_{L5} and L_{R5} each independently indicate a fifth linker;
Z_{L} indicates a first functional substance;
Z_{R} indicates a second functional substance;
T_{L1} and T_{R1} each independently indicate a monovalent group; and
the average percent modification r_{L} of said immunoglobulin unit with the first modified moiety and the average percent modification r_{R} of said immunoglobulin unit with the second modified moiety are individually 65 to 135%.

138. The conjugate or a salt thereof according to claim 137, wherein
said conjugate or a salt thereof comprises a structural unit represented by the following formula (VIIb-1): 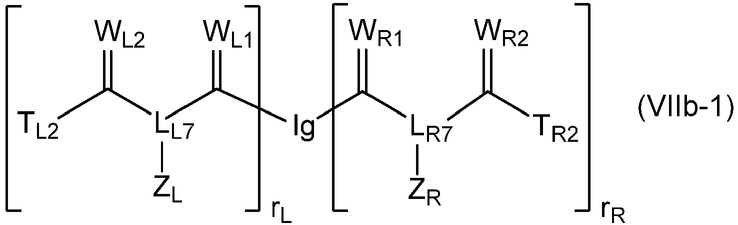 wherein
Ig indicates said immunoglobulin unit;
W_{L1} and W_{L2}, and W_{R1} and W_{R2} each independently indicate an oxygen atom or a sulfur atom;
L_{L7} and L_{R7} each independently indicate a seventh linker;
Z_{L} indicates a first functional substance;
Z_{R} indicates a second functional substance;
T_{L2} and T_{R2} each independently indicate a monovalent group; and
the average percent modification r_{L} of said immunoglobulin unit with the first modified moiety and the average percent modification r_{R} of said immunoglobulin unit with the second modified moiety are individually 65 to 135%.

139. The conjugate or a salt thereof according to claim 132, wherein
said conjugate or a salt thereof comprises a structural unit represented by the following formula (VIIc): 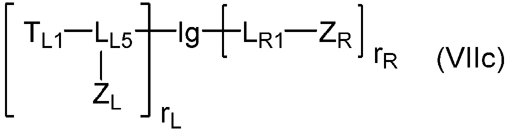 wherein
Ig indicates said immunoglobulin unit;
L_{R1} indicates a first linker;
L_{L5} indicates a fifth linker;
Z_{L} indicates a first functional substance;
Z_{R} indicates a second functional substance;
T_{L1} indicates a monovalent group; and
the average percent modification r_{L} of said immunoglobulin unit with the first modified moiety and the average percent modification r_{R} of said immunoglobulin unit with the second modified moiety are individually 65 to 135%.

140. The conjugate or a salt thereof according to claim 139, wherein
said conjugate or a salt thereof comprises a structural unit represented by the following formula (VIIc-1): 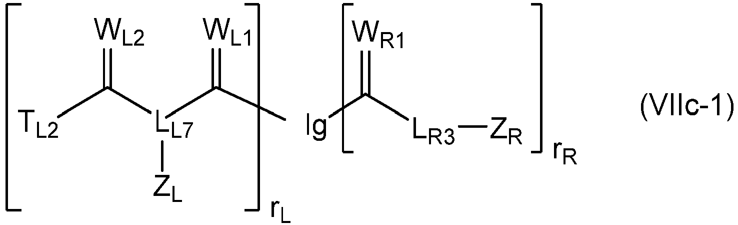 wherein
Ig indicates said immunoglobulin unit;
W_{L1} and W_{L2}, and W_{R1} each independently indicate an oxygen atom or a sulfur atom;
L_{R3} indicates a third linker;
L_{L7} indicates a seventh linker;
Z_{L} indicates a first functional substance;
Z_{R} indicates a second functional substance;
T_{L2} indicates a monovalent group; and
the average percent modification r_{L} of said immunoglobulin unit with the first modified moiety and the average percent modification r_{R} of said immunoglobulin unit with the second modified moiety are individually 65 to 135%.

141. The conjugate or a salt thereof according to claim 132, wherein the first and second functional substances are each independently a drug, a labelling substance, an affinity substance, a transporting substance, or a stabilizer.

142. The conjugate or a salt thereof according to claim 141, wherein one of the first and second functional substances is a full-length antibody or a fragment thereof, or both of the first and second functional substances are each independently a full-length antibody or a fragment thereof.

143. The conjugate or a salt thereof according to claim 132, wherein the conjugate further comprises an additional modified moiety.

144. The conjugate or a salt thereof according to claim 143, wherein the additional modified moiety is an additional modified moiety comprising a functional substance, and said additional modified moiety comprising a functional substance is contained in a constant region in a heavy chain of the antibody.

145. The conjugate or a salt thereof according to claim 144, wherein the additional modified moiety comprising a functional substance is introduced into said constant regions in two heavy chains via modification of the amino group in the side chain of a lysine residue present at one or more positions in said constant regions in two heavy chains.

146. The conjugate or a salt thereof according to claim 145, wherein said one or more positions in the constant regions in two heavy chains are positions 246/248, 288/290, or 317 in a human IgG heavy chain according to the EU numbering.
